# EUROPEAN PATENT APPLICATION

(11) **EP 4 650 353 A1**
(43) Date of publication of application: **19.11.2025**
(21) Application number: 23915833.0
(22) Date of filing: 28.12.2023
(51) Int. Cl.: C07D 405/14, C07D 471/04, A61P 3/10, A61K 31/454

(54) **4-ALKOXY BENZIMIDAZOLE-6-CARBOXYLIC ACID DERIVATIVE SERVING AS GLP-1 RECEPTOR AGONIST**

(30) Priority: 13.01.2023 CN 202310072648; 13.09.2023 CN 202311183126
(71) Applicant: Shanghai Institute of Materia Medica, Chinese Academy of Sciences, Shanghai 201203 (CN)
(72) Inventor: WANG, Kai, Shanghai 201203 (CN); XIE, Xin, Shanghai 201203 (CN); SHEN, Jianhua, Shanghai 201203 (CN); GUO, Shimeng, Shanghai 201203 (CN); CHEN, Lili, Shanghai 201203 (CN); YUN, Ying, Shanghai 201203 (CN); XU, Tifei, Shanghai 201203 (CN); WANG, Xiaoyan, Shanghai 201203 (CN)
(74) Representative: Dompatent
(86) International application number: PCT/CN2023/142906
(87) International publication number: WO 2024/149080

(57) **Abstract**

Disclosed in the present invention is a 4-alkoxy benzimidazole-6-carboxylic acid derivative serving as a GLP-1 receptor agonist. The structure of the 4-alkoxy benzimidazole-6-carboxylic acid derivative is represented by general formula (II), and the definition of each substituent is as recited in the description and the claims. The compound in the present invention serves as a GLP-1 receptor agonist, and can be used to prevent and/or treat diseases or symptoms associated with dysregulation of GLP-1 receptor signaling pathway. (II)

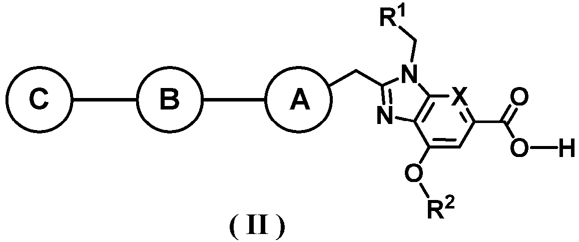

## Description

### Technical Field

The present invention relates to the field of pharmaceutical chemistry, and in particular to a 4-alkoxybenzimidazole-6-carboxylic acid compound, a preparation method thereof and use thereof in preparing a medicament. The present invention further relates to the pharmacological effects of the compounds and pharmaceutical compositions containing the compounds as GLP-1 receptor agonists, and use thereof in treating diseases such as diabetes, obesity, metabolic syndrome, non-alcoholic fatty liver disease (NASH), Alzheimer's disease, and the like.

### Background Art

Diabetes is the third most serious chronic disease threatening human health after cancer and cardiovascular and cerebrovascular diseases. According to the eighth edition of the Global Diabetes Atlas released by the International Diabetes Federation (IDF), there are currently more than 460 million diabetes patients in the world. It is estimated that by 2045, there will be nearly 700 million diabetes patients. The situation is not optimistic. As a metabolic disease with multiple causes, diabetes is characterized by chronic hyperglycemia, accompanied by disorders of sugar, lipid and protein metabolism caused by defects in insulin secretion or action. Two major forms of diabetes are currently recognized, type 1 diabetes (insulin-dependent diabetes mellitus, IDDM) and type 2 diabetes (non-insulin-dependent diabetes mellitus, NIDDM). Type 2 diabetes accounts for more than 90% of the total number of diabetic patients, which is characterized by decreased pancreatic β-cell function and resistance to insulin in peripheral tissues such as liver, skeletal muscle, fat, etc., leading to glucose and lipid metabolism disorders. Other diseases associated with it include hepatic insulin resistance, glucose intolerance, diabetic neuropathy, diabetic nephropathy, diabetic retinopathy, obesity, dyslipidemia, hypertension, hyperinsulinemia and non-alcoholic fatty liver disease (NAFLD). In the past decade, three new glucose-lowering drugs have been widely used in the clinical treatment of type 2 diabetes, namely glucagon-like peptide-1 (GLP-1) receptor agonists, dipeptidyl peptidase-4 (DPP-4) inhibitors, and sodium-glucose cotransporter 2 (SGLT2) inhibitors. Among them, GLP-1 receptor agonists have the advantages of being strong, blood sugar-dependent, and not increasing the risk of hypoglycemia in lowering blood sugar. They have now become one of the first-line hypoglycemic drugs, and their market share is also growing. Another major feature of GLP-1 receptor agonists is that they have "multi-functionality". In addition to lowering blood sugar, its weight loss effect is also very prominent. In a large-scale Phase III clinical trial of obese patients, semaglutide is able to reduce body weight by more than 15% and is considered by the industry to be a drug that will change the treatment landscape for obesity. In addition, GLP-1 receptor agonists can lower blood pressure, improve dyslipidemia, reduce fatty liver, and fight Alzheimer's disease. In particular, it has a protective effect on the heart and kidney, can significantly reduce the incidence of cardiovascular events, and delay the progression of diabetic nephropathy. For patients suffering from multiple of the above diseases, GLP-1 receptor agonists will undoubtedly bring huge long-term benefits.

GLP-1 receptors are members of the class B family of peptide hormone G protein-coupled receptors (GPCRs) and are distributed throughout the body's tissues, including pancreatic islet cells, lung, kidney, brain, hypothalamus, cardiovascular system, gastrointestinal tract, vagus nerve in the skin, etc. GLP-1 receptors may also exist in the liver, adipose tissue, and skeletal muscle. Its natural agonist ligand is GLP-1 (glucagon-like peptide-1) is a peptide hormone encoded by the glucagon gene and secreted by intestinal L cells and belongs to the incretin family. Physiologically, after the body ingests nutrients through food, GLP-1 is released from intestinal L cells into the circulation at low picomolar levels (5 to 15 pmol/L). These levels of GLP-1 help activate the GLP-1 receptors on pancreatic beta cells and stimulate insulin secretion in a glucose-dependent manner, while inhibiting glucagon secretion, thereby lowering postprandial blood sugar and maintaining a constant level. GLP-1 also has neuromodulatory functions, can reduce appetite, inhibit gastric emptying and promote the growth of pancreatic β cells. 50%-70% of the glucose taken orally by the human body is metabolized through the GLP-1/insulin pathway, so GLP-1 is an important glucose-lowering polypeptide. In patients with type 2 diabetes, the secretion level of endogenous GLP-1 is significantly decreased, which is an important reason for the uncontrolled blood sugar in patients. Natural GLP-1 is easily degraded and inactivated by dipeptidyl peptidase-4 (DPP-4) in the body and has an extremely short half-life of only 1-2 minutes. Therefore, it is not suitable for the clinical treatment of diabetes. The development of metabolically stable exogenous GLP-1 analogs has been an important research direction for the treatment of type 2 diabetes.

Currently, the GLP-1 receptor agonists on the market and the drugs under development are mainly concentrated in peptide analogs. The GLP-1 analog exenatide is the first GLP-1 receptor agonist approved for the treatment of type 2 diabetes. Exenatide is equivalent to natural GLP-1 in activating the GLP-1 receptor and shows effective blood sugar control effects when the plasma concentration is in the range of 40 to 70 pmol/L. Other potent GLP-1 receptor agonists, including liraglutide, dulaglutide, and semaglutide, have also been approved for marketing. Although these drugs provide patients with better blood sugar control, they still only account for a limited proportion of diabetes prescriptions. One of the important reasons is that they are all large-molecule peptide drugs that need to be administered through invasive subcutaneous injections, and patient compliance is poor compared to oral administration. A breakthrough in GLP-1 therapy is the development of a semaglutide tablet co-formulated with the absorption enhancer sodium N-(8-(2-hydroxybenzoyl)amino) caprylate for oral administration, but the oral bioavailability is less than 1%. In addition, since drug absorption is significantly affected by food and gastric juice, the oral dosing regimen of semaglutide for patients is limited. Specifically, the tablets must be taken with a sufficient amount of water after an overnight fast and at least 30 minutes before breakfast or other medications. Therefore, the development of small molecule GLP-1 receptor agonists that are convenient for oral administration could provide a more standardized drug formulation and a simpler administration method.

WO2018109607 discloses a compound represented by the following general formula and use thereof as a GLP-1 receptor agonist, wherein Z¹ is CH or N. Example 4A-01 is a representative compound in this application, which is currently in Phase 2 clinical research, with a research and development code of PF-06882961, and is used as a reference compound hereinafter.

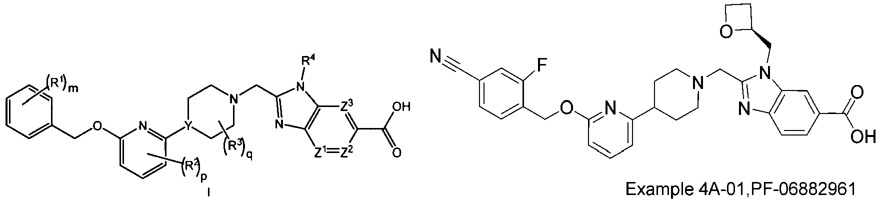

WO2019239319 discloses a compound represented by the following general formula, wherein Z¹ is CR^{Z} or N, and R^{Z} is further defined to H, F, Cl or -CH₃. Example 10 is a representative compound in this application and is used as a reference compound hereinafter.

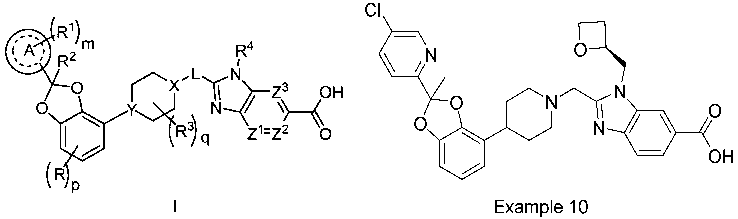

WO2019239371 discloses a compound shown in the following general formula, wherein A can be the structure shown in A1, Z¹ is CR^{Z} or N, and R^{Z} is further defiend to H, F, Cl or -CH₃. Example 1 is a representative compound in this application and is used as a reference compound hereinafter.

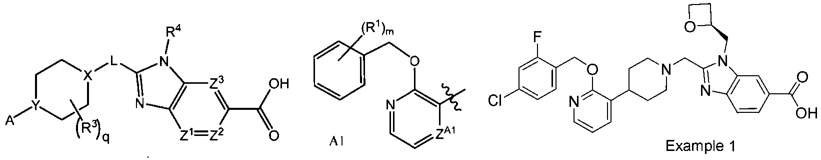

WO2022109182 discloses a compound shown in the following general formula, wherein X¹ is defined as N, CH or CR¹¹, and R¹¹ is defined more broadly, including many groups, but the examples only show compounds in which R¹¹ is a halogen, alkyl, cycloalkyl, heteroaromatic ring, or alkynyl. Among them, the best effect is achieved when R¹¹ is -F, while the compounds having other substituents have poor effects. Example 21 is one of the most active compounds and is used as a reference compound hereinafter.

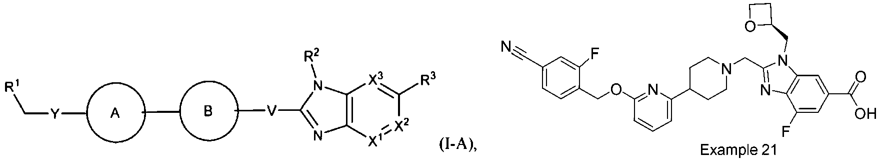

CN2022114139828 discloses a compound of the following general formula, wherein A is a 5-6 membered aryl or heteroaryl, and R⁴ is H, halogen or C1-C6 alkyl. Compounds 2, 10 and 63 in this application are used as reference compounds hereinafter.

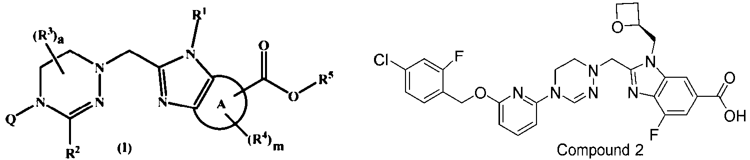

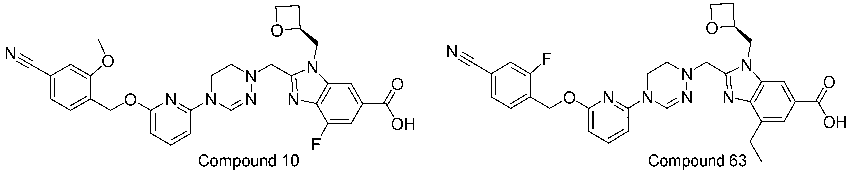

### Summary of the invention

The object of the present invention is to provide a class of 4-alkoxybenzimidazole-6-carboxylic acid compounds with very potent GLP-1 receptor agonist activity, a preparation method thereof and medical use thereof.

In a first aspect, the present invention provides a compound represented by general formula (I), a stereoisomer, deuterated derivative and pharmaceutically acceptable salt thereof: 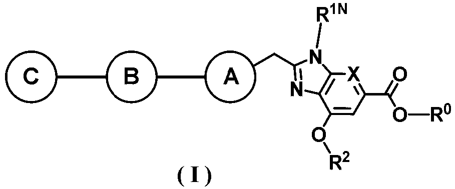
wherein, X is CR^{X} or N; R^{X} is H or halogen; R⁰ is H or methyl;
R^{1N} is 4-8 membered heterocyclyl, C1-C6 alkyl substituted by 4-8 membered heterocyclyl, C1-C6 alkyl substituted by 5-8 membered heteroaryl, or C1-C6 alkyl substituted by C3-C8 cycloalkyl, wherein the C1-C6 alkyl, the 4-8 membered heterocyclyl, the 5-8 membered heteroaryl, the C3-C8 cycloalkyl is not substituted by other groups or is optionally substituted by one or more groups selected from a group consisting of halogen, C1-C6 alkyl, C1-C6 haloalkyl, C1-C6 alkyl substituted by cyano, C1-C6 alkoxy, C1-C6 haloalkoxy, and cyano;
R² is C1-C6 alkyl, C1-C6 haloalkyl, deuterated C1-C6 alkyl, C1-C6 alkyl substituted by cyano, C3-C8 cycloalkyl, C1-C6 alkyl substituted by C3-C8 cycloalkyl, phenyl, C1-C6 alkyl substituted by phenyl, 5-8 membered heteroaryl, C1-C6 alkyl substituted by 5-8 membered heteroaryl, 4-8 membered heterocyclyl or C1-C6 alkyl substituted by 4-8 membered heterocyclyl, and the cycloalkyl, phenyl, heteroaryl, heterocyclyl is unsubstituted or optionally substituted by one or more groups selected from a group consisting of halogen, C1-C6 alkyl, C1-C6 alkoxy, C1-C6 haloalkyl, C1-C6 haloalkoxy, cyano; 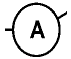
is 5-6 membered heterocyclyl, phenyl, 5-6 membered heteroaryl or C5-C8 cycloalkyl, wherein the heterocyclyl, phenyl, heteroaryl or cycloalkyl is unsubstituted or optionally substituted by m R³ groups; 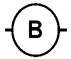
is selected from a group consisting of: 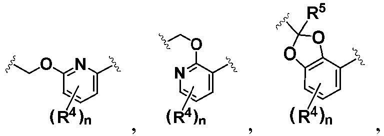 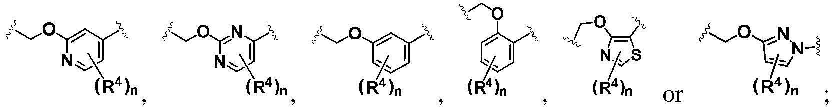 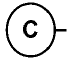
is C6-C10 aryl or 5-12 membered heteroaryl, wherein the aryl or heteroaryl is unsubstituted or optionally substituted by p R⁶ groups or q R7 groups;
each R³, R⁴, R⁷ is independently H, halogen, C1-C6 alkyl or C1-C6 haloalkyl;
R⁵ is H, C1-C6 alkyl or C1-C6 haloalkyl;
each R⁶ is independently H, halogen, cyano, C1-C6 alkyl, C1-C6 alkoxy, C2-C6 alkenyl, C2-C6 alkynyl, C3-C8 cycloalkyl, 4-8 membered heterocyclyl, C1-C6 haloalkyl, C1-C6 haloalkoxy, C3-C8 cycloalkyl substituted C1-C6 alkyl, hydroxy substituted C1-C6 alkyl, C1-C6 alkoxy substituted C1-C6 alkyl, amino substituted C1-C6 alkyl, C1-C6 alkylamino substituted C1-C6 alkyl, C6-C10 aryl, 5-8 membered heteroaryl, nitro or C1-C6 alkyl substituted by cyano, hydroxy, -SR⁸, -N(R⁸)₂, -C(O)OR⁸, -C(O)N(R⁸)₂, -C(O)R⁸, -S(O)R⁸, -S(O)₂R⁸, -S(O)₂N(R⁸)₂ or - N(R⁸)C(O)R⁸;
each R⁸ is independently H, C1-C6 alkyl, C3-C8 cycloalkyl, C1-C6 haloalkyl, hydroxy-substituted C1-C6 alkyl, C1-C6 alkoxy-substituted C1-C6 alkyl, amino-substituted C1-C6 alkyl or C1-C6 alkylamino-substituted C1-C6 alkyl;
m, n, p, and q are each independently an integer of 1-4.

In another preferred embodiment, m is 1, 2, 3 or 4. In another preferred embodiment, n is 1, 2, 3 or 4.

In another preferred embodiment, p is 1, 2, 3 or 4. In another preferred embodiment, q is 1, 2, 3 or 4.

In another preferred embodiment, X is CH.

In another preferred embodiment, R⁰ is H.

In another preferred embodiment, R^{1N} is methyl substituted by 4-5 membered heterocyclyl, methyl substituted by 5-6 membered heteroaryl, or methyl substituted by C3-C4 cycloalkyl, and the heterocyclyl, heteroaryl, cycloalkyl is unsubstituted or optionally substituted by 1-2 C1-C3 alkyls.

In another preferred embodiment, R^{1N} is

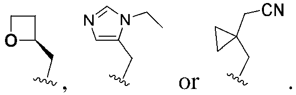

In another preferred embodiment, R² is C1-C4 alkyl, C1-C4 haloalkyl, deuterated C1-C4 alkyl, C3-C6 cycloalkyl or C3-C6 cycloalkyl substituted C1-C4 alkyl, phenyl, phenyl substituted C1-C4 alkyl, 5-6 membered heteroaryl, 5-6 membered heteroaryl substituted C1-C4 alkyl, 4-6 membered heterocyclyl or 4-6 membered heterocyclyl substituted C1-C4 alkyl, the cycloalkyl is unsubstituted or optionally substituted by one or more groups selected from a group consisting of halogen, C1-C6 alkyl. In another preferred embodiment, R² is C1-C3 alkyl, C1-C3 haloalkyl, deuterated C1-C3 alkyl, C3-C5 cycloalkyl, C1-C3 alkyl substituted by C3-C5 cycloalkyl, phenyl, C1-C3 alkyl substituted by phenyl, 5-6 membered heteroaryl, C1-C3 alkyl substituted by 5-6 membered heteroaryl, 4-6 membered heterocyclyl or C1-C3 alkyl substituted by 4-6 membered heterocyclyl.

In another preferred embodiment, R² is methyl, difluoromethyl, trideuterated methyl, ethyl, trifluoroethyl, propyl, isopropyl, cyclopropyl, cyclopropylmethyl, benzyl, furanylmethyl or pyridyl.

In another preferred embodiment, 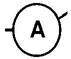 is selected from the following group:

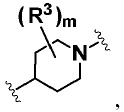

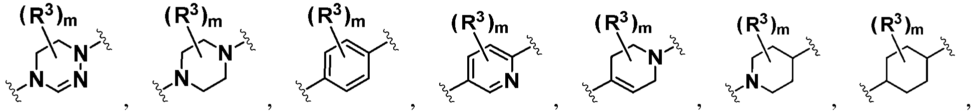

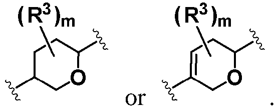

In another preferred embodiment, 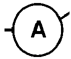 is

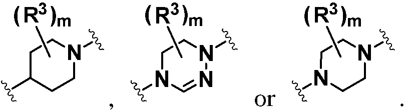

In another preferred embodiment, 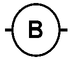 is

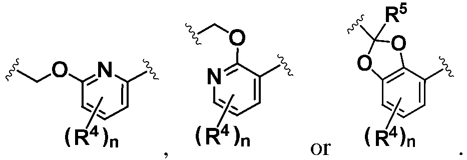

In another preferred embodiment, 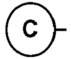 is selected from the following group: 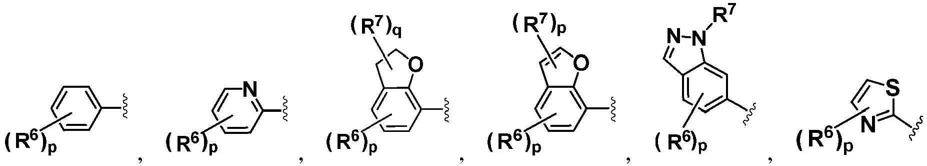 or

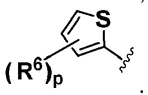

In another preferred embodiment, 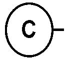 is

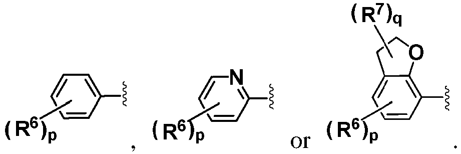

In another preferred embodiment, each of R³, R⁴, R⁷ is independently H or halogen; preferably, R³, R⁴, and R⁷ are all H.

In another preferred embodiment, R⁵ is C1-C3 alkyl; in another preferred embodiment, R⁵ is methyl.

In another preferred embodiment, each R⁶ is independently H, halogen, cyano, C1-C4 alkyl, C1-C4 alkoxy, C1-C4 haloalkyl or C1-C4 haloalkoxy. In another preferred embodiment, each R⁶ is independently H, halogen, cyano, C1-C3 alkoxy or C1-C3 haloalkyl.

In another preferred embodiment, each R⁶ is independently H, F, Cl, cyano, methoxy, or trifluoromethyl.

Furthermore, the present invention provides a compound represented by general formula (II), stereoisomer, deuterated compoundand pharmaceutically acceptable salt thereof: 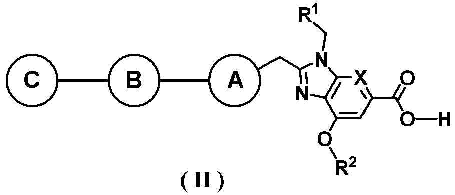
wherein, X is CR^{X} or N; R^{X} is H or halogen;
R¹ is 4-8 membered heterocyclyl, 5-8 membered heteroaryl or C3-C8 cycloalkyl, wherein the heterocyclyl, heteroaryl or cycloalkyl is unsubstituted or optionally substituted by one or more groups selected from a group consisting of halogen, C1-C6 alkyl, C1-C6 haloalkyl, C1-C6 alkyl substituted by cyano, C1-C6 alkoxy, C1-C6 haloalkoxy or cyano;
R² is C1-C6 alkyl, C1-C6 haloalkyl, deuterated C1-C6 alkyl, C1-C6 alkyl substituted by cyano, C3-C8 cycloalkyl, C1-C6 alkyl substituted by C3-C8 cycloalkyl, phenyl, C1-C6 alkyl substituted by phenyl, 5-8 membered heteroaryl, C1-C6 alkyl substituted by 5-8 membered heteroaryl, 4-8 membered heterocyclyl or C1-C6 alkyl substituted by 4-8 membered heterocyclyl, and the cycloalkyl, phenyl, heteroaryl, heterocyclyl is unsubstituted or optionally substituted by one or more groups selected from a group consisting of halogen, C1-C6 alkyl, C1-C6 alkoxy, C1-C6 haloalkyl, C1-C6 haloalkoxy, cyano; 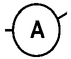
is selected from a group consisting of: 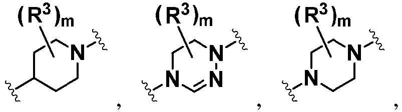 wherein N on the ring is linked to -CH₂; 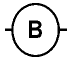
is selected from a group consisting of: 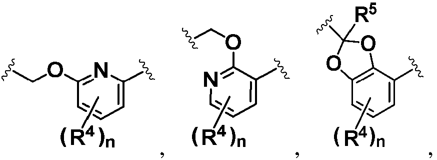 wherein benzene ring or pyridine ring is attached to 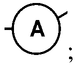 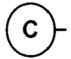
is selected from a group consisting of: 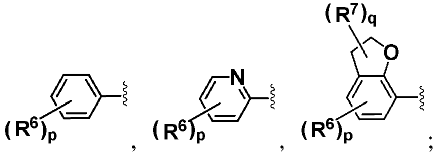 each R³, R⁴, R⁷ is independently H, halogen or C1-C6 alkyl;
R⁵ is H, C1-C6 alkyl or C1-C6 haloalkyl;
each R⁶ is independently H, halogen, cyano, C1-C6 alkyl, C1-C6 alkoxy, C1-C6 haloalkyl or C1-C6 haloalkoxy;
m, n, p, and q are each independently an integer of 1-4.

In another preferred embodiment, m is 1, 2, 3 or 4.

In another preferred embodiment, n is 1, 2, 3 or 4.

In another preferred embodiment, p is 1, 2, 3 or 4.

In another preferred embodiment, q is 1, 2, 3 or 4.

In another preferred embodiment, X is CH.

In another preferred embodiment, R¹ is 4-6 membered heterocyclyl; preferably, R¹ is

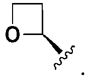

In another preferred embodiment, R² is C1-C4 alkyl, C1-C4 haloalkyl, deuterated C1-C4 alkyl, C3-C8 cycloalkyl or C3-C8 cycloalkyl substituted C1-C4 alkyl, phenyl substituted C1-C4 alkyl, 5-6 membered heteroaryl, C1-C4 alkyl substituted by 5-6 membered heteroaryl, or C1-C4 alkyl substituted by 5-6 membered heterocyclyl, wherein the cycloalkyl, heteroaryl and heterocyclyl are unsubstituted or optionally substituted by one or more groups selected from a group consisting of halogen atoms and C1-C4 alkyl. In another preferred embodiment, R² is C1-C3 alkyl, C1-C3 haloalkyl, deuterated C1-C3 alkyl, C3-C5 cycloalkyl, C1-C3 alkyl substituted by C3-C5 cycloalkyl, C1-C4 alkyl substituted by phenyl, 5-6 membered heteroaryl, C1-C3 alkyl substituted by 5-6 membered heteroaryl, or C1-C3 alkyl substituted by 5-6 membered heterocyclyl.

In another preferred embodiment, R² is methyl, difluoromethyl, trideuterated methyl, ethyl, trifluoroethyl, propyl, isopropyl, cyclopropyl, cyclopropylmethyl, benzyl, furanylmethyl or pyridyl.

In another preferred embodiment, each of R³, R⁴, and R⁷ is independently H or halogen; preferably, R³, R⁴, and R⁷ are all H.

In another preferred embodiment, R⁵ is C1-C3 alkyl; in another preferred embodiment, R⁵ is methyl.

In another preferred embodiment, each R⁶ is each independently H, halogen, cyano, C1-C4 alkyl, C1-C4 alkoxy, C1-C4 haloalkyl or C1-C4 haloalkoxy; preferably, each R⁶ is each independently H, halogen, cyano, C1-C3 alkoxy or C1-C3 haloalkyl; more preferably, each R⁶ is each independently H, F, Cl, cyano, methoxy or trifluoromethyl.

In another preferred embodiment, the compound represented by general formula (II) has the following structural formulas (II-a) to (II-k): 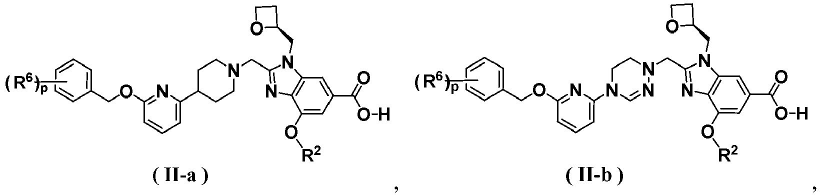 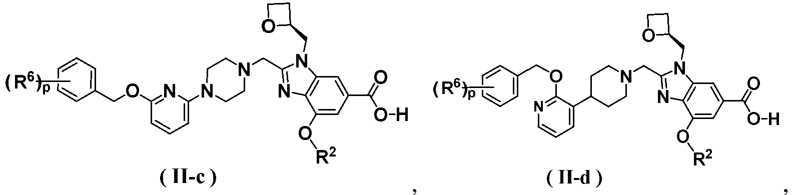 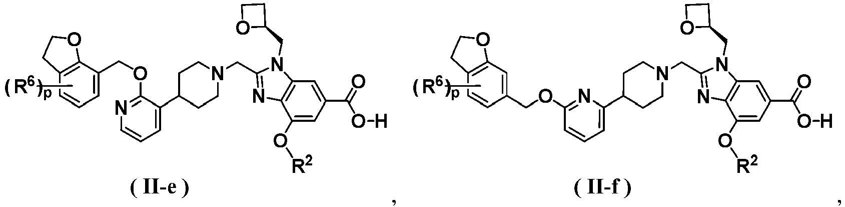 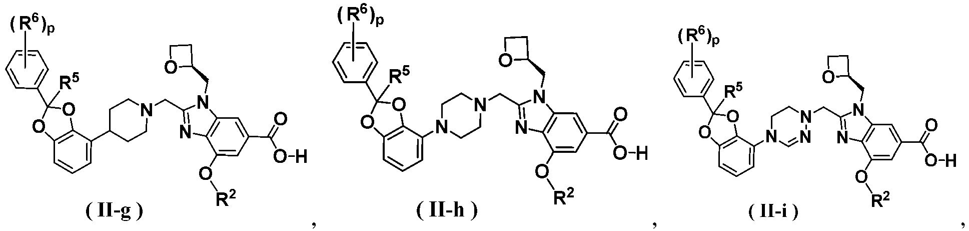 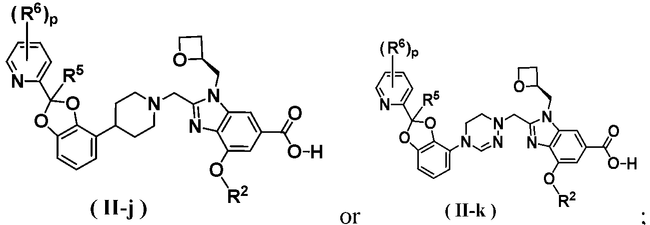 wherein, the definitions of R², R⁵, R⁶ and p are the same as those in the general formula (II).

In another preferred embodiment, the compound is selected from: Compound 1-Compound 90.

In another preferred embodiment, the pharmaceutically acceptable salt is an ammonium salt or Tris salt of the compound.

The second aspect of the present invention provides a pharmaceutical composition comprising a safe and effective amount of a compound represented by general formula (I) or (II), stereoisomer, deuterated compound or pharmaceutically acceptable salt thereof as an active ingredient, and a pharmaceutically acceptable excipient.

In the present invention, the "effective amount" means that the disease or side effects of the subject receiving the treatment with the dose is cured, improved, effectively prevented, or the incidence of the disease or side effect is significantly reduced compared with the subject not receiving the treatment with the dose; in addition, it also includes an effective dose for enhancing normal physiological function. "Safe" means that the amount of active ingredient is sufficient to significantly improve the condition without causing serious side effects.

In the pharmaceutical composition, the compound of the present invention is used as an active ingredient, and its weight accounts for 0.1 to 99.9% of the total weight of the pharmaceutical composition, and the rest are pharmaceutically acceptable excipients; the preferred ratio of the compound of the present invention to the excipients is: the compound of the present invention as an active ingredient accounts for more than 60% of the total weight, and the rest accounts for 0-40% of the total weight, and the amount of the rest is preferably 1-20%, and most preferably 1-10%. Typically, the pharmaceutical composition contains 1-2000 mg of active ingredient per dose, more preferably, 10-200 mg of active ingredient per dose. Preferably, the "one dose" is one tablet.

In addition to the compound represented by general formula (I) or (II) as an active ingredient, the pharmaceutical composition may further contain one or more other therapeutic agents. In a preferred embodiment, the other therapeutic agent is a therapeutic agent for diabetes, a therapeutic agent for cardiovascular disease, or a therapeutic agent for obesity.

The pharmaceutical excipients are pharmaceutically acceptable carriers, excipients, sustained-release agents, odorants, flavoring agents, etc. "Pharmaceutically acceptable carrier" refers to: one or more compatible solid or liquid fillers or gel substances, which are suitable for human use and must be sufficiently pure and sufficiently low in toxicity. "Compatibility" herein means that the components in the composition can be blended with the active ingredient of the present invention and with each other without significantly reducing the efficacy of the active ingredient. Examples of pharmaceutically acceptable carriers include, but are not limited to, cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), etc.

The compounds or pharmaceutical compositions of the present invention can be prepared into various dosage forms based on conventional processes in the field of pharmaceutical preparations, such as tablets, capsules, powders, syrups, solutions, suspensions, sprays, creams, ointments, gels, transdermal patches, etc., and can be present in suitable solid or liquid carriers or diluents. The pharmaceutical composition of the present invention can also be stored in a suitable sterile device for injection or infusion. From the standpoint of ease of preparation and administration, preferred pharmaceutical compositions are solid compositions, particularly tablets and solid-filled or liquid-filled capsules.

The compounds or pharmaceutical compositions of the present invention can be used clinically on mammals, including humans and animals. The administration method is not particularly limited, and representative administration methods include but are not limited to oral administration, nasal inhalation, topical administration to the skin, intravenous injection, intramuscular injection, subcutaneous injection, and the like. In another preferred embodiment, the preferred administration route of the compound or pharmaceutical composition of the present invention is oral administration.

Solid dosage forms of the compounds or pharmaceutical compositions of the present invention for oral administration include capsules, tablets, pills, powders and granules. Solid carriers include starch, lactose, dicalcium phosphate, microcrystalline cellulose, sucrose and kaolin, while liquid carriers include sterile water, polyethylene glycol, non-ionic surfactants and edible oils (such as corn oil, peanut oil and sesame oil), as long as they are suitable for the characteristics of the active ingredient and the specific administration method required. Adjuvants commonly used in preparing pharmaceutical compositions may also advantageously be included, such as flavoring agents, coloring agents, preservatives and antioxidants such as vitamin E, vitamin C, BHT and BHA.

Liquid dosage forms for oral administration include pharmaceutically acceptable emulsions, solutions, suspensions, syrups or tinctures. In addition to the active ingredient, the liquid dosage form may contain an inert diluent conventionally used in the art, such as water or other solvents, solubilizers and emulsifiers, for example, ethanol, isopropanol, ethyl carbonate, ethyl acetate, propylene glycol, 1,3-butylene glycol, dimethylformamide and oil, in particular cottonseed oil, peanut oil, corn germ oil, olive oil, castor oil and sesame oil or mixtures of these substances. Besides such inert diluents, the composition may also include adjuvants such as wetting agents, emulsifying and suspending agents, sweetening, flavoring, and perfuming agents. In addition to the active ingredients, suspensions may contain suspending agents such as, for example, ethoxylated isostearyl alcohols, polyoxyethylene sorbitol and sorbitan esters, microcrystalline cellulose, aluminum methanol and agar, or mixtures of these substances.

Injectable preparations include, but are not limited to, sterile, injectable, aqueous, oily solutions, suspensions, emulsions and the like. These preparations can also be formulated with suitable parenteral diluents, dispersants, wetting agents, suspending agents, etc. Such injectable formulations can be sterilized by filtration through a bacteria-retaining filter. These preparations may also be formulated with a bactericide which is dissolved or dispersed in the injectable medium or by other methods known in the art.

When used as a pharmaceutical preparation, the compound or pharmaceutical composition of the present invention can be used once a day or in divided doses. Regardless of the method of use, the optimal dose for an individual should be determined based on the specific treatment. Usually, it starts with a small dose and gradually increases until the most suitable dose is found.

The third aspect of the present invention provides use of a compound represented by general formula (I) or (II) or stereoisomer, deuterated product thereof, pharmaceutically acceptable salt thereof, and a pharmaceutical composition containing the compound as an active ingredient for preparing a GLP-1 receptor agonist, or for preparing a drug for preventing and/or treating diseases or symptoms associated with disorders of the GLP-1 receptor signaling pathway.

In another preferred embodiment, the disease or symptom associated with the disorder of the GLP-1 receptor signaling pathway is selected from a group consisting of: diabetes, metabolic syndrome, diabetic complications, obesity, non-alcoholic fatty liver disease (NAFLD), non-alcoholic steatohepatitis (NASH), Parkinson's disease, dementia, hyperglycemia, glucose intolerance, atherosclerosis, hypertension, hyperlipidemia, coronary heart disease, cerebral infarction or stroke.

In another preferred embodiment, the disease or symptom associated with the disorder of the GLP-1 receptor signaling pathway is type II diabetes or obesity.

The fourth aspect of the present invention provides a method for preparing a compound represented by general formula (I) or (II), a stereoisomer thereof, a deuterated product thereof or a pharmaceutically acceptable salt thereof.

The compounds of the present invention can be prepared by a variety of synthetic methods, steps and routes. The synthetic routes shown in the following routes A to F are representative general schemes. They are combined with the synthetic methods of the specific compounds in the preparation examples to form the preparation methods of the compounds of the present invention. It should also be understood that the preparation methods of the compounds of the present invention are not limited to the synthetic routes shown in Route A to Route F, which are only used for explanation and do not limit the present invention in any way.

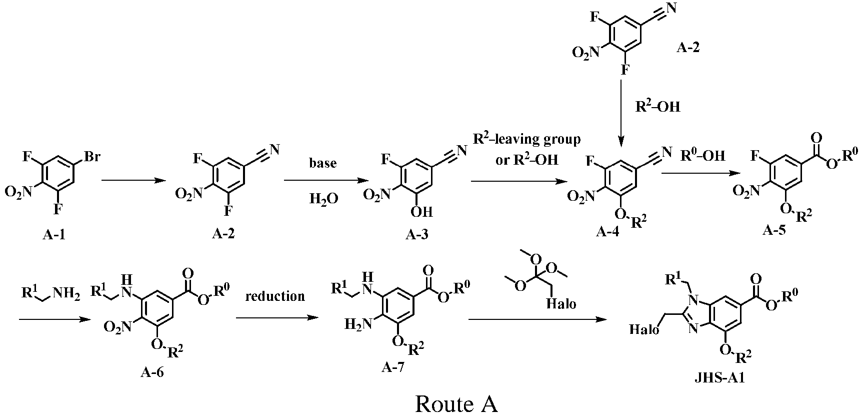

Route A can be used to synthesize the intermediate JHS-A1 of the compound of the present invention. The substituents are defined as described in the general formula (I) or (II). The reaction steps include:
(1.1) Intermediate A-1 undergoes a coupling reaction with a cyanide to generate intermediate A-2; the coupling reaction is usually carried out under the action of a palladium catalyst or a copper catalyst, a ligand and a base; the cyanide is, for example, but not limited to, zinc cyanide, cuprous cyanide, potassium ferrocyanide; the palladium catalyst or the copper catalyst is, for example, but not limited to, palladium acetate, tris(dibenzylideneacetone)palladium, tetrakis(triphenylphosphine)palladium, cuprous iodide; the ligand is, for example, but not limited to, 1,1'-bis(diphenylphosphino)ferrocene, 2-di-tert-butylphosphino-2',4',6'-triisopropylbiphenyl, 2-dicyclohexylphosphino-2',4',6'-triisopropylbiphenyl; the base is, for example but not limited to, cesium carbonate, potassium carbonate, sodium carbonate, 1,8-diazabicyclo[5.4.0]undec-7-ene, triethylamine; the reaction is carried out in a suitable organic solvent, such as N,N-dimethylformamide, dimethyl sulfoxide, N-methylpyrrolidone; the reaction temperature is generally room temperature to 150 °C.
(1.2) Intermediate A-2 is reacted in the presence of a base and water to generate intermediate A-3; the base is, for example but not limited to, cesium carbonate, potassium carbonate, sodium carbonate; the reaction is carried out in a suitable organic solvent, such as N,N-dimethylformamide, dimethyl sulfoxide, N-methylpyrrolidone; the reaction temperature is generally room temperature to 100 °C.
(1.3) Intermediate A-3 undergoes nucleophilic substitution reaction with the reagent 'R²-leaving group' under the action of a base, or intermediate A-3 undergoes Mitsunobu reaction with R²-OH under the action of triphenylphosphine, DIAD or DEAD to generate intermediate A-4; the 'leaving group' is a leaving group, such as but not limited to -Cl, -Br, -I, -OTs, -OMs; the base is such as but not limited to cesium carbonate, potassium carbonate, N,N-diisopropylethylamine; the nucleophilic substitution reaction is carried out in a suitable organic solvent, such as acetonitrile, N,N-dimethylformamide, dimethyl sulfoxide, N-methylpyrrolidone; the Mitsunobu reaction is carried out in a suitable organic solvent, such as tetrahydrofuran, diethyl ether, dichloromethane and toluene; the reaction temperature is generally room temperature to 100 °C.
(1.4) Intermediate A-2 is reacted with R²-OH in the presence of a base to undergo an aromatic nucleophilic substitution reaction to generate intermediate A-4; the base may be, but is not limited to, sodium hydride, sodium tert-butoxide, potassium tert-butoxide; the reaction is carried out in a suitable organic solvent, such as N,N-dimethylformamide, dimethyl sulfoxide, N-methylpyrrolidone; the reaction temperature is generally room temperature to 120 °C.
(1.5) Intermediate A-4 is reacted with R⁰-OH under acidic conditions to generate A-5; the acid is, for example but not limited to, thionyl chloride, hydrochloric acid, sulfuric acid; the reaction temperature is generally room temperature to 100 °C.
(1.6) Intermediate A-5 is reacted with a primary amine under the action of a base to undergo an aromatic nucleophilic substitution reaction to generate intermediate A-6; the base is, for example but not limited to, potassium carbonate, cesium carbonate, triethylamine, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 120 °C.
(1.7) Intermediate A-6 is subjected to reduction reaction to generate intermediate A-7; the reduction reaction is carried out under the catalysis of palladium/carbon, in a hydrogen atmosphere or under acidic conditions, under the action of a reducing agent; the reducing agent is for example but not limited to iron powder and zinc powder; the acidic conditions are for example but not limited to acetic acid, hydrochloric acid, and aqueous ammonium chloride solution; the reaction temperature is generally room temperature; the reaction solvent includes protic solvents and aprotic solvents, for example but not limited to methanol, ethanol, ethyl acetate, THF, etc.
(1.8) Intermediate A-7 is reacted with 2-halo-1,1,1-trimethoxyethane under the catalysis of an acid reagent to generate intermediate JHS-A1; the catalytic acid reagent is for example but not limited to p-toluenesulfonic acid, p-toluenesulfonic acid pyridinium salt, acetic acid, etc.; the reaction is carried out in a suitable organic solvent, such as acetonitrile, toluene; the reaction temperature is generally room temperature to 80 °C.

When A in the general formula (I) or (II) is 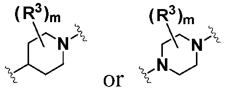 and B is 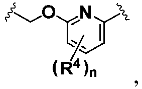 the preparation can be carried out according to route B, and the definitions of the substituents are as described in the general formula (I) or (II), and the reaction steps include:
(2.1) Intermediate B-1 and intermediate B-2 undergo aromatic nucleophilic substitution reaction under the action of a base to generate intermediate B-3; the base is, for example but not limited to, potassium tert-butoxide, sodium hydride, sodium tert-butoxide; the reaction is carried out in a suitable organic solvent, such as tert-butanol, tetrahydrofuran, 1,4-dioxane, N,N-dimethylformamide; the reaction temperature is generally room temperature to 80 °C.
(2.2) Intermediate B-3 undergoes Suzuki coupling reaction with a boron ester raw material (purchased commercially or synthesized according to literature methods) to generate intermediate B-4; the Suzuki coupling reaction is usually carried out under the action of a palladium catalyst and a base; the palladium catalyst is, for example, but not limited to, bis(triphenylphosphine)palladium dichloride (II), 1,1'-bis(diphenylphosphino) ferrocenepalladium dichloride (II), tetrakis(triphenylphosphine)palladium (0); the base is, for example, but not limited to, triethylamine, N,N-diisopropylethylamine, potassium carbonate, cesium carbonate; the reaction is carried out in a suitable organic solvent or a mixed solvent of an organic solvent and water, such as N,N-dimethylformamide, toluene, 1,4-dioxane; the reaction temperature is generally room temperature to 100 °C. 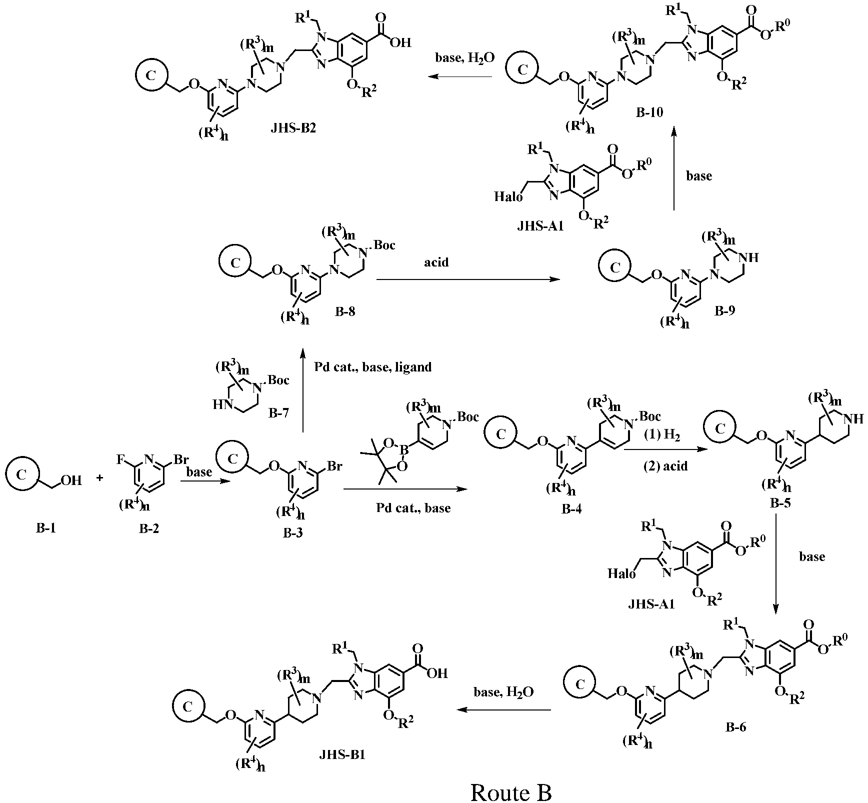
(2.3) After the intermediate B-4 is subjected to hydrogenation, the tert-butyloxycarbonyl (Boc) protecting group is removed under the action of an acid to generate the intermediate B-5; the hydrogenation reaction is carried out under the catalysis of palladium/carbon, nickel, etc.; the reaction temperature is generally room temperature; the reaction solvent includes protic solvents and non-protic solvents, such as but not limited to methanol, ethanol, ethyl acetate, tetrahydrofuran; the acid for the Boc removal reaction is, for example, but not limited to, trifluoroacetic acid and hydrochloric acid; the reaction temperature is generally room temperature; the reaction solvent is, for example, but not limited to, dichloromethane, tetrahydrofuran, 1,4-dioxane.
(2.4) Intermediate B-5 and intermediate JHS-A1 undergo nucleophilic substitution reaction under the action of a base to generate intermediate B-6; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(2.5) Intermediate B-6 is hydrolyzed under alkaline conditions to generate compound JHS-B1. The base may be, but is not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.
(2.6) Intermediate B-3 and intermediate B-7 undergo Buchwald-Hartwig coupling reaction to generate intermediate B-8. The Buchwald-Hartwig coupling reaction is usually carried out under the action of a palladium catalyst, a ligand and a base. The palladium catalyst is, for example, but not limited to, palladium acetate and tris(dibenzylideneacetone)palladium. The ligand is, for example, but not limited to, 4,5-bis(diphenylphosphine)-9,9-dimethyloxanthene, 1,1'-binaphthyl-2,2'-bis(diphenylphosphine) and 2-dicyclohexylphosphine-2',6'-diisopropoxy-1,1'-biphenyl. The base is, for example, but not limited to, cesium carbonate, sodium tert-butoxide and potassium phosphate. The reaction is carried out in an appropriate organic solvent, such as 1,4-dioxane, toluene and N,N-dimethylformamide. The reaction temperature is generally from room temperature to 140 °C.
(2.7) Intermediate B-8 is deprotected to remove Boc protecting group under the action of acid to generate intermediate B-9. The acid is, for example, but not limited to, trifluoroacetic acid and hydrochloric acid. The reaction temperature is generally room temperature. The reaction solvent is, for example, but not limited to, dichloromethane, tetrahydrofuran, and 1,4-dioxane.
(2.8) Intermediate B-9 and intermediate JHS-A1 undergo nucleophilic substitution reaction under the action of a base to generate intermediate B-10; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(2.9) Intermediate B-10 is hydrolyzed under alkaline conditions to generate compound JHS-B2. The base may be, for example, but not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.

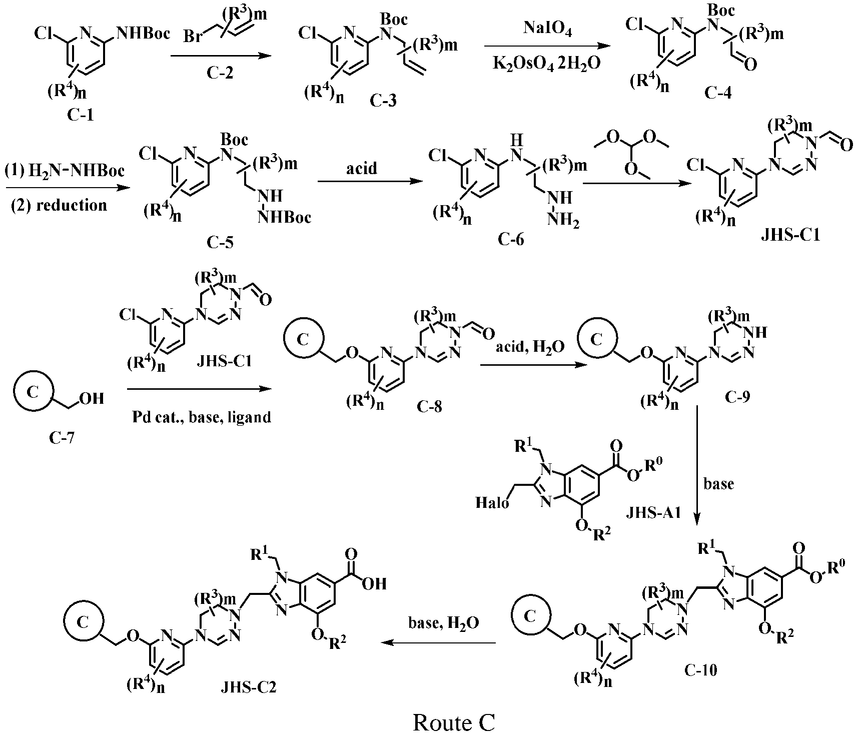

When A in the general formula (I) or (II) is A 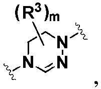 and B is 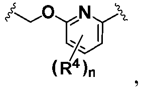 the preparation can be carried out according to route C, and the definitions of the substituents are as described in the general formula (I) or (II), and the reaction steps include:
(3.1) Intermediate C-1 and intermediate C-2 undergo nucleophilic substitution reaction under the action of a base to generate intermediate C-3; the base is, for example but not limited to, cesium carbonate, potassium hydroxide, potassium tert-butoxide; the reaction is carried out in a suitable organic solvent, for example but not limited to N,N-dimethylformamide, dimethyl sulfoxide, N-methylpyrrolidone; the reaction temperature is generally room temperature to 100 °C.
(3.2) Intermediate C-3 undergoes oxidative cleavage reaction under the action of sodium periodate and potassium osmate dihydrate to generate intermediate C-4; the reaction is carried out in a mixed system of an organic solvent and water, such as tetrahydrofuran, water, etc.; the reaction temperature is generally room temperature.
(3.3) Intermediate C-4 is reacted with tert-butyloxycarbonylhydrazine in the presence of a reducing agent to undergo a reductive amination reaction to generate intermediate C-5; the reducing agent may be, for example but not limited to, sodium triacetoxyborohydride, sodium cyanoborohydride, or sodium borohydride; the reaction is carried out in an organic solvent, such as 1,2-dichloroethane, tetrahydrofuran, toluene, etc.; the reaction temperature is generally room temperature.
(3.4) Intermediate C-5 is deprotected to remove Boc protecting group under the action of acid to generate intermediate C-6. The acid is, for example, but not limited to, trifluoroacetic acid and hydrochloric acid. The reaction temperature is generally room temperature. The reaction solvent is, for example, but not limited to, dichloromethane, tetrahydrofuran, and 1,4-dioxane.
(3.5) Intermediate C-6 is reacted with trimethyl orthoformate to produce intermediate JHS-C1; the reaction solvent is usually acetic acid; the reaction temperature is generally room temperature to 120 °C.
(3.6) Intermediate C-7 undergoes a coupling reaction with intermediate JHS-C1 to generate intermediate C-8; the coupling reaction is usually carried out under the action of a palladium catalyst, a ligand and a base; the palladium catalyst is, for example, but not limited to, palladium acetate and tris(dibenzylideneacetone)palladium; the ligand is, for example, but not limited to, 4,5-bis(diphenylphosphine)-9,9-dimethyloxanthene, 1,1'-binaphthyl-2,2'-bis(diphenylphosphine) and 2-dicyclohexylphosphine-2',6'-diisopropoxy-1,1'-biphenyl; the base is, for example, but not limited to, cesium carbonate, sodium tert-butoxide and potassium phosphate; the reaction is carried out in an appropriate organic solvent, such as 1,4-dioxane, toluene and N,N-dimethylformamide; the reaction temperature is generally from room temperature to 140 °C.
(3.7) Intermediate C-8 is hydrolyzed under acidic or alkaline conditions to generate intermediate C-9; the acid is, for example, but not limited to, hydrochloric acid; the alkaline conditions are, for example, but not limited to, sodium hydroxide or hydrazine hydrate; the reaction temperature is generally room temperature to 50 °C.
(3.8) Intermediate C-9 and intermediate JHS-A1 undergo nucleophilic substitution reaction under the action of a base to generate intermediate C-10; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(3.9) Intermediate C-10 is hydrolyzed under alkaline conditions to generate compound JHS-C2. The base may be, for example but not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.

When A in the general formula (I) or (II) is 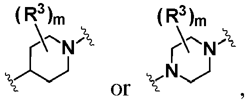 and B is 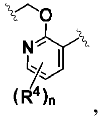 the compound can be prepared according to route D, wherein the definitions of the substituents are as described in the general formula (I) or (II), and the reaction steps include:
(4.1) Intermediate B-1 and intermediate D-1 undergo aromatic nucleophilic substitution reaction under the action of a base to generate intermediate D-2; the base is, for example, but not limited to, potassium tert-butoxide, sodium hydride, sodium tert-butoxide; the reaction is carried out in a suitable organic solvent, for example, but not limited to, tetrahydrofuran, 1,4-dioxane, N,N-dimethylformamide; the reaction temperature is generally room temperature to 80 °C.
(4.2) Intermediate D-2 undergoes Suzuki coupling reaction with a boron ester raw material (purchased commercially or synthesized according to literature methods) to generate intermediate D-3; the Suzuki coupling reaction is usually carried out in the presence of a palladium catalyst and a base; the palladium catalyst is, for example but not limited to, bis(triphenylphosphine)palladium dichloride (II), 1,1'-bis(diphenylphosphino) ferrocenepalladium dichloride (II), tetrakis(triphenylphosphine)palladium (0); the base is, for example but not limited to, triethylamine, N,N-diisopropylethylamine, potassium carbonate, cesium carbonate; the reaction is carried out in an appropriate organic solvent or a mixed solvent of an organic solvent and water, such as N,N-dimethylformamide, toluene, 1,4-dioxane; the reaction temperature is generally room temperature to 100 °C. 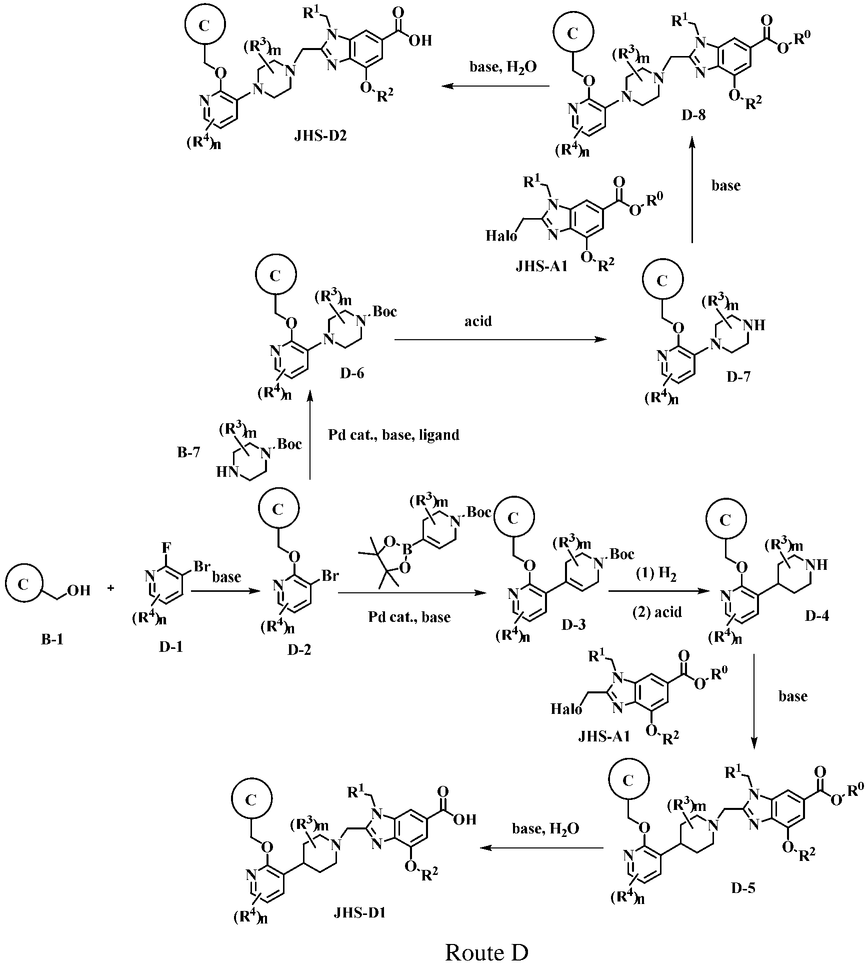
(4.3) After the intermediate D-3 is subjected to hydrogenation reaction, it is further deprotected under the action of an acid to generate the intermediate D-4; the hydrogenation reaction is carried out under the catalysis of palladium/carbon, nickel, etc.; the reaction temperature is generally room temperature; the reaction solvent includes protic solvents and non-protic solvents, such as but not limited to methanol, ethanol, ethyl acetate, tetrahydrofuran; the acid for the deprotection reaction of the Boc protecting group is such as but not limited to trifluoroacetic acid and hydrochloric acid; the reaction temperature is generally room temperature; the reaction solvent is such as but not limited to dichloromethane, tetrahydrofuran, 1,4-dioxane.
(4.4) Intermediate D-4 and intermediate JHS-A1 undergo nucleophilic substitution reaction under the action of a base to generate intermediate D-5; the base is, for example but not limited to, potassium carbonate, cesium carbonate, N,N-diisopropylethylamine; the reaction is carried out in a suitable organic solvent, for example but not limited to acetonitrile, N,N-dimethylformamide, dimethyl sulfoxide, N-methylpyrrolidone; the reaction temperature is generally room temperature to 100 °C.
(4.5) Intermediate D-5 is hydrolyzed under alkaline conditions to generate compound JHS-D1. The base may be, but is not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.
(4.6) Intermediate D-2 undergoes Buchwald-Hartwig coupling reaction with intermediate B-7 to generate intermediate D-6. The Buchwald-Hartwig coupling reaction is usually carried out under the action of a palladium catalyst, a ligand and a base. The palladium catalyst may be, but not limited to, palladium acetate and tris(dibenzylideneacetone)palladium. The ligand may be, but not limited to, 4,5-bis(diphenylphosphine)-9,9-dimethyloxanthene, 1,1'-binaphthyl-2,2'-bis(diphenylphosphine) and 2-dicyclohexylphosphine-2',6'-diisopropoxy-1,1'-biphenyl. The base may be, but not limited to, cesium carbonate, sodium tert-butoxide and potassium phosphate. The reaction is carried out in an appropriate organic solvent, such as 1,4-dioxane, toluene and N,N-dimethylformamide. The reaction temperature is generally from room temperature to 140 °C.
(4.7) Intermediate D-6 is deprotected to remove Boc protecting group under the action of acid to generate intermediate D-7. The acid is, for example, but not limited to, trifluoroacetic acid and hydrochloric acid. The reaction temperature is generally room temperature. The reaction solvent is, for example, but not limited to, dichloromethane, tetrahydrofuran, and 1,4-dioxane.
(4.8) Intermediate D-7 is reacted with intermediate JHS-A1 under the action of a base to produce intermediate D-8 by nucleophilic substitution reaction; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(4.9) Intermediate D-8 is hydrolyzed under alkaline conditions to generate compound JHS-D2. The base may be, but is not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.

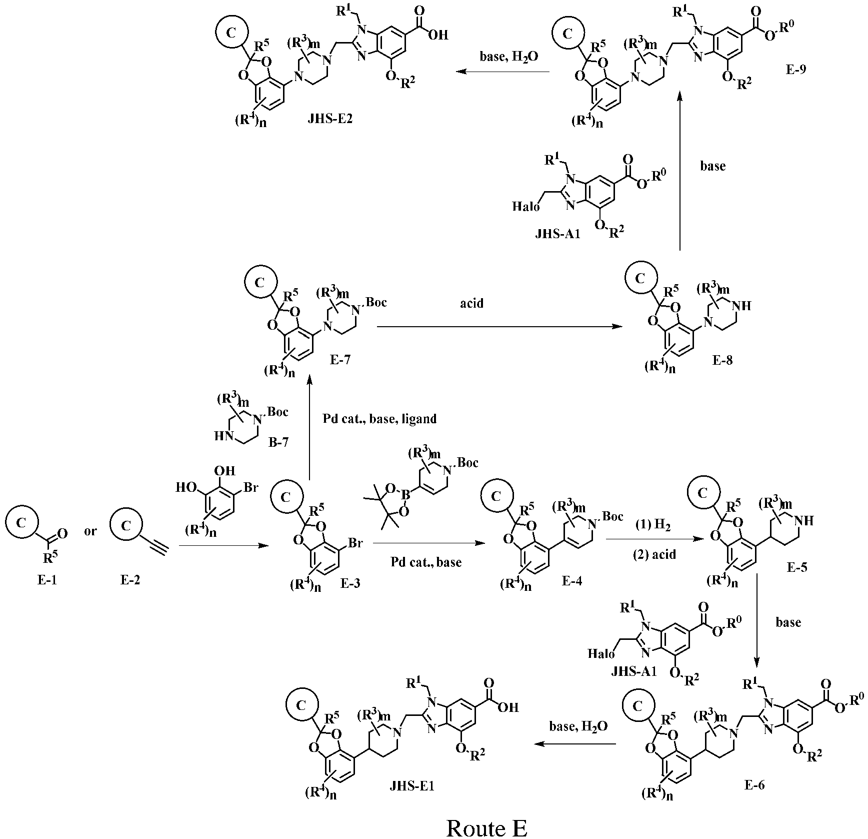

When A in the general formula (I) or (II) is 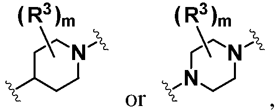 and B is 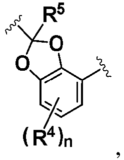 the compound can be prepared according to route E, wherein the definitions of the substituents are as described in the general formula (I) or (II), and the reaction steps include:
(5.1) Intermediate E-1 is reacted with catechol (purchased commercially or synthesized according to literature methods) under acid catalysis to generate intermediate E-3; the acid catalyst is, for example, but not limited to p-toluenesulfonic acid and pyridinium p-toluenesulfonate; the reaction is carried out in a suitable organic solvent, such as toluene; the reaction temperature is generally room temperature to 160 °C; in another preparation method, intermediate E-2 is reacted with catechol (purchased commercially or synthesized according to literature methods) under the action of a ruthenium catalyst to generate intermediate E-3; the ruthenium catalyst is, for example, but not limited to triruthenium dodecacarbonyl; the reaction is carried out in a suitable organic solvent, such as toluene; the reaction temperature is generally room temperature to 120 °C.
(5.2) Intermediate E-3 undergoes Suzuki coupling reaction with a boron ester raw material (purchased commercially or synthesized according to literature methods) to generate intermediate E-4; the Suzuki coupling reaction is usually carried out in the presence of a palladium catalyst and a base; the palladium catalyst is, for example but not limited to, bis(triphenylphosphine)palladium dichloride (II), 1,1'-bis(diphenylphosphino)ferrocenepalladium dichloride (II), tetrakis(triphenylphosphine)palladium (0); the base is, for example but not limited to, triethylamine, N,N-diisopropylethylamine, potassium carbonate, cesium carbonate; the reaction is carried out in an appropriate organic solvent or a mixed solvent of an organic solvent and water, such as N,N-dimethylformamide, toluene, 1,4-dioxane; the reaction temperature is generally room temperature to 100 °C.
(5.3) After the intermediate E-4 is hydrogenated, the Boc protecting group is removed under the action of an acid to generate the intermediate E-5; the hydrogenation reaction is carried out under the catalysis of palladium/carbon, nickel, etc.; the reaction temperature is generally room temperature; the reaction solvent includes protic solvents and non-protic solvents, such as but not limited to methanol, ethanol, ethyl acetate, tetrahydrofuran; the acid for the deprotection reaction is, for example, but not limited to trifluoroacetic acid and hydrochloric acid; the reaction temperature is generally room temperature; the reaction solvent is, for example, but not limited to, dichloromethane, tetrahydrofuran, 1,4-dioxane.
(5.4) Intermediate E-5 and intermediate JHS-A1 undergo nucleophilic substitution reaction under the action of a base to generate intermediate E-6; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(5.5) Intermediate E-6 is hydrolyzed under alkaline conditions to generate compound JHS-E1. The base may be, but is not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.
(5.6) Intermediate E-3 undergoes Buchwald-Hartwig coupling reaction with intermediate B-7 to generate intermediate E-7. The Buchwald-Hartwig coupling reaction is usually carried out under the action of a palladium catalyst, a ligand and a base. The palladium catalyst may be, but not limited to, palladium acetate and tris(dibenzylideneacetone)palladium. The ligand may be, but not limited to, 4,5-bis(diphenylphosphine)-9,9-dimethyloxanthene, 1,1'-binaphthyl-2,2'-bis(diphenylphosphine) and 2-dicyclohexylphosphine-2',6'-diisopropoxy-1,1'-biphenyl. The base may be, but not limited to, cesium carbonate, sodium tert-butoxide and potassium phosphate. The reaction is carried out in an appropriate organic solvent, such as 1,4-dioxane, toluene and N,N-dimethylformamide. The reaction temperature is generally from room temperature to 140 °C.
(5.7) Intermediate E-7 is deprotected to remove Boc protecting group to generate intermediate E-8 under the action of acid, such as but not limited to trifluoroacetic acid and hydrochloric acid; the reaction temperature is generally room temperature; the reaction solvent is such as but not limited to dichloromethane, tetrahydrofuran, 1,4-dioxane.
(5.8) Intermediate E-8 and intermediate JHS-A1 undergo nucleophilic substitution reaction under the action of a base to generate intermediate E-9; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(5.9) Intermediate E-9 is hydrolyzed under alkaline conditions to generate compound JHS-E2. The base may be, but is not limited to, lithium hydroxide or sodium hydroxide. The reaction is carried out in an appropriate organic solvent, such as acetonitrile, tetrahydrofuran, 1,4-dioxane, or methanol. The reaction temperature is generally from room temperature to 50 °C.

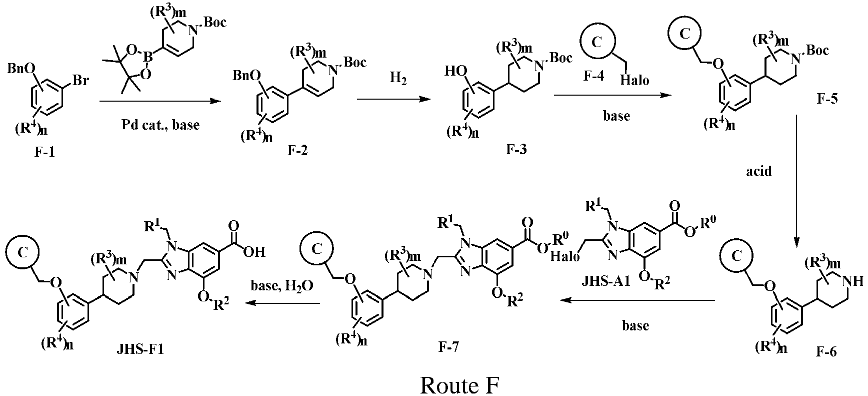

When A in the general formula (I) or (II) is 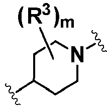 and B is 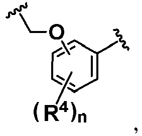 the compound can be prepared according to route F, wherein the definitions of the substituents are as described in the general formula (I), and the reaction steps include:
(6.1) Intermediate F-1 undergoes Suzuki coupling reaction with a boron ester raw material (purchased commercially or synthesized according to literature methods) to generate intermediate F-2; the Suzuki coupling reaction is usually carried out in the presence of a palladium catalyst and a base; the palladium catalyst is, for example, but not limited to, bis(triphenylphosphine)palladium dichloride (II), 1,1'-bis(diphenylphosphino)ferrocenepalladium dichloride (II), tetrakis(triphenylphosphine)palladium (0); the base is, for example, but not limited to, triethylamine, N,N-diisopropylethylamine, potassium carbonate, cesium carbonate; the reaction is carried out in a suitable organic solvent or a mixed solvent of an organic solvent and water, such as N,N-dimethylformamide, toluene, 1,4-dioxane; the reaction temperature is generally room temperature to 100 °C.
(6.2) Under the action of hydrogen and palladium/carbon, the double bond of intermediate F-2 is reduced and the benzyl protecting group is removed to generate intermediate F-3; the reaction temperature is generally room temperature; the reaction solvent includes protic solvents and aprotic solvents, such as but not limited to methanol, ethanol, ethyl acetate, tetrahydrofuran.
(6.3) Intermediate F-3 and intermediate F-4 undergo nucleophilic substitution reaction in the presence of a base to generate intermediate F-5; the base may be, but is not limited to, triethylamine, N,N-diisopropylethylamine, potassium carbonate, cesium carbonate; the reaction is carried out in a suitable organic solvent, such as acetonitrile, N,N-dimethylformamide, dimethyl sulfoxide; the reaction temperature is generally room temperature to 100 °C.
(6.4) Intermediate F-5 is deprotected under the action of an acid to generate intermediate F-6. The acid may be, but not limited to, trifluoroacetic acid or hydrochloric acid. The reaction temperature is generally room temperature. The reaction solvent may be, but not limited to, dichloromethane, tetrahydrofuran, or 1,4-dioxane.
(6.5) Intermediate F-6 and intermediate JHS-A1 undergo nucleophilic substitution reaction under the action of a base to generate intermediate F-7; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(6.6) Intermediate F-7 is hydrolyzed under alkaline conditions to generate compound JHS-F1. The base may be, but is not limited to, lithium hydroxide or sodium hydroxide. The reaction is carried out in a suitable organic solvent, such as acetonitrile, tetrahydrofuran, 1,4-dioxane, or methanol. The reaction temperature is generally from room temperature to 50 °C.

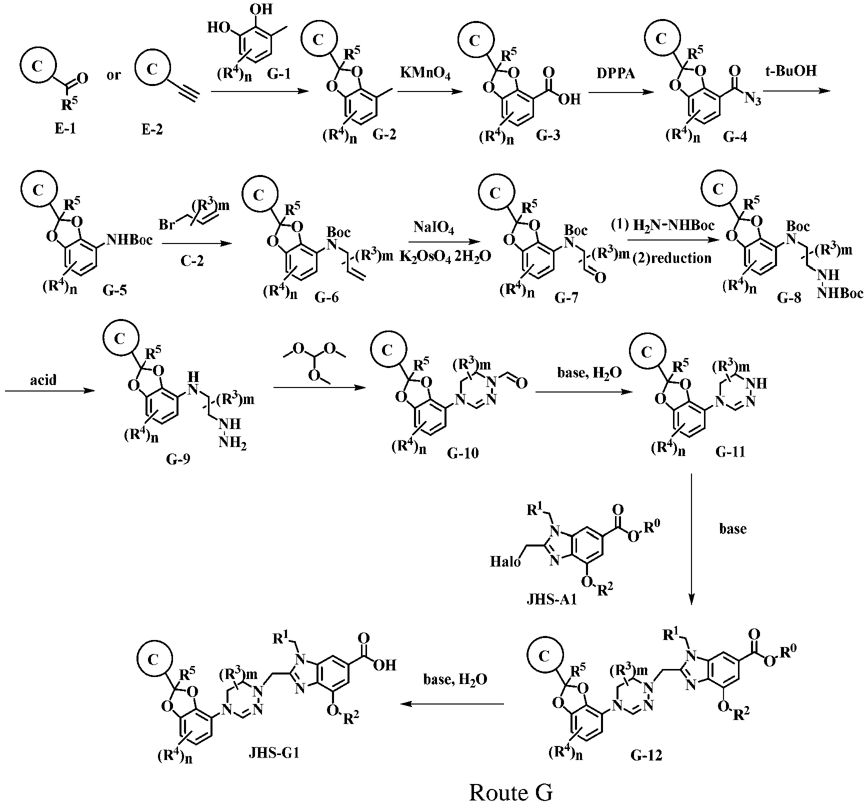

When A in the general formula (I) or (II) is 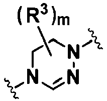 and B is 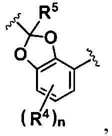 the compound can be prepared according to route G, wherein the definitions of the substituents are as described in the general formula (I) or (II), and the reaction steps include:
(7.1) Intermediate E-1 is reacted with catechol (G-1, purchased commercially or synthesized according to literature methods) under acid catalysis to generate intermediate G-2; the acid catalyst is, for example, but not limited to p-toluenesulfonic acid and pyridinium p-toluenesulfonate; the reaction is carried out in a suitable organic solvent, such as toluene; the reaction temperature is generally room temperature to 160 °C; in another preparation method, intermediate E-2 is reacted with catechol (G-1, purchased commercially or synthesized according to literature methods) under the action of a ruthenium catalyst to generate intermediate G-2; the ruthenium catalyst is, for example, but not limited to triruthenium dodecacarbonyl; the reaction is carried out in a suitable organic solvent, such as toluene; the reaction temperature is generally room temperature to 120 °C.
(7.2) Intermediate G-2 is oxidized by a strong oxidant in an alkaline system to generate intermediate G-3; the alkaline system is, for example, but not limited to, a pyridine system; the strong oxidant is, for example, but not limited to, potassium permanganate; the reaction temperature is generally room temperature to 100 °C.
(7.3) Intermediate G-3 is reacted with an azidating agent in the presence of a base to generate intermediate G-4; the azidating agent may be, but is not limited to, diphenylphosphoryl azide; the base may be, but is not limited to, triethylamine, N,N-diisopropylethylamine, 1,8-diazabicyclo[5.4.0]undec-7-ene; the reaction is carried out in an appropriate organic solvent, such as dichloromethane; the reaction temperature is generally 0 °C to room temperature.
(7.4) Intermediate G-4 is heated in a tert-butanol system to rearrange and reacted with tert-butanol to produce intermediate G-5; the reaction temperature is generally 50 °C to 120 °C.
(7.5) Intermediate G-5 and intermediate C-2 undergo nucleophilic substitution reaction under the action of a base to generate intermediate G-6; the base is, for example but not limited to, cesium carbonate, potassium hydroxide, potassium tert-butoxide; the reaction is carried out in a suitable organic solvent, for example but not limited to N,N-dimethylformamide, dimethyl sulfoxide, N-methylpyrrolidone; the reaction temperature is generally room temperature to 100 °C.
(7.6) Intermediate G-6 undergoes oxidative cleavage reaction under the action of sodium periodate and potassium osmate dihydrate to generate intermediate G-7; the reaction is carried out in a mixed system of organic solvent and water, such as tetrahydrofuran, water, etc.; the reaction temperature is generally room temperature.
(7.7) Intermediate G-7 is reacted with tert-butyloxycarbonylhydrazine in the presence of a reducing agent to undergo a reductive amination reaction to generate intermediate G-8; the reducing agent may be, for example but not limited to, sodium triacetoxyborohydride, sodium cyanoborohydride, or sodium borohydride; the reaction is carried out in an organic solvent, such as 1,2-dichloroethane, tetrahydrofuran, toluene, etc.; the reaction temperature is generally room temperature.
(7.8) Intermediate G-8 is deprotected to remove Boc protecting group under the action of acid to generate intermediate G-9. The acid is, for example, but not limited to, trifluoroacetic acid and hydrochloric acid. The reaction temperature is generally 0 °C to room temperature. The reaction solvent is, for example, but not limited to, dichloromethane, tetrahydrofuran, and 1,4-dioxane.
(7.9) Intermediate G-9 is reacted with trimethyl orthoformate to produce intermediate G-10; the reaction solvent is, for example but not limited to, acetic acid and 1,4-dioxane; the reaction temperature is generally room temperature to 120 °C.
(7.10) Intermediate G-10 is hydrolyzed under acidic or alkaline conditions to generate intermediate G-11; the acid is, for example, but not limited to, hydrochloric acid; the alkaline conditions are, for example, but not limited to, sodium hydroxide or hydrazine hydrate; the reaction temperature is generally room temperature to 80 °C.
(7.11) Intermediate G-11 and intermediate JHS-A1 undergo nucleophilic substitution reaction under the action of a base to generate intermediate G-12; the base is, for example but not limited to, potassium carbonate, cesium carbonate, N,N-diisopropylethylamine; the reaction is carried out in a suitable organic solvent, such as acetonitrile, N,N-dimethylformamide, dimethyl sulfoxide, N-methylpyrrolidone; the reaction temperature is generally room temperature to 100 °C.
(7.12) Intermediate G-12 is hydrolyzed under alkaline conditions to generate compound JHS-G1. The base may be, but is not limited to, lithium hydroxide or sodium hydroxide. The reaction is carried out in an appropriate organic solvent, such as acetonitrile, tetrahydrofuran, 1,4-dioxane, or methanol. The reaction temperature is generally from room temperature to 50 °C.

In the present invention, appropriate protecting groups and methods for protection and deprotection using such protecting groups are well known to those skilled in the art, and examples thereof can be found in literature or reference books, such as T. Greene and P. Wuts, Protecting Groups in Organic Synthesis (4th edition), John Wiley & Sons (2007), which are incorporated herein by reference in their entirety. Those skilled in the art can adjust the reaction conditions appropriately according to literature data or actual conditions encountered during the synthesis process.

It should be understood that within the scope of the present invention, the above-mentioned technical features and the technical features specifically described below (such as examples) can be combined with each other to form new or preferred technical solutions. Each feature disclosed in the specification may be replaced by any alternative feature that provides the same, equal or similar purpose. Due to limited space, it will not be repeated one by one here.

### Brief description of the drawings

Figure 1 shows the concentration-activation effect curve of the compounds in recruiting β-arrestin 1 (n=3).
Figure 2 shows the concentration-activation effect curve of the compounds in recruiting β-arrestin 2 (n=3).
Figure 3 shows the blood glucose concentration-time curves of the ammonium salt of the positive control PF-06882961 and compound 20 in an oral glucose tolerance test in mice (n=5; means±SEM; **, p<0.01 vs blank control; ***, p<0.001 vs blank control).
Figure 4 shows the blood glucose area under the curve AUC₍₀₋₉₀₎ from 0 to 90 minutes of the ammonium salt of the positive control PF-06882961 and compound 20 in an oral glucose tolerance test in mice (n=5; means±SEM; ***, p<0.001 vs blank control; #, p<0.05, compound 20 vs PF).
Figure 5 shows the blood glucose concentration-time curves of the ammonium salt of the positive control PF-06882961 and compound 19 in an oral glucose tolerance test in mice (n=8; means±SEM; *, p<0.05 vs blank control; **, p<0.01 vs blank control; ***, p<0.001 vs blank control).
Figure 6 shows the blood glucose area under the curve AUC₍₀₋₉₀₎ from 0 to 90 minutes (A) and the blood glucose area under the curve AUC₍₁₈₀₋₂₇₀₎ from 180 to 270 minutes (B) of the ammonium salt of the positive control PF-06882961 and compound 19 in an oral glucose tolerance test in mice (n=8; means±SEM; **, p<0.01 vs blank control; ***, p<0.001 vs blank control; ###, p<0.001, compound 19 vs PF).
Figure 7 shows the changes in food intake of mice within 2.5, 5 and 12 hours after oral administration of the ammonium salt of the positive control PF-06882961 and compound 19 (n=5; means±SEM; *, p<0.05 vs blank control; **, p<0.01 vs blank control; ***, p<0.001 vs blank control).
Figure 8 shows the changes in food intake of mice within 2.5, 5 and 12 hours after oral administration of the ammonium salt of the positive control PF-06882961 and compound 76-1 (n=7; means±SD; *, p<0.05 vs blank control; **, p<0.01 vs blank control; ***, p<0.001 vs blank control).
Figure 9 shows the effects of ammonium salt of positive control PF-06882961 and compound 76-1 on body weight of HFD-induced obese mice (n=9; mean±SD; *, p<0.05 vs blank control; **, p<0.01 vs blank control; ***, p<0.001 vs blank control).
Figure 10 shows the effects of ammonium salt of positive control PF-06882961 and compound 76-1 on fat (left) and lean (right) content in HFD-induced obese mice (n=9; mean±SD; ***, p<0.001 vs blank control).
Figure 11 shows the blood glucose concentration-time curve (A) and blood glucose area under the curve (B) of ammonium salt of positive control PF-06882961 and compound 76-1 in an oral glucose tolerance test in mice (n=6; means±SEM; **, p<0.01 vs blank control; ***, p<0.001 vs blank control).
Figure 12 shows the changes in food intake of mice within 2.5, 5, 12 and 24 hours after oral administration of Tris salt of positive control PF-06882961, compounds 81-1, and 82-1 (n=8; means±SD; *, p<0.05 vs blank control; **, p<0.01 vs blank control; ***, p<0.001 vs blank control; #, p<0.05 compound 81-1 vs PF-06882961).

### Detailed description

After extensive and in-depth research, the inventors have developed a 4-alkoxybenzimidazole-6-carboxylic acid compound and found that the introduction of -OR² at the 4-position of benzimidazole has achieved unexpected effects compared to the substituents such as -H, halogen, alkyl, and cycloalkyl disclosed in the prior art, which are as follows: (1) the GLP-1 receptor agonist activity of the compound is increased by several to dozens of times, reaching the level of endogenous GLP-1; (2) the activation effect on the β-arrestin pathway of the GLP-1 receptor downstream is weakened, thereby increasing the signal bias. These biological features enable the compounds of the present invention to exhibit better effects in reducing blood sugar and inhibiting diet in animal experiments. Therefore, when used for treatment, it is expected to translate into better clinical efficacy and increase the clinical benefits of patients to a greater extent.

### Term

In the present invention, unless otherwise specified, the terms used have the general meanings well known to those skilled in the art.

In the present invention, the halogen is F, Cl, Br or I.

In the present invention, the term "C1-C6" refers to having 1, 2, 3, 4, 5 or 6 carbon atoms, "C3-C8" refers to having 3, 4, 5, 6, 7 or 8 carbon atoms, "C1-C3" refers to having 1, 2 or 3 carbon atoms, and so on. "5-6 membered" means having 5 or 6 ring atoms, "4-8 membered" means having 4, 5, 6, 7 or 8 ring atoms, and so on.

In the present invention, the term "alkyl" refers to a saturated straight or branched hydrocarbon moiety. For example, the term "C1-C6 alkyl" refers to a straight or branched alkyl having 1 to 6 carbon atoms, including but not limited to methyl, ethyl, n-propyl, isopropyl, n-butyl, isobutyl, sec-butyl, tert-butyl, n-pentyl, isopentyl, neopentyl, tert-pentyl and n-hexyl, etc.

In the present invention, the term "alkoxy" refers to a "-O-alkyl" group. For example, the term "C1-C6 alkoxy" refers to a straight or branched alkoxy group having 1 to 6 carbon atoms, including but not limited to methoxy, ethoxy, n-propoxy, isopropoxy, n-butoxy, tert-butoxy, and the like.

In the present invention, the term "alkenyl" refers to a straight chain or branched hydrocarbon moiety containing at least one double bond. For example, the term "C2-C6 alkenyl" refers to a straight chain or branched alkenyl group having 2 to 6 carbon atoms and containing at least one double bond, including but not limited to ethenyl, propenyl, butenyl, isobutenyl, pentenyl and hexenyl.

In the present invention, the term "alkynyl" refers to a straight chain or branched alkynyl group containing at least one triple bond. For example, the term "C2-C6 alkynyl" refers to a straight chain or branched alkynyl group containing at least one triple bond having 2 to 6 carbon atoms, including but not limited to ethynyl, propynyl, butynyl, isobutynyl, pentynyl and hexynyl.

In the present invention, the term "cycloalkyl" represents a non-aromatic cyclic aliphatic hydrocarbyl group with a specific number of ring carbon atoms. For example, the term "C3-C8 cycloalkyl" represents a cyclic aliphatic hydrocarbyl group composed of 3 to 8 ring carbon atoms, and so on; it should also be understood that the "cycloalkyl" described in the present invention includes not only monocyclic aliphatic hydrocarbyl groups, but also includes fused, spiro and bridged ring systems composed of multiple cyclic aliphatic hydrocarbons; the "cycloalkyl" described in the present invention includes not only aliphatic hydrocarbyl groups with fully saturated carbon atoms, but also aliphatic hydrocarbyl groups with unsaturated bonds in some carbon atoms, but does not include aromatic rings composed of multiple unsaturated carbons; examples of "cycloalkyl" described in the present invention include but are not limited to: 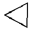 , 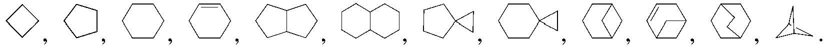 It should also be understood that when the "cycloalkyl" is used as a substituent or linker, the connection site with the molecular body can occur at any chemical bond-allowed position on the "cycloalkyl".

In the present invention, the term "aryl" refers to a monocyclic system and a polycyclic system composed of a specific number of carbon atoms and complying with the Hückel rule; it should be understood that when the "aryl" described in the present invention is a polycyclic system, it not only includes the case where all rings are aromatic rings, but also includes the case where only one of the rings is aromatic and the other rings are non-aromatic aliphatic rings; for example, the term "C6-C10 aryl" refers to a cyclic system having 6 to 10 carbon atoms and at least one of the rings is an aromatic ring, and so on; examples of the "aryl" described in the present invention include but are not limited to 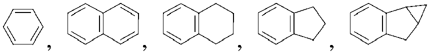 and the like. It should be understood that when the "aryl" is used as a substituent or a linker, the connection site with the main body of the molecule occurs on the aromatic ring.

In the present invention, the term "heterocyclyl" refers to a saturated or partially unsaturated, and non-aromatic cyclic group with a specific number of ring atoms, containing 1-4 ring heteroatoms (such as N, O or S). It should be understood that the "heterocyclyl" described in the present invention includes not only monocyclic heterocyclic systems, but also polycyclic heterocyclic systems, such as fused, spiro and bridged rings. When the "heterocyclyl" is a polycyclic system, at least one ring contains a ring heteroatom, and the other rings can be heterocyclic or aliphatic rings. For example, the term "4-8 membered heterocyclyl" refers to a saturated or partially unsaturated monocyclic or polycyclic system with 4 to 8 ring atoms, at least one of which is a heteroatom, and so on. Examples of the "heterocyclyl" described in the present invention include but are not limited to 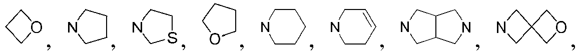 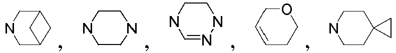 and the like. It should be understood that when the "heterocyclyl" is used as a substituent or linker, the connection site with the main body of the molecule can occur at any position on the "heterocyclyl" where chemical bonds are allowed.

In the present invention, the term "heteroaryl" refers to an aromatic cyclic group with a specific number of ring atoms, containing 1-4 ring heteroatoms (such as N, O or S); it should be understood that the "heteroaryl" described in the present invention includes not only monocyclic heteroaromatic systems, but also polycyclic heteroaromatic systems; when the "heteroaryl" is a polycyclic heteroaromatic system, at least one ring is aromatic, the other rings may be aromatic or non-aromatic, and the heteroatoms may be in the aromatic ring or in the non-aromatic ring; for example, the term "5-12 membered heteroaryl" refers to a monocyclic or polycyclic system with 5 to 12 ring atoms, at least one of which is a heteroatom, and at least one ring is aromatic; examples of the "heteroaryl" described in the present invention include but are not limited to 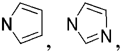 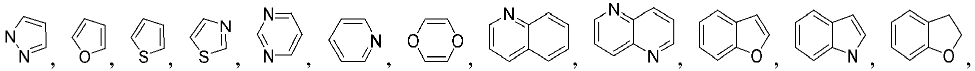 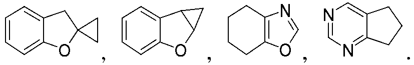 It should be understood that when the "heteroaryl" is used as a substituent or linker, the connection site with the molecular body occurs on the aromatic ring.

The term "independently" used in the present invention means that when several substituents defined simultaneously are selected from the same series of candidate groups, they do not affect each other and may be the same or different.

The term "substituted" as used herein means replaced by one or more groups. If no specific atom is specified, it means that it can occur on any atom that has not reached saturation with substituents.

The "pharmaceutically acceptable salt" mentioned in the present invention refers to a salt formed by a positively charged group on the compound represented by the general formula (I) or (II) and an anion, or a salt formed by a negatively charged group on the compound represented by the general formula (I) or (II) and a cation. Suitable anions include, but are not limited to, chloride, bromide, iodide, sulfate, nitrate, phosphate, citrate, methanesulfonate, trifluoroacetate, acetate, malate, toluenesulfonate, tartrate, fumarate, glutamate, glucuronate, lactate, glutarate or maleate, and the like. Suitable cations include, but are not limited to, sodium ion, potassium ion, magnesium ion, calcium ion, ammonium ion, and the like.

In another preferred embodiment, the pharmaceutically acceptable salts of the present invention refer to salts formed by the compound represented by general formula (I) or (II) with the following acids: hydrofluoric acid, hydrochloric acid, hydrobromic acid, phosphoric acid, acetic acid, oxalic acid, sulfuric acid, nitric acid, methanesulfonic acid, aminosulfonic acid, salicylic acid, trifluoromethanesulfonic acid, naphthalenesulfonic acid, maleic acid, citric acid, acetic acid, lactic acid, tartaric acid, succinic acid, oxalic acid, pyruvic acid, malic acid, glutamic acid, p-toluenesulfonic acid, naphthalenesulfonic acid, ethanesulfonic acid, naphthalenedisulfonic acid, malonic acid, fumaric acid, propionic acid, oxalic acid, trifluoroacetic acid, stearic acid, pamoic acid, hydroxymaleic acid, phenylacetic acid, benzoic acid, glutamic acid, ascorbic acid, p-aminobenzenesulfonic acid, 2-acetoxybenzoic acid and isethionic acid; or a sodium salt, potassium salt, calcium salt, aluminum salt or ammonium salt formed by the compound represented by the general formula (I) or (II) and an inorganic base; or a methylamine salt, ethylamine salt or ethanolamine salt formed by the compound represented by the general formula (I) or (II) and an organic base.

In another preferred embodiment, the pharmaceutically acceptable salt is a sodium salt.

In another preferred embodiment, the pharmaceutically acceptable salt is an ammonium salt.

In another preferred embodiment, the pharmaceutically acceptable salt is preferably 1,3-dihydroxy-2-(hydroxymethyl)propyl-2-amine (also known as tris(hydroxymethyl)aminomethane, tromethamine, etc., abbreviated as Tris) salt.

The compound of the general formula (I) or (II) of the present invention or a pharmaceutically acceptable salt thereof is obtained by distillation, crystallization or recrystallization from water or an organic solvent, and the compound may contain the solvent molecules used. In addition, different crystallization conditions may lead to different crystalline forms of the compound. Therefore, the compounds represented by the general formula (I) or (II) or their pharmaceutically acceptable salts containing different chemical dosages of crystallization solvents and all crystalline forms are within the scope of the present invention.

Some compounds represented by the general formula (I) or (II) of the present invention have chiral centers, potential chiral centers or unsaturated bonds, and can form various forms of stereoisomers, such as racemates, enantiomers, diastereomers, E/Z isomers, cis-trans isomers, tautomers, etc. Unless otherwise indicated, in the specification and appended claims, a given chemical formula or name is intended to encompass all forms of stereoisomers, as well as mixtures consisting of individual isomers in varying proportions, and pharmaceutically acceptable salts thereof. A person skilled in the art can use common laboratory separation methods to separate the compounds containing asymmetric centers in the present invention to obtain single isomers, but this does not destroy the novelty of the compounds of the present invention.

Replacing hydrogen atoms with deuterium atoms to change the physical and chemical properties of a compound has become a structural modification method well known to those skilled in the art. Unless otherwise stated, the present invention intends that the deuterated forms of the compounds represented by formula (I) or (II) are also included in the invention.

"Diabetic complications" are complications caused by diabetes or hyperglycemia, and may be acute complications or chronic complications. The term "acute complications" includes ketoacidosis and infectious diseases (e.g., skin infections, soft tissue infections, biliary system infections, respiratory system infections, urinary tract infections), and "chronic complications" include, for example, microangiopathy (e.g., nephropathy, retinopathy), neuropathy (e.g., sensory nerve disorders, motor nerve disorders, autonomic nerve disorders), and gangrene. Major complications of diabetes include diabetic retinopathy, diabetic nephropathy, and diabetic neuropathy.

"Coronary heart disease" includes asymptomatic myocardial ischemia (latent coronary heart disease), myocardial infarction, angina pectoris, ischemic heart failure (ischemic cardiomyopathy) and sudden death.

"Dementia" includes, for example, Alzheimer's disease, early onset dementia (EOD), vascular dementia, and diabetic dementia.

The compounds of the present invention may be administered alone or in combination with other drugs known to treat or ameliorate similar conditions.

The compounds of the present invention may be used in combination with one or more other drugs to treat, prevent or ameliorate diseases for which the compounds of the present invention or other drugs may be effective, where the combined use of these drugs is safer or more effective than the use of any one drug alone. The other drugs can be administered simultaneously with, before or after the compound of the present invention via common administration routes and dosages. When the compound of the present invention is used contemporaneously with one or more other drugs, a pharmaceutical composition in unit dosage form containing the other drugs and the compound of the present invention is preferred. However, drug combinations may also include therapies in which the compounds described herein and one or more other drugs are administered in various overlapping schedules. When used in combination with one or more other active ingredients, the compound of the present invention and the other drug may be used in lower doses than when used alone.

The drugs or active ingredients that can be used in combination with the compounds of the present invention include, but are not limited to, the following drugs for treating obesity: glucagon-like peptide-1 (GLP-1) analogs (such as Semaglutide), glucagon (GCG) analogs, peptide YY (PYY) analogs, oxyntomodulating peptide (OXM) analogs, pancreatic polypeptide (PP) analogs, cholecystokinin (CCK) analogs, leptin analogs, amylin analogs, fibroblast growth factor 21 (FGF21) analogs, neuropeptide Y receptor type 2 (Y-2R) agonists, melanocortin receptor 4 (MC4R) agonists, glucose-dependent insulinotropic polypeptide (GIP) receptor agonists, lipase inhibitors (such as orlistat), AMPK agonists, neuropeptide Y5 receptor antagonists, GPR40 agonists, cannabinoid type 1 receptor blockers, naltrexone/bupropion, lorcaserin, phentermine/topiramate obesity vaccine, or GLP-1 receptor-based dual or multiple receptor modulators, such as GLP-1/GIP dual receptor agonists (such as Tirzepatide), GLP-1 receptor agonist/GIP receptor antagonist conjugates, GLP-1/GCG dual receptor agonists, GLP-1/GIP/GCG triple receptor agonists, GLP-1/FGF21 fusion protein, and the like.

Drugs or active ingredients that can be used in combination with the compounds of the present invention include, but are not limited to, the following drugs for treating diabetes: biguanides, thiazolidinediones, glinides, sulfonylureas, DPP4 inhibitors, SGLT1 and/or SGLT2 inhibitors, GPR40 agonists, α-glucosidase inhibitors, glucokinase agonists, insulin, insulin analogs, GLP-1 analogs, or dual or multiple receptor modulators based on the GLP-1 receptor, such as GLP-1/GIP dual receptor agonists (such as Tirzepatide), GLP-1 receptor agonist/GIP receptor antagonist conjugates, GLP-1/GCG dual receptor agonists, GLP-1/GIP/GCG triple receptor agonists, GLP-1/FGF21 fusion proteins, and the like.

Drugs or active ingredients that can be used in combination with the compounds of the present invention include, but are not limited to, the following drugs for treating NASH: FXR receptor agonists, PPARα/δ agonists, fibroblast growth factor 19/21 analogs, thyroid hormone receptor β agonists, SGLT1 and/or SGLT2 inhibitors, acetyl-CoA carboxylase inhibitors, chemokine receptor-2/5 inhibitors, anti-apoptotic signal-regulating kinase 1 inhibitors, ATP-binding transporter 1 agonists, 5-lipoxygenase inhibitors, or vascular adhesion protein 1 inhibitors.

When using the pharmaceutical composition, a safe and effective amount of the compound of the present invention is administered to a mammal (such as a human) in need of treatment, wherein the dosage when administered is a pharmaceutically effective dosage. For a person weighing 60 kg, the daily dosage is usually 1 to 2000 mg, preferably 20 to 500 mg. Of course, the specific dosage should also take into account factors such as the route of administration and the patient's health status, which are all within the skills of a skilled physician.

The present invention will be further described below with reference to examples. It should be particularly pointed out that these examples are only used to illustrate the present invention, but not to limit the present invention in any way. Unless defined otherwise, all technical and scientific terms used herein have the same meanings as commonly understood by one skilled in the art. In addition, any methods and materials similar or equivalent to those described herein can be applied to the present invention. The preferred embodiments and materials described herein are for exemplary purposes only.

### Preparation Example

Unless otherwise specified, the fillers used for column chromatography separation are all silica gel. The experimental methods for which specific conditions are not specified in the following examples are generally performed under conventional conditions (such as the conditions described in Sambrook et al., Molecular Cloning: A Laboratory Manual (New York: Cold Spring Harbor Laboratory Press, 1989) or under conditions recommended by the manufacturer. All parameters and other descriptions in the examples are based on quality unless otherwise stated. Unless otherwise indicated, percentages and parts are by weight.

### Example 1: Preparation of (S)-2-((4-(6-((4-cyano-2-fluorobenzyl)oxy)pyridin-2-yl)piperidin-1-yl)methyl)-4-methoxy-1-(oxetan-2-ylmethyl)-1H-benzo[d]imidazole-6-carboxylic acid (Compound 1)

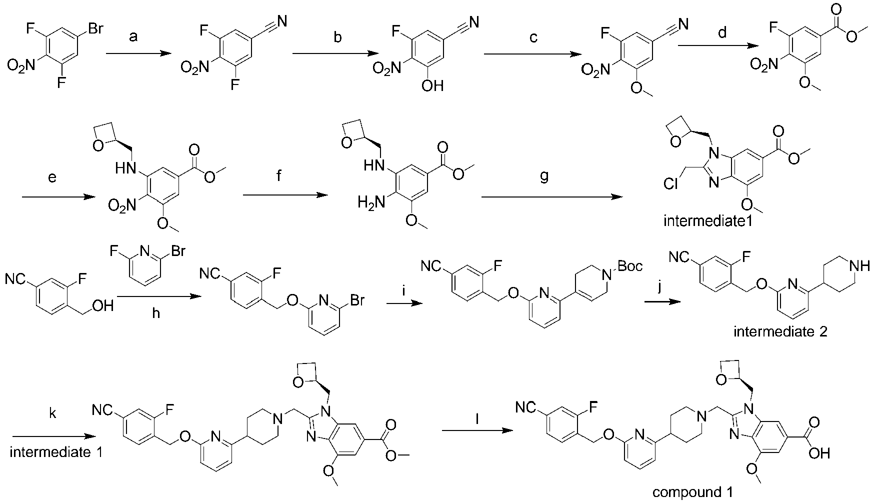

Methyl (S)-2-(chloromethyl)-4-methoxy-1-(oxetan-2-ylmethyl)-1H-benzo[d]imidazole-6-carboxylater (Intermediate 1) was prepared according to the route shown in the above route A:
Step a: 5-bromo-1,3-difluoro-2-nitrobenzene (5 g, 21.1 mmol), zinc cyanide (4.96 g, 42.2 mmol), 1,1'-bis(diphenylphosphino)ferrocene (468 mg, 0.84 mmol) and tris(dibenzylideneacetone)palladium (386 mg, 0.42 mmol) were dissolved in 30 ml of N,N-dimethylformamide and reacted at 110 °C for 12 hours under nitrogen protection. After the reaction was completed, the mixture was extracted with ethyl acetate three times and washed three times with saturated brine. The organic phase was dried over anhydrous magnesium sulfate and filtered, the filtrate was evaporated to dryness, and the crude product was separated by column chromatography to obtain 3.57 g of 3,5-difluoro-4-nitrobenzonitrile.
Step b: 3,5-difluoro-4-nitrobenzonitrile (3.57 g, 19.4 mmol) was dissolved in 20 ml of N,N-dimethylformamide, followed by addition of potassium carbonate (5.35 g, 38.8 mmol) and water (698 mg, 38.8 mmol), and the mixture was reacted at 80 °C for 4 hours. After the reaction is completed, the pH is adjusted to 4-5 with 2M hydrochloric acid solution, the mixture was extracted three times with ethyl acetate, washed three times with saturated brine, and the organic phase was dried over anhydrous magnesium sulfate and filtered. The filtrate was evaporated to dryness, and the crude product is separated by column chromatography to obtain 2.82 g of 3-fluoro-5-hydroxy-4-nitrobenzonitrile.
Step c: 3-fluoro-5-hydroxy-4-nitrobenzonitrile (2.82 g, 15.5 mmol) was dissolved in 20 ml of N,N-dimethylformamide, followed by the addition of potassium carbonate (4.28 g, 31.0 mmol). After stirring at room temperature for 15 minutes, iodomethane (2.87 g, 20.2 mmol) was added and the mixture was reacted at 50 °C for 3 hours. After the reaction was completed, the mixture was extracted three times with ethyl acetate and washed three times with saturated brine. The organic phase was dried over anhydrous magnesium sulfate and filtered. The filtrate was evaporated to dryness and the crude product was separated by column chromatography to obtain 2.98 g of 3-fluoro-5-methoxy-4-nitrobenzonitrile.
Step d: 30 ml of methanol was placed in a round-bottom flask, and under nitrogen protection, 10 ml of thionyl chloride was added dropwise under an ice-salt bath, followed by the addition of 3-fluoro-5-methoxy-4-nitrobenzonitrile (2.98 g, 15.2 mmol). The reaction mixture was moved to room temperature and stirred overnight, and then moved to 80 °C for reaction for 2 hours. After the reaction was completed, the mixture was concentrated under reduced pressure and the crude product was separated by column chromatography to obtain 3.4 g of methyl 3-fluoro-5-methoxy-4-nitrobenzoate. LCMS(ESI): m/z = 229.0 (M+H)⁺.
Step e: methyl 3-fluoro-5-methoxy-4-nitrobenzoate (3.4 g, 14.8 mmol) was dissolved in 20 ml of N,N-dimethylformamide, followed by the addition of potassium carbonate (4.08 g, 29.6 mmol) and (S)-oxetane-2-methylamine (1.3 g, 15.5 mmol), and the reaction was carried out at 80 °C for 2 hours. After the reaction was completed, the mixture was extracted three times with ethyl acetate, washed three times with saturated brine, the organic phase was dried over anhydrous magnesium sulfate and filtered, the filtrate was evaporated to dryness, and the crude product was separated by column chromatography to obtain 3.9 g of methyl (S)-3-methoxy-4-nitro-5-((oxetan-2-ylmethyl)amino)benzoate. LCMS (ESI): m/z = 296.1 (M+H)⁺.
Step f: methyl (S)-3-methoxy-4-nitro-5-((oxetan-2-ylmethyl)amino)benzoate (1 g, 3.4 mmol) was dissolved in a mixed solvent of 10 ml of methanol and 10 ml of ethyl acetate, 100 mg of 10% palladium carbon (m/m, 55% water content) was added, hydrogen was replaced, and the mixture was stirred at room temperature for 2 hours. After the reaction was completed, the mixture was filtered and the filtrate was concentrated under reduced pressure to obtain 860 mg of methyl (S)-4-amino-3-methoxy-5-((oxetan-2-ylmethyl)amino)benzoate. LCMS(ESI): m/z = 266.1 (M+H)⁺.
Step g: methyl (S)-4-amino-3-methoxy-5-((oxetan-2-ylmethyl)amino)benzoate (860 mg, 3.2 mmol) was dissolved in 15 ml of acetonitrile, 2-chloro-1,1,1-trimethoxyethane (986 mg, 6.4 mmol) and p-toluenesulfonic acid (110 mg, 0.64 mmol) were added, and the mixture was reacted at 60 °C for 1 hour. After the reaction was completed, the mixture was concentrated under reduced pressure, and the crude product was separated by column chromatography to obtain 830 mg of methyl (S)-2-(chloromethyl)-4-methoxy-1-(oxetan-2-ylmethyl)-1H-benzo[d]imidazole-6-carboxylate (intermediate 1). LCMS(ESI): m/z = 324.1 (M+H)⁺.

3-fluoro-4-(((6-(piperidin-4-yl)pyridin-2-yl)oxy)methyl)benzonitrile (Intermediate 2) was prepared according to the route shown in the above route B:
Step h: 3-fluoro-4-(hydroxymethyl)benzonitrile (1 g, 6.6 mmol) was dissolved in 20 ml of THF. Potassium tert-butoxide (1.5 g, 13.2 mmol) was added under ice bath. After stirring for 15 minutes, 2-bromo-6-fluoropyridine (1.7 g, 9.9 mmol) was added. The mixture was moved to room temperature and stirred for 3 hours. After the reaction was completed, the mixture was extracted three times with ethyl acetate, washed three times with saturated aqueous ammonium chloride solution, the organic phase was dried over anhydrous magnesium sulfate and filtered, the filtrate was concentrated under reduced pressure, and the crude product was separated by column chromatography to obtain 1.7 g of 4-(((6-bromopyridin-2-yl)oxy)methyl)-3-fluorobenzonitrile. LCMS(ESI): m/z = 306.0 (M+H)⁺.
Step i: 4-(((6-bromopyridin-2-yl)oxy)methyl)-3-fluorobenzonitrile (1.7 g, 5.6 mmol), N-Boc-1,2,5,6-tetrahydropyridine-4-boronic acid pinacol ester (2.1 g, 6.7 mmol), cesium carbonate (3.7 g, 11.2 mmol) and 1,1'-bis(diphenylphosphino)ferrocenepalladium(II) chloride (205 mg, 0.28 mmol) were dissolved in 20 ml of 1,4-dioxane and 4 ml of water, protected by nitrogen, and reacted at 100 °C for 3 hours. After the reaction was completed, the mixture was extracted three times with ethyl acetate and washed three times with saturated brine. The organic phase was dried over anhydrous magnesium sulfate and filtered. The filtrate was evaporated to dryness and the crude product was separated by column chromatography to obtain 1.8 g of tert-butyl 6-((4-cyano-2-fluorobenzyl)oxy)-3',6'-dihydro-[2,4'-bipyridine]-1'(2'H)-carboxylate. LCMS (ESI): m/z = 409.2 (M+H)⁺.
Step j: tert-butyl 6-((4-cyano-2-fluorobenzyl)oxy)-3',6'-dihydro-[2,4'-bipyridine]-1'(2'H)-carboxylate (1.8 g, 4.4 mmol) was dissolved in 20 ml of ethyl acetate, 180 mg of 10% palladium carbon (55% water) was added, hydrogen was replaced, and the mixture was stirred at room temperature for 3 hours. After the reaction was completed, the reaction mixture was filtered and the filtrate was concentrated under reduced pressure. The obtained oil was dissolved in 20 ml of dichloromethane, and 4 ml of trifluoroacetic acid was added, followed by stirring at room temperature for 4 hours. After the reaction was completed, the reaction solution was concentrated under reduced pressure, saturated sodium bicarbonate aqueous solution was added and extracted three times with dichloromethane, the organic phase was collected, dried over anhydrous magnesium sulfate, and the filtrate was evaporated to dryness to obtain 960 mg of 3-fluoro-4-(((6-(piperidin-4-yl)pyridin-2-yl)oxy)methyl)benzonitrile (Intermediate 2). LCMS(ESI): m/z = 311.1 (M+H)⁺.

(S)-2-((4-(6-((4-cyano-2-fluorobenzyl)oxy)pyridin-2-yl)piperidin-1-yl)methyl)-4-methoxy-1-(oxetan-2-ylmethyl)-1H-benzo[d]imidazole-6-carboxylic acid (Compound 1) was prepared according to the route shown in the above route B:
Step k: methyl (S)-2-(chloromethyl)-4-methoxy-1-(oxetan-2-ylmethyl)-1H-benzo[d]imidazole-6-carboxylate (Intermediate 1, 50 mg, 0.15 mmol), 3-fluoro-4-(((6-(piperidin-4-yl)pyridin-2-yl)oxy)methyl)benzonitrile (Intermediate 2, 56 mg, 0.18 mmol), potassium carbonate (41 mg, 0.3 mmol) and a catalytic amount of potassium iodide were dissolved in 5 ml of acetonitrile and reacted at 80 °C for 2 hours. After the reaction was completed, the reaction mixture was filtered and the filtrate was concentrated under reduced pressure. The mixture was separated by preparative thin layer chromatography to obtain 72 mg of methyl (S)-2-((4-(6-((4-cyano-2-fluorobenzyl)oxy)pyridin-2-yl)piperidin-1-yl)methyl)-4-methoxy-1-(oxetan-2-ylmethyl)-1H-benzo[d]imidazole-6-carboxylate. LCMS (ESI): m/z = 599.3 (M+H)⁺.
Step 1: methyl (S)-2-((4-(6-((4-cyano-2-fluorobenzyl)oxy)pyridin-2-yl)piperidin-1-yl)methyl)-4-methoxy-1-(oxetan-2-ylmethyl)-1H-benzo[d]imidazole-6-carboxylate (72 mg, 0.12 mmol) was dissolved in 3 ml of 1,4-dioxane, 2 M NaOH (300 µL, 0.6 mmol) was added, and the reaction was carried out at 40 °C overnight. After the reaction was complete, the pH was adjusted to 5-6 with 2M hydrochloric acid, and preparative liquid separation was performed to obtain 56 mg of (S)-2-((4-(6-((4-cyano-2-fluorobenzyl)oxy)pyridin-2-yl)piperidin-1-yl)methyl)-4-methoxy-1-(oxetan-2-ylmethyl)-1H-benzo[d]imidazole-6-carboxylic acid (Compound 1). LCMS (ESI): m/z = 585.2 (M+H)⁺. ¹H NMR (500 MHz, DMSO) δ 7.92 - 7.86 (m, 2H), 7.73 - 7.68 (m, 2H), 7.65 (t, *J* = 7.7 Hz, 1H), 7.26 (s, 1H), 6.89 (d, *J =* 7.3 Hz, 1H), 6.72 (d, *J =* 8.1 Hz, 1H), 5.47 (s, 2H), 5.14 - 5.05 (m, 1H), 4.80 - 4.72 (m, 1H), 4.68 - 4.60 (m, 1H), 4.51 - 4.43 (m, 1H), 4.40 - 4.32 (m, 1H), 3.96 (s, 3H), 3.91 (d, *J =* 13.4 Hz, 1H), 3.76 (d, *J =* 13.5 Hz, 1H), 2.97 (d, *J =* 11.3 Hz, 1H), 2.83 (d, *J =* 11.3 Hz, 1H), 2.73 - 2.65 (m, 1H), 2.64 - 2.53 (m, 1H), 2.46 - 2.39 (m, 1H), 2.25 - 2.11 (m, 2H), 1.80 - 1.59 (m, 4H).

### Example 2: Preparation of (S)-2-((4-(6-((4-cyano-2-fluorobenzyl)oxy)pyridin-2-yl)piperidin-1-yl)methyl)-4-ethoxy-1-(oxetan-2-ylmethyl)-1H-benzo[d]imidazole-6-carboxylic acid (Compound 2)

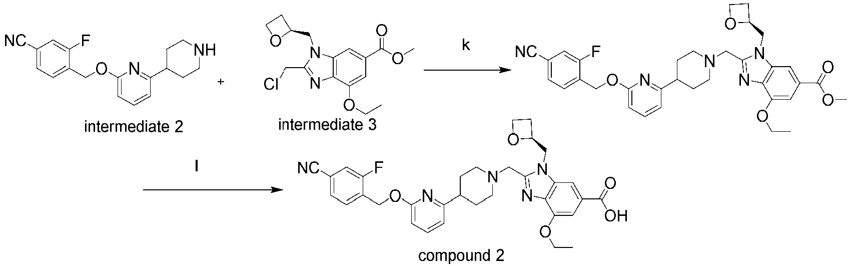

Methyl (S)-2-(chloromethyl)-4-ethoxy-1-(oxetan-2-ylmethyl)-1H-benzo[d]imidazole-6-carboxylate (Intermediate 3) was prepared according to the route shown in the above route A. For the specific synthesis method, refer to Intermediate 1. LCMS (ESI): m/z = 338.1 (M+H)⁺.

(S)-2-((4-(6-((4-cyano-2-fluorobenzyl)oxy)pyridin-2-yl)piperidin-1-yl)methyl)-4-ethoxy-1-(oxetan-2-ylmethyl)-1H-benzo[d]imidazole-6-carboxylic acid (Compound 2) was prepared according to the route shown in the aforementioned route B. The specific synthesis method was the same as Example 1, except that Intermediate 1 was replaced by Intermediate 3. LCMS(ESI): m/z = 599.3 (M+H)⁺. ¹H NMR (500 MHz, DMSO) δ 7.92 - 7.87 (m, 2H), 7.73 - 7.68 (m, 2H), 7.65 (t, *J* = 7.8 Hz, 1H), 7.25 (d, *J* = 1.2 Hz, 1H), 6.89 (d, *J* = 7.3 Hz, 1H), 6.73 (d, *J* = 8.2 Hz, 1H), 5.47 (s, 2H), 5.14 - 5.06 (m, 1H), 4.81 - 4.73 (m, 1H), 4.68 - 4.61 (m, 1H), 4.51 - 4.44 (m, 1H), 4.42 - 4.32 (m, 1H), 4.25 (q, *J =* 6.9 Hz, 2H), 3.91 (d, *J =* 13.4 Hz, 1H), 3.77 (d, *J =* 13.4 Hz, 1H), 2.96 (d, *J* = 11.1 Hz, 1H), 2.83 (d, *J =* 11.3 Hz, 1H), 2.75 - 2.64 (m, 1H), 2.63 - 2.53 (m, 1H), 2.48 - 2.37 (m, 1H), 2.25 - 2.11 (m, 2H), 1.84 - 1.56 (m, 4H), 1.43 (t, *J* = 7.0 Hz, 3H).

### Example 3: Preparation of (S)-2-((4-(6-((4-cyano-2-fluorobenzyl)oxy)pyridin-2-yl)piperidin-1-yl)methyl)-1-(oxetan-2-ylmethyl)-4-propoxy-1H-benzo[d]imidazole-6-carboxylic acid (Compound 3)

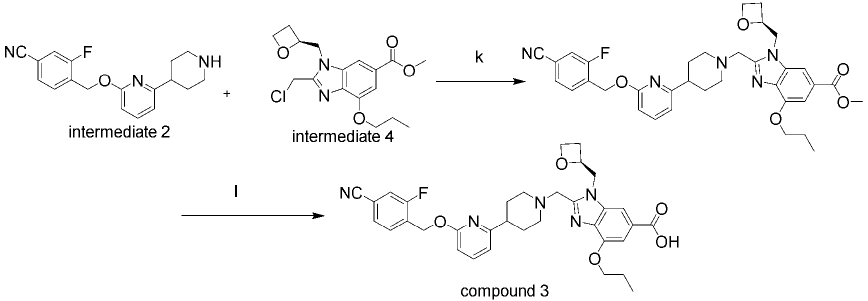

Methyl (S)-2-(Chloromethyl)-1-(oxetan-2-ylmethyl)-4-propoxy-1H-benzo[d]imidazole-6-carboxylate (Intermediate 4) was prepared according to the route shown in the above route A. For the specific synthesis method, refer to Intermediate 1. LCMS(ESI): m/z = 352.1 (M+H)⁺.

(S)-2-((4-(6-((4-cyano-2-fluorobenzyl)oxy)pyridin-2-yl)piperidin-1-yl)methyl)-1-(oxetan-2-ylmethyl)-4-propoxy-1H-benzo[d]imidazole-6-carboxylic acid (Compound 3) was prepared according to the route shown in the aforementioned route B. The specific synthesis method was the same as that in Example 1, except that Intermediate 1 was replaced by Intermediate 4. LCMS(ESI): m/z = 613.3 (M+H)⁺. ¹H NMR (500 MHz, DMSO) δ 7.93-7.88 (m, 2H), 7.77 - 7.60 (m, 3H), 7.27 - 7.21 (m, 1H), 6.90 (d, *J =* 7.5 Hz, 1H), 6.74 (d, *J =* 7.9 Hz, 1H), 5.48 (s, 2H), 5.10 - 5.07 (m, 1H), 4.82 - 4.74 (m, 1H), 4.69 - 4.62 (m, 1H), 4.52 - 4.44 (m, 1H), 4.38 - 4.34 (m, 1H), 4.16 (t, *J =* 6.7 Hz, 2H), 3.91 (d, *J =* 13.4 Hz, 1H), 3.77 (d, *J =* 13.4 Hz, 1H), 2.96 (d, *J =* 11.1 Hz, 1H), 2.82 (d, *J =* 11.2 Hz, 1H), 2.74 - 2.65 (m, 1H), 2.66 - 2.57 (m, 1H), 2.47 - 2.36 (m, 1H), 2.30 - 2.06 (m, 2H), 1.87 - 1.79 (m, 2H), 1.78 - 1.57 (m, 4H), 1.04 (t, *J =* 7.4 Hz, 3H).

### Example 4: Preparation of (S)-2-((4-(6-((4-cyano-2-methoxybenzyl)oxy)pyridin-2-yl)piperidin-1-yl)methyl)-4-methoxy-1-(oxetan-2-ylmethyl)-1H-benzo[d]imidazole-6-carboxylic acid (Compound 4)

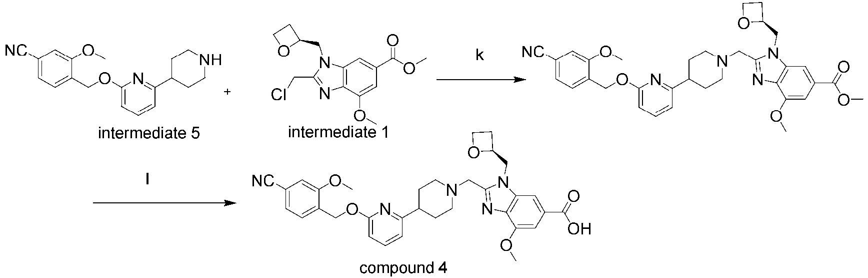

3-Methoxy-4-(((6-(piperidin-4-yl)pyridin-2-yl)oxy)methyl)benzonitrile (Intermediate 5) was prepared according to the route shown in route B. The specific synthesis method was the same as that of Intermediate 2. LCMS (ESI): m/z = 323.2 (M+H)⁺.

(S)-2-((4-(6-((4-cyano-2-methoxybenzyl)oxy)pyridin-2-yl)piperidin-1-yl)methyl)-4-methoxy-1-(oxetan-2-ylmethyl)-1H-benzo[d]imidazole-6-carboxylic acid (Compound 4) was prepared according to the route shown in route B. The specific synthesis method was the same as Example 1, except that Intermediate 2 was replaced by Intermediate 5. LCMS (ESI): m/z = 597.3 (M+H)⁺. ¹H NMR (500 MHz, DMSO) δ 7.91 (d, *J =* 1.3 Hz, 1H), 7.64 (dd, *J =* 8.2, 7.3 Hz, 1H), 7.57 - 7.51 (m, 2H), 7.42 (dd, *J =* 7.6, 1.5 Hz, 1H), 7.26 (d, *J =* 1.3 Hz, 1H), 6.87 (d, *J =* 7.3 Hz, 1H), 6.72 (d, *J =* 8.2 Hz, 1H), 5.38 (s, 2H), 5.13 - 5.04 (m, 1H), 4.75 (dd, *J =* 15.2, 7.1 Hz, 1H), 4.63 (dd, *J =* 15.2, 2.9 Hz, 1H), 4.50 - 4.42 (m, 1H), 4.39 - 4.31 (m, 1H), 3.96 (s, 3H), 3.89 (s, 3H), 3.91 (d, *J =* 13.1 Hz, 1H), 3.75 (d, *J =* 13.0 Hz, 1H), 2.97 (d, *J =* 11.0 Hz, 1H), 2.84 (d, *J =* 11.0 Hz, 1H), 2.74 - 2.63 (m, 1H), 2.62 - 2.54 (m, 1H), 2.46 - 2.38 (m, 1H), 2.28 - 2.08 (m, 2H), 1.82 - 1.59 (m, 4H).

### Example 5: Preparation of (S)-2-((4-(6-((4-cyano-2-fluorobenzyl)oxy)pyridin-2-yl)piperidin-1-yl)methyl)-4-(cyclopropylmethoxy)-1-(oxetan-2-ylmethyl)-1H-benzo[d]imidazole-6-carboxylic acid (Compound 5)

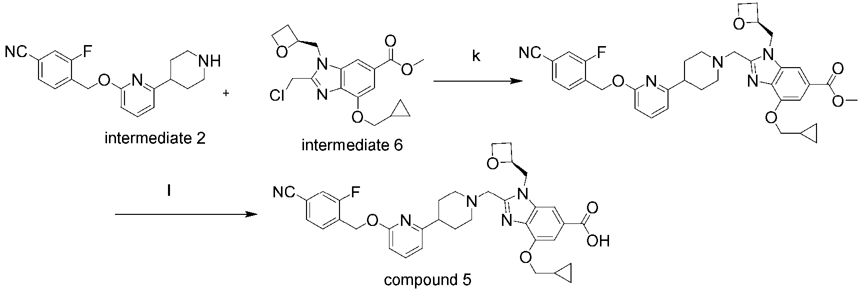

Methyl (S)-2-(Chloromethyl)-4-(cyclopropylmethoxy)-1-(oxetan-2-ylmethyl)-1H-benzo[d]imidazole-6-carboxylate (Intermediate 6) was prepared according to the route shown in route A. The specific synthesis method was the same as that of Intermediate 1. LCMS(ESI): m/z = 364.1 (M+H)⁺.

(S)-2-((4-(6-((4-cyano-2-fluorobenzyl)oxy)pyridin-2-yl)piperidin-1-yl)methyl)-4-(cyclopropylmethoxy)-1-(oxetan-2-ylmethyl)-1H-benzo[d]imidazole-6-carboxylic acid (Compound 5) was prepared according to the route shown in route B. The specific synthesis method was the same as Example 1, except that Intermediate 1 was replaced by Intermediate 6. LCMS(ESI): m/z = 625.3 (M+H)⁺. ¹H NMR (600 MHz, DMSO) δ 7.89 (d, *J =* 9.8 Hz, 1H), 7.73 - 7.67 (m, 3H), 7.65 (t, *J =* 7.8 Hz, 1H), 7.26 (s, 1H), 6.89 (d, *J =* 7.3 Hz, 1H), 6.72 (d, *J =* 8.1 Hz, 1H), 5.47 (s, 2H), 5.13 - 5.06 (m, 1H), 4.73 - 4.66 (m, 1H), 4.62 - 4.56 (m, 1H), 4.50 - 4.44 (m, 1H), 4.39 - 4.32 (m, 1H), 3.99 (d, *J =* 7.0 Hz, 2H), 3.88 (d, *J =* 13.3 Hz, 1H), 3.76 (d, *J =* 13.3 Hz, 1H), 2.96 (d, *J =* 11.1 Hz, 1H), 2.85 (d, *J =* 11.2 Hz, 1H), 2.72 - 2.63 (m, 1H), 2.61 - 2.55 (m, 1H), 2.47 - 2.40 (m, 1H), 2.23 - 2.11 (m, 2H), 1.81 - 1.60 (m, 4H), 0.88 - 0.83 (m, 1H), 0.64 - 0.57 (m, 2H), 0.40 - 0.35 (m, 2H).

### Example 6: Preparation of (S)-2-((4-(6-((4-cyano-2-fluorobenzyl)oxy)pyridin-2-yl)piperidin-1-yl)methyl)-4-(furan-2-ylmethoxy)-1-(oxetan-2-ylmethyl)-1H-benzo[d]imidazole-6-carboxylic acid (Compound 6)

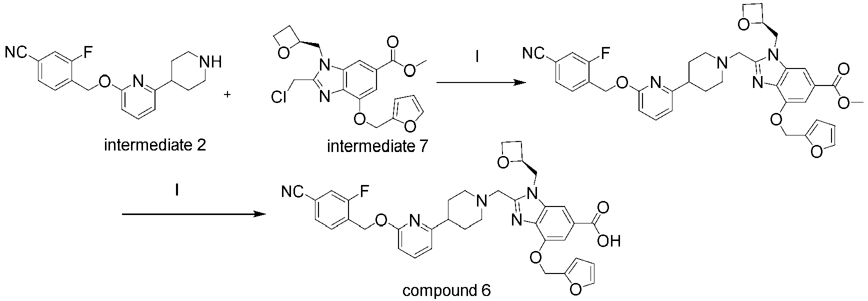

Methyl (S)-2-(chloromethyl)-4-(furan-2-ylmethoxy)-1-(oxetan-2-ylmethyl)-1H-benzo[d]imidazole-6-carboxylate (Intermediate 7) was prepared according to the route shown in route A. The specific synthesis method was the same as that of Intermediate 1. LCMS(ESI): m/z = 390.1 (M+H)⁺.

(S)-2-((4-(6-((4-cyano-2-fluorobenzyl)oxy)pyridin-2-yl)piperidin-1-yl)methyl)-4-(furan-2-ylmethoxy)-1-(oxetan-2-ylmethyl)-1H-benzo[d]imidazole-6-carboxylic acid (Compound 6) was prepared according to the route shown in route B. The specific synthesis method was the same as Example 1, except that Intermediate 1 was replaced by Intermediate 7. LCMS(ESI): m/z = 651.3 (M+H)⁺.

### Example 7: Preparation of (S)-4-(benzyloxy)-2-((4-(6-((4-cyano-2-fluorobenzyl)oxy)pyridin-2-yl)piperidin-1-yl)methyl)-1-(oxetan-2-ylmethyl)-1H-benzo[d]imidazole-6-carboxylic acid (Compound 7)

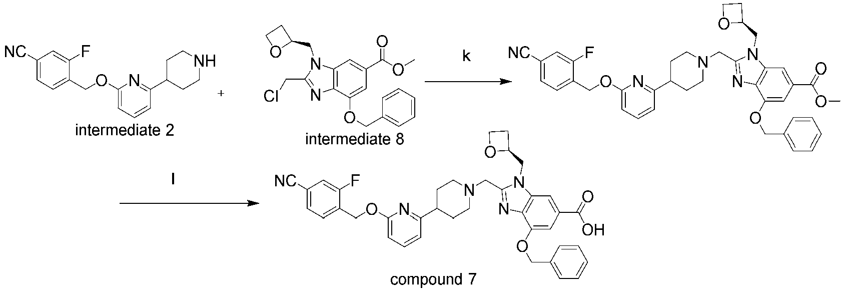

Methyl (S)-4-(benzyloxy)-2-(chloromethyl)-1-(oxetan-2-ylmethyl)-1H-benzo[d]imidazole-6-carboxylate (Intermediate 8) was prepared according to the route shown in route A. For the specific synthesis method, refer to Intermediate 1. LCMS(ESI): m/z = 400.1 (M+H)⁺.

(S)-4-(Benzyloxy)-2-((4-(6-((4-cyano-2-fluorobenzyl)oxy)pyridin-2-yl)piperidin-1-yl)methyl)-1-(oxetan-2-ylmethyl)-1H-benzo[d]imidazole-6-carboxylic acid (Compound 7) was prepared according to the route shown in route B. The specific synthesis method was the same as Example 1, except that Intermediate 1 was replaced by Intermediate 8. LCMS(ESI): m/z = 661.3 (M+H)⁺. ¹H NMR (600 MHz, DMSO) δ 7.89 (d, *J* = 9.9 Hz, 1H), 7.80 (s, 1H), 7.72 - 7.67 (m, 2H), 7.64 (t, *J =* 7.7 Hz, 1H), 7.54 - 7.50 (m, 2H), 7.44 - 7.37 (m, 3H), 7.37 - 7.32 (m, 1H), 6.89 (d, *J* = 7.3 Hz, 1H), 6.72 (d, *J =* 8.2 Hz, 1H), 5.47 (s, 2H), 5.31 (s, 2H), 5.13 - 5.06 (m, 1H), 4.76 - 4.68 (m, 1H), 4.65 - 4.58 (m, 1H), 4.51 - 4.44 (m, 1H), 4.40 - 4.33 (m, 1H), 3.89 (d, *J =* 13.4 Hz, 1H), 3.75 (d, *J =* 13.3 Hz, 1H), 2.95 (d, *J =* 11.1 Hz, 1H), 2.84 (d, *J =* 11.2 Hz, 1H), 2.75 - 2.64 (m, 1H), 2.62 - 2.54 (m, 1H), 2.47 - 2.38 (m, 1H), 2.23 - 2.11 (m, 2H), 1.80 - 1.59 (m, 4H).

### Example 8: Preparation of (S)-2-((4-(6-((4-cyano-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 8)

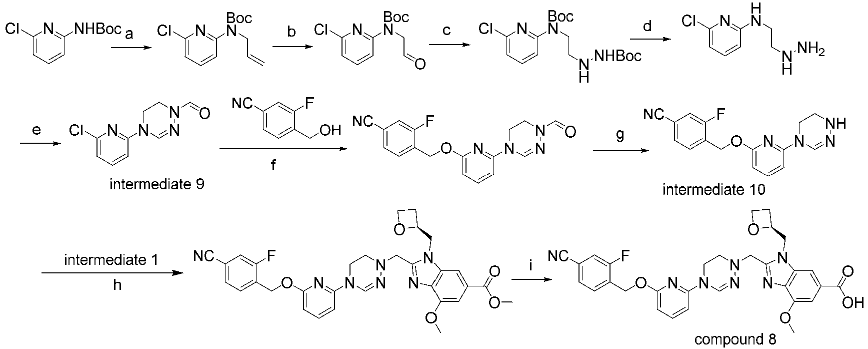

4-(6-Chloropyridin-2-yl)-5,6-dihydro-1,2,4-triazine-1(4H)-carbaldehyde (Intermediate 9) was prepared according to the route shown in route C.

Step a: 2-Boc-amino-6-chloropyridine (2 g, 8.7 mmol) was dissolved in 30 ml of N,N-dimethylformamide, and cesium carbonate (5.7 g, 17.4 mmol) and 3-bromopropylene (2.1 g, 17.4 mmol) were added. The mixture was reacted at 70 °C for 4 hours. After the reaction was completed, the mixture was extracted with ethyl acetate three times and washed with saturated brine three times. The organic phase was dried over anhydrous magnesium sulfate and then filtered. The filtrate was concentrated under reduced pressure. The crude product was separated by column chromatography to obtain 2.2 g of tert-butyl allyl (6-chloropyridin-2-yl) carbamate. LCMS (ESI): m/z = 268.1 (M+H)⁺.

Step b: Tert-butyl allyl (6-chloropyridin-2-yl) carbamate (2.2 g, 8.2 mmol) was dissolved in a mixed solvent of 20 ml of tetrahydrofuran and 20 ml of water, potassium osmate (VI) dihydrate (26 mg, 0.08 mmol) and sodium periodate (5.3 g, 24.6 mmol) were added, and the mixture was stirred at room temperature for 1 hour. After the reaction was completed, 100 ml of saturated sodium thiosulfate aqueous solution was poured into the mixture, which was then stirred for 10 minutes, extracted three times with ethyl acetate. The organic phase was dried over anhydrous magnesium sulfate and filtered, the filtrate was concentrated under reduced pressure, and the crude product was separated by column chromatography to obtain 1.8 g of tert-butyl (6-chloropyridin-2-yl) (2-oxoethyl) carbamate. LCMS (ESI): m/z = 270.1 (M+H)⁺.

Step c: Tert-butyl (6-chloropyridin-2-yl) (2-oxoethyl) carbamate (1.8 g, 6.7 mmol) was dissolved in 30 ml of ultra-dry 1,2-dichloroethane, tert-butyl carbazate (1.3 g, 10 mmol) was added, stirred at room temperature for 1 hour, then sodium triacetoxyborohydride (4.3 g, 20.1 mmol) and sodium cyanoborohydride (1.3 g, 20.1 mmol) were added and reacted at room temperature for 1 hour. After the reaction was completed, the reaction was quenched with a saturated aqueous sodium bicarbonate solution. The mixture was extracted three times with dichloromethane, the organic phase was dried over anhydrous magnesium sulfate and then filtered, the filtrate was concentrated under reduced pressure, and the crude product was separated by column chromatography to obtain 1.9 g of tert-butyl 2-(2-((tert-butoxycarbonyl)(6-chloropyridin-2-yl)amino)ethyl)hydrazine-1-carboxylate. LCMS (ESI): m/z = 386.2 (M+H)⁺.

Step d: Tert-butyl 2-(2-((tert-butoxycarbonyl)(6-chloropyridin-2-yl)amino)ethyl)hydrazine-1-carboxylate (1.9 g, 4.9 mmol) was dissolved in 20 ml of dichloromethane, 4M hydrogen chloride-1,4-dioxane solution (6.1 mL, 24.5 mmol) was added dropwise, and the mixture was stirred at room temperature for 4 hours. After the reaction was completed, the reaction solution was concentrated under reduced pressure to obtain 911 mg of 6-chloro-N-(2-hydrazinoethyl)pyridin-2-amine hydrochloride. LCMS(ESI): m/z = 186.1 (M+H)⁺.

Step e: 6-Chloro-N-(2-hydrazinoethyl)pyridin-2-amine hydrochloride (911 mg, 4.9 mmol) was dissolved in 15 ml of acetic acid, 3 ml of trimethyl orthoformate was added, and the mixture was reacted at 100 °C for 3 hours under nitrogen protection. After the reaction was completed, the reaction solution was concentrated under reduced pressure and the crude product was separated by column chromatography to obtain 880 mg of 4-(6-chloropyridin-2-yl)-5,6-dihydro-1,2,4-triazine-1(4H)-carbaldehyde (Intermediate 9). LCMS(ESI): m/z = 224.1 (M+H)⁺.

(S)-2-((4-(6-((4-cyano-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 8) was prepared according to the route shown in route C.

Step f: 4-(6-chloropyridin-2-yl)-5,6-dihydro-1,2,4-triazine-1(4H)-carbaldehyde (100 mg, 0.45 mmol), 3-fluoro-4-(hydroxymethyl)benzonitrile (88 mg, 0.58 mmol), tris(dibenzylideneacetone)dipalladium (46 mg, 0.05 mmol), 2-dicyclohexylphosphine-2',6'-diisopropoxy-1,1'-biphenyl (47 mg, 0.1 mmol), cesium carbonate (293 mg, 0.9 mmol) and 8 ml of toluene were added into a pressure tube, protected by nitrogen, and reacted at 120 °C for 3 hours. After the reaction was completed, the reaction mixture was filtered and the filtrate was concentrated under reduced pressure. The crude product was separated by column chromatography to obtain 140 mg of 3-fluoro-4-((6-(1-formyl-5,6-dihydro-1,2,4-triazin-4(1H)-yl)pyridin-2-yl)oxy)methyl)benzonitrile. LCMS (ESI): m/z = 339.1 (M+H)⁺.

Step g: 3-Fluoro-4-((6-(1-formyl-5,6-dihydro-1,2,4-triazin-4(1H)-yl)pyridin-2-yl)oxy)methyl)benzonitrile (140 mg, 0.41 mmol) was dissolved in a mixed solvent of 4 ml of dichloromethane and 4 ml of methanol. 4 M hydrochloric acid (410 µL, 1.64 mmol) was added dropwise under ice bath, and the mixture was moved to room temperature and stirred overnight. After the reaction was completed, the pH was adjusted to neutral with 2M NaOH. The mixture was extracted three times with dichloromethane, the organic phase was dried over anhydrous magnesium sulfate and then filtered, the filtrate was concentrated under reduced pressure, and the crude product was separated by column chromatography to obtain 115 mg of 4-((6-(5,6-dihydro-1,2,4-triazin-4(1H)-yl)pyridin-2-yl)oxy)methyl)-3-fluorobenzonitrile (intermediate 10). LCMS(ESI): m/z = 311.1 (M+H)⁺.

Step h: Methyl (S)-2-(chloromethyl)-4-methoxy-1-(oxetan-2-ylmethyl)-1H-benzo[d]imidazole-6-carboxylate (Intermediate 1, 50 mg, 0.15 mmol), 4-((6-(5,6-dihydro-1,2,4-triazin-4(1H)-yl)pyridin-2-yl)oxy)methyl)-3-fluorobenzonitrile (Intermediate 10, 47 mg, 0.15 mmol), potassium carbonate (41 mg, 0.3 mmol) and a catalytic amount of potassium iodide were dissolved in 5 ml of acetonitrile and reacted at 80 °C for 2 hours. After the reaction was completed, the reaction mixture was filtered and the filtrate was concentrated under reduced pressure. The mixture was separated by preparative thin layer chromatography to obtain 68 mg of (S)-2-((4-(6-((4-cyano-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylate. LCMS (ESI): m/z = 599.2 (M+H)⁺.

Step i: Methyl (S)-2-((4-(6-((4-cyano-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylate (68 mg, 0.11 mmol) was dissolved in 3 ml of 1,4-dioxane, 2 M NaOH (300 µL, 0.6 mmol) was added and the reaction was carried out at 40 °C overnight. After the reaction was completed, the pH was adjusted to 5-6 with 2M hydrochloric acid, and preparative liquid separation was performed to obtain 50 mg of (S)-2-((4-(6-((4-cyano-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 8). LCMS (ESI): m/z = 585.2 (M+H)⁺. ¹H NMR (500 MHz, DMSO) δ 7.94 - 7.88 (m, 2H), 7.74 - 7.62 (m, 4H), 7.33 (d, *J* = 1.0 Hz, 1H), 6.54 (d, *J* = 8.1 Hz, 1H), 6.45 (d, *J* = 8.0 Hz, 1H), 5.46 (s, 2H), 5.09 - 5.00 (m, 1H), 4.71 - 4.63 (m, 1H), 4.60 - 4.53 (m, 1H), 4.51 - 4.39 (m, 2H), 4.36 - 4.28 (m, 2H), 3.90 (s, 3H), 3.73 (t, *J* = 5.3 Hz, 2H), 3.05 (t, *J =* 5.3 Hz, 2H), 2.71 - 2.61 (m, 1H), 2.44 - 2.36 (m, 1H).

### Example 9: Preparation of (S)-2-((4-(6-((4-cyano-2-fluorobenzyl)oxy)pyridin-2-yl)-5,6-dihydro-1,2,4-triazin-1(4H)-yl)methyl)-4-ethoxy-1-(oxetan-2-ylmethyl)-1H-benzo[d]imidazole-6-carboxylic acid (Compound 9)

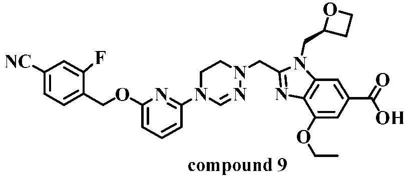

(S)-2-((4-(6-((4-cyano-2-fluorobenzyl)oxy)pyridin-2-yl)-5,6-dihydro-1,2,4-triazin-1(4H)-yl)methyl)-4-ethoxy-1-(oxetan-2-ylmethyl)-1H-benzo[d]imidazole-6-carboxylic acid (Compound 9) was prepared according to the route shown in route C. The specific synthesis method was the same as Example 8, except that Intermediate 1 was replaced by Intermediate 3. LCMS (ESI): m/z = 599.2 (M+H)⁺.1H NMR (500 MHz, DMSO) δ 7.89 - 7.84 (m, 2H), 7.71 - 7.60 (m, 4H), 7.31 (s, 1H), 6.51 (d, *J =* 8.0 Hz, 1H), 6.42 (d, *J =* 8.0 Hz, 1H), 5.44 (s, 2H), 5.06 - 4.98 (m, 1H), 4.69 - 4.61 (m, 1H), 4.58 - 4.51 (m, 1H), 4.48 - 4.37 (m, 2H), 4.34 - 4.25 (m, 2H), 4.19 (q, *J =* 7.0 Hz, 2H), 3.71 (t, *J =* 5.2 Hz, 2H), 3.02 (t, *J =* 5.2 Hz, 2H), 2.68 - 2.58 (m, 1H), 2.41 - 2.31 (m, 1H), 1.39 (t, *J* = 7.0 Hz, 3H).

### Example 10: Preparation of (S)-2-((4-(6-((4-cyano-2-fluorobenzyl)oxy)pyridin-2-yl)-5,6-dihydro-1,2,4-triazin-1(4H)-yl)methyl)-1-(oxetan-2-ylmethyl)-4-propoxy-1H-benzo[d]imidazole-6-carboxylic acid (Compound 10)

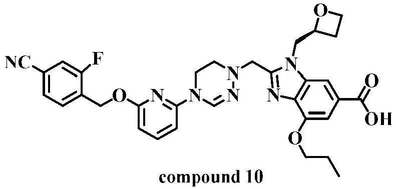

(S)-2-((4-(6-((4-cyano-2-fluorobenzyl)oxy)pyridin-2-yl)-5,6-dihydro-1,2,4-triazin-1(4H)-yl)methyl)-1-(oxetan-2-ylmethyl)-4-propoxy-1H-benzo[d]imidazole-6-carboxylic acid (Compound 10) was prepared according to the route shown in route C. The specific synthesis method was the same as Example 8, except that Intermediate 1 was replaced by Intermediate 4. LCMS (ESI): m/z = 613.2 (M+H)⁺.¹H NMR (500 MHz, DMSO) δ 7.86 (d, *J =* 9.6 Hz, 2H), 7.71 - 7.59 (m, 4H), 7.33 (d, *J* = 1.1 Hz, 1H), 6.51 (d, *J =* 8.0 Hz, 1H), 6.42 (d, *J* = 8.0 Hz, 1H), 5.43 (s, 2H), 5.06 - 4.98 (m, 1H), 4.70 - 4.62 (m, 1H), 4.59 - 4.51 (m, 1H), 4.50 - 4.37 (m, 2H), 4.34 - 4.26 (m, 2H), 4.10 (t, *J* = 6.7 Hz, 2H), 3.71 (t, *J* = 5.2 Hz, 2H), 3.02 (t, *J* = 5.2 Hz, 2H), 2.68 - 2.58 (m, 1H), 2.41 - 2.31 (m, 1H), 1.84 - 1.72 (m, 2H), 1.01 (t, *J =* 7.4 Hz, 3H).

### Example 11: Preparation of (S)-2-((4-(6-((4-cyano-2-fluorobenzyl)oxy)pyridin-2-yl)-5,6-dihydro-1,2,4-triazin-1(4H)-yl)methyl)-4-(difluoromethoxy)-1-(oxetan-2-ylmethyl)-1H-benzo[d]imidazole-6-carboxylic acid (Compound 11)

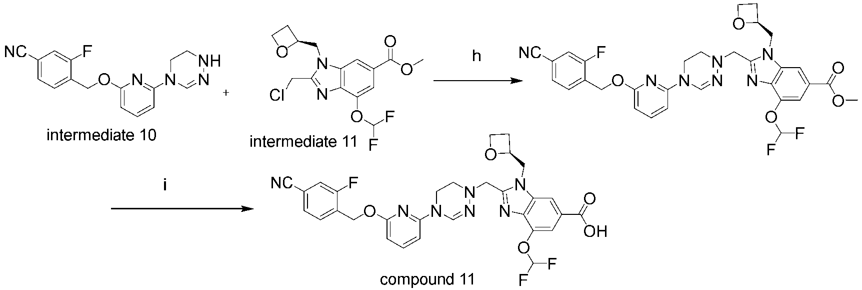

Methyl (S)-2-(Chloromethyl)-4-(difluoromethoxy)-1-(oxetan-2-ylmethyl)-1H-benzo[d]imidazole-6-carboxylate (Intermediate 11) was prepared according to the route shown in route A. The specific synthesis method is the same as that of Intermediate 1. LCMS (ESI): m/z = 360.1 (M+H)⁺.

(S)-2-((4-(6-((4-cyano-2-fluorobenzyl)oxy)pyridin-2-yl)-5,6-dihydro-1,2,4-triazin-1(4H)-yl)methyl)-4-(difluoromethoxy)-1-(oxetan-2-ylmethyl)-1H-benzo[d]imidazole-6-carboxylic acid (Compound 11) was prepared according to the route shown in route C. The specific synthesis method was the same as Example 8, except that Intermediate 1 was replaced by Intermediate 11. LCMS (ESI): m/z = 621.2 (M+H)⁺.¹H NMR (500 MHz, DMSO) δ 7.99 (d, *J =* 1.2 Hz, 1H), 7.93 - 7.87 (m, 2H), 7.74 - 7.66 (m, 3H), 7.66 - 7.42 (m, 2H), 6.55 (d, *J =* 8.0 Hz, 1H), 6.45 (d, *J =* 8.0 Hz, 1H), 5.46 (s, 2H), 5.10 - 5.01 (m, 1H), 4.83 - 4.73 (m, 1H), 4.69 - 4.61 (m, 1H), 4.52 - 4.44 (m, 2H), 4.39 - 4.31 (m, 2H), 3.75 (t, *J =* 5.1 Hz, 2H), 3.10 (t, *J =* 5.5 Hz, 2H), 2.75 - 2.63 (m, 1H), 2.45 - 2.36 (m, 1H).

### Example 12: Preparation of (S)-2-((4-(6-((4-cyano-2-fluorobenzyl)oxy)pyridin-2-yl)-5,6-dihydro-1,2,4-triazin-1(4H)-yl)methyl)-4-(methoxy-d3)-1-(oxetan-2-ylmethyl)-1H-benzo[d]imidazole-6-carboxylic acid (Compound 12)

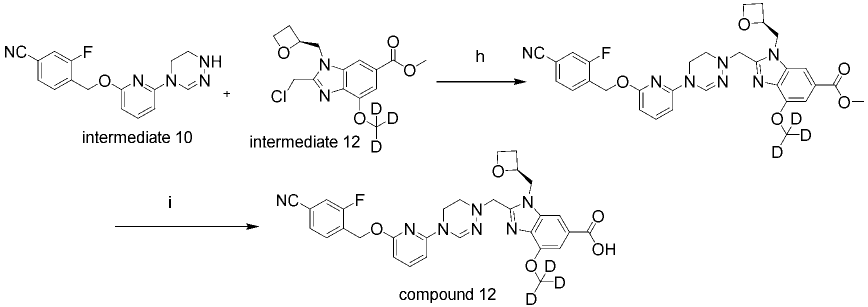

Methyl (S)-2-(Chloromethyl)-4-(methoxy-d3)-1-(oxetan-2-ylmethyl)-1H-benzo[d]imidazole-6-carboxylater (Intermediate 12) was prepared according to the route shown in route A. The specific synthesis method is the same as that of Intermediate 1. LCMS (ESI): m/z = 327.1 (M+H)⁺.

(S)-2-((4-(6-((4-cyano-2-fluorobenzyl)oxy)pyridin-2-yl)-5,6-dihydro-1,2,4-triazin-1(4H)-yl)methyl)-4-(methoxy-d3)-1-(oxetan-2-ylmethyl)-1H-benzo[d]imidazole-6-carboxylic acid (Compound 12) was prepared according to the route shown in route C. The specific synthesis method was the same as Example 8, except that Intermediate 1 was replaced by Intermediate 12. LCMS (ESI): m/z = 588.2 (M+H)⁺. ¹H NMR (600 MHz, DMSO) δ 7.92 - 7.87 (m, 2H), 7.74 (s, 1H), 7.73 - 7.63 (m, 3H), 7.32 (s, 1H), 6.54 (d, *J =* 8.0 Hz, 1H), 6.45 (d, *J =* 8.0 Hz, 1H), 5.46 (s, 2H), 5.08 - 5.01 (m, 1H), 4.74 - 4.68 (m, 1H), 4.63 - 4.57 (m, 1H), 4.50 - 4.40 (m, 2H), 4.39 - 4.25 (m, 2H), 3.73 (t, *J =* 5.2 Hz, 2H), 3.06 (t, *J =* 5.2 Hz, 2H), 2.70 - 2.61 (m, 1H), 2.42 - 2.33 (m, 1H).

### Example 13: Preparation of (S)-2-((4-(6-((4-cyano-2-fluorobenzyl)oxy)pyridin-2-yl)-5,6-dihydro-1,2,4-triazin-1(4H)-yl)methyl)-4-cyclopropyloxy-1-(oxetan-2-ylmethyl)-1H-benzo[d]imidazole-6-carboxylic acid (Compound 13)

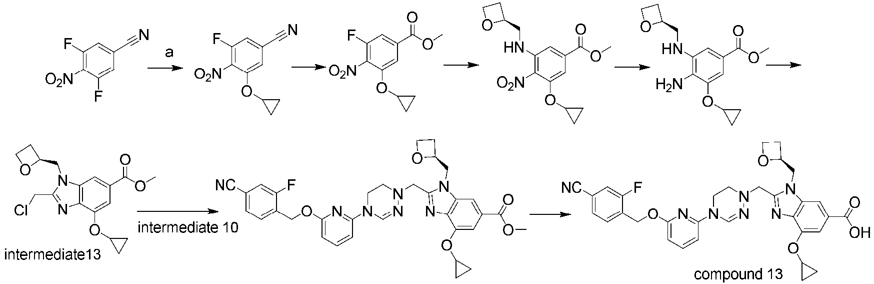

Methyl (S)-2-(chloromethyl)-4-cyclopropoxy-1-(oxetan-2-ylmethyl)-1H-benzo[d]imidazole-6-carboxylate (Intermediate 13) was prepared according to the route shown in route A:
Step a: Cyclopropanol (377 mg, 6.5 mmol) was dissolved in 20 ml of N,N-dimethylformamide, followed by the addition of potassium hydroxide (5.35 g, 38.8 mmol) and 3,5-difluoro-4-nitrobenzonitrile (1 g, 5.4 mmol), and the reaction was carried out at 80 °C overnight. After the reaction was completed, the pH was adjusted to 7 with 2M hydrochloric acid. The mixture was extracted three times with ethyl acetate, washed three times with saturated brine, the organic phase was dried over anhydrous magnesium sulfate and then filtered, the filtrate was concentrated under reduced pressure, and the crude product was separated by column chromatography to obtain 720 mg of 3-cyclopropyloxy-5-fluoro-4-nitrobenzonitrile.

The subsequent reaction steps were carried out according to the preparation of Intermediate 1 to obtain methyl (S)-2-(chloromethyl)-4-cyclopropoxy-1-(oxetan-2-ylmethyl)-1H-benzo[d]imidazole-6-carboxylate (Intermediate 13). LCMS (ESI): m/z = 350.1 (M+H)⁺.

(S)-2-((4-(6-((4-cyano-2-fluorobenzyl)oxy)pyridin-2-yl)-5,6-dihydro-1,2,4-triazin-1(4H)-yl)methyl)-4-cyclopropyloxy-1-(oxetan-2-ylmethyl)-1H-benzo[d]imidazole-6-carboxylic acid (Compound 13) was prepared according to the route shown in route C. The specific synthesis method was the same as Example 8, except that Intermediate 1 was replaced by Intermediate 13. LCMS (ESI): m/z = 611.2 (M+H)⁺.¹H NMR (500 MHz, DMSO) δ 7.91 (d, *J =* 9.7 Hz, 2H), 7.74 - 7.64 (m, 4H), 7.62 (d, *J =* 1.1 Hz, 1H), 6.54 (d, *J =* 8.0 Hz, 1H), 6.45 (d, *J =* 8.0 Hz, 1H), 5.46 (s, 2H), 5.08 - 5.00 (m, 1H), 4.72 - 4.64 (m, 1H), 4.60 - 4.53 (m, 1H), 4.51 - 4.38 (m, 2H), 4.36 - 4.28 (m, 2H), 4.02 - 3.95 (m, 1H), 3.73 (t, *J =* 5.3 Hz, 2H), 3.03 (t, *J =* 5.3 Hz, 2H), 2.71 - 2.61 (m, 1H), 2.44 - 2.33 (m, 1H), 0.84 - 0.78 (m, 2H), 0.75 - 0.70 (m, 2H).

### Example 14: Preparation of (S)-2-((4-(6-((4-cyano-2-fluorobenzyl)oxy)pyridin-2-yl)-5,6-dihydro-1,2,4-triazin-1(4H)-yl)methyl)-1-(oxetan-2-ylmethyl)-4-(pyridin-3-yloxy)-1H-benzo[d]imidazole-6-carboxylic acid (Compound 14)

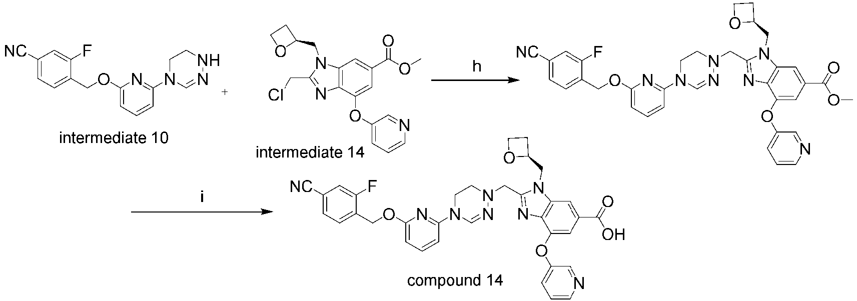

Methyl (S)-2-(chloromethyl)-1-(oxetan-2-ylmethyl)-4-(pyridin-3-yloxy)-1H-benzo[d]imidazole-6-carboxylate (Intermediate 14) was prepared according to the route shown in route A. The specific synthesis method is the same as that of Intermediate 13. LCMS (ESI): m/z = 387.1 (M+H)⁺.

(S)-2-((4-(6-((4-cyano-2-fluorobenzyl)oxy)pyridin-2-yl)-5,6-dihydro-1,2,4-triazin-1(4H)-yl)methyl)-1-(oxetan-2-ylmethyl)-4-(pyridin-3-yloxy)-1H-benzo[d]imidazole-6-carboxylic acid (Compound 14) was prepared according to the route shown in route C. The specific synthesis method was the same as Example 8, except that Intermediate 1 was replaced by Intermediate 14. LCMS (ESI): m/z = 648.2 (M+H)⁺.¹H NMR (600 MHz, DMSO) δ 8.40 (s, 1H), 8.35 - 8.30 (m, 1H), 8.01 (s, 1H), 7.92 - 7.87 (m, 2H), 7.73 - 7.63 (m, 3H), 7.40 - 7.36 (m, 2H), 7.34 (s, 1H), 6.55 (d, *J* = 8.0 Hz, 1H), 6.45 (d, *J* = 7.9 Hz, 1H), 5.46 (s, 2H), 5.11 - 5.04 (m, 1H), 4.83 - 4.76 (m, 1H), 4.69 - 4.63 (m, 1H), 4.52 - 4.42 (m, 2H), 4.40 - 4.29 (m, 2H), 3.73 (t, *J =* 5.1 Hz, 2H), 3.07 - 2.98 (m, 2H), 2.75 - 2.65 (m, 1H), 2.46 - 2.39 (m, 1H).

### Example 15: Preparation of (S)-2-((4-(6-((4-cyano-2,3-dihydrobenzofuran-7-yl)methoxy)pyridin-2-yl)piperidin-1-yl)methyl)-4-methoxy-1-(oxetan-2-ylmethyl)-1H-benzo[d]imidazole-6-carboxylic acid (Compound 15)

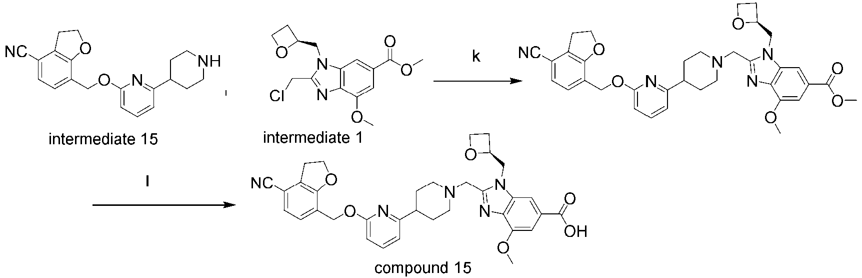

7-(((6-(piperidin-4-yl)pyridin-2-yl)oxy)methyl)-2,3-dihydrobenzofuran-4-carbonitrile (Intermediate 15) was prepared according to the route shown in route B. The specific synthesis method is the same as that of Intermediate 2. LCMS (ESI): m/z = 335.2 (M+H)⁺.

(S)-2-((4-(6-((4-cyano-2,3-dihydrobenzofuran-7-yl)methoxy)pyridin-2-yl)piperidin-1-yl)methyl)-4-methoxy-1-(oxetan-2-ylmethyl)-1H-benzo[d]imidazole-6-carboxylic acid (Compound 15) was prepared according to the route shown in route B. The specific synthesis method was the same as Example 1, except that Intermediate 2 was replaced by Intermediate 15. LCMS (ESI): m/z = 609.3 (M+H)⁺.¹H NMR (600 MHz, DMSO) δ 7.89 (s, 1H), 7.62 (t, *J =* 7.8 Hz, 1H), 7.35 (d, *J =* 7.9 Hz, 1H), 7.27 - 7.23 (m, 2H), 6.86 (d, *J =* 7.3 Hz, 1H), 6.68 (d, *J* = 8.2 Hz, 1H), 5.31 (s, 2H), 5.12 - 5.05 (m, 1H), 4.76 - 4.72 (m, 1H), 4.70 (t, *J =* 8.8 Hz, 2H), 4.65 - 4.59 (m, 1H), 4.50 - 4.43 (m, 1H), 4.38 - 4.32 (m, 1H), 3.96 (s, 3H), 3.91 (d, *J =* 13.5 Hz, 1H), 3.75 (d, *J =* 13.5 Hz, 1H), 3.38 (t, *J =* 8.8 Hz, 2H), 2.99 (d, *J =* 11.2 Hz, 1H), 2.85 (d, *J =* 11.3 Hz, 1H), 2.75 - 2.65 (m, 1H), 2.62 - 2.52 (m, 1H), 2.47 - 2.39 (m, 1H), 2.26 - 2.13 (m, 2H), 1.80 - 1.62 (m, 4H).

### Example 16: Preparation of (S)-2-((4-(6-((4-chloro-2,3-dihydrobenzofuran-7-yl)methoxy)pyridin-2-yl)piperidin-1-yl)methyl)-4-methoxy-1-(oxetan-2-ylmethyl)-1H-benzo[d]imidazole-6-carboxylic acid (Compound 16)

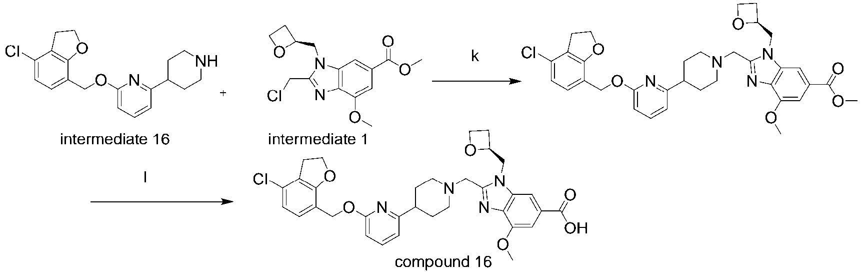

2-((4-chloro-2,3-dihydrobenzofuran-7-yl)methoxy)-6-(piperidin-4-yl)pyridine (Intermediate 16) was prepared according to the route shown in route B. The specific synthesis method was the same as that of Intermediate 2. LCMS (ESI): m/z = 344.1 (M+H)⁺.

(S)-2-((4-(6-((4-chloro-2,3-dihydrobenzofuran-7-yl)methoxy)pyridin-2-yl)piperidin-1-yl)methyl)-4-methoxy-1-(oxetan-2-ylmethyl)-1H-benzo[d]imidazole-6-carboxylic acid (Compound 16) was prepared according to the route shown in route B. The specific synthesis method was the same as Example 1, except that Intermediate 2 was replaced by Intermediate 16. LCMS (ESI): m/z = 618.2 (M+H)⁺.¹H NMR (600 MHz, DMSO) δ 7.95 (s, 1H), 7.66 (t, *J =* 7.7 Hz, 1H), 7.32 (s, 1H), 7.29 (d, *J =* 8.2 Hz, 1H), 6.93 (d, *J =* 8.2 Hz, 1H), 6.91 (d, *J* = 7.3 Hz, 1H), 6.69 (d, *J =* 8.2 Hz, 1H), 5.29 (s, 2H), 5.19 - 5.12 (m, 1H), 4.84 - 4.77 (m, 1H), 4.72 (t, *J =* 8.7 Hz, 2H), 4.72 - 4.66 (m, 1H), 4.55 - 4.49 (m, 1H), 4.45 - 4.38 (m, 1H), 4.02 (s, 3H), 3.98 (d, *J =* 13.5 Hz, 1H), 3.82 (d, *J* = 13.4 Hz, 1H), 3.28 (t, *J =* 8.7 Hz, 2H), 3.06 (d, *J =* 11.2 Hz, 1H), 2.92 (d, *J =* 11.3 Hz, 1H), 2.81 - 2.71 (m, 1H), 2.70 - 2.62 (m, 1H), 2.53 - 2.44 (m, 1H), 2.32 - 2.19 (m, 2H), 1.89 - 1.72 (m, 4H).

### Example 17: Preparation of (S)-2-((4-(6-((4-chloro-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 17)

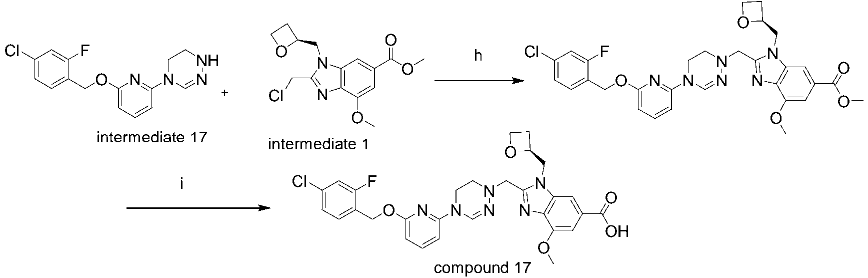

44-(6-((4-chloro-2-fluorobenzyl)oxy)pyridin-2-yl)-1,4,5,6-tetrahydro-1,2,4-triazine (Intermediate 17) was prepared according to the route shown in route C. The specific synthesis method was the same as that of Intermediate 10. LCMS (ESI): m/z = 320.1 (M+H)⁺.

(S)-2-((4-(6-((4-chloro-2-fluorobenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 17) was prepared according to the route shown in route C. The specific synthesis method was the same as Example 8, except that Intermediate 10 was replaced by Intermediate 17. LCMS (ESI): m/z = 594.2 (M+H)⁺.¹H NMR (600 MHz, DMSO) δ 7.93 (s, 1H), 7.81 (s, 1H), 7.64 (t, *J =* 8.0 Hz, 1H), 7.53 (t, *J =* 8.1 Hz, 1H), 7.47 (dd, *J =* 10.0, 2.1 Hz, 1H), 7.32 - 7.27 (m, 2H), 6.53 (d, *J =* 8.0 Hz, 1H), 6.40 (d, *J =* 8.0 Hz, 1H), 5.35 (s, 2H), 5.08 - 5.01 (m, 1H), 4.78 - 4.71 (m, 1H), 4.66 - 4.60 (m, 1H), 4.50 - 4.43 (m, 2H), 4.38 - 4.28 (m, 2H), 3.94 (s, 3H), 3.76 (t, *J* = 5.2 Hz, 2H), 3.13 - 3.03 (m, 2H), 2.71 - 2.62 (m, 1H), 2.42 - 2.33 (m, 1H).

### Example 18: Preparation of (S)-2-((4-(6-((4-cyano-2-methoxybenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 18)

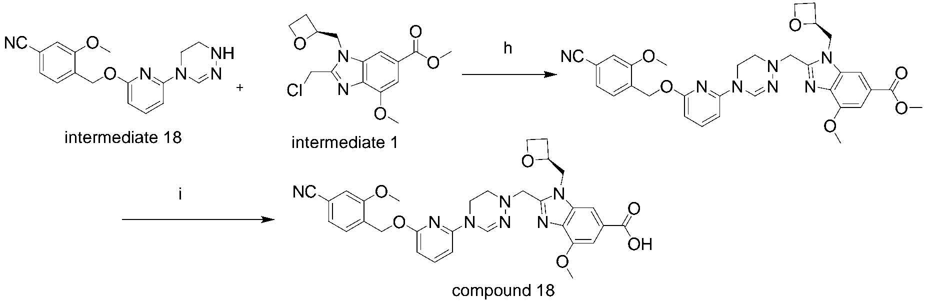

4-((6-(5,6-dihydro-1,2,4-triazine-4(1H)-yl)pyridin-2-yl)oxy)methyl)-3-methoxybenzonitrile (Intermediate 18) was prepared according to the route shown in route C. The specific synthesis method was the same as that of Intermediate 10. LCMS (ESI): m/z = 323.1 (M+H)⁺.

(S)-2-((4-(6-((4-cyano-2-methoxybenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 18) was prepared according to the route shown in route C. The specific synthesis method was the same as Example 8, except that Intermediate 10 was replaced by Intermediate 18. LCMS (ESI): m/z = 597.2 (M+H)⁺.¹H NMR (600 MHz, DMSO) δ 7.85 (s, 1H), 7.75 (s, 1H), 7.65 (t, *J* = 8.0 Hz, 1H), 7.51 (s, 1H), 7.49 (d, *J =* 7.7 Hz, 1H), 7.41 (dd, *J* = 7.8, 1.5 Hz, 1H), 7.32 (s, 1H), 6.50 (d, *J* = 8.0 Hz, 1H), 6.45 (d, *J* = 8.0 Hz, 1H), 5.35 (s, 2H), 5.08 - 5.00 (m, 1H), 4.76 - 4.69 (m, 1H), 4.63 - 4.57 (m, 1H), 4.50 - 4.40 (m, 2H), 4.36 - 4.28 (m, 2H), 3.93 (s, 3H), 3.89 (s, 3H), 3.71 (t, *J =* 5.2 Hz, 2H), 3.10 - 3.00 (m, 2H), 2.70 - 2.61 (m, 1H), 2.42 - 2.33 (m, 1H).

### Example 19: Preparation of (S)-2-((4-(6-((4-chloro-2-fluorobenzyl)oxy)pyridin-2-yl)piperidin-1-yl)methyl)-4-methoxy-1-(oxetan-2-ylmethyl)-1H-benzo[d]imidazole-6-carboxylic acid (Compound 19)

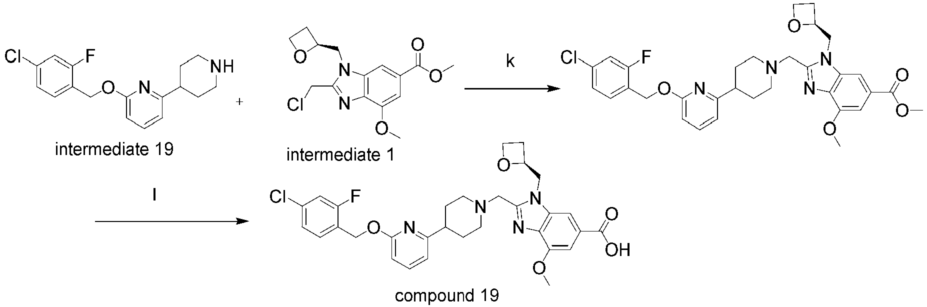

2-((4-chloro-2-fluorobenzyl)oxy)-6-(piperidin-4-yl)pyridine (Intermediate 19) was prepared according to the route shown in route B. The specific synthesis method is the same as that of Intermediate 2. LCMS (ESI): m/z = 320.1 (M+H)⁺.

(S)-2-((4-(6-((4-chloro-2-fluorobenzyl)oxy)pyridin-2-yl)piperidin-1-yl)methyl)-4-methoxy-1-(oxetan-2-ylmethyl)-1H-benzo[d]imidazole-6-carboxylic acid (Compound 19) was prepared according to the route shown in route B. The specific synthesis method was the same as Example 1, except that Intermediate 2 was replaced by Intermediate 19. LCMS (ESI): m/z = 594.2 (M+H)⁺.¹H NMR (500 MHz, DMSO) δ 7.73 (d, *J =* 1.1 Hz, 1H), 7.63 (dd, *J =* 8.2, 7.3 Hz, 1H), 7.57 (t, *J =* 8.2 Hz, 1H), 7.47 (dd, *J =* 10.0, 2.1 Hz, 1H), 7.32 (d, *J =* 1.1 Hz, 1H), 7.30 (dd, *J* = 8.2, 2.1 Hz, 1H), 6.88 (d, *J =* 7.3 Hz, 1H), 6.67 (d, *J =* 8.2 Hz, 1H), 5.38 (s, 2H), 5.15 - 5.06 (m, 1H), 4.73 - 4.65 (m, 1H), 4.65 - 4.52 (m, 1H), 4.51 - 4.43 (m, 1H), 4.40 - 4.33 (m, 1H), 3.91 (s, 3H), 3.88 (d, *J =* 13.3 Hz, 1H), 3.74 (d, *J =* 13.3 Hz, 1H), 2.98 (d, *J =* 11.1 Hz, 1H), 2.86 (d, *J =* 11.2 Hz, 1H), 2.72 - 2.64 (m, 1H), 2.64 - 2.56 (m, 1H), 2.49 - 2.39 (m, 1H), 2.25 - 2.10 (m, 2H), 1.86 - 1.63 (m, 4H).

### Example 20: Preparation of (S)-2-((4-(6-((4-chloro-2-methoxybenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 20)

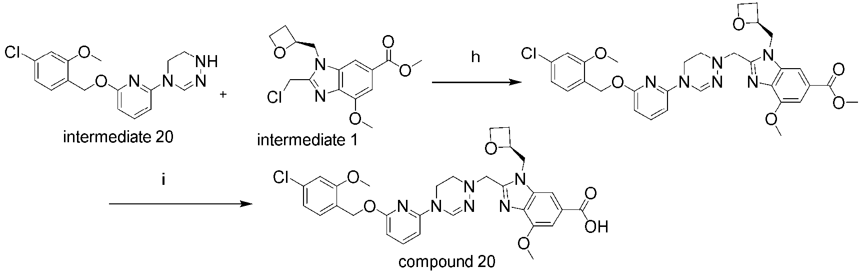

4-(6-((4-chloro-2-methoxybenzyl)oxy)pyridin-2-yl)-1,4,5,6-tetrahydro-1,2,4-triazine (Intermediate 20) was prepared according to the route shown in route C. The specific synthesis method was the same as that of Intermediate 10. LCMS (ESI): m/z = 332.1 (M+H)⁺.

(S)-2-((4-(6-((4-chloro-2-methoxybenzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 20)) was prepared according to the route shown in route C. The specific synthesis method was the same as Example 8, except that Intermediate 10 was replaced by Intermediate 20. LCMS (ESI): m/z = 606.2 (M+H)⁺.¹H NMR (600 MHz, DMSO) δ 7.91 (s, 1H), 7.78 (s, 1H), 7.63 (t, *J* = 8.0 Hz, 1H), 7.34 (d, *J* = 8.1 Hz, 1H), 7.31 (s, 1H), 7.10 (d, *J* = 2.0 Hz, 1H), 6.99 (dd, *J =* 8.1, 2.0 Hz, 1H), 6.49 (d, *J* = 8.0 Hz, 1H), 6.40 (d, *J* = 8.0 Hz, 1H), 5.26 (s, 2H), 5.08 - 5.01 (m, 1H), 4.77 - 4.71 (m, 1H), 4.65 - 4.59 (m, 1H), 4.50 - 4.42 (m, 2H), 4.37 - 4.28 (m, 2H), 3.94 (s, 3H), 3.83 (s, 3H), 3.74 (t, *J =* 5.2 Hz, 2H), 3.11 - 3.02 (m, 2H), 2.70 - 2.62 (m, 1H), 2.42 - 2.33 (m, 1H).

### Example 21: Preparation of (S)-2-((4-(6-((4-chloro-2-fluorobenzyl)oxy)pyridin-2-yl)-5,6-dihydro-1,2,4-triazin-1(4H)-yl)methyl)-4-ethoxy-1-(oxetan-2-ylmethyl)-1H-benzo[d]imidazole-6-carboxylic acid (Compound 21)

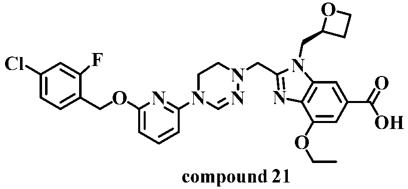

(S) -2-((4-(6-((4-chloro-2-fluorobenzyl)oxy)pyridin-2-yl)-5,6-dihydro-1,2,4-triazin-1(4H)-yl)methyl)-4-ethoxy-1-(oxetan-2-ylmethyl)-1H-benzo[d]imidazole-6-carboxylic acid (Compound 21)) was prepared according to the route shown in route C. The specific synthesis method was the same as Example 8, except that Intermediate 1 was replaced by Intermediate 3 and Intermediate 10 was replaced by Intermediate 17. LCMS (ESI): m/z = 608.2 (M+H)⁺.1H NMR (600 MHz, DMSO) δ 7.94 (s, 1H), 7.66 - 7.63 (m, 2H), 7.53 (t, *J* = 8.2 Hz, 1H), 7.47 (dd, *J =* 9.9, 2.1 Hz, 1H), 7.32 (s, 1H), 7.30 (dd, *J =* 8.2, 2.2 Hz, 1H), 6.52 (d, *J* = 8.0 Hz, 1H), 6.40 (d, *J =* 8.0 Hz, 1H), 5.36 (s, 2H), 5.08 - 5.01 (m, 1H), 4.71 - 4.65 (m, 1H), 4.60 - 4.54 (m, 1H), 4.49 - 4.41 (m, 2H), 4.36 - 4.28 (m, 2H), 4.21 (q, *J* = 7.0 Hz, 2H), 3.75 (t, *J =* 5.3 Hz, 2H), 3.08 - 3.02 (m, 2H), 2.70 - 2.61 (m, 1H), 2.43 - 2.34 (m, 1H), 1.41 (t, *J =* 7.0 Hz, 3H).

### Example 22: Preparation of (S)-2-((4-(6-((2-fluoro-4-(trifluoromethyl)benzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 22)

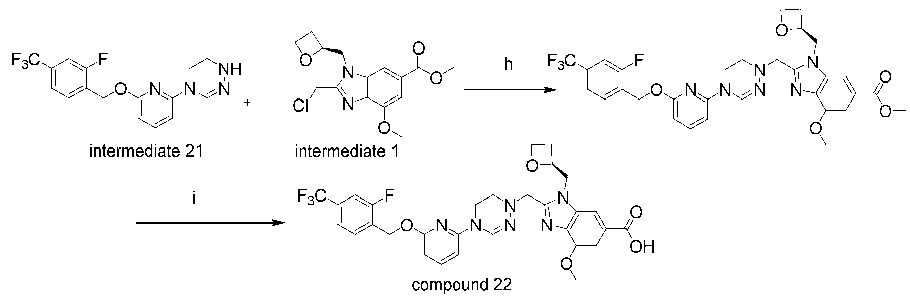

4-(6-((2-fluoro-4-(trifluoromethyl)benzyl)oxy)pyridin-2-yl)-1,4,5,6-tetrahydro-1,2,4-triazine (Intermediate 21) was prepared according to the route shown in route C. The specific synthesis method was the same as that of Intermediate 10. LCMS (ESI): m/z = 354.1 (M+H)⁺.

(S)-2-((4-(6-((2-fluoro-4-(trifluoromethyl)benzyl)oxy)pyridin-2-yl)-5,6-dihydro-1,2,4-triazin-1(4H)-yl)methyl)-4-methoxy-1-(oxetan-2-ylmethyl)-1H-benzo[d]imidazole-6-carboxylic acid (Compound 22)) was prepared according to the route shown in route C. The specific synthesis method was the same as Example 8, except that Intermediate 10 was replaced by Intermediate 21. LCMS (ESI): m/z = 628.2 (M+H)⁺.1H NMR (600 MHz, DMSO) δ 7.92 (s, 1H), 7.76 - 7.70 (m, 3H), 7.66 (t, *J =* 8.0 Hz, 1H), 7.61 (d, *J =* 8.0 Hz, 1H), 7.34 (s, 1H), 6.54 (d, *J =* 8.0 Hz, 1H), 6.44 (d, *J =* 8.0 Hz, 1H), 5.47 (s, 2H), 5.08 - 5.01 (m, 1H), 4.73 - 4.66 (m, 1H), 4.62 - 4.56 (m, 1H), 4.49 - 4.41 (m, 2H), 4.36 - 4.28 (m, 2H), 3.92 (s, 3H), 3.74 (t, *J =* 5.3 Hz, 2H), 3.10 - 3.02 (m, 2H), 2.70 - 2.61 (m, 1H), 2.42 - 2.33 (m, 1H).

### Example 23: Preparation of (S)-4-methoxy-2-((4-(6-((2-methoxy-4-(trifluoromethyl)benzyl)oxy)pyridin-2-yl)-5,6-dihydro-1,2,4-triazin-1(4H)-yl)methyl)-1-(oxetan-2-ylmethyl)-1H-benzo[d]imidazole-6-carboxylic acid (Compound 23)

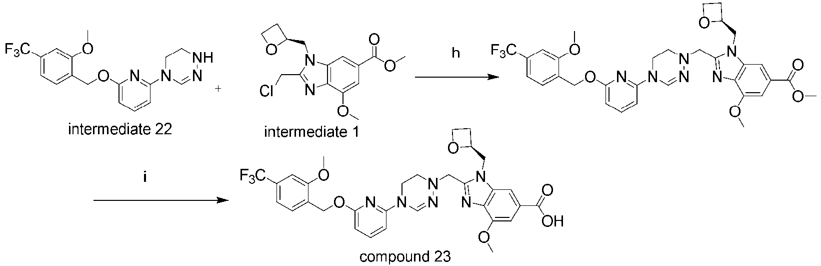

4-(6-((2-methoxy-4-(trifluoromethyl)benzyl)oxy)pyridin-2-yl)-1,4,5,6-tetrahydro-1,2,4-triazine (Intermediate 22) was prepared according to the route shown in route C. The specific synthesis method was the same as that of Intermediate 10. LCMS (ESI): m/z = 366.1 (M+H)⁺.

(S)-4-methoxy-2-((4-(6-((2-methoxy-4-(trifluoromethyl)benzyl)oxy)pyridin-2-yl)-5,6-dihydro-1,2,4-triazin-1(4H)-yl)methyl)-1-(oxetan-2-ylmethyl)-1H-benzo[d]imidazole-6-carboxylic acid (Compound 23)) was prepared according to the route shown in route C. The specific synthesis method was the same as Example 8, except that Intermediate 10 was replaced by Intermediate 22. LCMS (ESI): m/z = 640.2 (M+H)⁺.¹H NMR (600 MHz, DMSO) δ 7.88 (s, 1H), 7.77 (s, 1H), 7.65 (t, *J =* 8.0 Hz, 1H), 7.54 (d, *J =* 8.0 Hz, 1H), 7.34 - 7.28 (m, 3H), 6.51 (d, *J =* 8.0 Hz, 1H), 6.44 (d, *J* = 7.9 Hz, 1H), 5.37 (s, 2H), 5.08 - 5.01 (m, 1H), 4.76 - 4.70 (m, 1H), 4.64 - 4.58 (m, 1H), 4.49 - 4.42 (m, 2H), 4.36 - 4.28 (m, 2H), 3.93 (s, 3H), 3.91 (s, 3H), 3.73 (t, *J* = 5.2 Hz, 2H), 3.10 - 3.01 (m, 2H), 2.70 - 2.61 (m, 1H), 2.41 - 2.33 (m, 1H).

### Example 24: Preparation of (S)-4-ethoxy-2-((4-(6-((2-fluoro-4-(trifluoromethyl)benzyl)oxy)pyridin-2-yl)-5,6-dihydro-1,2,4-triazin-1(4H)-yl)methyl)-1-(oxetan-2-ylmethyl)-1H-benzo[d]imidazole-6-carboxylic acid (Compound 24)

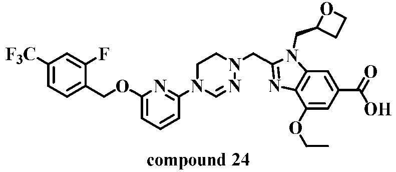

(S)-4-ethoxy-2-((4-(6-((2-fluoro-4-(trifluoromethyl)benzyl)oxy)pyridin-2-yl)-5,6-dihydro-1,2,4-triazin-1(4H)-yl)methyl)-1-(oxetan-2-ylmethyl)-1H-benzo[d]imidazole-6-carboxylic acid (Compound 24)) was prepared according to the route shown in route C. The specific synthesis method was the same as Example 8, except that Intermediate 1 was replaced by Intermediate 3, and Intermediate 10 was replaced by Intermediate 21. LCMS (ESI): m/z = 642.2 (M+H)⁺. ¹H NMR (600 MHz, DMSO) δ 7.91 (s, 1H), 7.88 (s, 1H), 7.72 (d, *J* = 9.1 Hz, 2H), 7.66 (t, *J* = 8.0 Hz, 1H), 7.60 (d, *J* = 8.0 Hz, 1H), 7.25 (s, 1H), 6.55 (d, *J =* 8.0 Hz, 1H), 6.45 (d, *J =* 8.0 Hz, 1H), 5.47 (s, 2H), 5.08 - 5.01 (m, 1H), 4.81 - 4.74 (m, 1H), 4.69 - 4.63 (m, 1H), 4.49 - 4.43 (m, 2H), 4.39 - 4.29 (m, 2H), 4.27 (q, *J* = 7.0 Hz, 2H), 3.75 (t, *J =* 5.2 Hz, 2H), 3.13 - 3.03 (m, 2H), 2.70 - 2.62 (m, 1H), 2.41 - 2.32 (m, 1H), 1.42 (t, *J* = 6.9 Hz, 3H).

### Example 25: Preparation of (S)-2-((4-(6-((4-chloro-2-fluorobenzyl)oxy)pyridin-2-yl)-5,6-dihydro-1,2,4-triazin-1(4H)-yl)methyl)-4-cyclopropyloxy-1-(oxetan-2-ylmethyl)-1H-benzo[d]imidazole-6-carboxylic acid (Compound 25)

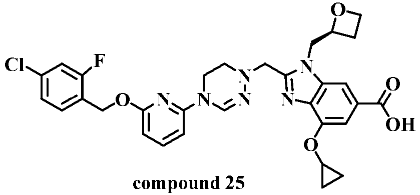

(S)-2-((4-(6-((4-chloro-2-fluorobenzyl)oxy)pyridin-2-yl)-5,6-dihydro-1,2,4-triazin-1(4H)-yl)methyl)-4-cyclopropyloxy-1-(oxetan-2-ylmethyl)-1H-benzo[d]imidazole-6-carboxylic acid (Compound 25)) was prepared according to the route shown in route C. The specific synthesis method was the same as Example 8, except that Intermediate 1 was replaced by Intermediate 13, and Intermediate 10 was replaced by Intermediate 17. LCMS (ESI): m/z = 620.2 (M+H)⁺.¹H NMR (600 MHz, DMSO) δ 7.93 (s, 1H), 7.68 - 7.60 (m, 3H), 7.53 (t, *J =* 8.2 Hz, 1H), 7.47 (dd, *J =* 10.1, 2.1 Hz, 1H), 7.30 (dd, *J* = 8.4, 2.1 Hz, 1H), 6.52 (d, *J =* 8.0 Hz, 1H), 6.40 (d, *J =* 8.0 Hz, 1H), 5.36 (s, 2H), 5.07 - 5.00 (m, 1H), 4.71 - 4.64 (m, 1H), 4.60 - 4.54 (m, 1H), 4.50 - 4.39 (m, 2H), 4.35 - 4.28 (m, 2H), 4.01 - 3.95 (m, 1H), 3.74 (t, *J =* 5.4 Hz, 2H), 3.06 - 3.01 (m, 2H), 2.70 - 2.61 (m, 1H), 2.43 - 2.34 (m, 1H), 0.83 - 0.78 (m, 2H), 0.74 - 0.68 (m, 2H).

### Example 26: Preparation of (S)-2-((4-(6-((4-chloro-2-fluorobenzyl)oxy)pyridin-2-yl)-5,6-dihydro-1,2,4-triazin-1(4H)-yl)methyl)-4-(methoxy-d3)-1-(oxetan-2-ylmethyl)-1H-benzo[d]imidazole-6-carboxylic acid (Compound 26)

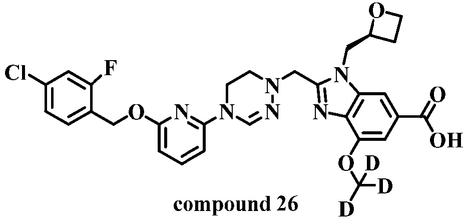

(S)-2-((4-(6-((4-chloro-2-fluorobenzyl)oxy)pyridin-2-yl)-5,6-dihydro-1,2,4-triazin-1(4H)-yl)methyl)-4-(methoxy-d3)-1-(oxetan-2-ylmethyl)-1H-benzo[d]imidazole-6-carboxylic acid (Compound 26)) was prepared according to the route shown in route C. The specific synthesis method was the same as Example 8, except that Intermediate 1 was replaced by Intermediate 12, and Intermediate 10 was replaced by Intermediate 17. LCMS (ESI): m/z = 597.2 (M+H)⁺.¹H NMR (600 MHz, DMSO) δ 7.94 (s, 1H), 7.68 - 7.61 (m, 2H), 7.53 (t, *J =* 8.2 Hz, 1H), 7.47 (dd, *J =* 9.9, 2.1 Hz, 1H), 7.34 (s, 1H), 7.30 (dd, *J =* 8.1, 2.1 Hz, 1H), 6.52 (d, *J =* 8.1 Hz, 1H), 6.40 (d, *J* = 7.9 Hz, 1H), 5.36 (s, 2H), 5.08 - 5.01 (m, 1H), 4.72 - 4.65 (m, 1H), 4.61 - 4.54 (m, 1H), 4.50 - 4.39 (m, 2H), 4.36 - 4.28 (m, 2H), 3.75 (t, *J =* 5.3 Hz, 2H), 3.07 - 3.04 (m, 2H), 2.70 - 2.62 (m, 1H), 2.43 - 2.34 (m, 1H).

### Example 27: Preparation of (S)-2-((4-(6-((2-fluoro-4-(trifluoromethyl)benzyl)oxy)pyridin-2-yl)piperidin-1-yl)methyl)-4-methoxy-1-(oxetan-2-ylmethyl)-1H-benzo[d]imidazole-6-carboxylic acid (Compound 27)

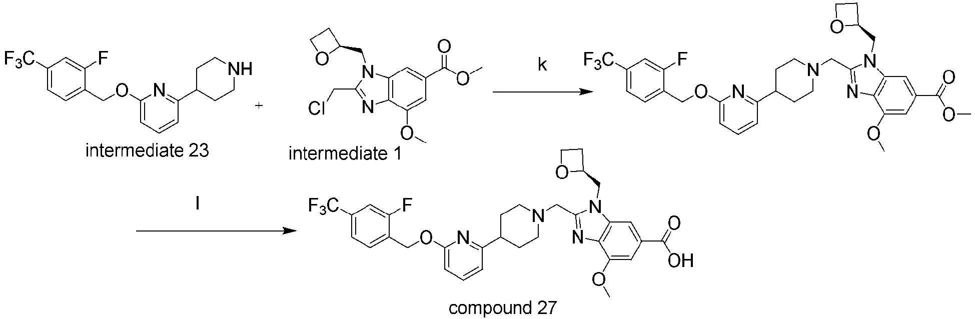

2-((2-Fluoro-4-(trifluoromethyl)benzyl)oxy)-6-(piperidin-4-yl)pyridine (Intermediate 23) was prepared according to the route shown in route B. The specific synthesis method is the same as that of Intermediate 2. LCMS (ESI): m/z = 354.1 (M+H)⁺.

(S)-2-((4-(6-((2-fluoro-4-(trifluoromethyl)benzyl)oxy)pyridin-2-yl)piperidin-1-yl)methyl)-4-methoxy-1-(oxetan-2-ylmethyl)-1H-benzo[d]imidazole-6-carboxylic acid (Compound 27)) was prepared according to the route shown in route B. The specific synthesis method was the same as Example 1, except that Intermediate 2 was replaced by Intermediate 23. LCMS (ESI): m/z = 628.2 (M+H)⁺.¹H NMR (500 MHz, DMSO) δ 7.83 (d, *J =* 1.3 Hz, 1H), 7.72 (t, *J =* 7.6 Hz, 1H), 7.68 (dd, *J =* 10.0, 1.8 Hz, 1H), 7.65 - 7.60 (m, 1H), 7.57 (dd, *J =* 8.1, 1.8 Hz, 1H), 7.26 (d, *J =* 1.2 Hz, 1H), 6.86 (d, *J =* 7.3 Hz, 1H), 6.69 (d, *J =* 8.2 Hz, 1H), 5.46 (s, 2H), 5.12 - 5.03 (m, 1H), 4.76 - 4.68 (m, 1H), 4.64 - 4.56 (m, 1H), 4.51 - 4.41 (m, 1H), 4.37 - 4.29 (m, 1H), 3.92 (s, 3H), 3.88 (d, *J = 13.4* Hz, 1H), 3.73 (d, *J =* 13.4 Hz, 1H), 2.94 (d, *J =* 11.2 Hz, 1H), 2.81 (d, *J =* 11.2 Hz, 1H), 2.72 - 2.61 (m, 1H), 2.61 - 2.52 (m, 1H), 2.45 - 2.35 (m, 1H), 2.22 - 2.09 (m, 2H), 1.80 - 1.55 (m, 4H).

### Example 28: Preparation of (S)-4-ethoxy-2-((4-(6-((2-fluoro-4-(trifluoromethyl)benzyl)oxy)pyridin-2-yl)piperidin-1-yl)methyl)-1-(oxetan-2-ylmethyl)-1H-benzo[d]imidazole-6-carboxylic acid (Compound 28)

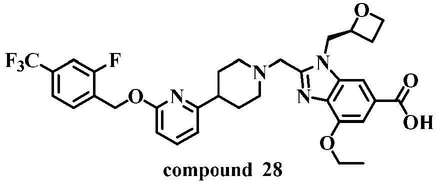

(S)-4-ethoxy-2-((4-(6-((2-fluoro-4-(trifluoromethyl)benzyl)oxy)pyridin-2-yl)piperidin-1-yl)methyl)-1-(oxetan-2-ylmethyl)-1H-benzo[d]imidazole-6-carboxylic acid (Compound 28)) was prepared according to the route shown in route B. The specific synthesis method was the same as Example 1, except that Intermediate 1 was replaced by Intermediate 3, and Intermediate 2 was replaced by Intermediate 23. LCMS (ESI): m/z = 642.3 (M+H)⁺.¹H NMR (500 MHz, DMSO) δ 7.85 (d, *J =* 1.3 Hz, 1H), 7.72 (t, *J =* 7.6 Hz, 1H), 7.68 (dd, *J =* 10.2, 1.7 Hz, 1H), 7.62 (dd, *J =* 8.2, 7.4 Hz, 1H), 7.57 (dd, *J =* 8.0, 1.8 Hz, 1H), 7.22 (d, *J =* 1.3 Hz, 1H), 6.86 (d, *J* = 7.3 Hz, 1H), 6.69 (d, *J =* 8.1 Hz, 1H), 5.46 (s, 2H), 5.12 - 5.03 (m, 1H), 4.78 - 4.70 (m, 1H), 4.65 - 4.58 (m, 1H), 4.49 - 4.41 (m, 1H), 4.39 - 4.29 (m, 1H), 4.23 (q, *J* = 7.0 Hz, 2H), 3.89 (d, *J =* 13.4 Hz, 1H), 3.74 (d, *J =* 13.3 Hz, 1H), 2.94 (d, *J =* 11.3 Hz, 1H), 2.80 (d, *J =* 11.3 Hz, 1H), 2.72 - 2.62 (m, 1H), 2.62 - 2.52 (m, 1H), 2.45 - 2.35 (m, 1H), 2.24 - 2.09 (m, 2H), 1.79 - 1.56 (m, 4H), 1.40 (t, *J* = 6.9 Hz, 3H).

### Example 29: Preparation of (S)-2-((4-(6-((4-chloro-2-fluorobenzyl)oxy)pyridin-2-yl)piperidin-1-yl)methyl)-4-ethoxy-1-(oxetan-2-ylmethyl)-1H-benzo[d]imidazole-6-carboxylic acid (Compound 29)

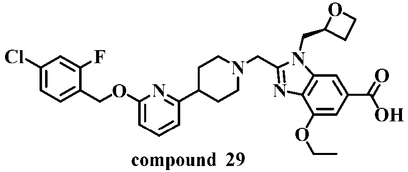

(S)-2-((4-(6-((4-chloro-2-fluorobenzyl)oxy)pyridin-2-yl)piperidin-1-yl)methyl)-4-ethoxy-1-(oxetan-2-ylmethyl)-1H-benzo[d]imidazole-6-carboxylic acid (Compound 29)) was prepared according to the route shown in route B. The specific synthesis method was the same as Example 1, except that Intermediate 1 was replaced by Intermediate 3, and Intermediate 2 was replaced by Intermediate 19. LCMS (ESI): m/z = 608.2 (M+H)⁺.¹H NMR (500 MHz, DMSO) δ 7.86 (s, 1H), 7.61 (t, *J =* 7.8 Hz, 1H), 7.54 (t, *J =* 8.2 Hz, 1H), 7.44 (dd, *J* = 9.9, 2.1 Hz, 1H), 7.27 (dd, *J =* 8.2, 2.1 Hz, 1H), 7.22 (d, *J =* 1.2 Hz, 1H), 6.85 (d, *J =* 7.3 Hz, 1H), 6.65 (d, *J =* 8.2 Hz, 1H), 5.36 (s, 2H), 5.12 - 5.04 (m, 1H), 4.78 - 4.70 (m, 1H), 4.65 - 4.58 (m, 1H), 4.48 - 4.40 (m, 1H), 4.38 - 4.30 (m, 1H), 4.23 (q, *J =* 6.9 Hz, 2H), 3.90 (d, *J =* 13.4 Hz, 1H), 3.74 (d, *J =* 13.4 Hz, 1H), 2.96 (d, *J* = 11.2 Hz, 1H), 2.82 (d, *J* = 11.3 Hz, 1H), 2.73 - 2.63 (m, 1H), 2.62 - 2.55 (m, 1H), 2.46 - 2.35 (m, 1H), 2.24 - 2.10 (m, 2H), 1.81 - 1.60 (m, 4H), 1.40 (t, *J =* 7.0 Hz, 3H).

### Example 30: Preparation of (S)-2-((4-(6-((4-chloro-2-fluorobenzyl)oxy)pyridin-2-yl)piperazin-1-yl)methyl)-4-methoxy-1-(oxetan-2-ylmethyl)-1H-benzo[d]imidazole-6-carboxylic acid (Compound 30)

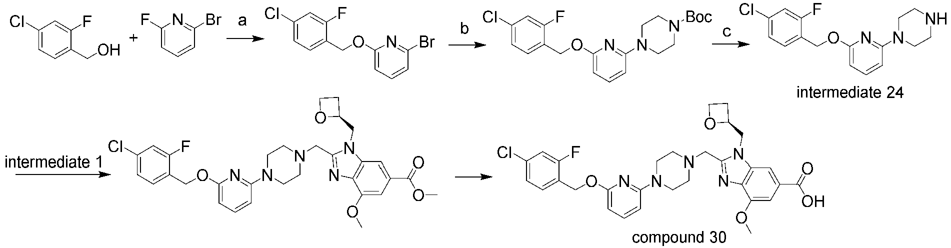

1-(6-((4-chloro-2-fluorobenzyl)oxy)pyridin-2-yl)piperazine (Intermediate 24) was prepared according to the route shown in route B:
Step a: (4-chloro-2-fluorophenyl)methanol (1 g, 6.3 mmol) was dissolved in 20 ml of THF. Potassium tert-butoxide (1.4 g, 12.6 mmol) was added under ice bath. After stirring for 15 minutes, 2-bromo-6-fluoropyridine (1.7 g, 9.9 mmol) was added. The mixture was moved to room temperature and stirred for 3 hours. After the reaction was completed, the mixture was extracted three times with ethyl acetate and washed three times with saturated aqueous ammonium chloride solution. The organic phase was dried over anhydrous magnesium sulfate and filtered. The filtrate was concentrated under reduced pressure. The crude product was separated by column chromatography to obtain 1.7 g of 2-bromo-6-((4-chloro-2-fluorobenzyl)oxy)pyridine. LCMS (ESI): m/z = 315.0 (M+H)⁺.
Step b: 2-Bromo-6-((4-chloro-2-fluorobenzyl)oxy)pyridine (500 mg, 1.6 mmol), 1-tert-butyloxycarbonylpiperazine (446 mg, 2.4 mmol), tris(dibenzylideneacetone)palladium (73 mg, 0.08 mmol), 1,1'-binaphthyl-2,2'-bisdiphenylphosphine (100 mg, 0.16 mmol), cesium carbonate (1 g, 3.2 mmol) and 15 ml of toluene were added into a pressure tube, protected by nitrogen, and reacted at 120 °C for 4 hours. After the reaction was completed, the reaction mixture was filtered and the filtrate was concentrated under reduced pressure. The crude product was separated by column chromatography to obtain 593 mg of tert-butyl 4-(6-((4-chloro-2-fluorobenzyl)oxy)pyridin-2-yl)piperazine-1-carboxylate. LCMS (ESI): m/ z = 421.2 (M+H)⁺.
Step c: tert-Butyl 4-(6-((4-chloro-2-fluorobenzyl)oxy)pyridin-2-yl)piperazine-1-carboxylate (593 mg, 1.4 mmol) was dissolved in 10 ml of dichloromethane, 2 ml of trifluoroacetic acid was added, and the mixture was stirred at room temperature for 4 hours. After the reaction was completed, the reaction solution was concentrated under reduced pressure, saturated sodium bicarbonate aqueous solution was added and extracted three times with dichloromethane. The organic phase was collected, dried over anhydrous magnesium sulfate, and filtered. The filtrate was evaporated to dryness to obtain 400 mg of 1-(6-((4-chloro-2-fluorobenzyl)oxy)pyridin-2-yl)piperazine (Intermediate 24). LCMS (ESI): m/z = 321.1 (M+H)⁺.

(S)-2-((4-(6-((4-chloro-2-fluorobenzyl)oxy)pyridin-2-yl)piperazin-1-yl)methyl)-4-methoxy-1-(oxetan-2-ylmethyl)-1H-benzo[d]imidazole-6-carboxylic acid (Compound 30)) was prepared according to the route shown in route B. The specific synthesis method was the same as Example 1, except that Intermediate 2 was replaced by Intermediate 24. LCMS (ESI): m/z = 595.2 (M+H)⁺.¹H NMR (500 MHz, DMSO) δ 7.85 (s, 1H), 7.52 (t, *J =* 8.2 Hz, 1H), 7.48 - 7.44 (m, 2H), 7.33 - 7.27 (m, 2H), 6.33 (d, *J =* 8.2 Hz, 1H), 6.09 (d, *J =* 7.8 Hz, 1H), 5.30 (s, 2H), 5.13 - 5.05 (m, 1H), 4.77 - 4.69 (m, 1H), 4.65 - 4.57 (m, 1H), 4.52 - 4.44 (m, 1H), 4.40 - 4.32 (m, 1H), 3.94 (s, 3H), 3.92 (d, *J =* 13.4 Hz, 1H), 3.78 (d, *J =* 13.4 Hz, 1H), 3.45 (t, *J =* 5.1 Hz, 8H), 2.74 - 2.63 (m, 1H), 2.47 - 2.38 (m, 1H).

### Example 31: Preparation of (S)-2-((4-(2-((4-chloro-2-fluorobenzyl)oxy)pyridin-3-yl)piperidin-1-yl)methyl)-4-methoxy-1-(oxetan-2-ylmethyl)-1H-benzo[d]imidazole-6-carboxylic acid (Compound 31)

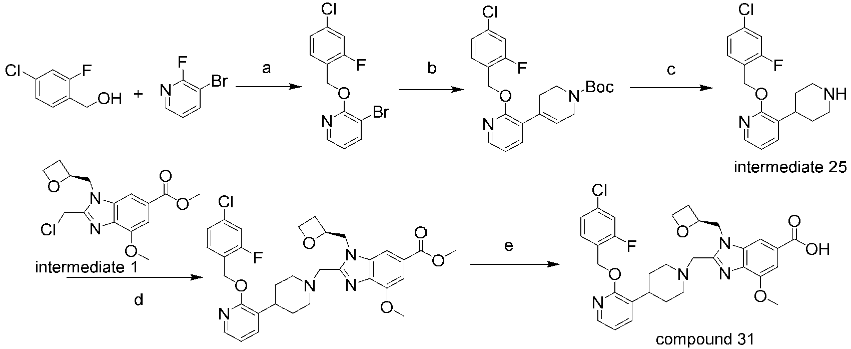

2-((4-chloro-2-fluorobenzyl)oxy)-3-(piperidin-4-yl)pyridine (Intermediate 25) was prepared according to the route shown in route D:
Step a: (4-chloro-2-fluorophenyl)methanol (1 g, 6.3 mmol) was dissolved in 20 ml of THF. Potassium tert-butoxide (1.4 g, 12.6 mmol) was added under ice bath. After stirring for 15 minutes, 2-fluoro-3-bromopyridine (1.7 g, 9.9 mmol) was added. The mixture was moved to room temperature and stirred for 3 hours. After the reaction was completed, the mixture was extracted three times with ethyl acetate and washed three times with saturated aqueous ammonium chloride solution. The organic phase was dried over anhydrous magnesium sulfate and filtered. The filtrate was concentrated under reduced pressure. The crude product was separated by column chromatography to obtain 1.7 g of 3-bromo-2-((4-chloro-2-fluorobenzyl)oxy)pyridine. LCMS (ESI): m/z = 315.0 (M+H)⁺.
Step b: 3-Bromo-2-((4-chloro-2-fluorobenzyl)oxy)pyridine (1.7 g, 5.4 mmol), N-Boc-1,2,5,6-tetrahydropyridine-4-boronic acid pinacol ester (2.1 g, 6.7 mmol), cesium carbonate (3.7 g, 11.2 mmol) and 1,1'-bis(diphenylphosphino)ferrocenepalladium dichloride (II) (205 mg, 0.28 mmol) were dissolved in 20 ml of 1,4-dioxane and 4 ml of water, protected by nitrogen, and reacted at 100 °C for 3 hours. After the reaction was completed, the mixture was extracted three times with ethyl acetate and washed three times with saturated brine. The organic phase was dried over anhydrous magnesium sulfate and filtered. The filtrate was evaporated to dryness and the crude product was separated by column chromatography to obtain 1.8 g of tert-butyl 2-((4-chloro-2-fluorobenzyl)oxy)-3',6'-dihydro-[3,4'-bipyridine]-1'(2'H)-carboxylate. LCMS (ESI): m/z = 418.2 (M+H)⁺.
Step c: tert-Butyl 2-((4-chloro-2-fluorobenzyl)oxy)-3',6'-dihydro-[3,4'-bipyridine]-1'(2'H)-carboxylate (1.8 g, 4.3 mmol) was dissolved in 20 ml of ethyl acetate, 180 mg of 10% palladium carbon (55% water) was added, hydrogen was replaced, and the mixture was stirred at room temperature for 3 hours. After the reaction was completed, the reaction mixture was filtered and the filtrate was concentrated under reduced pressure. The obtained oil was dissolved in 20 ml of dichloromethane, and 4 ml of trifluoroacetic acid was added, followed by stirring at room temperature for 4 hours. After the reaction was completed, the reaction solution was concentrated under reduced pressure, saturated sodium bicarbonate aqueous solution was added and extracted three times with dichloromethane, the organic phase was collected, dried over anhydrous magnesium sulfate, and filtered, and the filtrate was concentrated under reduced pressure to obtain 1 g of 2-((4-chloro-2-fluorobenzyl)oxy)-3-(piperidin-4-yl)pyridine (Intermediate 25). LCMS (ESI): m/z = 320.1 (M+H)⁺.

(S)-2-((4-(2-((4-chloro-2-fluorobenzyl)oxy)pyridin-3-yl)piperidin-1-yl)methyl)-4-methoxy-1-(oxetan-2-ylmethyl)-1H-benzo[d]imidazole-6-carboxylic acid (Compound 31) was prepared according to the route shown in route D:
Step d: Methyl (S)-2-(Chloromethyl)-4-methoxy-1-(oxetan-2-ylmethyl)-1H-benzo[d]imidazole-6-carboxylate (Intermediate 1, 50 mg, 0.15 mmol), 2-((4-chloro-2-fluorobenzyl)oxy)-3-(piperidin-4-yl)pyridine (Intermediate 25, 58 mg, 0.18 mmol), potassium carbonate (41 mg, 0.3 mmol) and a catalytic amount of potassium iodide were dissolved in 5 ml of acetonitrile and reacted at 80 °C for 2 hours. After the reaction was completed, the reaction mixture was filtered and the filtrate was concentrated under reduced pressure. The mixture was separated by preparative thin layer chromatography to obtain 75 mg of methyl (S)-2-((4-(2-((4-chloro-2-fluorobenzyl)oxy)pyridin-3-yl)piperidin-1-yl)methyl)-4-methoxy-1-(oxetan-2-ylmethyl)-1H-benzo[d]imidazole-6-carboxylate. LCMS (ESI): m/z = 608.2 (M+H)⁺.
Step e: methyl ((S)-2-((4-(2-((4-chloro-2-fluorobenzyl)oxy)pyridin-3-yl)piperidin-1-yl)methyl)-4-methoxy-1-(oxetan-2-ylmethyl)-1H-benzo[d]imidazole-6-carboxylate (75 mg, 0.12 mmol) was dissolved in 3 ml of 1,4-dioxane, 2M NaOH (300 µL, 0.6 mmol) was added, and the reaction was carried out at 40 °C overnight. After the reaction was completed, the pH was adjusted to 5-6 with 2M hydrochloric acid, and preparative liquid separation was performed to obtain 60 mg of (S)-2-((4-(2-((4-chloro-2-fluorobenzyl)oxy)pyridin-3-yl)piperidin-1-yl)methyl)-4-methoxy-1-(oxetan-2-ylmethyl)-1H-benzo[d]imidazole-6-carboxylic acid (Compound 31). LCMS (ESI): m/z = 594.2 (M+H)⁺.¹H NMR (500 MHz, DMSO) δ 7.98 (dd, *J =* 5.0, 1.8 Hz, 1H), 7.82 (d, *J* = 1.2 Hz, 1H), 7.57 (dd, *J* = 7.5, 1.9 Hz, 1H), 7.52 (t, *J* = 8.2 Hz, 1H), 7.47 (dd, *J* = 10.1, 2.1 Hz, 1H), 7.31 (dd, *J* = 8.2, 2.0 Hz, 1H), 7.26 (d, *J* = 1.2 Hz, 1H), 6.95 (dd, *J* = 7.4, 4.9 Hz, 1H), 5.38 (s, 2H), 5.08 - 5.00 (m, 1H), 4.74 - 4.65 (m, 1H), 4.60 - 4.55 (m, 1H), 4.49 - 4.41 (m, 1H), 4.35 - 4.27 (m, 1H), 3.92 (s, 3H), 3.86 (d, *J* = 13.4 Hz, 1H), 3.74 (d, *J* = 13.4 Hz, 1H), 2.94 (d, *J* = 11.2 Hz, 1H), 2.84 (d, *J* = 11.2 Hz, 1H), 2.78 - 2.69 (m, 1H), 2.69 - 2.57 (m, 1H), 2.42 - 2.31 (m, 1H), 2.23 - 2.09 (m, 2H), 1.79 - 1.67 (m, 2H), 1.67 - 1.51 (m, 2H).

### Example 32: Preparation of (S)-2-((4-(2-((4-chloro-2-fluorobenzyl)oxy)pyridin-3-yl)piperidin-1-yl)methyl)-4-ethoxy-1-(oxetan-2-ylmethyl)-1H-benzo[d]imidazole-6-carboxylic acid (Compound 32)

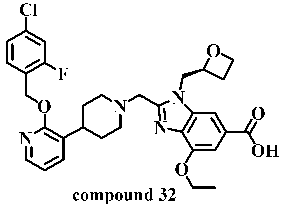

(S)-2-((4-(2-((4-chloro-2-fluorobenzyl)oxy)pyridin-3-yl)piperidin-1-yl)methyl)-4-ethoxy-1-(oxetan-2-ylmethyl)-1H-benzo[d]imidazole-6-carboxylic acid (Compound 32) was prepared according to the route shown in route D. The specific synthesis method was the same as Example 31, except that Intermediate 1 was replaced by Intermediate 3. LCMS (ESI): m/z = 608.2 (M+H)⁺.¹H NMR (500 MHz, DMSO) δ 8.01 (dd, *J =* 5.0, 1.8 Hz, 1H), 7.87 (s, 1H), 7.61 (d, *J =* 7.3 Hz, 1H), 7.55 (t, *J =* 8.1 Hz, 1H), 7.51 (dd, *J* = 9.9, 2.1 Hz, 1H), 7.34 (dd, *J =* 8.2, 2.1 Hz, 1H), 7.24 (s, 1H), 6.98 (dd, *J* = 7.4, 4.9 Hz, 1H), 5.41 (s, 2H), 5.09 - 5.04 (m, 1H), 4.78 - 4.70 (m, 1H), 4.64 - 4.58 (m, 1H), 4.51 - 4.43 (m, 1H), 4.37 - 4.30 (m, 1H), 4.24 (q, *J =* 6.9 Hz, 2H), 3.90 (d, *J* = 13.4 Hz, 1H), 3.77 (d, *J =* 13.4 Hz, 1H), 2.96 (d, *J =* 11.1 Hz, 1H), 2.84 (d, *J =* 11.0 Hz, 1H), 2.80 - 2.72 (m, 1H), 2.71 - 2.64 (m, 1H), 2.42 - 2.32 (m, 1H), 2.24 - 2.10 (m, 2H), 1.80 - 1.70 (m, 2H), 1.69 - 1.55 (m, 2H), 1.42 (t, *J =* 6.9 Hz, 3H).

### Example 33: Preparation of (S)-2-((4-(2-((4-cyano-2-fluorobenzyl)oxy)pyridin-3-yl)piperidin-1-yl)methyl)-4-ethoxy-1-(oxetan-2-ylmethyl)-1H-benzo[d]imidazole-6-carboxylic acid (Compound 33)

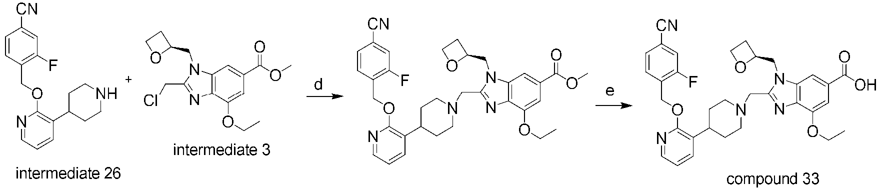

(S)-2-((4-(2-((4-cyano-2-fluorobenzyl)oxy)pyridin-3-yl)piperidin-1-yl)methyl)-4-ethoxy-1-(oxetan-2-ylmethyl)-1H-benzo[d]imidazole-6-carboxylic acid (Compound 33) was prepared according to the route shown in route D. The specific synthesis method was the same as Example 31, except that Intermediate 1 was replaced by Intermediate 3, and Intermediate 25 was replaced by Intermediate 26. LCMS (ESI): m/z = 599.3 (M+H)⁺.¹H NMR (500 MHz, DMSO) δ 8.00 (dd, *J =* 5.0, 1.8 Hz, 1H), 7.93 (dd, *J* = 9.9, 1.5 Hz, 1H), 7.86 (d, *J =* 1.2 Hz, 1H), 7.75 (dd, *J =* 7.8, 1.6 Hz, 1H), 7.68 (t, *J* = 7.6 Hz, 1H), 7.63 (dd, *J* = 7.4, 1.9 Hz, 1H), 7.25 (d, *J =* 1.3 Hz, 1H), 7.00 (dd, *J =* 7.4, 5.0 Hz, 1H), 5.51 (s, 2H), 5.12 - 5.03 (m, 1H), 4.79 - 4.71 (m, 1H), 4.66 - 4.59 (m, 1H), 4.52 - 4.44 (m, 1H), 4.38 - 4.30 (m, 1H), 4.24 (q, *J =* 6.9 Hz, 2H), 3.91 (d, *J =* 13.3 Hz, 1H), 3.78 (d, *J =* 13.3 Hz, 1H), 2.98 (d, *J* = 11.1 Hz, 1H), 2.87 (d, *J =* 11.1 Hz, 1H), 2.84 - 2.76 (m, 1H), 2.74 - 2.64 (m, 1H), 2.44 - 2.34 (m, 1H), 2.28 - 2.13 (m, 2H), 1.83 - 1.74 (m, 2H), 1.70 - 1.56 (m, 2H), 1.42 (t, *J* = 6.9 Hz, 3H).

### Example 34: Preparation of (S)-2-((4-(2-((4-cyano-2-methoxybenzyl)oxy)pyridin-3-yl)piperidin-1-yl)methyl)-4-methoxy-1-(oxetan-2-ylmethyl)-1H-benzo[d]imidazole-6-carboxylic acid (Compound 34)

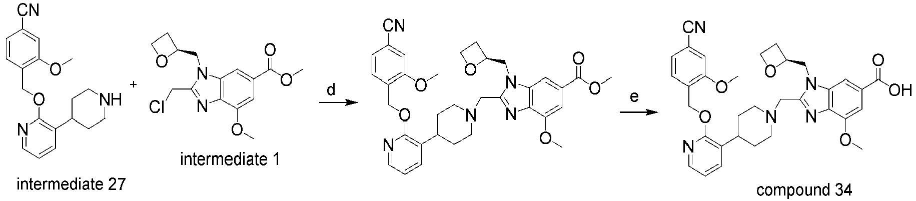

(S)-2-((4-(2-((4-cyano-2-methoxybenzyl)oxy)pyridin-3-yl)piperidin-1-yl)methyl)-4-methoxy-1-(oxetan-2-ylmethyl)-1H-benzo[d]imidazole-6-carboxylic acid (Compound 34) was prepared according to the route shown in route D. The specific synthesis method was the same as Example 31, except that Intermediate 25 was replaced by Intermediate 27. LCMS (ESI): m/z = 597.3 (M+H)⁺.¹H NMR (600 MHz, DMSO) δ 7.99 (dd, *J =* 4.9, 1.8 Hz, 1H), 7.90 (d, *J =* 1.1 Hz, 1H), 7.61 (dd, *J =* 7.5, 1.9 Hz, 1H), 7.52 (d, *J =* 1.3 Hz, 1H), 7.50 - 7.44 (m, 2H), 7.26 (s, 1H), 6.98 (dd, *J =* 7.4, 4.9 Hz, 1H), 5.41 (s, 2H), 5.11 - 5.04 (m, 1H), 4.79 - 4.72 (m, 1H), 4.66 - 4.60 (m, 1H), 4.51 - 4.44 (m, 1H), 4.38 - 4.31 (m, 1H), 3.96 (s, 3H), 3.92 (d, *J =* 13.6 Hz, 1H), 3.90 (s, 3H), 3.79 (d, *J =* 13.5 Hz, 1H), 3.00 (d, *J =* 11.1 Hz, 1H), 2.88 (d, *J =* 12.1 Hz, 1H), 2.86 - 2.79 (m, 1H), 2.75 - 2.65 (m, 1H), 2.44 - 2.35 (m, 1H), 2.29 - 2.15 (m, 2H), 1.84 - 1.75 (m, 2H), 1.71 - 1.57 (m, 2H).

### Example 35: Preparation of (S)-2-((4-(2-((4-cyano-2,3-dihydrobenzofuran-7-yl)methoxy)pyridin-3-yl)piperidin-1-yl)methyl)-4-methoxy-1-(oxetan-2-ylmethyl)-1H-benzo[d]imidazole-6-carboxylic acid (Compound 35)

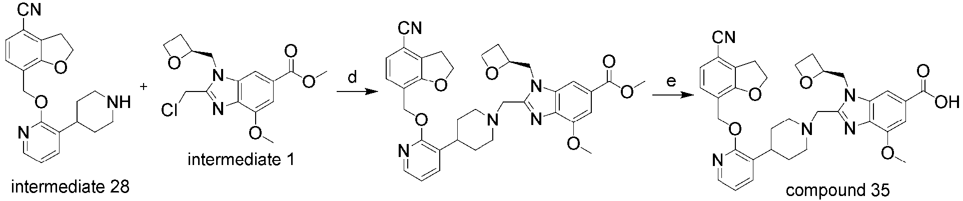

(S)-2-((4-(2-((4-cyano-2,3-dihydrobenzofuran-7-yl)methoxy)pyridin-3-yl)piperidin-1-yl)methyl)-4-methoxy-1-(oxetan-2-ylmethyl)-1H-benzo[d]imidazole-6-carboxylic acid (Compound 35) was prepared according to the route shown in route D. The specific synthesis method was the same as Example 31, except that Intermediate 25 was replaced by Intermediate 28. LCMS (ESI): m/z = 609.3 (M+H)⁺.¹H NMR (600 MHz, DMSO) δ 7.99 (dd, *J* = 4.9, 1.8 Hz, 1H), 7.90 (s, 1H), 7.60 (dd, *J =* 7.5, 1.9 Hz, 1H), 7.33 - 7.25 (m, 3H), 6.97 (dd, *J =* 7.3, 5.0 Hz, 1H), 5.35 (s, 2H), 5.11 - 5.04 (m, 1H), 4.79 - 4.72 (m, 1H), 4.69 (t, *J =* 8.8 Hz, 2H), 4.65 - 4.59 (m, 1H), 4.51 - 4.44 (m, 1H), 4.38 - 4.31 (m, 1H), 3.96 (s, 3H), 3.91 (d, *J =* 13.3 Hz, 1H), 3.78 (d, *J =* 13.4 Hz, 1H), 3.41 (t, *J* = 8.8 Hz, 2H), 2.99 (d, *J* = 11.2 Hz, 1H), 2.87 (d, *J =* 11.3 Hz, 1H), 2.84 - 2.76 (m, 1H), 2.74 - 2.65 (m, 1H), 2.44 - 2.35 (m, 1H), 2.27 - 2.14 (m, 2H), 1.82 - 1.74 (m, 2H), 1.69 - 1.55 (m, 2H).

### Example 36: Preparation of 2-((4-(2-(4-chloro-2-fluorophenyl)-2-methylbenzo[d][1,3]dioxol-4-yl)piperidin-1-yl)methyl)-4-methoxy-1-(((S)-oxetan-2-yl)methyl)-1H-benzo[d]imidazole-6-carboxylic acid (Compound 36)

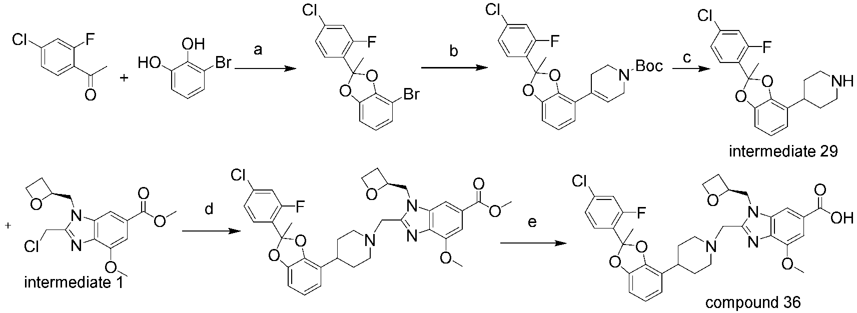

4-(2-(4-chloro-2-fluorophenyl)-2-methylbenzo[d][1,3]dioxy-4-yl)piperidine (Intermediate 29) was prepared according to the route shown in route E:
Step a: 4'-chloro-2'-fluoroacetophenone (10 g, 58.1 mmol), 3-bromobenzene-1,2-diol (10 g, 53.3 mmol) and p-toluenesulfonic acid (1 g, 5.8 mmol) were dissolved in 100 ml of toluene, protected by nitrogen and a water separator was used. The mixture was reacted at 160 °C for 72 hours. After the reaction was completed, the reaction solution was concentrated under reduced pressure, and the crude product was separated by column chromatography to obtain 3.5 g of 4-bromo-2-(4-chloro-2-fluorophenyl)-2-methylbenzo[d][1,3]diazole. LCMS (ESI): m/z = 342.0 (M+H)⁺.
Step b: 4-Bromo-2-(4-chloro-2-fluorophenyl)-2-methylbenzo[d] [1,3]diazole (1 g, 2.9 mmol), N-Boc-1,2,5,6-tetrahydropyridine-4-boronic acid pinacol ester (1.1 g, 3.5 mmol), cesium carbonate (1.9 g, 5.8 mmol) and 1,1'-bis(diphenylphosphino)ferrocenepalladium(II) chloride (106 mg, 0.15 mmol) were dissolved in 20 ml of 1,4-dioxane and 4 ml of water, and reacted at 100 °C for 3 hours under nitrogen protection. After the reaction is completed, the mixture is extracted three times with ethyl acetate, washed three times with saturated brine, the organic phase is dried over anhydrous magnesium sulfate and filtered, the filtrate is evaporated to dryness, and the crude product is separated by column chromatography to obtain 1.1 g of tert-butyl 4-(2-(4-chloro-2-fluorophenyl)-2-methylbenzo[d][1,3]dioxy-4-yl)-3,6-dihydropyridine-1(2H)-carboxylate. LCMS (ESI): m/z = 445.2 (M+H)⁺.
Step c: tert-Butyl 4-(2-(4-chloro-2-fluorophenyl)-2-methylbenzo[d][1,3]dioxy-4-yl)-3,6-dihydropyridine-1(2H)-carboxylate (1.1 g, 2.5 mmol) was dissolved in 20 ml of ethyl acetate, 110 mg of 10% palladium carbon (55% water) was added, hydrogen was replaced, and the mixture was stirred at room temperature for 3 hours. After the reaction was completed, the reaction mixture was filtered and the filtrate was concentrated under reduced pressure. The obtained oil was dissolved in 20 ml of dichloromethane, and 4 ml of trifluoroacetic acid was added, followed by stirring at room temperature for 4 hours. After the reaction was completed, the reaction solution was concentrated under reduced pressure, saturated sodium bicarbonate aqueous solution was added and extracted three times with dichloromethane. The organic phase was collected, dried over anhydrous magnesium sulfate, and filtered. The filtrate was evaporated to dryness to obtain 790 mg of 4-(2-(4-chloro-2-fluorophenyl)-2-methylbenzo[d][1,3]dioxy-4-yl)piperidine (Intermediate 29). LCMS (ESI): m/z = 347.1 (M+H)⁺.

2-((4-(2-(4-Chloro-2-fluorophenyl)-2-methylbenzo[d] [1,3]dioxy-4-yl)piperidin-1-yl)methyl)-4-methoxy-1-(((S)-oxetan-2-yl)methylmethyl)-1H-benzo[d]imidazole-6-carboxylic acid (Compound 36) was prepared according to the route shown in route E:.
Step d: Methyl (S)-2-(Chloromethyl)-4-methoxy-1-(oxetan-2-ylmethyl)-1H-benzo[d]imidazole-6-carboxylate (Intermediate 1, 50 mg, 0.15 mmol), 4-(2-(4-chloro-2-fluorophenyl)-2-methylbenzo[d][1,3]dioxy-4-yl)piperidine (Intermediate 29, 62 mg, 0.18 mmol), potassium carbonate (41 mg, 0.3 mmol) and a catalytic amount of potassium iodide were dissolved in 5 ml of acetonitrile and reacted at 80 °C for 2 hours. After the reaction was completed, the reaction mixture was filtered and the filtrate was concentrated under reduced pressure. The mixture was separated by preparative thin layer chromatography to obtain 67 mg of methyl 2-((4-(2-(4-chloro-2-fluorophenyl)-2-methylbenzo[d][1,3]dioxy-4-yl)piperidin-1-yl)methyl)-4-methoxy-1-(((S)-oxetan-2-yl)methylmethyl)-1H-benzo[d]imidazole-6-carboxylate. LCMS (ESI): m/z = 635.2 (M+H)⁺.
Step e: methyl 2-((4-(2-(4-chloro-2-fluorophenyl)-2-methylbenzo[d][1,3]dioxy-4-yl)piperidin-1-yl)methyl)-4-methoxy-1-(((S)-oxetan-2-yl)methyl)-1H-benzo[d]imidazole-6-carboxylate (67 mg, 0.11 mmol) was dissolved in 3 ml of 1,4-dioxane, 2M NaOH (300 µL, 0.6 mmol) was added, and the reaction was carried out at 40 °C overnight. After the reaction was completed, the pH was adjusted to 5-6 with 2M hydrochloric acid, and preparative liquid separation was performed to obtain 50 mg of 2-((4-(2-(4-chloro-2-fluorophenyl)-2-methylbenzo[d][1,3]dioxy-4-yl)piperidin-1-yl)methyl)-4-methoxy-1-(((S)-oxetan-2-yl)methylmethyl)-1H-benzo[d]imidazole-6-carboxylic acid (Compound 36). LCMS (ESI): m/z = 621.2 (M+H)⁺.¹H NMR (600 MHz, DMSO) δ 7.76 (d, *J =* 2.3 Hz, 1H), 7.59 - 7.52 (m, 2H), 7.34 (dd, *J =* 8.5, 2.0 Hz, 1H), 7.31 (s, 1H), 6.80 - 6.73 (m, 3H), 5.13 - 5.06 (m, 1H), 4.72 - 4.65 (m, 1H), 4.60 - 4.55 (m, 1H), 4.50 - 4.42 (m, 1H), 4.39 - 4.32 (m, 1H), 3.92 (s, 3H), 3.89 (dd, *J =* 13.4, 3.4 Hz, 1H), 3.74 (d, *J =* 13.3 Hz, 1H), 2.99 (d, *J =* 10.8 Hz, 1H), 2.87 (d, *J =* 11.2 Hz, 1H), 2.73 - 2.63 (m, 2H), 2.48 - 2.40 (m, 1H), 2.25 - 2.19 (m, 1H), 2.19 - 2.12 (m, 1H), 2.02 (s, 3H), 1.83 - 1.64 (m, 4H).

### Example 37: Preparation of 2-((4-(2-(4-chloro-2-fluorophenyl)-2-methylbenzo[d][1,3]dioxol-4-yl)piperidin-1-yl)methyl)-4-ethoxy-1-(((S)-oxetan-2-yl)methyl)-1H-benzo[d]imidazole-6-carboxylic acid (Compound 37)

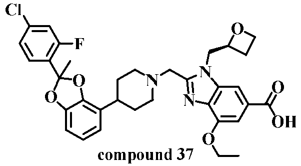

2-((4-(2-(4-chloro-2-fluorophenyl)-2-methylbenzo[d] [1,3]dioxy-4-yl)piperidin-1-yl)methyl)-4-ethoxy-1-(((S)-oxetan-2-yl)methyl)-1H-benzo[d]imidazole-6-carboxylic acid (Compound 37) was prepared according to the route shown in route E. The specific synthesis method was the same as Example 36, except that Intermediate 1 was replaced by Intermediate 3. LCMS (ESI): m/z = 635.2 (M+H)⁺.¹H NMR (500 MHz, DMSO) δ 7.85 (s, 1H), 7.58 - 7.50 (m, 2H), 7.32 (dd, *J =* 8.5, 2.1 Hz, 1H), 7.23 (s, 1H), 6.79 - 6.76 (m, 2H), 6.75 - 6.71 (m, 1H), 5.12 - 5.03 (m, 1H), 4.76 - 4.68 (m, 1H), 4.64 - 4.56 (m, 1H), 4.49 - 4.39 (m, 1H), 4.38 - 4.29 (m, 1H), 4.23 (q, *J* = 7.0 Hz, 2H), 3.90 (dd, *J* = 13.4, 2.8 Hz, 1H), 3.74 (d, *J* = 13.4 Hz, 1H), 3.00 - 2.94 (m, 1H), 2.87 - 2.80 (m, 1H), 2.72 - 2.59 (m, 2H), 2.45 - 2.37 (m, 1H), 2.26 - 2.10 (m, 2H), 2.00 (s, 3H), 1.77 - 1.63 (m, 4H), 1.40 (t, *J* = 7.0 Hz, 3H).

### Example 38: Preparation of 2-((4-(2-(5-chloropyridin-2-yl)-2-methylbenzo[d][1,3]dioxol-4-yl)piperidin-1-yl)methyl)-4-methoxy-1-(((S)-oxetan-2-yl)methyl)-1H-benzo[d]imidazole-6-carboxylic acid (Compound 38)

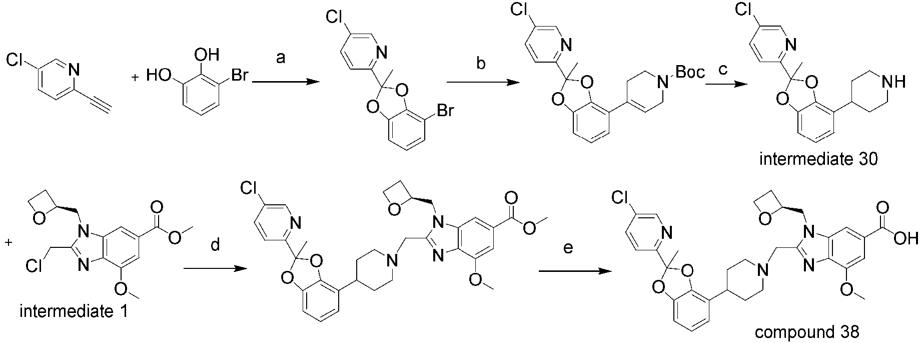

5-Chloro-2-(2-methyl-4-(piperidin-4-yl)benzo[d][1,3]dioxy-2-yl)pyridine (Intermediate 30) was prepared according to the route shown in route E:
Step a: 5-chloro-2-alkynylpyridine (1 g, 7.3 mmol), 3-bromobenzene-1,2-diol (1.8 g, 9.5 mmol), triruthenium dodecacarbonyl (192 mg, 0.3 mmol) and 20 ml of toluene were added into a pressure tube, protected by nitrogen, and reacted at 100 °C overnight. After the reaction was completed, the insoluble catalyst was removed by filtration, the filtrate was concentrated under reduced pressure, and the crude product was separated by column chromatography to obtain 900 mg of 2-(4-bromo-2-methylbenzo[d][1,3]dioxol-2-yl)-5-chloropyridine. LCMS (ESI): m/z = 325.0 (M+H)⁺.

For the subsequent steps, refer to the preparation method of intermediate 29 to obtain 5-chloro-2-(2-methyl-4-(piperidin-4-yl)benzo[d][1,3]dioxy-2-yl)pyridine (intermediate 30). LCMS (ESI): m/z = 330.1 (M+H)⁺.

2-((4-(2-(5-chloropyridin-2-yl)-2-methylbenzo[d][1,3]dioxy-4-yl)piperidin-1-yl)methyl)-4-methoxy-1-(((S)-oxetan-2-yl)methyl)-1H-benzo[d]imidazole-6-carboxylic acid (Compound 38) was prepared according to the route shown in route E. The specific synthesis method was the same as Example 36, except that Intermediate 29 was replaced by Intermediate 30. LCMS (ESI): m/z = 604.2 (M+H)⁺.¹H NMR (500 MHz, DMSO) δ 8.70 (t, *J =* 2.3 Hz, 1H), 7.98 (dd, *J =* 8.5, 2.5 Hz, 1H), 7.70 (s, 1H), 7.58 (d, *J =* 8.4 Hz, 1H), 7.29 (d, *J =* 1.1 Hz, 1H), 6.81 - 6.70 (m, 3H), 5.11 - 5.02 (m, 1H), 4.67 - 4.59 (m, 1H), 4.56 - 4.49 (m, 1H), 4.47 - 4.37 (m, 1H), 4.37 - 4.29 (m, 1H), 3.88 (s, 3H), 3.85 (dd, *J =* 13.3, 2.5 Hz, 1H), 3.70 (dd, *J =* 13.3, 3.4 Hz, 1H), 2.96 (d, *J =* 11.2 Hz, 1H), 2.84 (d, *J =* 11.3 Hz, 1H), 2.70 - 2.59 (m, 2H), 2.47 - 2.36 (m, 1H), 2.22 - 2.07 (m, 2H), 1.99 (s, 3H), 1.78 - 1.66 (m, 4H).

### Example 39: Preparation of 2-((4-(2-(5-chloropyridin-2-yl)-2-methylbenzo[d][1,3]dioxol-4-yl)piperidin-1-yl)methyl)-4-ethoxy-1-(((S)-oxetan-2-yl)methyl)-1H-benzo[d]imidazole-6-carboxylic acid (Compound 39)

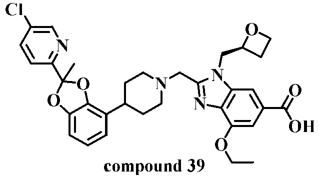

2-((4-(2-(5-chloropyridin-2-yl)-2-methylbenzo[d][1,3]dioxy-4-yl)piperidin-1-yl)methyl)-4-ethoxy-1-(((S)-oxetan-2-yl)methyl)-1H-benzo[d]imidazole-6-carboxylic acid (Compound 39) was prepared according to the route shown in route E. The specific synthesis method was the same as Example 36, except that Intermediate 1 was replaced by Intermediate 3, and Intermediate 29 was replaced by Intermediate 30. LCMS (ESI): m/z = 618.2 (M+H)⁺.¹H NMR (500 MHz, DMSO) δ 8.70 (t, *J* = 2.3 Hz, 1H), 7.98 (dd, *J =* 8.5, 2.5 Hz, 1H), 7.64 (d, *J =* 1.0 Hz, 1H), 7.58 (d, *J =* 8.5 Hz, 1H), 7.28 (d, *J* = 1.1 Hz, 1H), 6.81 - 6.71 (m, 3H), 5.11 - 5.02 (m, 1H), 4.66 - 4.57 (m, 1H), 4.55 - 4.48 (m, 1H), 4.47 - 4.37 (m, 1H), 4.37 - 4.28 (m, 1H), 4.17 (q, *J =* 7.0 Hz, 2H), 3.84 (dd, *J* = 13.4, 2.0 Hz, 1H), 3.70 (dd, *J* = 13.3, 2.8 Hz, 1H), 2.96 (d, *J* = 11.1 Hz, 1H), 2.84 (d, *J* = 11.3 Hz, 1H), 2.70 - 2.57 (m, 2H), 2.47 - 2.36 (m, 1H), 2.22 - 2.07 (m, 2H), 1.99 (s, 3H), 1.78 - 1.64 (m, 4H), 1.38 (t, *J =* 7.0 Hz, 3H).

### Example 40: Preparation of 2-((4-(2-(4-chloro-2-fluorophenyl)-2-methylbenzo[d][1,3]dioxol-4-yl)piperazin-1-yl)methyl)-4-methoxy-1-(((S)-oxetan-2-yl)methyl)-1H-benzo[d]imidazole-6-carboxylic acid (Compound 40)

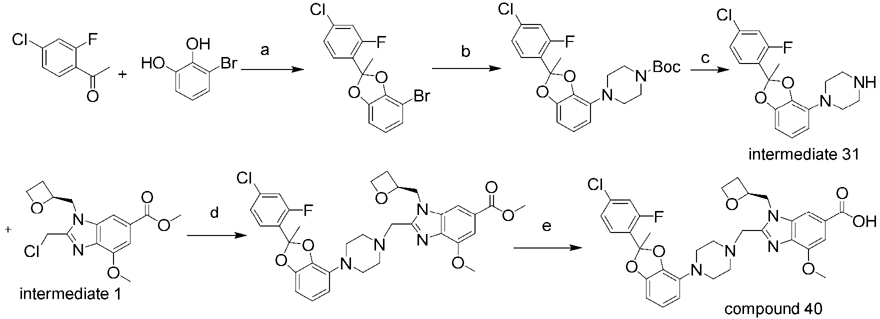

1-(2-(4-chloro-2-fluorophenyl)-2-methylbenzo[d][1,3]dioxy-4-yl)piperazine (Intermediate 31) was prepared according to the route shown in route E:
Step a: 4'-chloro-2'-fluoroacetophenone (10 g, 58.1 mmol), 3-bromobenzene-1,2-diol (10 g, 53.3 mmol) and p-toluenesulfonic acid (1 g, 5.8 mmol) were dissolved in 100 ml of toluene, protected by nitrogen and a water separator was used. The mixture was reacted at 160 °C for 72 hours. After the reaction was completed, the reaction solution was concentrated under reduced pressure, and the crude product was separated by column chromatography to obtain 3.5 g of 4-bromo-2-(4-chloro-2-fluorophenyl)-2-methylbenzo[d][1,3]diazole. LCMS (ESI): m/z = 342.0 (M+H)⁺.
Step b: 4-bromo-2-(4-chloro-2-fluorophenyl)-2-methylbenzo[d][1,3]diazole (550 mg, 1.6 mmol), 1-tert-butyloxycarbonylpiperazine (446 mg, 2.4 mmol), tris(dibenzylideneacetone)palladium (73 mg, 0.08 mmol), 1,1'-binaphthyl-2,2'-bis(diphenylphosphine) (100 mg, 0.16 mmol), cesium carbonate (1 g, 3.2 mmol) and 15 ml of toluene were added into a pressure tube, protected by nitrogen, and reacted at 120 °C for 4 hours. After the reaction was completed, the reaction mixture was filtered and the filtrate was concentrated under reduced pressure. The crude product was separated by column chromatography to obtain 600 mg of tert-butyl 4-(2-(4-chloro-2-fluorophenyl)-2-methylbenzo[d][1,3]dioxy-4-yl)piperazine-1-carboxylate. LCMS (ESI): m/z = 448.2 (M+H)⁺.
Step c: tert-Butyl 4-(2-(4-chloro-2-fluorophenyl)-2-methylbenzo[d][1,3]dioxy-4-yl)piperazine-1-carboxylate (600 mg, 1.4 mmol) was dissolved in 10 ml of dichloromethane, 2 ml of trifluoroacetic acid was added, and the mixture was stirred at room temperature for 4 hours. After the reaction was completed, the reaction solution was concentrated under reduced pressure, saturated sodium bicarbonate aqueous solution was added and extracted three times with dichloromethane, the organic phase was collected, dried over anhydrous magnesium sulfate, and filtered, and the filtrate was evaporated to dryness to obtain 400 mg of 1-(2-(4-chloro-2-fluorophenyl)-2-methylbenzo[d][1,3]dioxy-4-yl)piperazine (Intermediate 31). LCMS(ESI): m/z = 348.1 (M+H)⁺.

2-((4-(2-(4-Chloro-2-fluorophenyl)-2-methylbenzo [d] [1,3]dioxy-4-yl)piperazin-1-yl)methyl)-4-methoxy-1-(((S)-oxetan-2-yl)methyl)-1H-benzo[d]imidazole-6-carboxylic acid (Compound 40) was prepared according to the route shown in route E, and the subsequent steps were the same as those of Compound 36, except that Intermediate 29 was replaced by Intermediate 31. LCMS (ESI): m/z = 622.2 (M+H)⁺.¹H NMR (500 MHz, DMSO) δ 7.90 (d, *J* = 1.2 Hz, 1H), 7.59 - 7.51 (m, 2H), 7.36 - 7.30 (m, 1H), 7.27 (d, *J* = 1.2 Hz, 1H), 6.75 (t, *J* = 8.1 Hz, 1H), 6.56 (d, *J =* 7.8 Hz, 1H), 6.44 (d, *J* = 8.4 Hz, 1H), 5.12 - 5.03 (m, 1H), 4.79 - 4.69 (m, 1H), 4.66 - 4.57 (m, 1H), 4.51 - 4.43 (m, 1H), 4.40 - 4.30 (m, 1H), 3.96 (s, 3H), 3.93 (d, *J =* 13.4 Hz, 1H), 3.80 (d, *J* = 13.4 Hz, 1H), 3.17 (t, *J* = 5.1 Hz, 4H), 3.07 (t, *J* = 5.1 Hz, 4H), 2.73 - 2.65 (m, 1H), 2.46 - 2.36 (m, 1H), 2.01 (s, 3H).

### Example 41: Preparation of methyl (S)-2-((4-(6-((4-chloro-2-fluorobenzyl)oxy)pyridin-2-yl)piperidin-1-yl)methyl)-4-methoxy-1-(oxetan-2-ylmethyl)-1H-benzo[d]imidazole-6-carboxylate (Compound 41)

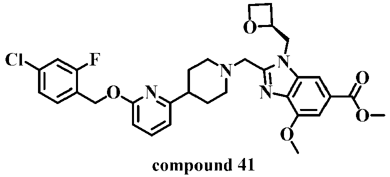

Methyl (S)-2-((4-(6-((4-chloro-2-fluorobenzyl)oxy)pyridin-2-yl)piperidin-1-yl)methyl)-4-methoxy-1-(oxetan-2-ylmethyl)-1H-benzo[d]imidazole-6-carboxylate (Compound 41) was prepared according to the route shown in route B and obtained as an intermediate of Compound 19. LCMS (ESI): m/z = 594.2 (M+H)⁺.¹H NMR (500 MHz, DMSO) δ 7.91 (d, *J =* 1.4 Hz, 1H), 7.61 (t, *J =* 7.7 Hz, 1H), 7.54 (t, *J =* 8.2 Hz, 1H), 7.44 (dd, *J =* 9.9, 2.1 Hz, 1H), 7.27 (dd, *J =* 8.3, 2.1 Hz, 1H), 7.24 (d, *J =* 1.3 Hz, 1H), 6.85 (d, *J =* 7.3 Hz, 1H), 6.65 (d, *J =* 8.1 Hz, 1H), 5.36 (s, 2H), 5.12 - 5.04 (m, 1H), 4.80 - 4.72 (m, 1H), 4.67 - 4.59 (m, 1H), 4.48 - 4.40 (m, 1H), 4.37 - 4.29 (m, 1H), 3.95 (s, 3H), 3.91 (d, *J =* 13.4 Hz, 1H), 3.85 (s, 3H), 3.74 (d, *J =* 13.5 Hz, 1H), 2.97 (d, *J =* 11.1 Hz, 1H), 2.82 (d, *J* = 11.3 Hz, 1H), 2.73 - 2.62 (m, 1H), 2.61 - 2.53 (m, 1H), 2.46 - 2.33 (m, 1H), 2.25 - 2.10 (m, 2H), 1.81 - 1.60 (m, 4H).

### Example 42: Preparation of (S)-2-((4-(3-((4-cyano-2-methoxybenzyl)oxy)phenyl)piperidin-1-yl)methyl)-4-methoxy-1-(oxetan-2-ylmethyl)-1H-benzo[d]imidazole-6-carboxylic acid

### (Compound 42)

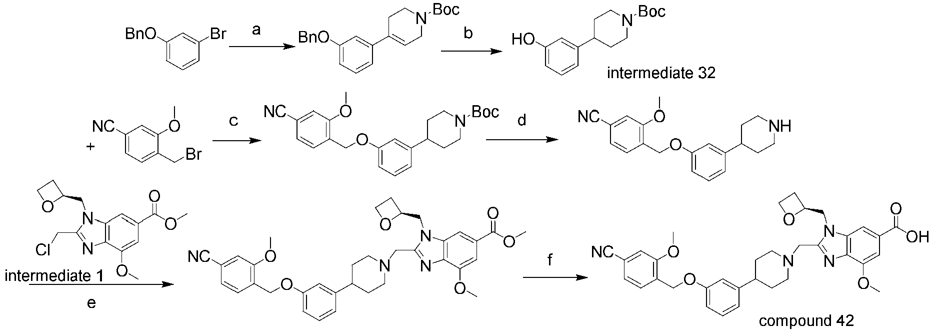

Tert-Butyl 4-(3-hydroxyphenyl)piperidine-1-carboxylate (Intermediate 32) was prepared according to the route shown in route F.

Step a: 1-(Benzyloxy)-3-bromobenzene (1.4 g, 5.4 mmol), N-Boc-1,2,5,6-tetrahydropyridine-4-boronic acid pinacol ester (2.1 g, 6.7 mmol), cesium carbonate (3.7 g, 11.2 mmol) and 1,1'-bis(diphenylphosphino)ferrocenepalladium dichloride (II) (205 mg, 0.28 mmol) were dissolved in 20 ml of 1,4-dioxane and 4 ml of water, and reacted at 100 °C for 3 hours under nitrogen protection. After the reaction was completed, the mixture was extracted three times with ethyl acetate and washed three times with saturated brine. The organic phase was dried over anhydrous magnesium sulfate and filtered. The filtrate was concentrated under reduced pressure. The crude product was separated by column chromatography to obtain 1.6 g of tert-butyl 4-(3-(benzyloxy)phenyl)-3,6-dihydropyridine-1(2H)-carboxylate. LCMS (ESI): m/z = 365.2 (M+H)⁺.

Step b: tert-Butyl 4-(3-(Benzyloxy)phenyl)-3,6-dihydropyridine-1(2H)-carboxylate (1.6 g, 4.3 mmol) was dissolved in 20 ml of ethyl acetate, 160 mg of 10% palladium carbon (55% water) was added, hydrogen was replaced, and the mixture was stirred at room temperature for 3 hours. After the reaction was completed, the solution was filtered and the filtrate was concentrated under reduced pressure to obtain 1.1 g of tert-butyl 4-(3-hydroxyphenyl)piperidine-1-carboxylate (Intermediate 32). LCMS (ESI): m/z = 277.2 (M+H)⁺.

(S)-2-((4-(3-((4-cyano-2-methoxybenzyl)oxy)phenyl)piperidin-1-yl)methyl)-4-methoxy-1-(oxetan-2-ylmethyl)-1H-benzo[d]imidazole-6-carboxylic acid (Compound 42) was prepared according to the route shown in route F.

Step c: tert-Butyl 4-(3-hydroxyphenyl)piperidine-1-carboxylate (Intermediate 32, 200 mg, 0.7 mmol) was dissolved in 5 ml of N,N-dimethylformamide, and cesium carbonate (456 mg, 1.4 mmol) and 4-(bromomethyl)-3-methoxybenzonitrile (225 mg, 1 mmol) were added, and the mixture was reacted at 70 °C for 2 hours. After the reaction was completed, the mixture was extracted three times with ethyl acetate and washed three times with saturated brine. The organic phase was dried over anhydrous magnesium sulfate and filtered. The filtrate was concentrated under reduced pressure. The crude product was separated by column chromatography to obtain 250 mg of tert-butyl 4-(3-((4-cyano-2-methoxybenzyl)oxy)phenyl)piperidine-1-carboxylate. LCMS (ESI): m/z = 422.2 (M+H)⁺.

Step d: tert-butyl 4-(3-((4-cyano-2-methoxybenzyl)oxy)phenyl)piperidine-1-carboxylate (250 mg, 0.6 mmol) was dissolved in 5 ml of dichloromethane, 0.5 ml of trifluoroacetic acid was added, and the mixture was stirred at room temperature for 2 hours. After the reaction was completed, the reaction solution was concentrated under reduced pressure, saturated sodium bicarbonate aqueous solution was added and extracted three times with dichloromethane, the organic phase was collected, dried over anhydrous magnesium sulfate, and filtered, and the filtrate was concentrated under reduced pressure to obtain 155 mg of 3-methoxy-4-((3-(piperidin-4-yl)phenoxy)methyl)benzonitrile. LCMS (ESI): m/z = 322.2 (M+H)⁺.

Step e: Methyl (S)-2-(Chloromethyl)-4-methoxy-1-(oxetan-2-ylmethyl)-1H-benzo[d]imidazole-6-carboxylate (Intermediate 1, 50 mg, 0.15 mmol), 3-methoxy-4-((3-(piperidin-4-yl)phenoxy)methyl)benzonitrile (Intermediate 32, 58 mg, 0.18 mmol), potassium carbonate (41 mg, 0.3 mmol) and a catalytic amount of potassium iodide were dissolved in 5 ml of acetonitrile and reacted at 80 °C for 2 hours. After the reaction was completed, the reaction mixture was filtered and the filtrate was concentrated under reduced pressure. The mixture was separated by preparative thin layer chromatography to obtain 65 mg of methyl (S)-2-((4-(3-((4-cyano-2-methoxybenzyl)oxy)phenyl)piperidin-1-yl)methyl)-4-methoxy-1-(oxetan-2-ylmethyl)-1H-benzo[d]imidazole-6-carboxylate. LCMS(ESI): m/z = 610.3 (M+H)⁺.

Step f: Methyl (S)-2-((4-(3-((4-cyano-2-methoxybenzyl)oxy)phenyl)piperidin-1-yl)methyl)-4-methoxy-1-(oxetan-2-ylmethyl)-1H-benzo[d]imidazole-6-carboxylate (65 mg, 0.11 mmol) was dissolved in 3 ml of 1,4-dioxane, 2 M NaOH (300 µL, 0.6 mmol) was added, and the reaction was carried out at 40 °C overnight. After the reaction was completed, the pH was adjusted to 5-6 with 2M hydrochloric acid, and preparative liquid separation was performed to obtain 50 mg of (S)-2-((4-(3-((4-cyano-2-methoxybenzyl)oxy)phenyl)piperidin-1-yl)methyl)-4-methoxy-1-(oxetan-2-ylmethyl)-1H-benzo[d]imidazole-6-carboxylic acid (Compound 42). LCMS (ESI): m/z = 596.3 (M+H)⁺.¹H NMR (500 MHz, DMSO) δ 7.91 (d, *J =* 1.3 Hz, 1H), 7.58 (d, *J =* 7.7 Hz, 1H), 7.54 (d, *J =* 1.5 Hz, 1H), 7.46 (dd, *J* = 7.7, 1.5 Hz, 1H), 7.26 (d, *J =* 1.3 Hz, 1H), 7.21 (t, *J =* 7.9 Hz, 1H), 6.88 (t, *J =* 2.0 Hz, 1H), 6.85 (d, *J =* 7.6 Hz, 1H), 6.81 (dd, *J =* 8.2, 2.5 Hz, 1H), 5.10 (s, 2H), 5.10 - 5.04 (m, 1H), 4.81 - 4.73 (m, 1H), 4.68 - 4.60 (m, 1H), 4.53 - 4.45 (m, 1H), 4.39 - 4.32 (m, 1H), 3.96 (s, 3H), 3.93 (d, *J =* 13.4 Hz, 1H), 3.90 (s, 3H), 3.78 (d, *J =* 13.4 Hz, 1H), 2.98 (d, *J =* 11.1 Hz, 1H), 2.86 (d, *J =* 11.1 Hz, 1H), 2.76 - 2.65 (m, 1H), 2.55 - 2.46 (m, 1H), 2.46 - 2.36 (m, 1H), 2.27 - 2.11 (m, 2H), 1.79 - 1.70 (m, 2H), 1.69 - 1.53 (m, 2H).

### Example 43: Preparation of (S)-2-((4-(3-((4-cyano-2,3-dihydrobenzofuran-7-yl)methoxy)phenyl)piperidin-1-yl)methyl)-4-methoxy-1-(oxetan-2-ylmethyl)-1H-benzo[d]imidazole-6-carboxylic acid (Compound 43)

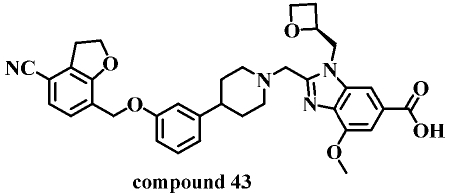

(S)-2-((4-(3-((4-cyano-2,3-dihydrobenzofuran-7-yl)methoxy)phenyl)piperidin-1-yl)methyl)-4-methoxy-1-(oxetan-2-ylmethyl)-1H-benzo[d]imidazole-6-carboxylic acid (Compound 43) was prepared according to the route shown in route F. The specific synthesis method was the same as that in Example 42, wherein the raw material 4-(bromomethyl)-3-methoxybenzonitrile was replaced by 7-(bromomethyl)-2,3-dihydrobenzofuran-4-carbonitrile. LCMS (ESI): m/z = 608.3 (M+H)⁺.¹H NMR (600 MHz, DMSO) δ 7.96 (d, *J =* 1.3 Hz, 1H), 7.45 (d, *J =* 7.9 Hz, 1H), 7.34 (d, *J =* 7.9 Hz, 1H), 7.32 (d, *J =* 1.3 Hz, 1H), 7.26 (t, *J =* 7.9 Hz, 1H), 6.94 (t, *J =* 2.2 Hz, 1H), 6.90 (d, *J =* 7.4 Hz, 1H), 6.87 (dd, *J =* 8.2, 2.5 Hz, 1H), 5.18 - 5.11 (m, 1H), 5.10 (s, 2H), 4.85 - 4.80 (m, 1H), 4.77 (t, *J =* 8.8 Hz, 2H), 4.74 - 4.66 (m, 1H), 4.57 - 4.52 (m, 1H), 4.45 - 4.38 (m, 1H), 4.01 (s, 3H), 3.97 (d, *J =* 13.4 Hz, 1H), 3.83 (d, *J =* 13.4 Hz, 1H), 3.47 (t, *J =* 8.8 Hz, 2H), 3.04 (d, *J =* 11.2 Hz, 1H), 2.92 (d, *J =* 11.3 Hz, 1H), 2.81 - 2.72 (m, 1H), 2.59 - 2.53 (m, 1H), 2.52 - 2.44 (m, 1H), 2.31 - 2.16 (m, 2H), 1.84 - 1.76 (m, 2H), 1.74 - 1.61 (m, 2H).

### Example 44: Preparation of (S)-2-((4-(2-((4-cyano-2-methoxybenzyl)oxy)phenyl)piperidin-1-yl)methyl)-4-methoxy-1-(oxetan-2-ylmethyl)-1H-benzo[d]imidazole-6-carboxylic acid (Compound 44)

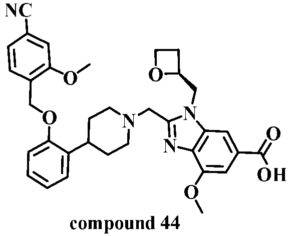

(S)-2-((4-(2-((4-cyano-2-methoxybenzyl)oxy)phenyl)piperidin-1-yl)methyl)-4-methoxy-1-(oxetan-2-ylmethyl)-1H-benzo[d]imidazole-6-carboxylic acid (Compound 44) was prepared according to the route shown in route F. The specific synthesis method is the same as Example 42. LCMS (ESI): m/z = 596.3 (M+H)⁺.¹H NMR (600 MHz, DMSO) δ 7.90 (d, *J =* 1.3 Hz, 1H), 7.57 (d, *J =* 7.7 Hz, 1H), 7.55 (d, *J =* 1.4 Hz, 1H), 7.50 (dd, *J =* 7.7, 1.5 Hz, 1H), 7.26 (d, *J =* 1.3 Hz, 1H), 7.20 (dd, *J =* 7.6, 1.7 Hz, 1H), 7.17 - 7.14 (m, 1H), 6.98 (d, *J =* 8.1 Hz, 1H), 6.93 (t, *J =* 7.5 Hz, 1H), 5.13 (s, 2H), 5.12 - 5.05 (m, 1H), 4.79 - 4.72 (m, 1H), 4.67 - 4.61 (m, 1H), 4.51 - 4.45 (m, 1H), 4.38 - 4.32 (m, 1H), 3.96 (s, 3H), 3.92 (d, *J =* 13.4 Hz, 1H), 3.90 (s, 3H), 3.78 (d, *J =* 13.4 Hz, 1H), 2.99 (d, *J* = 11.1 Hz, 1H), 2.96 - 2.91 (m, 1H), 2.87 (d, *J =* 11.2 Hz, 1H), 2.72 - 2.65 (m, 1H), 2.45 - 2.36 (m, 1H), 2.26 - 2.14 (m, 2H), 1.77 - 1.69 (m, 2H), 1.68 - 1.53 (m, 2H).

### Example 45: Preparation of (S)-2-((4-(2-((4-chloro-2,3-dihydrobenzofuran-7-yl)methoxy)phenyl)piperidin-1-yl)methyl)-4-methoxy-1-(oxetan-2-ylmethyl)-1H-benzo[d]imidazole-6-carboxylic acid (Compound 45)

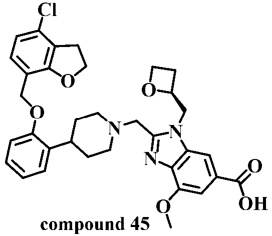

(S)-2-((4-(2-((4-chloro-2,3-dihydrobenzofuran-7-yl)methoxy)phenyl)piperidin-1-yl)methyl)-4-methoxy-1-(oxetan-2-ylmethyl)-1H-benzo[d]imidazole-6-carboxylic acid (Compound 45) was prepared according to the route shown in route F, and the specific synthesis method was the same as Example 42. LCMS (ESI): m/z = 617.2 (M+H)⁺.¹H NMR (600 MHz, DMSO) δ 7.89 (s, 1H), 7.27 (s, 1H), 7.21 (d, *J =* 8.2 Hz, 1H), 7.17 (dd, *J* = 7.8, 1.7 Hz, 1H), 7.16 - 7.12 (m, 1H), 7.02 (d, *J* = 8.2 Hz, 1H), 6.93 (d, *J* = 8.2 Hz, 1H), 6.90 (t, *J* = 7.4 Hz, 1H), 5.10 - 5.05 (m, 1H), 4.99 (s, 2H), 4.78 - 4.71 (m, 1H), 4.64 (t, *J* = 8.8 Hz, 2H), 4.63 - 4.59 (m, 1H), 4.51 - 4.45 (m, 1H), 4.37 - 4.31 (m, 1H), 3.95 (s, 3H), 3.90 (d, *J =* 13.4 Hz, 1H), 3.77 (d, *J =* 13.4 Hz, 1H), 3.25 (t, *J =* 8.7 Hz, 2H), 2.97 (d, *J* = 11.0 Hz, 1H), 2.93 - 2.87 (m, 1H), 2.85 (d, *J =* 11.3 Hz, 1H), 2.75 - 2.65 (m, 1H), 2.44 - 2.35 (m, 1H), 2.23 - 2.10 (m, 2H), 1.73 - 1.65 (m, 2H), 1.64 - 1.52 (m, 2H).

### Example 46: Preparation of (S)-2-((4-(2-((4-cyano-2,3-dihydrobenzofuran-7-yl)methoxy)phenyl)piperidin-1-yl)methyl)-4-methoxy-1-(oxetan-2-ylmethyl)-1H-benzo[d]imidazole-6-carboxylic acid (Compound 46)

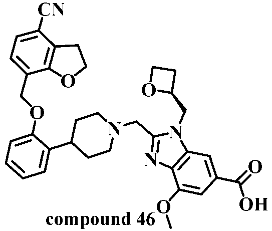

(S)-2-((4-(2-((4-cyano-2,3-dihydrobenzofuran-7-yl)methoxy)phenyl)piperidin-1-yl)methyl)-4-methoxy-1-(oxetan-2-ylmethyl)-1H-benzo[d]imidazole-6-carboxylic acid (Compound 46) was prepared according to the route shown in route F, and the specific synthesis method was the same as Example 42. LCMS (ESI): m/z = 608.2 (M+H)⁺.¹H NMR (600 MHz, DMSO) δ 7.90 (d, *J* = 1.2 Hz, 1H), 7.37 (d, *J* = 7.9 Hz, 1H), 7.32 (d, *J* = 7.9 Hz, 1H), 7.26 (d, *J =* 1.1 Hz, 1H), 7.19 (dd, *J =* 7.6, 1.7 Hz, 1H), 7.17 - 7.13 (m, 1H), 7.01 (d, *J =* 8.1 Hz, 1H), 6.92 (t, *J* = 7.4 Hz, 1H), 5.08 (s, 2H), 5.08 - 5.04 (m, 1H), 4.79 - 4.73 (m, 1H), 4.70 (t, *J =* 8.8 Hz, 2H), 4.66 - 4.60 (m, 1H), 4.52 - 4.45 (m, 1H), 4.38 - 4.31 (m, 1H), 3.96 (s, 3H), 3.91 (d, *J =* 13.5 Hz, 1H), 3.78 (d, *J =* 13.5 Hz, 1H), 3.42 (t, *J =* 8.8 Hz, 2H), 2.98 (d, *J =* 11.2 Hz, 1H), 2.95 - 2.90 (m, 1H), 2.86 (d, *J =* 11.2 Hz, 1H), 2.74 - 2.66 (m, 1H), 2.45 - 2.36 (m, 1H), 2.25 - 2.12 (m, 2H), 1.75 - 1.68 (m, 2H), 1.66 - 1.54 (m, 2H).

### Example 47: Preparation of (S)-2-((4-(2-((4-cyano-2-fluorobenzyl)oxy)phenyl)piperidin-1-yl)methyl)-4-methoxy-1-(oxetan-2-ylmethyl)-1H-benzo[d]imidazole-6-carboxylic acid (Compound 47)

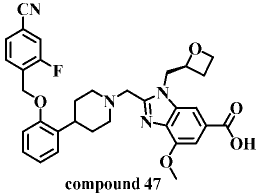

(S)-2-((4-(2-((4-cyano-2-fluorobenzyl)oxy)phenyl)piperidin-1-yl)methyl)-4-methoxy-1-(oxetan-2-ylmethyl)-1H-benzo[d]imidazole-6-carboxylic acid (Compound 47) was prepared according to the route shown in route F. The specific synthesis method is the same as Example 42. LCMS (ESI): m/z = 584.2 (M+H)⁺.¹H NMR (500 MHz, DMSO) δ 7.94 (dd, *J =* 9.9, 1.5 Hz, 1H), 7.90 (d, *J =* 1.3 Hz, 1H), 7.80 (dd, *J =* 7.9, 1.5 Hz, 1H), 7.74 (t, *J =* 7.5 Hz, 1H), 7.26 (d, *J =* 1.3 Hz, 1H), 7.21 (dd, *J* = 7.6, 1.7 Hz, 1H), 7.20 - 7.14 (m, 1H), 7.07 (dd, *J =* 8.3, 1.2 Hz, 1H), 6.95 (td, *J =* 7.5, 1.1 Hz, 1H), 5.26 (s, 2H), 5.12 - 5.03 (m, 1H), 4.79 - 4.71 (m, 1H), 4.66 - 4.59 (m, 1H), 4.52 - 4.44 (m, 1H), 4.38 - 4.31 (m, 1H), 3.95 (s, 3H), 3.91 (d, *J =* 13.5 Hz, 1H), 3.77 (d, *J =* 13.5 Hz, 1H), 2.98 (d, *J =* 11.0 Hz, 1H), 2.95 - 2.88 (m, 1H), 2.85 (d, *J =* 11.1 Hz, 1H), 2.74 - 2.67 (m, 1H), 2.45 - 2.36 (m, 1H), 2.26 - 2.11 (m, 2H), 1.75 - 1.66 (m, 2H), 1.65 - 1.51 (m, 2H).

### Example 48: Preparation of (S)-2-((4-(2-((4-chloro-2-fluorobenzyl)oxy)phenyl)piperidin-1-yl)methyl)-4-methoxy-1-(oxetan-2-ylmethyl)-1H-benzo[d]imidazole-6-carboxylic acid (Compound 48)

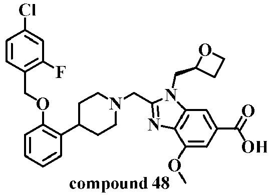

(S)-2-((4-(2-((4-chloro-2-fluorobenzyl)oxy)phenyl)piperidin-1-yl)methyl)-4-methoxy-1-(oxetan-2-ylmethyl)-1H-benzo[d]imidazole-6-carboxylic acid (Compound 48) was prepared according to the route shown in route F. The specific synthesis method is the same as Example 42. LCMS (ESI): m/z = 593.2 (M+H)⁺.¹H NMR (500 MHz, DMSO) δ 7.89 (s, 1H), 7.57 (t, *J =* 8.1 Hz, 1H), 7.52 (dd, *J* = 10.0, 2.1 Hz, 1H), 7.37 (dd, *J* = 8.3, 2.1 Hz, 1H), 7.26 (s, 1H), 7.21 - 7.12 (m, 2H), 7.08 (d, *J =* 8.0 Hz, 1H), 6.94 (t, *J =* 7.4 Hz, 1H), 5.14 (s, 2H), 5.10 - 5.03 (m, 1H), 4.78 - 4.70 (m, 1H), 4.65 - 4.57 (m, 1H), 4.52 - 4.44 (m, 1H), 4.38 - 4.30 (m, 1H), 3.95 (s, 3H), 3.89 (d, *J =* 13.5 Hz, 1H), 3.76 (d, *J =* 13.4 Hz, 1H), 2.95 (d, *J =* 11.1 Hz, 1H), 2.90 - 2.84 (m, 1H), 2.82 (d, *J =* 11.1 Hz, 1H), 2.74 - 2.59 (m, 1H), 2.44 - 2.34 (m, 1H), 2.21 - 2.08 (m, 2H), 1.72 - 1.53 (m, 4H).

### Example 49: Preparation of (S)-2-((4-(6-((4-chloro-2-methoxybenzyl)oxy)pyridin-2-yl)piperidin-1-yl)methyl)-4-methoxy-1-(oxetan-2-ylmethyl)-1H-benzo[d]imidazole-6-carboxylic acid (Compound 49)

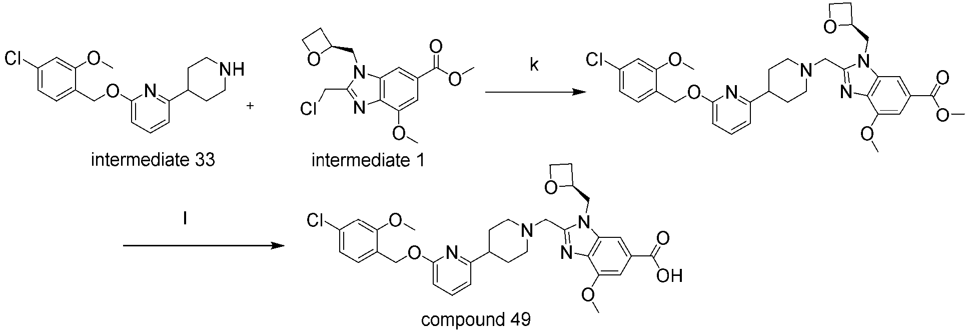

2-((4-chloro-2-methoxybenzyl)oxy)-6-(piperidin-4-yl)pyridine (Intermediate 33) was prepared according to the route shown in route B. The specific synthesis method was the same as that of Intermediate 2. LCMS (ESI): m/z = 332.1 (M+H)⁺.

(S)-2-((4-(6-((4-chloro-2-methoxybenzyl)oxy)pyridin-2-yl)piperidin-1-yl)methyl)-4-methoxy-1-(oxetan-2-ylmethyl)-1H-benzo[d]imidazole-6-carboxylic acid (Compound 49) was prepared according to the route shown in route B. The specific synthesis method was the same as Example 1, except that Intermediate 2 was replaced by Intermediate 33. LCMS (ESI): m/z = 606.2 (M+H)⁺.

### Example 50: Preparation of (S)-2-((4-(6-((4-chloro-2-methoxybenzyl)oxy)pyridin-2-yl)piperidin-1-yl)methyl)-4-ethoxy-1-(oxetan-2-ylmethyl)-1H-benzo[d]imidazole-6-carboxylic acid (Compound 50)

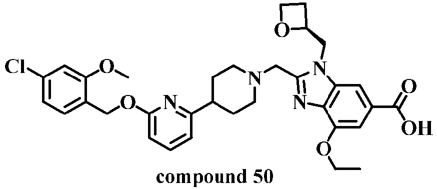

(S)-2-((4-(6-((4-chloro-2-methoxybenzyl)oxy)pyridin-2-yl)piperidin-1-yl)methyl)-4-ethoxy-1-(oxetan-2-ylmethyl)-1H-benzo[d]imidazole-6-carboxylic acid (Compound 50) was prepared according to the route shown in route B. The specific synthesis method was the same as Example 49, except that Intermediate 1 was replaced by Intermediate 3. LCMS (ESI): m/z = 620.2 (M+H)⁺.

### Example 51: Preparation of (S)-4-methoxy-2-((4-(6-((2-methoxy-4-(trifluoromethyl)benzyl)oxy)pyridin-2-yl)piperidin-1-yl)methyl)-1-(oxetan-2-ylmethyl)-1H-benzo[d]imidazole-6-carboxylic acid (Compound 51)

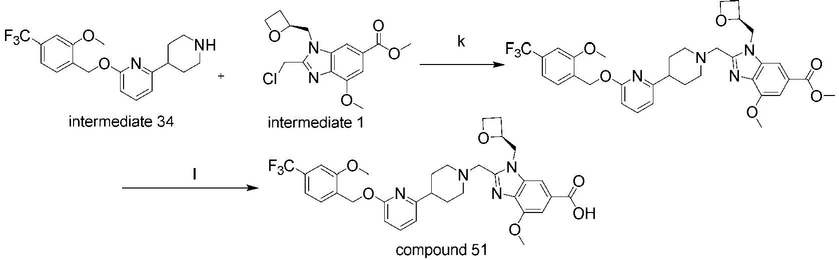

2-((2-methoxy-4-(trifluoromethyl)benzyl)oxy)-6-(piperidin-4-yl)pyridine (Intermediate 34) was prepared according to the route shown in route B. The specific synthesis method is the same as Intermediate 2. LCMS (ESI): m/z = 366.2 (M+H)⁺.

(S)-4-methoxy-2-((4-(6-((2-methoxy-4-(trifluoromethyl)benzyl)oxy)pyridin-2-yl)piperidin-1-yl)methyl)-1-(oxetan-2-ylmethyl)-1H-benzo[d]imidazole-6-carboxylic acid (Compound 51)) was prepared according to the route shown in route B. The specific synthesis method was the same as Example 1, except that Intermediate 2 was replaced by Intermediate 34. LCMS (ESI): m/z = 640.3 (M+H)⁺.

### Example 52: Preparation of (S)-4-ethoxy-2-((4-(6-((2-methoxy-4-(trifluoromethyl)benzyl)oxy)pyridin-2-yl)piperidin-1-yl)methyl)-1-(oxetan-2-ylmethyl)-1H-benzo[d]imidazole-6-carboxylic acid (Compound 52)

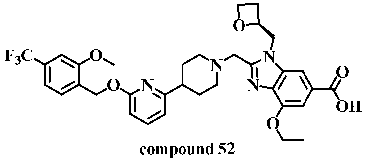

(S)-4-ethoxy-2-((4-(6-((2-methoxy-4-(trifluoromethyl)benzyl)oxy)pyridin-2-yl)piperidin-1-yl)methyl)-1-(oxetan-2-ylmethyl)-1H-benzo[d]imidazole-6-carboxylic acid (Compound 52) was prepared according to the route shown in route B. The specific synthesis method was the same as Example 51, except that Intermediate 1 was replaced by Intermediate 3. LCMS (ESI): m/z = 654.3 (M+H)⁺.

### Example 53: Preparation of 2-((4-(2-(2-fluoro-4-(trifluoromethyl)phenyl)-2-methylbenzo[d][1,3]dioxol-4-yl)piperidin-1-yl)methyl)-4-methoxy-1-(((S)-oxetan-2-yl)methyl)-1H-benzo[d]imidazole-6-carboxylic acid (Compound 53)

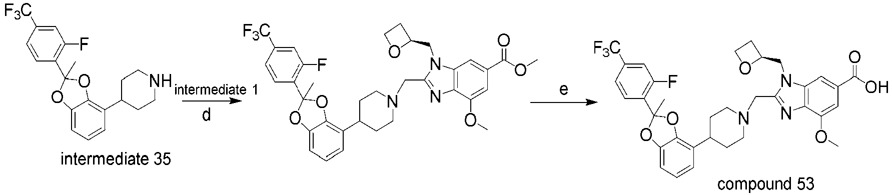

4-(2-(2-Fluoro-4-(trifluoromethyl)phenyl)-2-methylbenzo[d][1,3]dioxy-4-yl)piperidine (Intermediate 35) was prepared according to the route shown in route E. The specific synthesis method is the same as that of Intermediate 29. LCMS (ESI): m/z = 381.1 (M+H)⁺.

2-((4-(2-(2-fluoro-4-(trifluoromethyl)phenyl)-2-methylbenzo[d][1,3]dioxy-4-yl)piperidin-1-yl)methyl)-4-methoxy-1-(((S)-oxetan-2-yl)methyl)-1H-benzo[d]imidazole-6-carboxylic acid (Compound 53) was prepared according to the route shown in route E. The specific synthesis method was the same as Example 36, except that Intermediate 29 was replaced by Intermediate 35..LCMS (ESI): m/z = 655.2 (M+H)⁺.

### Example 54: Preparation of 4-ethoxy-2-((4-(2-(2-fluoro-4-(trifluoromethyl)phenyl)-2-methylbenzo[d][1,3]dioxol-4-yl)piperidin-1-yl)methyl)-1-(((S)-oxetan-2-yl)methyl)-1H-benzo[d]imidazole-6-carboxylic acid (Compound 54)

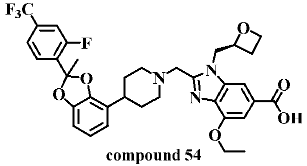

4-Ethoxy-2-((4-(2-(2-fluoro-4-(trifluoromethyl)phenyl)-2-methylbenzo[d][1,3]dioxy-4-yl)piperidin-1-yl)methyl)-1-(((S)-oxetan-2-yl)methyl)-1H-benzo[d]imidazole-6-carboxylic acid (Compound 54) was prepared according to the route shown in route E. The specific synthesis method was the same as Example 53, except that Intermediate 1 was replaced by Intermediate 3. LCMS (ESI): m/z = 669.3 (M+H)⁺.

### Example 55: Preparation of 4-methoxy-2-((4-(2-methyl-2-(5-(trifluoromethyl)pyridin-2-yl)benzo[d][1,3]dioxol-4-yl)piperidin-1-yl)methyl)-1-(((S)-oxetan-2-yl)methyl)-1H-benzo[d]imidazole-6-carboxylic acid (Compound 55)

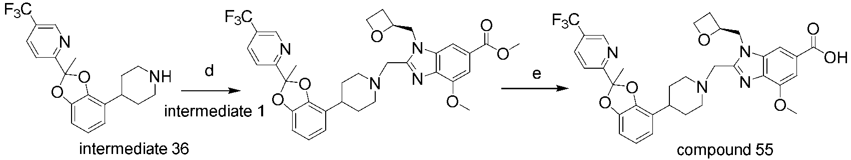

2-(2-Methyl-4-(piperidin-4-yl)benzo[d][1,3]dioxy-2-yl)-5-(trifluoromethyl)pyridine (Intermediate 36) was prepared according to the route shown in route E. The specific synthesis method is the same as that of Intermediate 30. LCMS (ESI): m/z = 364.1 (M+H)⁺.

4-Methoxy-2-((4-(2-methyl-2-(5-(trifluoromethyl)pyridin-2-yl)benzo[d][1,3]dioxy-4-yl)piperidin-1-yl)methyl)-1-(((S)-oxetan-2-yl)methyl)-1H-benzo[d]imidazole-6-carboxylic acid (Compound 55) was prepared according to the route shown in route E. The specific synthesis method was the same as Example 38, except that Intermediate 30 was replaced by Intermediate 36. LCMS (ESI): m/z = 638.2 (M+H)⁺.

### Example 56: Preparation of 4-ethoxy-2-((4-(2-methyl-2-(5-(trifluoromethyl)pyridin-2-yl)benzo[d][1,3]dioxol-4-yl)piperidin-1-yl)methyl)-1-(((S)-oxetan-2-yl)methyl)-1H-benzo[d]imidazole-6-carboxylic acid (Compound 56)

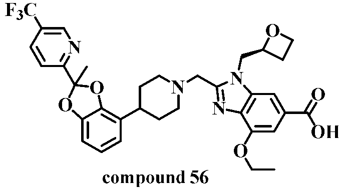

4-Ethoxy-2-((4-(2-methyl-2-(5-(trifluoromethyl)pyridin-2-yl)benzo[d][1,3]dioxy-4-yl)piperidin-1-yl)methyl)-1-(((S)-oxetan-2-yl)methyl)-1H-benzo[d]imidazole-6-carboxylic acid (Compound 56) was prepared according to the route shown in route E. The specific synthesis method was the same as Example 55, except that Intermediate 1 was replaced by Intermediate 3. LCMS (ESI): m/z = 652.3 (M+H)⁺.

### Example 57: Preparation of 2-((4-(2-(4-chloro-2-methoxyphenyl)-2-methylbenzo[d][1,3]dioxol-4-yl)piperidin-1-yl)methyl)-4-methoxy-1-(((S)-oxetan-2-yl)methyl)-1H-benzo[d]imidazole-6-carboxylic acid (Compound 57)

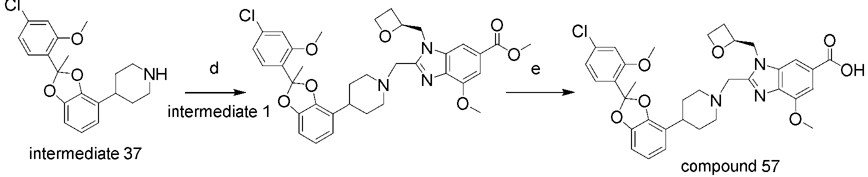

4-(2-(4-chloro-2-methoxyphenyl)-2-methylbenzo[d][1,3]dioxy-4-yl)piperidine (Intermediate 37) was prepared according to the route shown in route E. The specific synthesis method is the same as that of Intermediate 29. LCMS(ESI): m/z = 359.1 (M+H)⁺.

2-((4-(2-(4-chloro-2-methoxyphenyl)-2-methylbenzo[d] [1,3]dioxy-4-yl)piperidin-1-yl)methyl)-4-methoxy-1-(((S)-oxetan-2-yl)methyl)-1H-benzo[d]imidazole-6-carboxylic acid (Compound 57) was prepared according to the route shown in route E. The specific synthesis method was the same as Example 36, except that Intermediate 29 was replaced by Intermediate 37. LCMS(ESI): m/z = 633.2 (M+H)⁺.

### Example 58: Preparation of 2-((4-(2-(4-chloro-2-methoxyphenyl)-2-methylbenzo[d][1,3]dioxol-4-yl)piperidin-1-yl)methyl)-4-ethoxy-1-(((S)-oxetan-2-yl)methyl)-1H-benzo[d]imidazole-6-carboxylic acid (Compound 58)

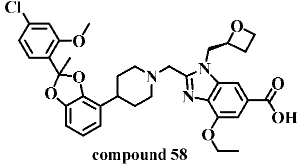

2-((4-(2-(4-chloro-2-methoxyphenyl)-2-methylbenzo[d][1,3]dioxy-4-yl)piperidin-1-yl)methyl)-4-ethoxy-1-(((S)-oxetan-2-yl)methyl)-1H-benzo[d]imidazole-6-carboxylic acid (Compound 58) was prepared according to the route shown in route E. The specific synthesis method was the same as Example 57, except that Intermediate 1 was replaced by Intermediate 3. LCMS(ESI): m/z = 647.2 (M+H)⁺.

### Example 59: Preparation of 4-methoxy-2-((4-(2-(2-methoxy-4-(trifluoromethyl)phenyl)-2-methylbenzo[d][1,3]dioxol-4-yl)piperidin-1-yl)methyl)-1-((S)-oxetan-2-yl)methyl]-1H-benzo[d]imidazole-6-carboxylic acid (Compound 59)

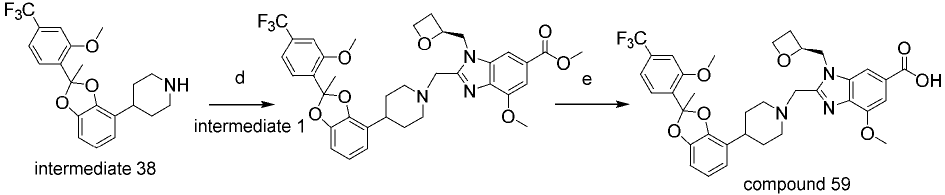

4-(2-(2-methoxy-4-(trifluoromethyl)phenyl)-2-methylbenzo[d][1,3]dioxy-4-yl)piperidine (Intermediate 38) was prepared according to the route shown in route E. The specific synthesis method is the same as that of Intermediate 29. LCMS(ESI): m/z = 393.2 (M+H)⁺.

4-methoxy-2-((4-(2-(2-methoxy-4-(trifluoromethyl)phenyl)-2-methylbenzo[d] [1,3]dioxy-4-yl)piperidin-1-yl)methyl)-1-((S)-oxetan-2-yl)methyl]-1H-benzo[d]imidazole-6-carboxylic acid (Compound 59) was prepared according to the route shown in route E, the specific synthesis method is the same as Example 36, except that intermediate 29 is replaced by intermediate 38. LCMS(ESI): m/z = 667.3 (M+H)⁺.

### Example 60: Preparation of 4-ethoxy-2-((4-(2-(2-methoxy-4-(trifluoromethyl)phenyl)-2-methylbenzo[d][1,3]dioxol-4-yl)piperidin-1-yl)methyl)-1-((S)-oxetan-2-yl)methyl]-1H-benzo[d]imidazole-6-carboxylic acid (Compound 60)

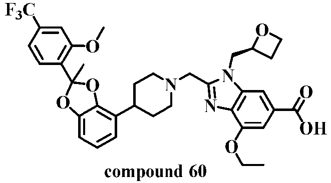

4-Ethoxy-2-((4-(2-(2-methoxy-4-(trifluoromethyl)phenyl)-2-methylbenzo[d][1,3]dioxy-4-yl)piperidin-1-yl)methyl)-1-((S)-oxetan-2-yl)methyl]-1H-benzo[d]imidazole-6-carboxylic acid (Compound 60) was prepared according to the route shown in route E. The specific synthesis method was the same as Example 59, except that Intermediate 1 was replaced by Intermediate 3. LCMS(ESI): m/z = 681.3 (M+H)⁺.

### Example 61: Preparation of 2-((4-(2-(5-chloropyridin-2-yl)-2-methylbenzo[d][1,3]dioxol-4-yl)piperidin-1-yl)methyl)-4-(methoxy-d3)-1-(((S)-oxetan-2-yl)methyl)-1H-benzo[d]imidazole-6-carboxylic acid (Compound 61)

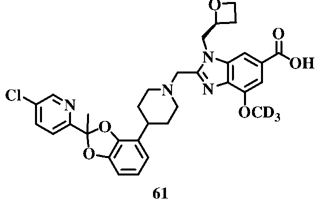

2-((4-(2-(5-chloropyridin-2-yl)-2-methylbenzo[d][1,3]dioxol-4-yl)piperidin-1-yl)methyl)-4-(methoxy-d3)-1-(((S)-oxetan-2-yl)methyl)-1H-benzo[d]imidazole-6-carboxylic acid (Compound 61) was prepared according to the route shown in route E. LCMS(ESI): m/z = 607.3 (M+H)⁺.

### Example 62: Preparation of 2-((4-(2-(5-chloropyridin-2-yl)-2-methylbenzo[d][1,3]dioxol-4-yl)piperidin-1-yl)methyl)-4-(difluoromethoxy)-1-(((S)-oxetan-2-yl)methyl)-1H-benzo[d]imidazole-6-carboxylic acid (Compound 62)

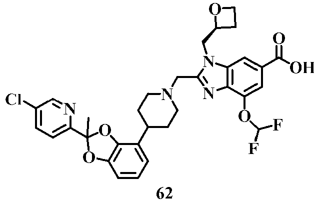

2-((4-(2-(5-chloropyridin-2-yl)-2-methylbenzo[d][1,3]dioxol-4-yl)piperidin-1-yl)methyl)-4-(difluoromethoxy)-1-(((S)-oxetan-2-yl)methyl)-1H-benzo[d]imidazole-6-carboxylic acid (Compound 62) was prepared according to the route shown in route E. LCMS(ESI): m/z = 640.2 (M+H)⁺.

### Example 63: Preparation of 2-((4-(2-(4-chloro-2-fluorophenyl)-2-methylbenzo[d][1,3]dioxol-4-yl)-5,6-dihydro-1,2,4-triazin-1(4H)-yl)methyl)-4-methoxy-1-(((S)-oxetan-2-yl)methyl)-1H-benzo[d]imidazole-6-carboxylic acid (Compound 63)

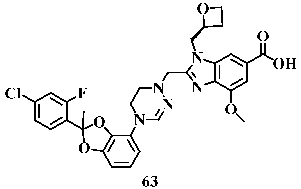

2-((4-(2-(4-Chloro-2-fluorophenyl)-2-methylbenzo[d][1,3]dioxol-4-yl)-5,6-dihydro-1,2,4-triazin-1(4H)-yl)methyl)-4-methoxy-1-(((S)-oxetan-2-yl)methyl)-1H-benzo[d]imidazole-6-carboxylic acid (Compound 63) was prepared according to the route shown in route G. LCMS(ESI): m/z = 621.2 (M+H)⁺.

### Example 64: Preparation of 2-((4-(2-(5-chloropyridin-2-yl)-2-methylbenzo[d][1,3]dioxol-4-yl)piperidin-1-yl)methyl)-1-(((S)-oxetan-2-yl)methyl)-4-(2,2,2-trifluoroethoxy)-1H-benzo[d]imidazole-6-carboxylic acid (Compound 64)

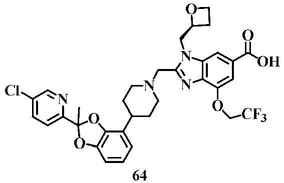

2-((4-(2-(5-chloropyridin-2-yl)-2-methylbenzo[d][1,3]dioxol-4-yl)piperidin-1-yl)methyl)-1-(((S)-oxetan-2-yl)methyl)-4-(2,2,2-trifluoroethoxy)-1H-benzo[d]imidazole-6-carboxylic acid (Compound 64) was prepared according to the route shown in route E. LCMS(ESI): m/z = 672.2 (M+H)⁺.

### Example 65: Preparation of 4-methoxy-2-((4-(2-methyl-2-(5-(trifluoromethyl)pyridin-2-yl)benzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 65)

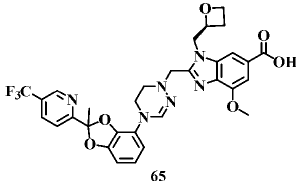

4-Methoxy-2-((4-(2-methyl-2-(5-(trifluoromethyl)pyridin-2-yl)benzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 65) was prepared according to the route shown in route G. LCMS(ESI): m/z = 638.2 (M+H)⁺.

### Example 66: Preparation of 4-ethoxy-2-((4-(2-methyl-2-(5-(trifluoromethyl)pyridin-2-yl)benzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 66)

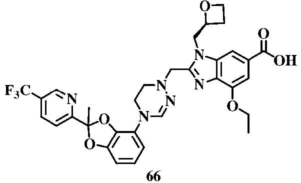

4-Ethoxy-2-((4-(2-methyl-2-(5-(trifluoromethyl)pyridin-2-yl)benzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 66) was prepared according to the route shown in route G. LCMS(ESI): m/z = 652.2 (M+H)⁺.

### Example 67: Preparation of 4-(methoxy-d3)-2-((4-(2-methyl-2-(5-(trifluoromethyl)pyridin-2-yl)benzo[d][1,3]dioxol-4-yl)piperidin-1-yl)methyl)-1-(((S)-oxetan-2-yl)methyl)-1H-benzo[d]imidazole-6-carboxylic acid (Compound 67)

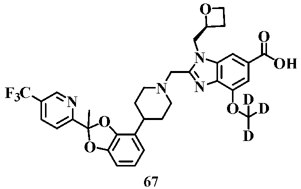

4-(Methoxy-d3)-2-((4-(2-methyl-2-(5-(trifluoromethyl)pyridin-2-yl)benzo[d][1,3]dioxol-4-yl)piperidin-1-yl)methyl)-1-(((S)-oxetan-2-yl)methyl)-1H-benzo[d]imidazole-6-carboxylic acid (Compound 67) was prepared according to the route shown in route E. LCMS(ESI): m/z = 641.3 (M+H)⁺.

### Example 68: Preparation of 2-((4-(2-(4-chloro-2-fluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 68)

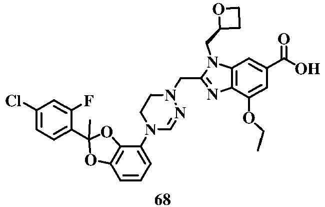

2-((4-(2-(4-Chloro-2-fluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 68) was prepared according to the route shown in route G. LCMS(ESI): m/z = 635.2 (M+H)⁺.

### Preparation of 2-((4-((S)-2-(4-chloro-2-fluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 68-1) and 2-((4-((R)-2-(4-chloro-2-fluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 68-2):

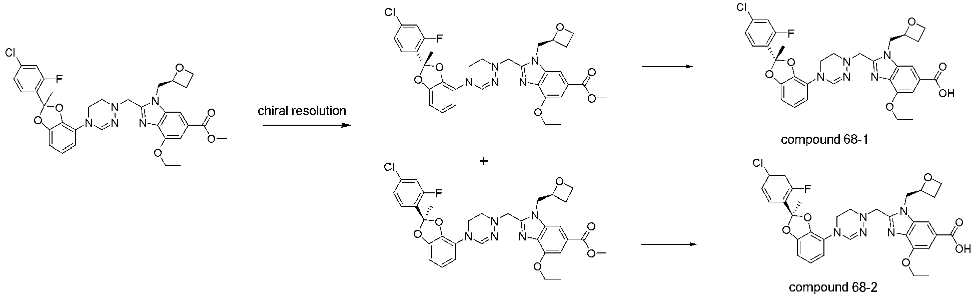

The chiral separation and preparation methods of compounds 68-1 and 68-2 are the same as those of the following compounds 76-1 and 76-2.

### Example 69: Preparation of 2-((4-(2-(5-chloropyridin-2-yl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 69)

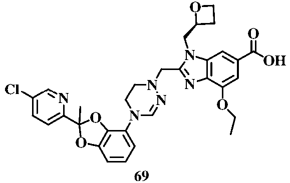

2-((4-(2-(5-chloropyridin-2-yl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 69) was prepared according to the route shown in route G. LCMS(ESI): m/z = 618.2 (M+H)⁺.

### Example 70: Preparation of 2-((4-(2-(5-chloropyridin-2-yl)-2-methylbenzo[d][1,3]dioxol-4-yl)-5,6-dihydro-1,2,4-triazin-1(4H)-yl)methyl)-4-(methoxy-d3)-1-(((S)-oxetan-2-yl)methyl)-1H-benzo[d]imidazole-6-carboxylic acid (Compound 70)

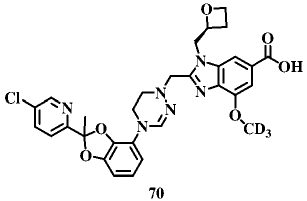

2-((4-(2-(5-chloropyridin-2-yl)-2-methylbenzo[d][1,3]dioxol-4-yl)-5,6-dihydro-1,2,4-triazin-1(4H)-yl)methyl)-4-(methoxy-d3)-1-(((S)-oxetan-2-yl)methyl)-1H-benzo[d]imidazole-6-carboxylic acid (Compound 70) was prepared according to the route shown in route G. LCMS(ESI): m/z = 607.2 (M+H)⁺.

### Example 71: Preparation of 2-((4-(2-(4-chloro-2-fluorophenyl)-2-methylbenzo[d][1,3]dioxol-4-yl)-5,6-dihydro-1,2,4-triazin-1(4H)-yl)methyl)-4-(methoxy-d3)-1-(((S)-oxetan-2-yl)methyl)-1H-benzo[d]imidazole-6-carboxylic acid (Compound 71)

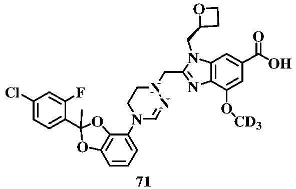

2-((4-(2-(4-Chloro-2-fluorophenyl)-2-methylbenzo[d][1,3]dioxol-4-yl)-5,6-dihydro-1,2,4-triazin-1(4H)-yl)methyl)-4-(methoxy-d3)-1-(((S)-oxetan-2-yl)methyl)-1H-benzo[d]imidazole-6-carboxylic acid (Compound 71) was prepared according to the route shown in route G. LCMS(ESI): m/z = 624.2 (M+H)⁺.

### Example 72: Preparation of 2-((4-(2-(5-chloropyridin-2-yl)-2-methylbenzo[d][1,3]dioxol-4-yl)piperidin-1-yl)methyl)-4-(ethoxy-d5)-1-(((S)-oxetan-2-yl)methyl)-1H-benzo[d]imidazole-6-carboxylic acid (Compound 72)

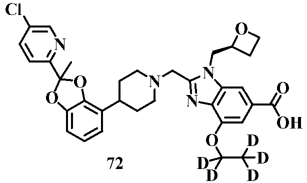

2-((4-(2-(5-chloropyridin-2-yl)-2-methylbenzo[d][1,3]dioxol-4-yl)piperidin-1-yl)methyl)-4-(ethoxy-d5)-1-(((S)-oxetan-2-yl)methyl)-1H-benzo[d]imidazole-6-carboxylic acid (Compound 72) was prepared according to the route shown in route E. LCMS(ESI): m/z = 623.3 (M+H)⁺.

### Example 73: Preparation of 2-((4-(2-(5-chloropyridin-2-yl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 73)

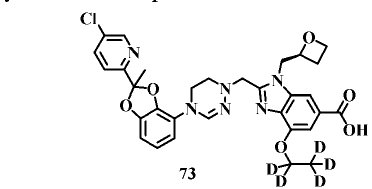

2-((4-(2-(5-chloropyridin-2-yl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 73) was prepared according to the route shown in route G. LCMS(ESI): m/z = 623.3 (M+H)⁺.

### Example 74: Preparation of 2-((4-(2-(4-chloro-2-fluorophenyl)-2-methylbenzo[d][1,3]dioxol-4-yl)piperidin-1-yl)methyl)-4-(methoxy-d3)-1-(((S)-oxetan-2-yl)methyl)-1H-benzo[d]imidazole-6-carboxylic acid (Compound 74)

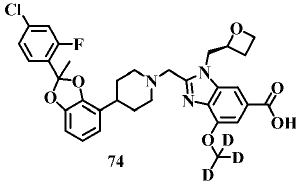

2-((4-(2-(4-Chloro-2-fluorophenyl)-2-methylbenzo[d][1,3]dioxol-4-yl)piperidin-1-yl)methyl)-4-(methoxy-d3)-1-(((S)-oxetan-2-yl)methyl)-1H-benzo[d]imidazole-6-carboxylic acid (Compound 74) was prepared according to the route shown in route E. LCMS(ESI): m/z = 624.3 (M+H)⁺.

### Example 75: Preparation of 2-((4-(2-(4-chloro-2-fluorophenyl)-2-methylbenzo[d][1,3]dioxol-4-yl)piperidin-1-yl)methyl)-4-(ethoxy-d5)-1-(((S)-oxetan-2-yl)methyl)-1H-benzo[d]imidazole-6-carboxylic acid (Compound 75)

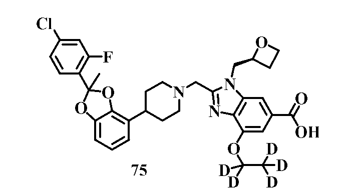

2-((4-(2-(4-Chloro-2-fluorophenyl)-2-methylbenzo[d] [1,3]dioxol-4-yl)piperidin-1-yl)methyl)-4-(ethoxy-d5)-1-(((S)-oxetan-2-yl)methyl)-1H-benzo[d]imidazole-6-carboxylic acid (Compound 75) was prepared according to the route shown in route E. LCMS(ESI): m/z = 640.3 (M+H)⁺.

### Example 76: Preparation of 2-((4-(2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 76)

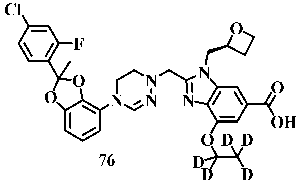

2-((4-(2-(4-Chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 76) was prepared according to the route shown in route G. LCMS(ESI): m/z = 640.3 (M+H)⁺.

### Preparation of 2-((4-((S)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 76-1) and 2-((4-((R)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 76-2):

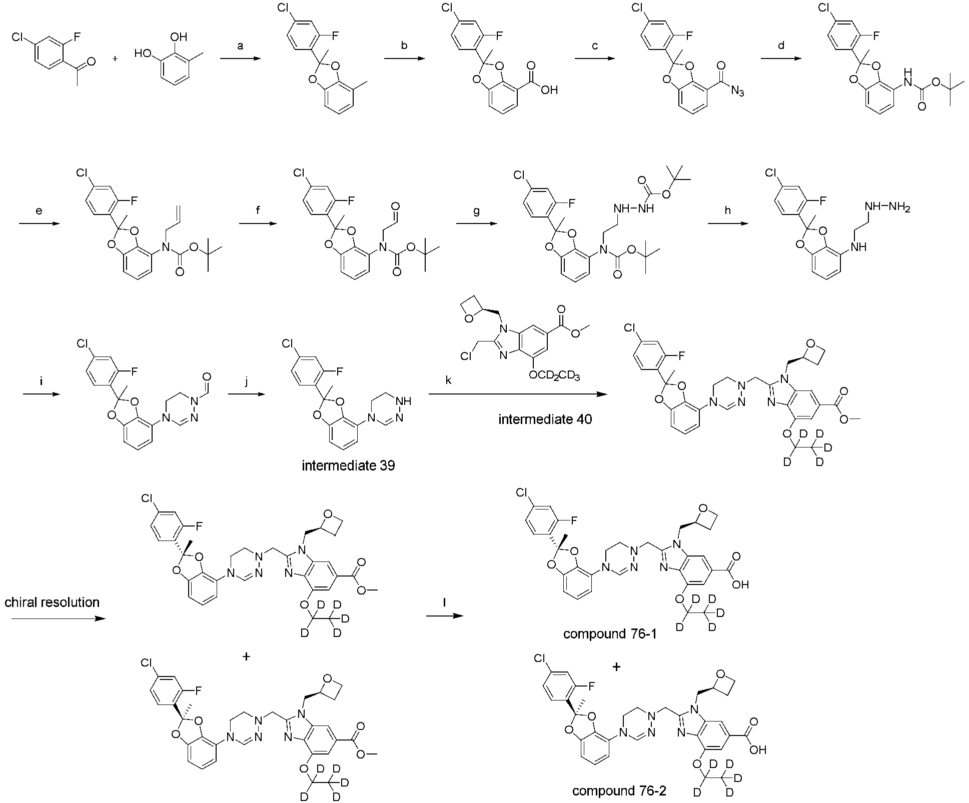

Step a: 4'-chloro-2'-fluoroacetophenone (20 g, 116.2 mmol), 3-methylbenzene-1,2-diol (14.4 g, 116.2 mmol) and p-toluenesulfonic acid (2 g, 11.6 mmol) were dissolved in 500 ml of toluene, protected by nitrogen and a water separator was used. The mixture was reacted at 160 °C for 72 hours. After the reaction was completed, the reaction solution was concentrated under reduced pressure, and the crude product was separated by column chromatography to obtain 25 g of 2-(4-chloro-2-fluorophenyl)-2,4-dimethylbenzo[d][1,3]dioxole. LCMS(ESI): m/z = 278.1 (M+H)⁺.

Step b: 2-(4-chloro-2-fluorophenyl)-2,4-dimethylbenzo[d][1,3]dioxole (25 g, 89.9 mmol) was dissolved in a mixture of 250 ml of pyridine and 250 ml of water. After heating to 80 °C, potassium permanganate (85 g, 539.4 mmol) was added in portions and stirred overnight. After the reaction is completed, the insoluble solid is filtered out, the filtrate is diluted with ethyl acetate and pyridine is washed out with 2M hydrochloric acid, the organic phase is collected and dried over anhydrous magnesium sulfate and filtered, the filtrate is concentrated under reduced pressure, and the crude product is separated by column chromatography to obtain 16 g of 2-(4-chloro-2-fluorophenyl)-2-methylbenzo[d][1,3]dioxole-4-carboxylic acid. LCMS(ESI): m/z = 308.0 (M+H)⁺.

Step c: 2-(4-Chloro-2-fluorophenyl)-2-methylbenzo[d][1,3]dioxole-4-carboxylic acid (16 g, 51.9 mmol) was dissolved in 250 ml of dichloromethane, triethylamine (15.8 g, 155.7 mmol) was added, and diphenylphosphoryl azide (18.6 g, 67.5 mmol) was added dropwise under nitrogen protection, and the mixture was stirred at room temperature for 2 hours. After the reaction was completed, the reaction solution was concentrated under reduced pressure at room temperature, and the crude product was separated by column chromatography to obtain 17 g of 2-(4-chloro-2-fluorophenyl)-2-methylbenzo[d][1,3]dioxole-4-carbonyl azide. LCMS(ESI): m/z = 333.0 (M+H)⁺.

Step d: 2-(4-chloro-2-fluorophenyl)-2-methylbenzo[d][1,3]dioxole-4-carbonyl azide (17 g, 51.1 mmol) and 150 ml of ultra-dry tert-butanol were added into a pressure tube, protected by nitrogen, and reacted at 90° C. overnight. After the reaction was completed, the reaction solution was concentrated under reduced pressure, and the crude product was separated by column chromatography to obtain 18 g of tert-butyl (2-(4-chloro-2-fluorophenyl)-2-methylbenzo[d][1,3]dioxol-4-yl)carbamate. LCMS(ESI): m/z = 379.1 (M+H)⁺.

Step e: tert-butyl (2-(4-chloro-2-fluorophenyl)-2-methylbenzo[d][1,3]dioxol-4-yl)carbamate (18 g, 47.5 mmol) was dissolved in 100 ml of N,N-dimethylformamide, cesium carbonate (31 g, 95 mmol) and 3-bromopropylene (11.5 g, 95 mmol) were added, and the reaction was carried out at 70 °C for 4 hours. After the reaction was completed, the mixture was extracted with ethyl acetate three times, washed with saturated brine three times, the organic phase was dried over anhydrous magnesium sulfate and filtered, the filtrate was concentrated under reduced pressure, and the crude product was separated by column chromatography to obtain 18.5 g of tert-butyl allyl (2-(4-chloro-2-fluorophenyl)-2-methylbenzo[d][1,3]dioxol-4-yl)carbamate. LCMS(ESI): m/z = 419.2 (M+H)⁺.

Step f: tert-butyl allyl(2-(4-chloro-2-fluorophenyl)-2-methylbenzo[d][1,3]dioxol-4-yl)carbamate (18.5 g, 44.1 mmol) was dissolved in a mixed solvent of 200 ml of tetrahydrofuran and 200 ml of water, potassium osmate (VI) dihydrate (162 mg, 0.44 mmol) and sodium periodate (37.7 g, 176.4 mmol) were added, and the mixture was stirred at room temperature for 1 hour. After the reaction was completed, the reaction mixture was filtered, the filtrate was diluted with EA and washed with a saturated aqueous sodium thiosulfate solution, the organic phase was collected and dried over anhydrous magnesium sulfate and filtered, the filtrate was concentrated under reduced pressure, and the crude product was separated by column chromatography to obtain 14 g of tert-butyl (2-(4-chloro-2-fluorophenyl)-2-methylbenzo[d][1,3]dioxol-4-yl)(2-oxoethyl)carbamate. LCMS(ESI): m/z = 421.1 (M+H)⁺.

Step g: tert-butyl (2-(4-chloro-2-fluorophenyl)-2-methylbenzo[d][1,3]dioxol-4-yl)(2-oxoethyl)carbamate (14 g, 33.2 mmol) was dissolved in 200 ml of ultra-dry 1,2-dichloroethane, tert-butyl carbazate (8.8 g, 66.4 mmol) wan added and stirred at room temperature for 1 hour, then sodium triacetoxyborohydride (14 g, 66.4 mmol) and sodium cyanoborohydride (6.3 g, 99.6 mmol) were added and reacted at room temperature for 1 hour. After the reaction was completed, the reaction mixture was quenched with a saturated aqueous sodium bicarbonate solution, extracted three times with dichloromethane. The organic phase was dried over anhydrous magnesium sulfate and then filtered, the filtrate was concentrated under reduced pressure, and the crude product was separated by column chromatography to obtain 15.5 g of tert-butyl 2-(2-((tert-butoxycarbonyl)(2-(4-chloro-2-fluorophenyl)-2-methylbenzo[d] [1,3]dioxol-4-yl)amino)ethyl)hydrazine-1-carboxylate. LCMS (ESI): m/z = 537.2 (M+H)⁺.

Step h: tert-butyl 2-(2-((tert-butoxycarbonyl)(2-(4-chloro-2-fluorophenyl)-2-methylbenzo[d][1,3]dioxol-4-yl)amino)ethyl)hydrazine-1-carboxylate (15.5 g, 28.9 mmol) was dissolved in 200 ml of dichloromethane, 4M hydrogen chloride-1,4-dioxane solution (58 mL, 231.2 mmol) was added dropwise, and stirred at room temperature for 4 hours. After the reaction was completed, the reaction solution was concentrated under reduced pressure to obtain 2-(4-chloro-2-fluorophenyl)-N-(2-hydrazinoethyl)-2-methylbenzo[d][1,3]dioxole-4-amine hydrochloride. LCMS(ESI): m/z = 337.1 (M+H)⁺.

Step i: 2-(4-chloro-2-fluorophenyl)-N-(2-hydrazinoethyl)-2-methylbenzo[d][1,3]dioxol-4-amine hydrochloride was dissolved in 100 ml of acetic acid, 50 ml of trimethyl orthoformate was added, and the mixture was reacted at 100 °C for 3 hours under nitrogen protection. After the reaction was completed, the reaction solution was concentrated under reduced pressure, and the crude product was separated by column chromatography to obtain 7.6 g of 4-(2-(4-chloro-2-fluorophenyl)-2-methylbenzo[d][1,3]dioxol-4-yl)-5,6-dihydro-1,2,4-triazine-1(4H)-carbaldehyde. LCMS(ESI): m/z = 375.1 (M+H)⁺.

Step j: 4-(2-(4-chloro-2-fluorophenyl)-2-methylbenzo[d][1,3]dioxol-4-yl)-5,6-dihydro-1,2,4-triazine-1(4H)-carbaldehyde (7.6 g, 20.3 mmol) was dissolved in 105 ml of a hydrolysis solution of methanol:tetrahydrofuran:water (3:3:1), sodium hydroxide (8.1 g, 203 mmol) was added, and the reaction was carried out at room temperature for 6 hours. After the reaction was completed, 100 ml of saturated ammonium chloride solution was poured into the reaction mixture, and the mixture was extracted with dichloromethane three times. The organic phase was dried over anhydrous magnesium sulfate and filtered. The filtrate was concentrated under reduced pressure. The crude product was separated by column chromatography to obtain 6.5 g of 4-(2-(4-chloro-2-fluorophenyl)-2-methylbenzo[d][1,3]dioxol-4-yl)-1,4,5,6-tetrahydro-1,2,4-triazine (Intermediate 39). LCMS(ESI): m/z = 347.1 (M+H)⁺.

Step k: 4-(2-(4-chloro-2-fluorophenyl)-2-methylbenzo[d][1,3]dioxol-4-yl)-1,4,5,6-tetrahydro-1,2,4-triazine (Intermediate 39, 6.5 g, 18.7 mmol), methyl (S)-2-(chloromethyl)-4-(ethoxy-d5)-1-(oxetan-2-ylmethyl)-1H-benzo[d]imidazole-6-carboxylate (Intermediate 40, 7.1 g, 20.6 mmol), potassium carbonate (5.2 g, 37.4 mmol) and a catalytic amount of potassium iodide were dissolved in 100 ml of acetonitrile and reacted at 80 °C for 2 hours. After the reaction was completed, the reaction mixture was filtered and the filtrate was concentrated under reduced pressure. The crude product was separated by column chromatography to obtain 10 g of methyl 2-((4-(2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-((S)-oxetan-2-yl)methyl)-1H-benzo[d]imidazole-6-carboxylate. LCMS(ESI): m/z = 654.3 (M+H)⁺.

A preparative liquid phase (Shimadzu, model: LC-20AD) and a CHIRALPAK IA (IA00CE-VD015) chiral chromatographic column (Shanghai Daicel, column size: 0.46 cm I.D. × 25 cm L) was used to separate the compound methyl 2-((4-(2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-((S)-oxetan-2-yl)methyl)-1H-benzo[d]imidazole-6-carboxylate, eluted with n-hexane/ethanol (60/40, V/V) as the mobile phase (flow rate: 1.0 ml/min; detection wavelength: 214 nm). The corresponding components were collected, and the solvent was removed by rotary evaporation to obtain two enantiomers methyl 2-((4-(((S)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-((S)-oxetan-2-yl)methyl)-1H-benzo[d]imidazole-6-carboxylate and methyl 2-((4-(((R)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylate, with retention times of 10.14 and 14.59 minutes, respectively. After detection, the ee values were both greater than 98%.

Step 1: methyl 2-((4-(((S)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylate (4.5 g, 6.9 mmol) was dissolved in 100 ml of 1,4-dioxane, 2M NaOH (14 mL, 27.6 mmol) was added and the reaction was carried out at 40 °C overnight. After the reaction was completed, the pH was adjusted to 5-6 with 2M hydrochloric acid, and 3.5 g of ammonium salt of compound 76-1 was obtained by preparative liquid separation (mobile phase: 0.1% ammonia water-acetonitrile). LCMS (ESI): m/z = 640.3 (M+H)⁺.

The ammonium salt of 76-1 was dissolved in a mixed solvent of DCM:MeOH (10:1), and 2M hydrochloric acid was added dropwise under ice bath to adjust the pH to 4-5. The organic phase was collected, dried and filtered, and the filtrate was concentrated under reduced pressure to obtain the prototype of 76-1.

Methyl 2-((4-(((R)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylate (4.6 g, 6.9 mmol) was dissolved in 100 mL of 1,4-dioxane, 2M NaOH (14 mL, 27.6 mmol) was added and the reaction was carried out at 40 °C overnight. After the reaction was completed, the pH was adjusted to 5-6 with 2M hydrochloric acid, and 3.5 g of ammonium salt of compound 76-2 was obtained by preparative liquid separation (mobile phase: 0.1% ammonia water-acetonitrile). LCMS(ESI): m/z = 640.3 (M+H)⁺.

The prototype of compound 76-2 was prepared in the same manner as compound 76-1.

### Preparation of tris(hydroxymethyl)aminomethane (Tris) salt of 2-((4-((S)-2-(4-chloro-2-fluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 76-1):

The prototype 76-1 and tris(hydroxymethyl)aminomethane were dissolved in methanol at a molar ratio of 1:1, and the mixture was concentrated under reduced pressure at 45 °C after ultrasonication for 10 minutes. The Tris salt of 76-1 was obtained after freeze-drying.

### Example 77: Preparation of 2-((4-(2-(4-chloro-2,3-difluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 77)

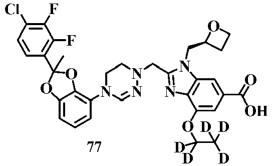

2-((4-(2-(4-Chloro-2,3-difluorophenyl)-2-methylbenzo[d] [1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 77) was prepared according to the route shown in route G. LCMS(ESI): m/z = 658.3 (M+H)⁺.

### Example 78: Preparation of 2-((4-(2-(4-chloro-2,3-difluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 78)

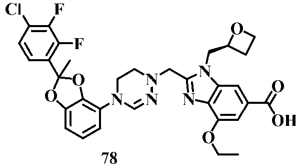

2-((4-(2-(4-Chloro-2,3-difluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 78) was prepared according to the route shown in route G. LCMS(ESI): m/z = 653.2 (M+H)⁺.

### Example 79: Preparation of 2-((4-(2-(4-chloro-2,5-difluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 79)

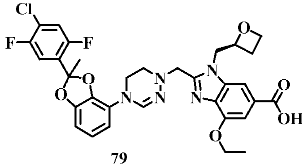

2-((4-(2-(4-Chloro-2,5-difluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 79) was prepared according to the route shown in route G. LCMS(ESI): m/z = 653.2 (M+H)⁺.

### Example 80: Preparation of 2-((4-(2-(4-chloro-2,5-difluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 80)

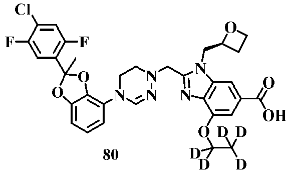

2-((4-(2-(4-Chloro-2,5-difluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 80) was prepared according to the route shown in route G. LCMS(ESI): m/z = 658.3 (M+H)⁺.

### Example 81: Preparation of 4-ethoxy-2-((4-(2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 81)

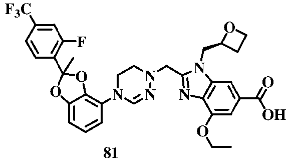

4-Ethoxy-2-((4-(2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 81) was prepared according to the route shown in route G. LCMS(ESI): m/z = 669.3 (M+H)⁺.

### Preparation of 4-ethoxy-2-((4-((S)-2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 81-1) and 4-ethoxy-2-((4-((R)-2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 81-2):

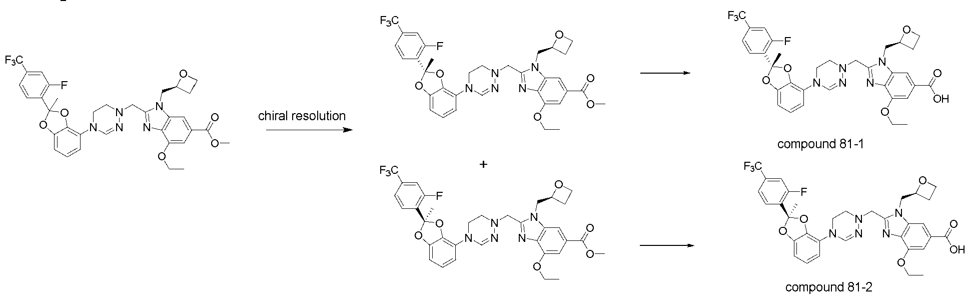

The chiral separation and preparation methods of compounds 81-1 and 81-2 are the same as those of compounds 76-1 and 76-2.

### Preparation of Tris salt of 4-ethoxy-2-((4-((S)-2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 81-1)

The prototype 81-1 and tris(hydroxymethyl)aminomethane were dissolved in methanol at a molar ratio of 1:1, and the mixture was concentrated under reduced pressure at 45°C after ultrasonication for 10 minutes. The Tris salt of 81-1 was obtained after freeze-drying.

### Example 82: Preparation of 4-(ethoxy-d5)-2-((4-(2-(2-fluoro-4-(trifluoromethyl)phenyl)-2-methylbenzo[d][1,3]dioxol-4-yl)-5,6-dihydro-1,2,4-triazin-1(4H)-yl)methyl)-1-((((S)-oxetan-2-yl)methyl)-1H-benzo[d]imidazole-6-carboxylic acid (Compound 82)

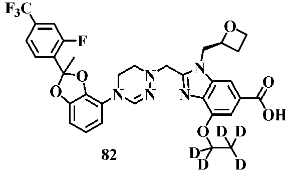

4-(Ethoxy-d5)-2-((4-(2-(2-fluoro-4-(trifluoromethyl)phenyl)-2-methylbenzo[d][1,3]dioxol-4-yl)-5,6-dihydro-1,2,4-triazin-1(4H)-yl)methyl)-1-((((S)-oxetan-2-yl)methyl)-1H-benzo[d]imidazole-6-carboxylic acid (Compound 82) was prepared according to the route shown in route G. LCMS(ESI): m/z = 675.3 (M+H)⁺.

### Preparation of 4-(ethoxy-d5)-2-((4-((S)-2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 82-1) and 4-(ethoxy-d5)-2-((4-((R)-2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 82-2):

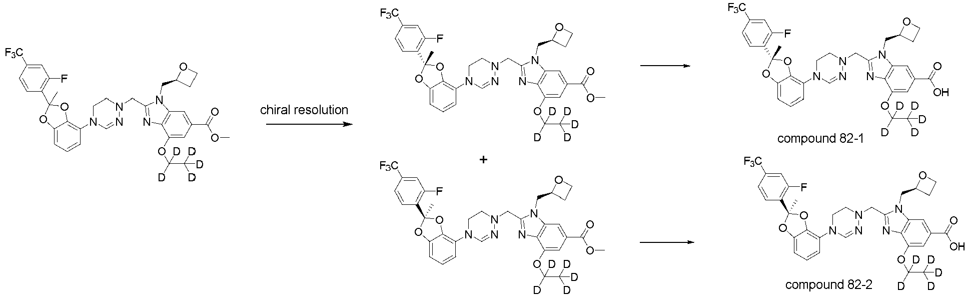

The chiral separation and preparation methods of compounds 82-1 and 82-2 are the same as those of compounds 76-1 and 76-2.

### Preparation of Tris salt of 4-(ethoxy-d5)-2-((4-((S)-2-(2-fluoro-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 82-1):

The prototype 82-1 and tris(hydroxymethyl)aminomethane were dissolved in methanol at a molar ratio of 1:1, and the mixture was concentrated under reduced pressure at 45°C after ultrasonication for 10 minutes. The Tris salt of 82-1 was obtained after freeze-drying.

### Example 83: 2-((4-(2-(4-chloro-2-methoxyphenyl)-2-methylbenzo[d][1,3]dioxol-4-yl)-5,6-dihydro-1,2,4-triazin-1(4H)-yl)methyl)-4-methoxy-1-(((S)-oxetan-2-yl)methyl)-1H-benzo[d]imidazole-6-carboxylic acid

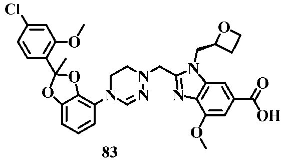

2-((4-(2-(4-Chloro-2-methoxyphenyl)-2-methylbenzo[d][1,3]dioxol-4-yl)-5,6-dihydro-1,2,4-triazin-1(4H)-yl)methyl)-4-methoxy-1-(((S)-oxetan-2-yl)methyl)-1H-benzo[d]imidazole-6-carboxylic acid (Compound 83) was prepared according to the route shown in route G. LCMS(ESI): m/z = 634.3 (M+H)⁺.

### Example 84: 2-((4-(2-(4-chloro-2-methoxyphenyl)-2-methylbenzo[d][1,3]dioxol-4-yl)-5,6-dihydro-1,2,4-triazin-1(4H)-yl)methyl)-4-(methoxy-d3)-1-(((S)-oxetan-2-yl)methyl)-1H-benzo[d]imidazole-6-carboxylic acid

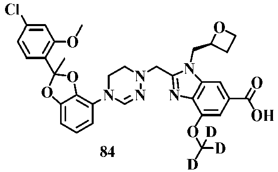

2-((4-(2-(4-Chloro-2-methoxyphenyl)-2-methylbenzo[d][1,3]dioxol-4-yl)-5,6-dihydro-1,2,4-triazin-1(4H)-yl)methyl)-4-(methoxy-d3)-1-(((S)-oxetan-2-yl)methyl)-1H-benzo[d]imidazole-6-carboxylic acid (Compound 84) was prepared according to the route shown in route G. LCMS(ESI): m/z = 637.3 (M+H)⁺.

### Example 85: 2-((4-(2-(4-chloro-2-methoxyphenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid

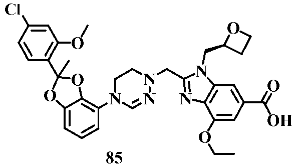

2-((4-(2-(4-Chloro-2-methoxyphenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 85) was prepared according to the route shown in route G. LCMS(ESI): m/z = 648.3 (M+H)⁺.

### Example 86: 2-((4-(2-(2,4-difluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid

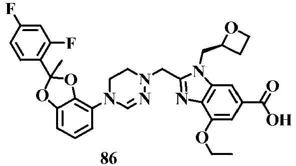

2-((4-(2-(2,4-difluorophenyl)-2-methylbenzo[d][1,3]dioxol-4-yl)-5,6-dihydro-1,2,4-triazin-1(4H)-yl)methyl)-4-ethoxy-1-(((S)-oxetan-2-yl)methyl)-1H-benzo[d]imidazole-6-carboxylic acid (Compound 86) was prepared according to the route shown in route G. LCMS(ESI): m/z = 620.3 (M+H)⁺.

### Example 87: 2-((4-(2-(2,4-difluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid

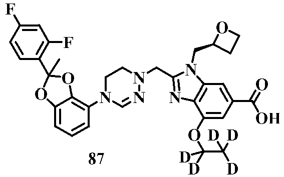

2-((4-(2-(2,4-difluorophenyl)-2-methylbenzo[d][1,3]dioxol-4-yl)-5,6-dihydro-1,2,4-triazin-1(4H)-yl)methyl)-4-(ethoxy-d5)-1-(((S)-oxetan-2-yl)methyl)-1H-benzo[d]imidazole-6-carboxylic acid (Compound 87) was prepared according to the route shown in route G. LCMS (ESI): m/z = 625.4 (M+H)⁺.

### Example 88: 4-methoxy-2-((4-(2-(2-methoxy-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid

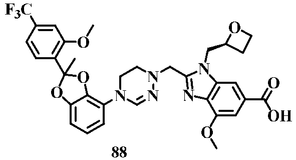

4-Methoxy-2-((4-(2-(2-methoxy-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 88) was prepared according to the route shown in route G. LCMS(ESI): m/z = 668.4 (M+H)⁺.

### Example 89: 4-(Methoxy-d3)-2-((4-(2-(2-methoxy-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid

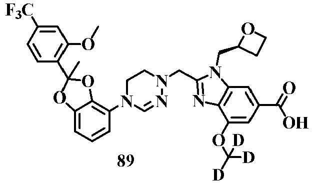

4-(Methoxy-d3)-2-((4-(2-(2-methoxy-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 89) was prepared according to the route shown in route G. LCMS(ESI): m/z = 671.3 (M+H)⁺.

### Example 90: 4-ethoxy-2-((4-(2-(2-methoxy-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid

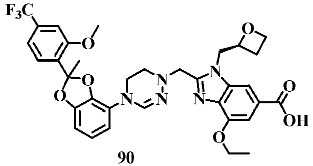

4-Ethoxy-2-((4-(2-(2-methoxy-4-(trifluoromethyl)phenyl)-2-methylbenzo[d][1,3]dioxol-4-yl)-5,6-dihydro-1,2,4-triazin-1(4H)-yl)methyl)-1-(((S)-oxetan-2-yl)methyl)-1H-benzo[d]imidazole-6-carboxylic acid (Compound 90) was prepared according to the route shown in route G. LCMS(ESI): m/z = 682.3 (M+H)⁺.

¹H NMR spectrum of the compounds:

| Compound | ¹H NMR |
|---|---|
| 56 | ¹H NMR (500 MHz, DMSO) δ 9.09 (d, *J =* 2.3 Hz, 1H), 8.35 - 8.29 (m, 1H), 7.81 (dd, *J* = 8.2, 1.9 Hz, 1H), 7.75 (d, *J* = 1.2 Hz, 1H), 7.29 (d, *J =* 1.2 Hz, 1H), 6.85 - 6.75 (m, 3H), 5.13 - 5.05 (m, 1H), 4.72 - 4.64 (m, 1H), 4.57 (dd, *J =* 15.2, 3.2 Hz, 1H), 4.50 - 4.39 (m, 1H), 4.38 - 4.31 (m, 1H), 4.22 (q, *J =* 7.0 Hz, 2H), 3.89 (dd, *J =* 13.3, 2.1 Hz, 1H), 3.74 (dd, *J* = 13.2, 2.5 Hz, 1H), 2.99 (d, *J* = 11.0 Hz, 1H), 2.87 (d, *J =* 11.1 Hz, 1H), 2.73 - 2.61 (m, 2H), 2.47 - 2.39 (m, 1H), 2.31 - 2.09 (m, 2H), 2.05 (s, 3H), 1.82 - 1.65 (m, 4H), 1.41 (t, *J* = 7.0 Hz, 3H). |
| 61 | ¹H NMR (500 MHz, DMSO) δ 8.72 (t, *J =* 2.2 Hz, 1H), 8.01 (dd, *J =* 8.5, 2.5 Hz, 1H), 7.70 (s, 1H), 7.60 (d, *J =* 8.4 Hz, 1H), 7.31 (s, 1H), 6.83 - 6.73 (m, 3H), 5.11 - 5.06 (m, 1H), 4.69 - 4.61 (m, 1H), 4.54 (dd, *J* = 15.3, 3.3 Hz, 1H), 4.49 - 4.40 (m, 1H), 4.38 - 4.32 (m, 1H), 3.87 (dd, *J =* 13.4, 2.6 Hz, 1H), 3.72 (dd, *J =* 13.3, 3.5 Hz, 1H), 2.99 (d, *J* = 11.1 Hz, 1H), 2.86 (d, *J =* 11.3 Hz, 1H), 2.71 - 2.61 (m, 2H), 2.47 - 2.40 (m, 1H), 2.25 - 2.17 (m, 1H), 2.17 - 2.09 (m, 1H), 2.01 (s, 3H), 1.80 - 1.68 (m, 4H). |
| 62 | ¹H NMR (500 MHz, DMSO) δ 8.72 (t, *J =* 2.6 Hz, 1H), 8.04 - 7.98 (m, 2H), 7.74 - 7.38 (m, 3H), 6.84 - 6.73 (m, 3H), 5.10 (d, *J =* 7.8 Hz, 1H), 4.79 - 4.69 (m, 1H), 4.61 (dd, *J =* 15.2, 3.0 Hz, 1H), 4.49 - 4.42 (m, 1H), 4.42 - 4.34 (m, 1H), 3.94 (dd, *J =* 13.6, 2.4 Hz, 1H), 3.76 (dd, *J =* 13.4, 3.2 Hz, 1H), 3.01 (d, *J =* 11.1 Hz, 1H), 2.86 (d, *J =* 11.1 Hz, 1H), 2.73 - 2.63 (m, 2H), 2.47 - 2.40 (m, 1H), 2.29 - 2.21 (m, 1H), 2.20 - 2.07 (m, 1H), 2.01 (s, 3H), 1.81 - 1.67 (m, 4H). |
| 63 | ¹H NMR (500 MHz, DMSO) δ 7.71 (s, 1H), 7.60 - 7.53 (m, 2H), 7.36 - 7.32 (m, 2H), 7.29 (d, *J* = 2.1 Hz, 1H), 6.85 (t, *J =* 8.1 Hz, 1H), 6.69 (d, *J =* 7.8 Hz, 1H), 6.65 (d, *J =* 8.4 Hz, 1H), 5.09 - 5.01 (m, 1H), 4.74 - 4.65 (m, 1H), 4.63 - 4.55 (m, 1H), 4.51 - 4.42 (m, 1H), 4.39 (dd, *J =* 13.8, 4.6 Hz, 1H), 4.35 - 4.25 (m, 2H), 3.92 (s, 3H), 3.86 - 3.77 (m, 1H), 3.74 - 3.67 (m, 1H), 3.13 - 2.98 (m, 2H), 2.72 - 2.64 (m, 1H), 2.45 - 2.37 (m, 1H), 2.04 (s, 3H). |
| 64 | ¹H NMR (500 MHz, DMSO) δ 8.74 - 8.70 (m, 1H), 8.01 (dd, *J =* 8.4, 2.5 Hz, 1H), 7.82 (d. *J* = 1.1 Hz, 1H), 7.61 (dd, *J* = 8.2, 1.7 Hz, 1H), 7.39 (d, *J* = 1.1 Hz, 1H), 6.81 - 6.74 (m, 3H), 5.14 - 5.06 (m, 1H), 5.01 (q, *J =* 9.0 Hz, 2H), 4.74 - 4.65 (m, 1H), 4.58 (dd, *J =* 15.0, 3.2 Hz, 1H), 4.50 - 4.40 (m, 1H), 4.40 - 4.33 (m, 1H), 3.91 (dd, *J =* 13.4, 2.0 Hz, 1H), 3.75 (dd, *J* = 13.3, 2.7 Hz, 1H), 2.99 (d, *J =* 11.0 Hz, 1H), 2.86 (d, *J =* 11.2 Hz, 1H), 2.71 - 2.59 (m, 2H), 2.47 - 2.40 (m, 1H), 2.26 - 2.19 (m, 1H), 2.19 - 2.09 (m, 1H), 2.01 (s, 3H), 1.81 - 1.67 (m, 4H). |
| 65 | ¹H NMR (500 MHz, DMSO) δ 9.10 - 9.06 (m, 1H), 8.31 (dd, *J* = 8.4, 2.3 Hz, 1H), 7.85 (d, *J =* 8.3 Hz, 1H), 7.74 (d, *J =* 1.1 Hz, 1H), 7.32 (dd, *J* = 7.2, 1.4 Hz, 2H), 6.86 (t, *J* = 8.1 Hz, 1H), 6.70 (d, *J* = 7.9 Hz, 1H), 6.67 (d, *J* = 8.8 Hz, 1H), 5.09 - 5.00 (m, 1H), 4.75 - 4.66 (m, 1H), 4.64 - 4.56 (m, 1H), 4.49 - 4.44 (m, 1H), 4.39 (dd, *J =* 13.8, 2.0 Hz, 1H), 4.35 - 4.26 (m, 2H), 3.93 (s, 3H), 3.85 - 3.79 (m, 1H), 3.77 - 3.70 (m, 1H), 3.13 - 3.00 (m, 2H), 2.72 - 2.63 (m, 1H), 2.44 - 2.33 (m, 1H), 2.07 (s, 3H). |
| 66 | ¹H NMR (500 MHz, DMSO) δ 9.08 (d, *J =* 2.1 Hz, 1H), 8.31 (dd, *J =* 8.5, 2.4 Hz, 1H), 7.85 (d, *J* = 8.3 Hz, 1H), 7.71 (s, 1H), 7.31 (d, *J =* 1.7 Hz, 1H), 7.28 (s, 1H), 6.86 (t, *J* = 8.2 Hz, 1H), 6.70 (d, *J =* 7.9 Hz, 1H), 6.67 (d, *J =* 8.5 Hz, 1H), 5.07 - 5.01 (m, 1H), 4.74 - 4.66 (m, 1H), 4.63 - 4.56 (m, 1H), 4.49 - 4.42 (m, 1H), 4.38 (dd, *J =* 13.8, 2.8 Hz, 1H), 4.33 - 4.26 (m, 2H), 4.22 (q, *J =* 6.9 Hz, 2H), 3.84 - 3.78 (m, 1H), 3.76 - 3.7C (m, 1H), 3.11 - 2.96 (m, 2H), 2.72 - 2.62 (m, 1H), 2.40 - 2.33 (m, 1H), 2.06 (s, 3H), 1.41 (t, *J =* 7.0 Hz, 3H). |
| 67 | ¹H NMR (500 MHz, DMSO) δ 9.08 (d, *J =* 2.4 Hz, 1H), 8.32 (d, *J =* 8.4 Hz, 1H), 7.85 (s, 1H), 7.80 (dd, *J =* 8.4, 2.6 Hz, 1H), 7.26 (d, *J =* 1.2 Hz, 1H), 6.82 - 6.75 (m, 3H), 5.13 - 5.04 (m, 1H), 4.71 (dd, *J* = 15.4, 7.0 Hz, 1H), 4.59 (dd, *J* = 15.3, 2.9 Hz, 1H), 4.49 - 4.39 (m, 1H), 4.38 - 4.30 (m, 1H), 3.90 (dd, *J =* 13.5, 3.5 Hz, 1H), 3.74 (dd, *J =* 13.4, 4.0 Hz, 1H), 2.99 (d, *J =* 11.2 Hz, 1H), 2.90 - 2.82 (m, 1H), 2.72 - 2.59 (m, 2H), 2.48 - 2.38 (m, 1H), 2.27 - 2.19 (m, 1H), 2.18 - 2.09 (m, 1H), 2.05 (s, 3H), 1.82 - 1.66 (m, 4H). |
| 68 | ¹H NMR (500 MHz, DMSO) δ 7.88 (s, 1H), 7.59 - 7.53 (m, 2H), 7.34 (d, *J =* 8.5 Hz, 1H), 7.27 (d, *J =* 17.9 Hz, 2H), 6.85 (t, *J =* 8.1 Hz, 1H), 6.70 (d, *J =* 7.8 Hz, 1H), 6.65 (d, *J =* 8.4 Hz, 1H), 5.08 - 5.02 (m, 1H), 4.81 - 4.74 (m, 1H), 4.65 (d, *J =* 15.7 Hz, 1H), 4.50 - 4.44 (m, 1H), 4.41 (dd, *J =* 13.7, 3.0 Hz, 1H), 4.34 - 4.28 (m, 2H), 4.26 (q, *J =* 7.1 Hz, 2H), 3.86 - 3.78 (m, 1H), 3.78 - 3.68 (m, 1H), 3.14 - 3.03 (m, 2H), 2.71 - 2.62 (m, 1H), 2.42 - 2.31 (m, 1H), 2.03 (s, 3H), 1.43 (t, *J =* 6.9 Hz, 3H). |
| 69 | ¹H NMR (600 MHz, DMSO) δ 8.71 (d, *J =* 2.4 Hz, 1H), 8.00 (dd, *J =* 8.5, 2.6 Hz, 1H), 7.84 (d, *J* = 1.3 Hz, 1H), 7.65 (d, *J* = 8.5 Hz, 1H), 7.28 (dd, *J* = 15.3, 1.4 Hz, 2H), 6.85 (t, *J =* 8.1 Hz, 1H), 6.70 - 6.62 (m, 2H), 5.07 - 5.00 (m, 1H), 4.79 - 4.72 (m, 1H), 4.68 - 4.61 (m, 1H), 4.49 - 4.42 (m, 1H), 4.40 (d, *J =* 13.8 Hz, 1H), 4.33 - 4.28 (m, 2H), 4.26 (q, *J =* 7.0 Hz, 2H), 3.83 - 3.78 (m, 1H), 3.77 - 3.68 (m, 1H), 3.12 - 2.99 (m, 2H), 2.71 - 2.61 (m, 1H), 2.40 - 2.32 (m, 1H), 2.02 (s, 3H), 1.42 (t, *J =* 7.0 Hz, 3H). |
| 70 | ¹H NMR (600 MHz, DMSO) δ 8.71 (d, *J =* 2.5 Hz, 1H), 8.00 (ddd, *J* = 8.5, 2.6, 1.1 Hz, 1H), 7.84 (d, *J =* 1.3 Hz, 1H), 7.65 (d, *J =* 8.5 Hz, 1H), 7.29 (d, *J =* 1.4 Hz, 2H), 6.85 (t, *J* = 8.1 Hz, 1H), 6.67 (dd, *J =* 9.7, 8.0 Hz, 2H), 5.08 - 5.01 (m, 1H), 4.79 - 4.71 (m, 1H), 4.67 - 4.61 (m, 1H), 4.49 - 4.43 (m, 1H), 4.42 - 4.38 (m, 1H), 4.34 - 4.27 (m, 2H), 3.83 - 3.79 (m, 1H), 3.77 - 3.70 (m, 1H), 3.13 - 3.01 (m, 2H), 2.71 - 2.62 (m, 1H), 2.40 - 2.33 (m, 1H), 2.02 (s, 3H). |
| 71 | ¹H NMR (600 MHz, DMSO) δ 7.85 (d, *J* = 1.3 Hz, 1H), 7.59 - 7.53 (m, 2H), 7.36 - 7.31 (m, 1H), 7.28 (t, *J =* 1.7 Hz, 2H), 6.85 (t, *J =* 8.1 Hz, 1H), 6.69 (d, *J =* 7.8 Hz, 1H), 6.65 (d, *J =* 8.4 Hz, 1H), 5.08 - 5.01 (m, 1H), 4.79 - 4.71 (m, 1H), 4.68 - 4.61 (m, 1H), 4.49 - 4.44 (m, 1H), 4.41 (dd, *J* = 13.8, 3.2 Hz, 1H), 4.35 - 4.27 (m, 2H), 3.85 - 3.78 (m, 1H), 3.77 - 3.68 (m, 1H), 3.14 - 3.01 (m, 2H), 2.75 - 2.64 (m, 1H), 2.44 - 2.34 (m, 1H), 2.03 (s, 3H). |
| 72 | ¹H NMR (600 MHz, DMSO) δ 8.72 (t, *J =* 2.7 Hz, 1H), 8.03 - 7.98 (m, 1H), 7.80 (d,*J* = 1.2 Hz, 1H), 7.60 (dd, *J =* 8.5, 2.5 Hz, 1H), 7.26 (d, *J =* 1.2 Hz, 1H), 6.81 - 6.74 (m, 3H), 5.12 - 5.05 (m, 1H), 4.73 - 4.66 (m, 1H), 4.58 (dd, *J =* 15.3, 3.0 Hz, 1H), 4.49 - 4.39 (m, 1H), 4.38 - 4.30 (m, 1H), 3.89 (dd, *J =* 13.4, 3.8 Hz, 1H), 3.74 (dd, *J =* 13.4, 4.7 Hz, 1H), 2.98 (d, *J =* 11.2 Hz, 1H), 2.84 (d, *J =* 11.6 Hz, 1H), 2.70 - 2.60 (m, 2H), 2.47 - 2.38 (m, 1H), 2.25 - 2.17 (m, 1H), 2.17 - 2.09 (m, 1H), 2.01 (s, 3H), 1.79 - 1.67 (m, 4H). |
| 73 | ¹H NMR (600 MHz, DMSO) δ 8.71 (d, *J =* 2.5 Hz, 1H), 8.02 - 7.98 (m, 1H), 7.71 (s, 1H), 7.65 (d, *J =* 8.5 Hz, 1H), 7.29 (t, *J =* 1.7 Hz, 2H), 6.85 (t, *J =* 8.1 Hz, 1H), 6.66 (t, *J =* 8.1 Hz, 2H), 5.07 - 5.00 (m, 1H), 4.74 - 4.66 (m, 1H), 4.63 - 4.56 (m, 1H), 4.49 - 4.42 (m, 1H), 4.38 (dd, *J =* 13.7, 2.1 Hz, 1H), 4.34 - 4.25 (m, 2H), 3.84 - 3.76 (m, 1H), 3.76 - 3.69 (m, 1H), 3.11 - 2.99 (m, 2H), 2.70 - 2.61 (m, 1H), 2.42 - 2.33 (m, 1H), 2.02 (s, 3H). |
| 74 | 1H NMR (600 MHz, DMSO) δ 7.87 - 7.84 (m, 1H), 7.60 - 7.52 (m, 2H), 7.34 (dd, *J* = 8.4, 2.1 Hz, 1H), 7.28 (d, *J =* 1.2 Hz, 1H), 6.80 - 6.70 (m, 3H), 5.14 - 5.06 (m, 1H), 4.76 - 4.69 (m, 1H), 4.64 - 4.57 (m, 1H), 4.50 - 4.42 (m, 1H), 4.40 - 4.32 (m, 1H), 3.91 (dd, *J =* 13.4, 3.3 Hz, 1H), 3.76 (d, *J=* 13.4 Hz, 1H), 3.02 - 2.96 (m, 1H), 2.89 - 2.83 (m, 1H), 2.74 - 2.62 (m, 2H), 2.48 - 2.39 (m, 1H), 2.27 - 2.20 (m, 1H), 2.18 - 2.09 (m, 1H), 2.02 (s, 3H), 1.81 - 1.64 (m, 4H). |
| 75 | ¹H NMR (600 MHz, DMSO) δ 7.82 (dd, *J =* 2.4, 1.3 Hz, 1H), 7.60 - 7.52 (m, 2H), 7.34 (dd, *J =* 8.4, 2.1 Hz, 1H), 7.27 (d, *J =* 1.2 Hz, 1H), 6.82 - 6.72 (m, 3H), 5.14 - 5.06 (m, 1H), 4.75 - 4.68 (m, 1H), 4.63 - 4.57 (m, 1H), 4.50 - 4.42 (m, 1H), 4.40 - 4.32 (m, 1H), 3.91 (dd, *J* = 13.4, 3.3 Hz, 1H), 3.76 (d, *J =* 13.3 Hz, 1H), 2.99 (dd, *J* = 12.0, 4.1 Hz, 1H), 2.88 - 2.83 (m, 1H), 2.73 - 2.62 (m, 2H), 2.48 - 2.39 (m, 1H), 2.26 - 2.20 (m, 1H), 2.18 - 2.11 (m, 1H), 2.02 (s, 3H), 1.81 - 1.64 (m, 4H). |
| 76 | ¹H NMR (600 MHz, DMSO) δ 7.81 (d, *J* = 1.3 Hz, 1H), 7.59 - 7.53 (m, 2H), 7.36 - 7.32 (m, 1H), 7.29 (dd, *J =* 5.5, 1.7 Hz, 2H), 6.85 (t, *J =* 8.1 Hz, 1H), 6.69 (d, *J =* 7.8 Hz, 1H), 6.65 (d, *J =* 8.5 Hz, 1H), 5.09 - 5.02 (m, 1H), 4.77 - 4.70 (m, 1H), 4.66 - 4.59 (m, 1H), 4.50 - 4.43 (m, 1H), 4.40 (dd, *J =* 13.7, 4.9 Hz, 1H), 4.36 - 4.27 (m, 2H), 3.85 - 3.78 (m, 1H), 3.75 - 3.68 (m, 1H), 3.13 - 3.00 (m, 2H), 2.71 - 2.62 (m, 1H), 2.43 - 2.34 (m, 1H), 2.03 (s, 3H). |
| 77 | ¹H NMR (600 MHz, DMSO) δ 7.78 (s, 1H), 7.49 (dd, *J =* 8.5, 6.6 Hz, 1H), 7.42 - 7.36 (m, 1H), 7.29 (dd, *J* = 5.0, 1.4 Hz, 2H), 6.86 (t, *J* = 8.1 Hz, 1H), 6.70 (d, *J* = 7.8 Hz, 1H), 6.67 (d, *J =* 8.4 Hz, 1H), 5.09 - 5.01 (m, 1H), 4.76 - 4.69 (m, 1H), 4.66 - 4.58 (m, 1H), 4.48 - 4.44 (m, 1H), 4.40 (dd, *J =* 13.7, 6.3 Hz, 1H), 4.34 - 4.27 (m, 2H), 3.86 - 3.78 (m, 1H), 3.75 - 3.69 (m, 1H), 3.13 - 3.00 (m, 2H), 2.71 - 2.62 (m, 1H), 2.43 - 2.34 (m, 1H), 2.05 (s, 3H). |
| 78 | ¹H NMR (600 MHz, DMSO) δ 7.68 (s, 1H), 7.49 (dd, *J =* 8.5, 6.7 Hz, 1H), 7.42 - 7.36 (m, 1H), 7.30 (dd, *J* = 7.6, 1.6 Hz, 2H), 6.86 (t, *J* = 8.1 Hz, 1H), 6.70 (d, *J* = 7.8 Hz, 1H), 6.67 (d, *J =* 8.4 Hz, 1H), 5.08 - 5.01 (m, 1H), 4.72 - 4.65 (m, 1H), 4.62 - 4.55 (m, 1H), 4.50 - 4.43 (m, 1H), 4.38 (dd, *J =* 13.7, 7.6 Hz, 1H), 4.34 - 4.26 (m, 2H), 4.22 (q, *J =* 7.0 Hz, 2H), 3.85 - 3.78 (m, 1H), 3.75 - 3.68 (m, 1H), 3.11 - 2.99 (m, 2H), 2.70 - 2.62 (m, 1H), 2.43 - 2.35 (m, 1H), 2.06 (s, 3H), 1.41 (t, *J =* 6.9 Hz, 3H). |
| 79 | ¹H NMR (600 MHz, DMSO) δ 7.78 (dd, *J =* 10.1, 5.9 Hz, 1H), 7.73 (d, *J =* 1.2 Hz, 1H), 7.55 (dd, *J =* 9.3, 6.5 Hz, 1H), 7.32 (d, *J =* 1.1 Hz, 1H), 7.29 (d, *J =* 1.1 Hz, 1H), 6.86 (t, *J =* 8.1 Hz, 1H), 6.72 (d, *J =* 7.7 Hz, 1H), 6.66 (d, *J =* 8.4 Hz, 1H), 5.09 - 5.02 (m, 1H), 4.74 - 4.66 (m, 1H), 4.63 - 4.56 (m, 1H), 4.50 - 4.43 (m, 1H), 4.39 (dd, *J =* 13.7, 6.6 Hz, 1H), 4.34 - 4.27 (m, 2H), 4.23 (q, *J =* 7.0 Hz, 2H), 3.84 - 3.77 (m, 1H), 3.74 - 3.67 (m, 1H), 3.11 - 2.99 (m, 2H), 2.71 - 2.62 (m, 1H), 2.43 - 2.34 (m, 1H), 2.04 (s, 3H), 1.42 (t, *J =* 7.0 Hz, 3H). |
| 80 | ¹H NMR (600 MHz, DMSO) δ 7.78 (dd, *J =* 10.1, 5.9 Hz, 1H), 7.71 (s, 1H), 7.55 (dd, *J* = 9.3, 6.4 Hz, 1H), 7.32 (s, 1H), 7.29 (d, *J =* 1.1 Hz, 1H), 6.86 (t, *J =* 8.1 Hz, 1H), 6.72 (d, *J* = 7.8 Hz, 1H), 6.66 (d, *J* = 8.4 Hz, 1H), 5.08 - 5.01 (m, 1H), 4.73 - 4.65 (m, 1H), 4.63 - 4.56 (m, 1H), 4.50 - 4.43 (m, 1H), 4.38 (dd, *J =* 13.7, 6.9 Hz, 1H), 4.34 - 4.26 (m, 2H), 3.84 - 3.77 (m, 1H), 3.74 - 3.67 (m, 1H), 3.11 - 2.98 (m, 2H), 2.71 - 2.62 (m, 1H), 2.43 - 2.34 (m, 1H), 2.04 (s, 3H). |
| 81 | ¹H NMR (600 MHz, DMSO) δ 7.82 (d, *J =* 11.0 Hz, 1H), 7.79 (t, *J =* 7.8 Hz, 1H), 7.75 (s, 1H), 7.65 (d, *J* = 8.1 Hz, 1H), 7.31 (d, *J =* 3.1 Hz, 2H), 6.86 (t, *J =* 8.2 Hz, 1H), 6.72 (d, *J=* 7.8 Hz, 1H), 6.66 (d, *J =* 8.4 Hz, 1H), 5.09 - 5.02 (m, 1H), 4.75 - 4.68 (m, 1H), 4.65 - 4.58 (m, 1H), 4.50 - 4.43 (m, 1H), 4.40 (dd, *J* = 13.7, 7.1 Hz, 1H), 4.33 - 4.28 (m, 2H), 4.24 (q, *J =* 6.9 Hz, 2H), 3.86 - 3.79 (m, 1H), 3.78 - 3.69 (m, 1H), 3.13 - 2.99 (m, 2H), 2.71 - 2.62 (m, 1H), 2.43 - 2.34 (m, 1H), 2.08 (s, 3H), 1.42 (t, *J =* 7.0 Hz, 3H). |
| 82 | ¹H NMR (600 MHz, DMSO) δ 7.82 (d, *J =* 11.0 Hz, 1H), 7.79 (t, *J =* 7.8 Hz, 1H), 7.7C (s, 1H), 7.65 (d, *J = 8.1* Hz, 1H), 7.32 (dd, *J* = 3.5, 2.1 Hz, 2H), 6.86 (t, *J =* 8.1 Hz, 1H), 6.71 (d, *J* = 7.8 Hz, 1H), 6.66 (d, *J =* 8.4 Hz, 1H), 5.09 - 5.02 (m, 1H), 4.73 - 4.66 (m, 1H), 4.63 - 4.56 (m, 1H), 4.50 - 4.43 (m, 1H), 4.39 (dd, *J =* 13.7, 7.9 Hz, 1H), 4.35 - 4.26 (m, 2H), 3.86 - 3.79 (m, 1H), 3.75 - 3.68 (m, 1H), 3.11 - 3.00 (m, 2H), 2.71 - 2.62 (m, 1H), 2.44 - 2.35 (m, 1H), 2.08 (s, 3H). |
| 83 | ¹H NMR (600 MHz, DMSO) δ 7.67 (s, 1H), 7.44 (d, *J =* 8.3 Hz, 1H), 7.34 (s, 1H), 7.31 (s, 1H), 7.20 (d, *J =* 1.9 Hz, 1H), 7.05 - 7.00 (m, 1H), 6.80 (t, *J =* 8.1 Hz, 1H), 6.64 (dd, *J =* 7.8, 1.0 Hz, 1H), 6.60 (d, *J =* 8.4 Hz, 1H), 5.09 - 5.02 (m, 1H), 4.74 - 4.65 (m, 1H), 4.63 - 4.55 (m, 1H), 4.50 - 4.44 (m, 1H), 4.39 (dd, *J* = 13.8, 1.8 Hz, 1H), 4.35 - 4.26 (m, 2H), 3.91 (s, 3H), 3.87 (s, 3H), 3.85 - 3.78 (m, 1H), 3.71 - 3.64 (m, 1H), 3.10 - 2.98 (m, 2H), 2.72 - 2.62 (m, 1H), 2.44 - 2.35 (m, 1H), 2.02 (s, 3H). |
| 84 | ¹H NMR (600 MHz, DMSO) δ 7.70 (s, 1H), 7.43 (d, *J =* 8.3 Hz, 1H), 7.32 (d, *J =* 1.1 Hz, 1H), 7.30 (s, 1H), 7.20 (d, *J =* 2.0 Hz, 1H), 7.04 - 7.00 (m, 1H), 6.79 (t, *J =* 8.1 Hz, 1H), 6.64 (d, *J =* 7.8 Hz, 1H), 6.60 (d, *J =* 8.4 Hz, 1H), 5.08 - 5.01 (m, 1H), 4.75 - 4.65 (m, 1H), 4.64 - 4.56 (m, 1H), 4.50 - 4.43 (m, 1H), 4.39 (dd, *J =* 13.9, 1.3 Hz, 1H), 4.34 - 4.27 (m, 2H), 3.86 (s, 3H), 3.84 - 3.78 (m, 1H), 3.72 - 3.64 (m, 1H), 3.11 - 2.98 (m, 2H), 2.71 - 2.62 (m, 1H), 2.43 - 2.34 (m, 1H), 2.01 (s, 3H). |
| 85 | ¹H NMR (600 MHz, DMSO) δ 7.88 (s, 1H), 7.43 (d, *J* = 8.3 Hz, 1H), 7.30(s, 1H), 7.26 (d, *J* = 1.3 Hz, 1H), 7.20 (t, *J =* 1.7 Hz, 1H), 7.04 - 6.99 (m, 1H), 6.80 (t, *J =* 8.1 Hz, 1H), 6.65 (dd, *J* = 7.8, 1.0 Hz, 1H), 6.60 (d, *J =* 8.4 Hz, 1H), 5.09 - 5.02 (m, 1H), 4.83 - 4.75 (m, 1H), 4.71 - 4.63 (m, 1H), 4.49 - 4.44 (m, 1H), 4.42 (d, *J =* 13.9 Hz, 1H), 4.35 - 4.29 (m, 2H), 4.27 (q, *J =* 7.0 Hz, 2H), 3.86 (s, 3H), 3.84 - 3.80 (m, 1H), 3.73 - 3.65 (m, 1H), 3.14 - 3.00 (m, 2H), 2.72 - 2.63 (m, 1H), 2.40 - 2.33 (m, 1H), 2.01 (s, 3H), 1.43 (t, *J* = 7.0 Hz, 3H). |
| 86 | ¹H NMR (600 MHz, DMSO) δ 7.71 (s, 1H), 7.64 - 7.57 (m, 1H), 7.39 - 7.34 (m, 1H), 7.31 (s, 1H), 7.30 (d, *J* = 2.7 Hz, 1H), 7.17 - 7.09 (m, 1H), 6.84 (t, *J =* 8.1 Hz, 1H), 6.68 (d, *J* = 7.8 Hz, 1H), 6.65 (d, *J =* 8.5 Hz, 1H), 5.08 - 5.01 (m, 1H), 4.73 - 4.66 (m, 1H), 4.63 - 4.56 (m, 1H), 4.49 - 4.43 (m, 1H), 4.38 (dd, *J =* 13.7, 5.4 Hz, 1H), 4.34 - 4.27 (m, 2H), 4.23 (q, *J =* 7.0 Hz, 2H), 3.85 - 3.79 (m, 1H), 3.75 - 3.68 (m, 1H), 3.11 - 2.99 (m, 2H), 2.71 - 2.62 (m, 1H), 2.43 - 2.34 (m, 1H), 2.03 (s, 3H), 1.42 (t, *J =* 7.0 Hz, 3H). |
| 87 | ¹H NMR (600 MHz, DMSO) δ 7.64 (s, 1H), 7.62 - 7.58 (m, 1H), 7.40 - 7.33 (m, 1H), 7.32 (s, 1H), 7.30 (d, *J =* 3.0 Hz, 1H), 7.15 - 7.09 (m, 1H), 6.84 (t, *J =* 8.1 Hz, 1H), 6.68 (d, *J =* 7.8 Hz, 1H), 6.64 (d, *J =* 8.4 Hz, 1H), 5.08 - 5.01 (m, 1H), 4.70 - 4.63 (m, 1H), 4.60 - 4.53 (m, 1H), 4.50 - 4.43 (m, 1H), 4.37 (dd, *J =* 13.7, 6.4 Hz, 1H), 4.33 - 4.24 (m, 2H), 3.85 - 3.77 (m, 1H), 3.74 - 3.67 (m, 1H), 3.12 - 2.98 (m, 2H), 2.70 - 2.62 (m, 1H), 2.44 - 2.35 (m, 1H), 2.03 (s, 3H). |
| 88 | ¹H NMR (600 MHz, DMSO) δ 7.88 (s, 1H), 7.65 (d, *J =* 8.0 Hz, 1H), 7.40 (d, *J =* 1.9 Hz, 1H), 7.35 - 7.32 (m, 1H), 7.31 (s, 1H), 7.28 (d, *J =* 1.3 Hz, 1H), 6.81 (t, *J =* 8.1 Hz, 1H), 6.67 (d, *J =* 7.8 Hz, 1H), 6.61 (d, *J =* 8.4 Hz, 1H), 5.09 - 5.02 (m, 1H), 4.83 - 4.74 (m, 1H), 4.72 - 4.63 (m, 1H), 4.49 - 4.44 (m, 1H), 4.42 (d, *J =* 13.9 Hz, 1H), 4.35 - 4.28 (m, 2H), 3.96 (s, 3H), 3.94 (s, 3H), 3.87 - 3.79 (m, 1H), 3.73 - 3.66 (m, 1H), 3.14 - 3.01 (m, 2H), 2.72 - 2.63 (m, 1H), 2.42 - 2.33 (m, 1H), 2.05 (s, 3H). |
| 89 | ¹H NMR (600 MHz, DMSO) δ 7.88 (s, 1H), 7.65 (d, *J =* 8.0 Hz, 1H), 7.40 (d, *J =* 1.9 Hz, 1H), 7.35 - 7.32 (m, 1H), 7.31 (s, 1H), 7.27 (d, *J* = 1.2 Hz, 1H), 6.81 (t, *J* = 8.1 Hz, 1H), 6.67 (d, *J* = 7.7 Hz, 1H), 6.61 (d, *J =* 8.4 Hz, 1H), 5.09 - 5.02 (m, 1H), 4.83 - 4.75 (m, 1H), 4.71 - 4.63 (m, 1H), 4.49 - 4.44 (m, 1H), 4.42 (d, *J =* 13.8 Hz, 1H), 4.35 - 4.28 (m, 2H), 3.94 (s, 3H), 3.87 - 3.79 (m, 1H), 3.73 - 3.66 (m, 1H), 3.15 - 3.01 (m, 2H), 2.75 - 2.63 (m, 1H), 2.42 - 2.33 (m, 1H), 2.05 (s, 3H). |
| 90 | ¹H NMR (600 MHz, DMSO) δ 7.87 (s, 1H), 7.65 (d, *J* = 8.0 Hz, 1H), 7.40 (s, 1H), 7.33 (d, *J =* 8.2 Hz, 1H), 7.31 (s, 1H), 7.26 (s, 1H), 6.81 (t, *J =* 8.1 Hz, 1H), 6.67 (d, *J* = 7.8 Hz, 1H), 6.61 (d, *J =* 8.4 Hz, 1H), 5.09 - 5.02 (m, 1H), 4.83 - 4.75 (m, 1H), 4.71 - 4.63 (m, 1H), 4.50 - 4.45 (m, 1H), 4.42 (dd, *J =* 13.7, 1.7 Hz, 1H), 4.34 - 4.30 (m, 2H), 4.26 (q, *J =* 7.0 Hz, 2H), 3.94 (s, 3H), 3.87 - 3.80 (m, 1H), 3.76 - 3.66 (m, 1H), 3.14 - 3.00 (m, 2H), 2.72 - 2.63 (m, 1H), 2.42 - 2.34 (m, 1H), 2.05 (s, 3H), 1.42 (t, *J =* 6.9 Hz, 3H). |
| 76-1 | ¹H NMR (500 MHz, DMSO) δ 7.78 (s, 1H), 7.61 - 7.53 (m, 2H), 7.34 (dd, *J* = 8.5, 2.1 Hz, 1H), 7.32 - 7.28 (m, 2H), 6.85 (t, *J =* 8.1 Hz, 1H), 6.70 (dd, *J =* 7.9, 1.0 Hz, 1H), 6.65 (dd, *J =* 8.5, 1.0 Hz, 1H), 5.09 - 5.00 (m, 1H), 4.73 (dd, *J =* 15.4, 6.9 Hz, 1H), 4.62 (dd, *J =* 15.3, 3.0 Hz, 1H), 4.50 - 4.42 (m, 1H), 4.39 (d, *J =* 13.7 Hz, 1H), 4.35 - 4.27 (m, 2H), 3.86 - 3.78 (m, 1H), 3.75 - 3.67 (m, 1H), 3.36 (s, 6H), 3.14 - 3.06 (m, 1H), 3.06 - 3.00 (m, 1H), 2.72 - 2.63 (m, 1H), 2.43 - 2.33 (m, 1H), 2.04 (s, 3H). |
| Tris salt | |
| 81-1 | ¹H NMR (500 MHz, DMSO) δ 7.85 - 7.76 (m, 3H), 7.65 (dd, *J =* 8.2, 1.8 Hz, 1H), 7.31 (s, 2H), 6.86 (t, *J =* 8.2 Hz, 1H), 6.72 (dd, *J =* 7.9, 1.0 Hz, 1H), 6.66 (dd, *J =* 8.5, 1.0 Hz, 1H), 5.09 - 5.01 (m, 1H), 4.73 (dd, *J =* 15.4, 6.8 Hz, 1H), 4.61 (d, *J =* 15.0 Hz, 1H), 4.50 - 4.42 (m, 1H), 4.39 (d, *J =* 13.7 Hz, 1H), 4.35 - 4.28 (m, 2H), 4.24 (q, *J =* 7.0 Hz, 2H), 3.87 - 3.79 (m, 1H), 3.76 - 3.67 (m, 1H), 3.37 (s, 6H), 3.14 - 3.08 (m, 1H), 3.07 - 2.99 (m, 1H), 2.70 - 2.61 (m, 1H), 2.44 - 2.34 (m, 1H), 2.08 (s, 3H), 1.42 (t, *J* = 7.0 Hz, 3H). |
| Tris salt | |
| 82-1 | ¹H NMR (500 MHz, DMSO) δ 7.83 (dd, *J =* 11.1, 1.7 Hz, 1H), 7.81 - 7.74 (m, 2H), 7.65 (dd, *J =* 8.3, 1.8 Hz, 1H), 7.33 - 7.29 (m, 2H), 6.86 (t, *J =* 8.1 Hz, 1H), 6.72 (d, *J* = 7.8 Hz, 1H), 6.66 (d, *J =* 8.4 Hz, 1H), 5.09 - 5.01 (m, 1H), 4.73 (dd, *J =* 15.3, 6.8 Hz, 1H), 4.61 (d, *J =* 15.0 Hz, 1H), 4.50 - 4.42 (m, 1H), 4.39 (d, *J =* 13.7 Hz, 1H), 4.36 - 4.25 (m, 2H), 3.87 - 3.79 (m, 1H), 3.76 - 3.68 (m, 1H), 3.38 (s, 6H), 3.14 - 3.07 (m, 1H), 3.06 - 2.99 (m, 1H), 2.74 - 2.62 (m, 1H), 2.43 - 2.34 (m, 1H), 2.08 (s, 3H). |
| Tris salt | |

### Pharmacological Examples

### 1. Determination of GLP-1 receptor-mediated agonist activity

(1) Test method: The agonist activity of the compound on the GLP-1 receptor was characterized by its effect on the cAMP level of the hGLP-1R/HEK293 cell line. The human GLP-1 receptor gene (hGLP-1R) was amplified from the cDNA of the human colorectal adenocarcinoma cell line NCl-H716 (Cell Bank of the Chinese Academy of Sciences, #TCHu210) and cloned into the HA-pcDNA3.1 vector (Addgene, #128034), with the HA-tag at the N-terminus of the hGLP-1R. hGLP-1R/HA-pcDNA3.1 plasmid was transfected into HEK293 cells to construct hGLP-1R/HEK293 stable cell line. The hGLP-1R/HEK293 cells were digested, centrifuged and resuspended in culture medium (DMEM+0.2% BSA, m/v), and the cell density was adjusted to 2×10⁵ cells/mL. IBMX (3-isobutyl-1-methylxanthine, final concentration of 0.5 mM) was added to the cell suspension and added to a 384-well plate at 5 µL (1×10³ cells)/well. The compound was dissolved in DMSO to prepare a stock solution with a concentration of 10 mM. The experiment started with a concentration of 100 µM which was diluted 10 times in a series of 7 dilution points, with the 8th point being DMSO. 2 µL of the test compound of different concentrations was added to 98 µL of culture medium, shaken and mixed, 5 µL was taken and added to a 384-well plate (final concentration of the test compound was 0-100 nM, final concentration of DMSO was 1%, v/v), which was then incubated in the incubator (37°C, 5% CO₂) in the dark for 30 min, then 10 µL of cAMP detection reagent (LANCE Ultra cAMP Detecion Kit, PerkinElmer, #TRF0264) was added, mixed well, and reacted at room temperature in the dark for 1 h. After the reaction was completed, the HTRF signal was read using the Envision 2104 multi-function microplate reader. All samples were tested in triplicate wells, and the ratio of the acceptor-donor excitation signal in each single well was calculated using the formula Ratio = Signal 665nM/Signal 620nM. The HTRF signal ratio and the corresponding compound concentration were analyzed and processed using GraphPad Prism 8 software, and the EC₅₀ value of the compound was obtained by fitting the S-type dose-response curve and calculating.
(2) Test results

**Table 1 Effects of the compounds of the present invention on cAMP levels in hGLP-1R/HEK293 cell lines**

| Compound | determined EC₅₀ (pM) | | Compound | determined EC₅₀ (pM) |
|---|---|---|---|---|
| PF-06882961 | 44.5 | | | |
| 1 | 4.8 | | 2 | 1.1 |
| 3 | 6.4 | | 4 | 0.8 |
| 5 | 12.0 | | 8 | 3.4 |
| 9 | 1.2 | | 10 | 4.9 |
| 11 | 13.3 | | 12 | 4.0 |
| 13 | 2.2 | | 16 | 3.3 |
| 15 | 1.9 | | 18 | 0.4 |
| 17 | 4.7 | | 20 | 0.6 |
| 19 | 2.6 | | 22 | 8.5 |
| 21 | 1.0 | | 24 | 5.3 |
| 23 | 1.5 | | 26 | 9.3 |
| 25 | 11.8 | | 28 | 7.4 |
| 27 | 8.7 | | 30 | 9.6 |
| 29 | 2.2 | | 32 | 14.2 |
| 31 | 37.0 | | 34 | 3.3 |
| 33 | 46.7 | | 36 | 8.3 |
| 35 | 8.2 | | 38 | 22.9 |
| 37 | 6.7 | | 40 | 11.1 |
| 39 | 8.0 | | 42 | 29.8 |
| 43 | 34.2 | | 44 | 9.5 |
| 45 | 50.9 | | 46 | 42.2 |
| 47 | 61.3 | | 48 | 43.4 |
| 49 | 0.8 | | 50 | 0.4 |
| 51 | 1.6 | | 52 | 1.2 |
| 53 | 9.3 | | 54 | 7.7 |
| 55 | 25.2 | | 56 | 9.8 |
| 57 | 2.8 | | 58 | 2.2 |
| 59 | 3.1 | | 60 | 2.6 |
| 61 | 10.3 | | 62 | 22.0 |
| 63 | 15.2 | | | |
| 65 | 49 | | 66 | 5.2 |
| 67 | 14.1 | | 68 | 6.4 |
| 69 | 11.1 | | 70 | 27.3 |
| 71 | 17.5 | | 72 | 10.0 |
| 73 | 8.4 | | 74 | 6.5 |
| 75 | 3.0 | | 76 | 4.5 |
| 76-1 | 1.6 | | 76-2 | 604.3 |
| 77 | 16.5 | | 78 | 11.5 |
| 79 | 44.2 | | 80 | 43.8 |
| 81 | 8.4 | | 82 | 13.1 |
| 81-1 | 1.8 | | 82-1 | 5.1 |
| 83 | 9.2 | | 84 | 9.8 |
| 85 | 5.0 | | 86 | 28.9 |
| 87 | 21.2 | | 88 | 8.7 |
| 89 | 7.5 | | 90 | 6.1 |

As can be seen from Table 1, the compounds of the present invention are very potent hGLP-1 receptor agonists, and the activities of most of the tested compounds exceed those of the reference compound PF-06882961.

### (3) Comparative data

The distinguishing structural feature of the compounds represented by the general formula (I) and (II) of the present invention is that the substituent at the 4-position of the "benzimidazole-6-carboxylic acid" skeleton is limited to -OR². In the prior art, the substituent at the 4-position is -H, halogen, alkyl, alkynyl, heterocyclyl and the like, but there is no clear revelation on OR², nor is there any disclosure on the effect when the substituent is -OR². Through a large amount of structural modification and evaluation work, the present invention finds that the introduction of - OR² can achieve unexpected effects, especially that the introduction of -OR² greatly enhances the agonist activity of the compound on the hGLP-1 receptor. The following will further illustrate the significant effect of introducing -OR² at the 4-position of "benzimidazole-6-carboxylic acid" through a comparison of the structures and activities of the compounds in Table 2.

It can be seen from Table 2 that compared with various types of compounds disclosed in the prior art, when other structural features are completely consistent, only by introducing or replacing other substituents with methoxy, ethoxy, cyclopropyloxy and the like at the 4-position of "benzimidazole-6-carboxylic acid", the activity of the compound can be increased by several times to dozens of times, and the activity of some of the compounds reaches or even exceeds the level of endogenous GLP-1₇₋₃₆.

**Table 2 Comparation of the effects of the compounds on the cAMP levels of the hGLP-1R/HEK293 cell line**

| Reference compound | Compound of the present invention |
|---|---|
| GLP-1₇₋₃₆ EC₅₀: 2.7 pM | |
| 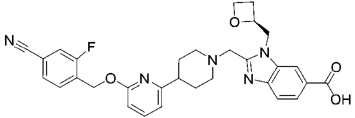 WO2018109607 , Example 4A-01/ PF-06882961, EC₅₀: 44.5 pM | 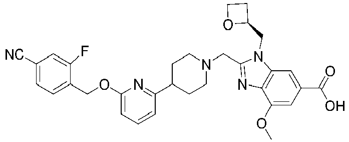 Compound 1, EC₅₀: 4.8 pM |
| 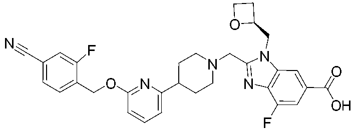 WO2022109182 , Example 21 EC₅₀: 26.1 pM | 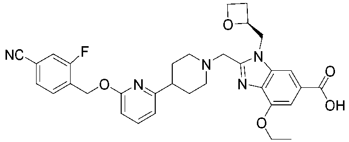 Compound 2, EC₅₀: 1.1 pM |
| 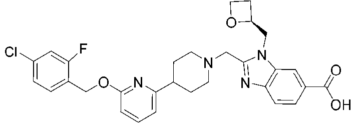 WO2018109607 , Example 3A-01 EC₅₀: 32.7 pM | 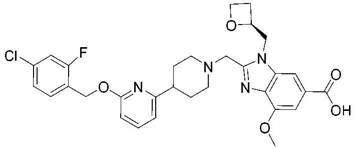 Compound 19, EC₅₀: 2.6 pM |
| 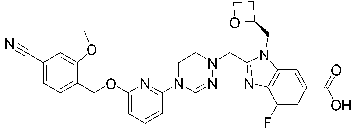 CN2022114139828 , Compound 10 EC₅₀: 6 pM | 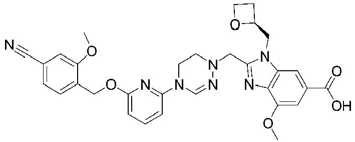 Compound 18, EC₅₀: 0.4 pM |
| 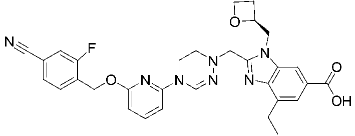 CN2022114139828 , Compound 63 EC₅₀: 363 pM | 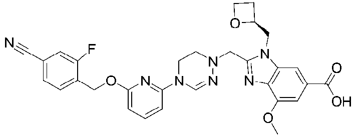 Compound 8, EC₅₀: 3.4 pM |
| | 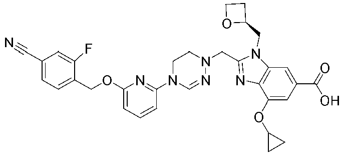 Compound 13, EC₅₀: 2.2 pM |
| 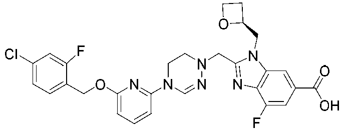 CN2022114139828 , Compound 2, EC₅₀: 49 pM | 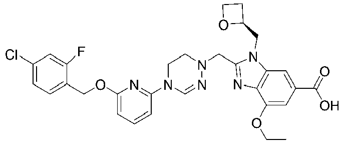 Compound 21, EC₅₀: 1 pM |
| 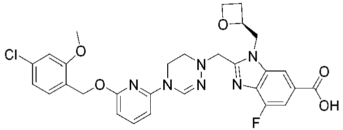 CN2022114139828 , Compound 51, EC₅₀: 4 pM | 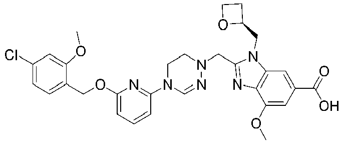 Compound 20, EC₅₀: 0.6 pM |
| 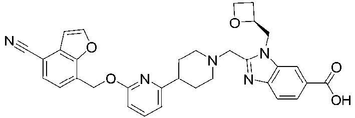 CN113480534 , Compound 3 EC₅₀: 12.7 pM | 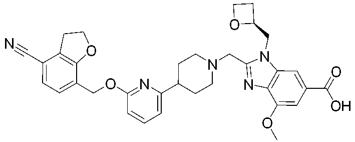 Compound 15, EC₅₀: 1.9 pM |
| 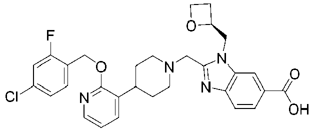 WO2019239371 , Example 1 EC₅₀: 45.8 pM | 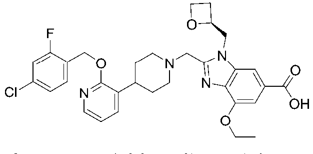 Compound 32, EC₅₀: 14.2 pM |
| 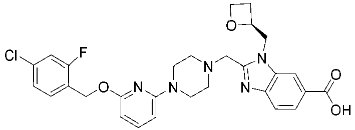 WO2018109607 , Example 10A-08 EC₅₀: 28.9 pM | 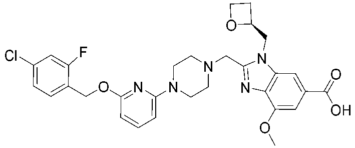 Compound 30, EC₅₀: 9.6 pM |
| 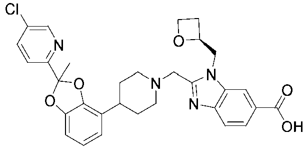 WO2019239319 , Example 10, EC₅₀: 77.3 pM | 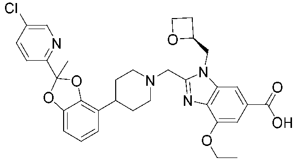 Compound 39, EC₅₀: 8.0 pM |
| 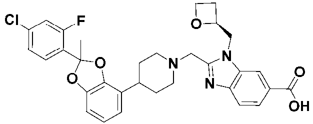 WO2019239319 , Example 7, EC₅₀: 22 pM | 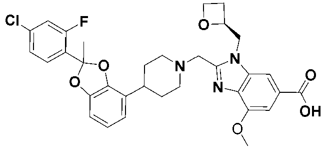 Compound 36, EC₅₀: 8.3 pM |
| | 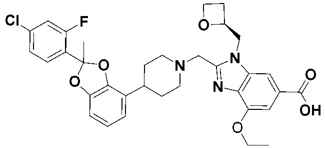 Compound 37, EC₅₀: 6.7 pM |
| 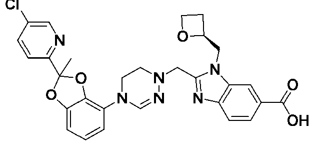 CN2022114139828 , Compound 36 EC₅₀: 336 pM | 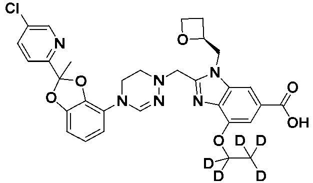 Compound 73, EC₅₀: 8.4 pM |
| 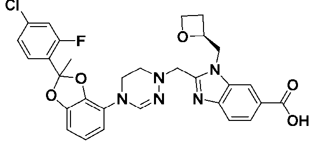 CN2022114139828 , Compound 37 EC₅₀: 137 pM | 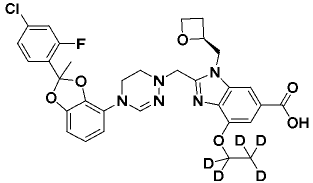 Compound 76, EC₅₀: 4.5 pM |
| 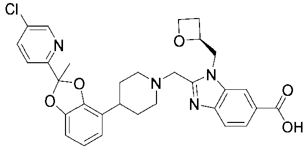 WO2019239319 , Example 10 EC₅₀: 77.3 pM | 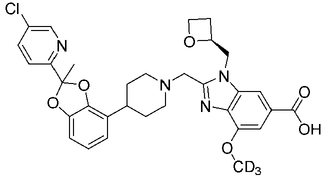 Compound 61, EC₅₀: 10.3 pM |

### 2. Evaluation of the compound's recruitment effect on β-arrestin

### (1) Test method

The Promega NanoBiT protein-protein reaction system was used to detect the recruitment of β-arrestin 1 and β-arrestin 2 by GLP-1R. The NanoBit Protein-Protein Reaction System was a dual-subunit system based on NanoLuc luciferase that could be used to detect protein-protein interactions in cells. The LgBiT (17.6 kDa) and SmBiT (11 amino acids) subunits were fused to the proteins of interest, respectively. When the proteins of interest interact, the two subunits came into close proximity to form an enzyme with catalytic activity, which could catalyze the luminescence of the luciferase substrate. Firstly, the human GLP-1R gene was connected to the N-terminus of LgBit, the β-arrestin 1/2 gene was cloned to the C-terminus of SmBit, and the fusion protein was introduced into the pcDNA3.0 vector to construct plasmids expressing hGLP-1R-LgBit and SmBit-β-arrestin 1/2 fusion proteins. The hGLP-1R-LgBit plasmid and SmBit-β-arrestin 1 (or SmBit-β-arrestin 2) plasmid were co-transfected into HEK293 cells at a ratio of 1 µg plasmid/2×10⁶ cells. Immediately after transfection, the cells were added to a 96-well white-bottom plate at a density of 4×10⁵ cells/well, and the cells were cultured in an incubator (37°C, 5% CO₂) for 20 hours.

After 20 hours, the liquid in the wells was discarded, 40 µL of fresh culture medium (DMEM+0.2% BSA, m/v) was added first, and then 10 µL of Nano-Glo Live Cell detection solution (Promega, #N2011) was added, and then the cells were placed in an incubator and incubated for 10 minutes. The compound was dissolved in DMSO and prepared into a storage solution with a concentration of 10mM. The experiment started with a concentration of 100µM. After 10-fold gradient dilution, 3 µL of different concentrations of the test compound was added to 97µL culture medium for later use (GLP-1 final concentration 0-10µM, other compounds final concentration 0-100µM, DMSO final concentration is 1%, v/v). After the cells were incubated for 10 min, 25 µL of culture medium containing the compound was added to each well and incubated at room temperature for 5 min. The fluorescence signal was then detected using an Envision 2104 multi-function microplate reader (PerkinElmer). All samples were tested in triplicate, and the fluorescence signal ratio and the corresponding compound concentration were analyzed using GraphPad Prism 8 software. The EC₅₀ value of the compound was obtained by fitting the S-type dose-response curve and calculating.

### (2) Test results

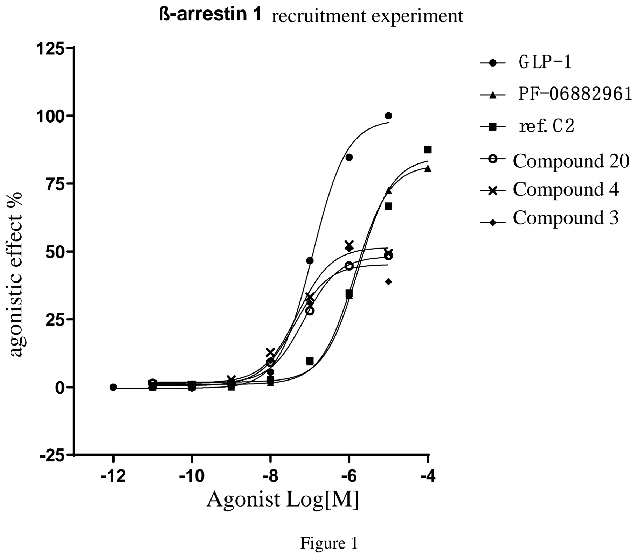

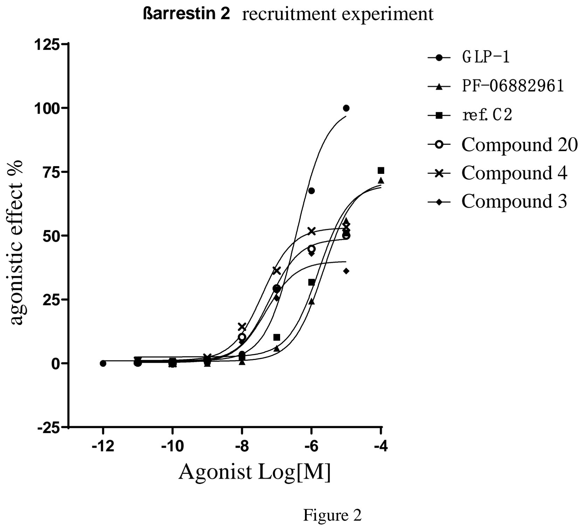

In addition to G protein, β-arrestin is another important signaling protein of GLP-1 receptor. As can be seen from Figures 1 and 2, the maximum agonistic effect (Eₘₐₓ) of endogenous GLP-1 recruiting β-arrestin 1 and β-arrestin 2 is 100%. PF-06882961 and ref. C2 are Example 4A-01 and Compound 2 disclosed in WO2018109607 and CN2022114139828, respectively, and their maximum agonist effects on β-arrestin 1 and β-arrestin 2 are between 75-80%. Compound 20, Compound 4 and Compound 3 are compounds disclosed in the present invention. Although their agonist activity on G protein cAMP signaling is increased by several to dozens of times, their maximum agonist effect on β-arrestin 1 and β-arrestin 2 recruitment is significantly reduced, ranging from 30-50%. It can be seen that the introduction of an alkoxy group at the 4-position of "benzimidazole-6-carboxylic acid" unexpectedly leads to an increase in the downstream signal bias of the GLP-1 receptor. Many studies have shown that β-arrestin mediates the desensitization of GLP-1 receptors, and reducing β-arrestin recruitment can enhance the effects of lowering blood sugar and reducing weight (Br J Pharmacol. 2022; 179: 492-510.). The compounds of the present invention have a stronger agonist effect on G protein signals and a weaker agonist effect on β-arrestin signals, and therefore have the potential to achieve better blood sugar-lowering and weight-reducing effects.

### 3. Pharmacodynamics experiments

### 3.1 Animals

Genetically engineered hGLP-1R gene knock-in mice, male, 8-10 weeks old, specific pathogen free (SPF) grade, were purchased from Biocytogen Jiangsu Gene Biotechnology Co., Ltd. All experimental animals were housed in the SPF-level environment of the National Compound Sample Library animal room, with a temperature of 24±2°C, a relative humidity of 40-60%RH, an air cleanliness level of 7, and a day and night light-dark cycle of 12h/12h; they were continuously supplied with cobalt-60 radiation-sterilized complete mouse granular feed (Shanghai Shilin Biotechnology Co., Ltd., maintenance feed for rats and mice), and were allowed to drink tap water (sterilized with high-pressure steam) without interruption, and were allowed to drink it freely. The cages were transparent polyetherimide cages (Suzhou Fengshi Experimental Animal Equipment Co., Ltd., CP-8 mouse cages), and the bedding was corn cobs (Tezhou Gumei Agricultural Technology Co., Ltd., used after high-pressure steam sterilization). There were 3-5 animals in each cage, and the cage card indicated the IACUC approval number, experiment number, experiment start time, person in charge, experimenter, animal source, group and animal number. The purchased mice were adapted to this breeding environment for at least 7 days before being used in experiments. The animal use methods in this study were approved by the IACUC Committee of the Shanghai Institute of Materia Medica.

### 3.2 Oral glucose tolerance test in mice

(1) Mouse model: above hGLP-1 receptor knock-in mice, 8-10 weeks old, 5-8 males per experimental group.
(2) Compound preparation: blank control group, ultrapure water, containing 1% DMSO (v/v); ammonium salt of PF-06882961, dose 1 mg/kg, dissolved in a small amount of DMSO and then added to pure water; ammonium salt of the compound to be tested, dose 1 mg/kg, dissolved in a small amount of DMSO and then added to pure water.
(3) Experimental procedure: Mice were starved overnight, and tail blood glucose was measured. 60 minutes after oral administration, blood glucose was measured again as zero-point blood glucose. Then, 2 g/kg of sugar was orally administered, and blood glucose was measured using a blood glucose meter 15 minutes, 30 minutes, 60 minutes, and 90 minutes after sugar administration. In order to explore the effect of long-term administration on glucose tolerance, 2 g/kg of sugar was orally administered again 240 min after the administration of the compound, and the blood glucose changes were measured at 180 min, 195 min, 210 min, 240 min, and 270 min.

### (4) Experimental results

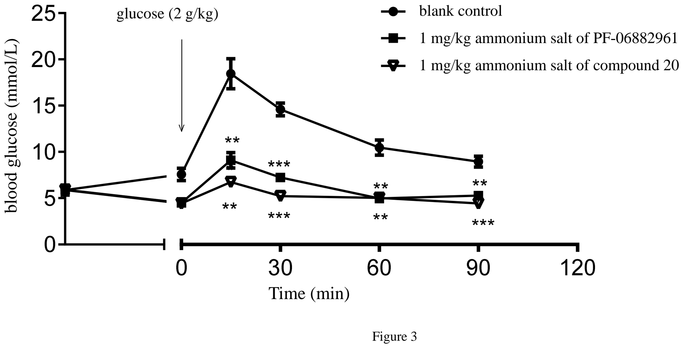

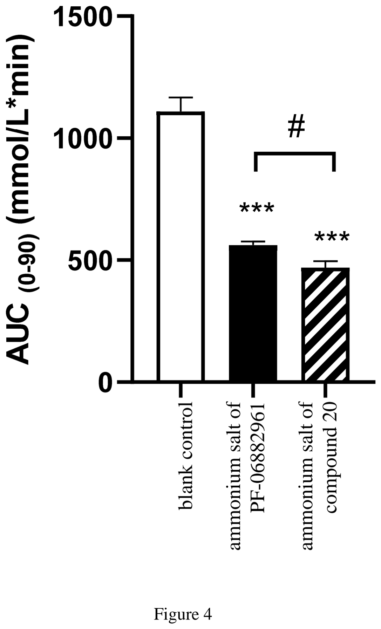

As shown in Figures 3 and 4, the ammonium salt of compound 20 of the present invention can significantly reduce blood glucose concentration and blood glucose area under the curve when orally administered at 1 mg/kg, and its effect is better than the positive control PF-06882961 ammonium salt at the same dose, and has better potential for treating diabetes.

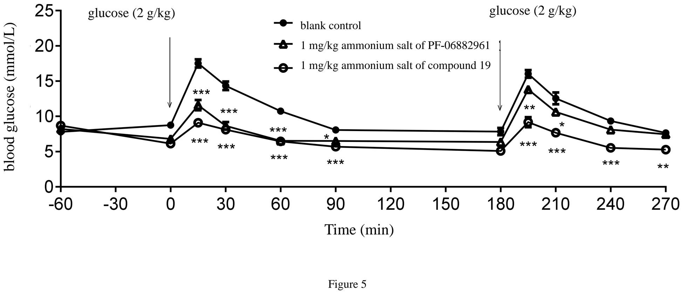

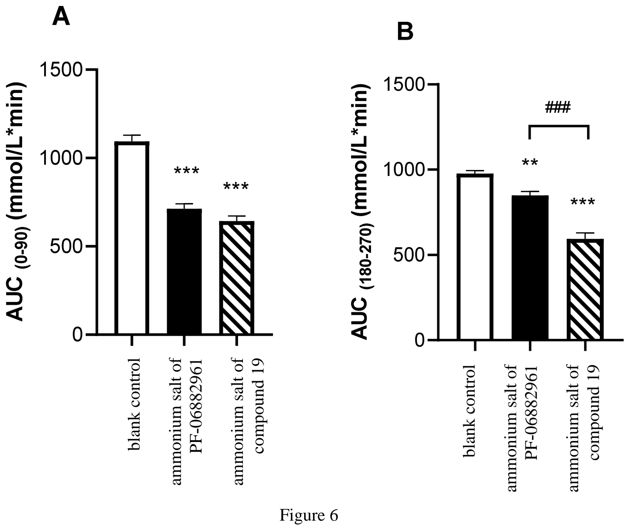

As shown in Figures 5 and 6, the ammonium salt of compound 19 of the present invention can significantly reduce blood glucose concentration and blood glucose area under the curve when orally administered at 1 mg/kg, and its effect is better than the positive control PF-06882961 ammonium salt at the same dose. In particular, the ammonium salt of compound 19 has a more significant effect in lowering blood sugar in a long-term oral glucose tolerance test, indicating that its efficacy is more lasting and it has better potential for treating diabetes.

### 3.3 Mouse food intake suppression experiment

### 3.3.1 Effect of compound 19

(1) Mouse model: above hGLP-1 receptor knock-in mice, 10 weeks old, 5 males per experimental group.
(2) Compound preparation: blank control group, ultrapure water, containing 10% DMSO (v/v); ammonium salt of PF-06882961, dose 30 mg/kg, dissolved in a small amount of DMSO and then added to the solvent; ammonium salt of the compound to be tested, dose 30 mg/kg, dissolved in a small amount of DMSO and then added to the solvent.
(3) Experimental procedures: Mice were fed a normal diet and drank water, and each mouse was placed in a cage. Drugs were administered orally by gavage at the beginning of the night cycle (7:00 PM). The weight of food in each cage was weighed at 0, 2.5, 5, 12 (7:00 AM at the beginning of the day cycle), and 24 hours after administration to calculate the food intake of the mice.
(4) Experimental results

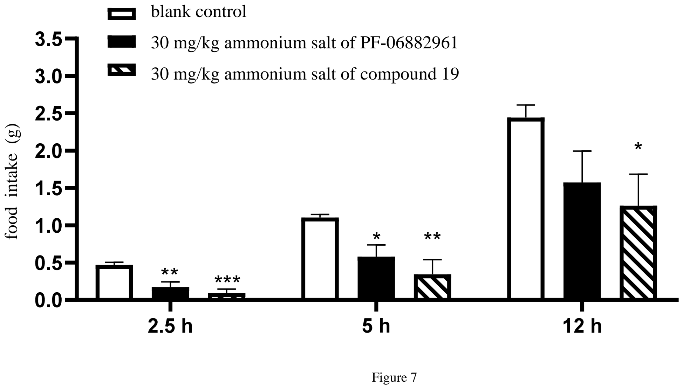

As shown in Figure 7, the ammonium salt of compound 19 of the present invention can significantly inhibit the food intake of experimental mice within 0 to 12 hours after oral administration, and its effect is better than the positive control PF-06882961 ammonium salt at the same dose. It is known that the peptidomimetic GLP-1 receptor agonists currently used in clinical practice can suppress appetite and reduce body weight. Therefore, the compound of the present invention, as a small molecule GLP-1 receptor agonist, also has the potential to be used for weight loss.

### 3.3.2 Effect of Compound 76-1

(1) Mouse model: above hGLP-1 receptor knock-in mice, 10 weeks old, 7 males per experimental group.
(2) Compound preparation: blank control group, ultrapure water, containing 10% DMSO (v/v); ammonium salt of PF-06882961, dose 10 or 30 mg/kg, dissolved in a small amount of DMSO and then added to the solvent; ammonium salt of the compound to be tested, dose 10 or 30 mg/kg, dissolved in a small amount of DMSO and then added to the solvent.
(3) Experimental procedure: Each mouse was placed in a cage and fasted for 6 h before the drug was administered orally by gavage at the start of the night cycle (7:00 PM). The weight of food in each cage was weighed at 0, 2.5, 5, 12 (7:00 AM at the start of the day cycle), and 24 h after administration to calculate the food intake of the mice.
(4) Experimental results

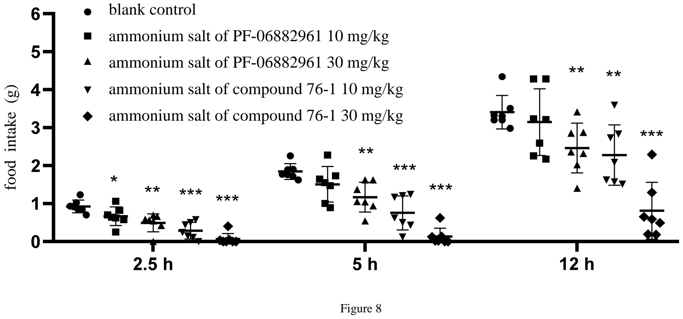

As shown in Figure 8, the ammonium salt of the compound 76-1 of the present invention can significantly inhibit the food intake of experimental mice within 0 to 12 hours after oral administration (10 or 30 mg/kg), and its effect is better than the positive control PF-06882961 ammonium salt at the same dose.

### 3.4 Long-term administration of compound 76-1 in hGLP-1R mice for weight loss

(1) Mouse model: above hGLP-1 receptor knock-in mice, male, 20 weeks old, weighing 35-40 g, 9 mice in each experimental group.
(2) Compound preparation: blank control group, ultrapure water, containing 5% DMSO (v/v); PF-06882961 ammonium salt, dose 30 mg/kg, dissolved in a small amount of DMSO and then added to the solvent; ammonium salt of the compound 76-1 to be tested, dose 30 mg/kg, dissolved in a small amount of DMSO and then added to the solvent.
(3) Experimental procedure: Three mice/cage were fed a high-fat diet (Research Diets, Catalog No. D12492) and the test compound was administered by gavage once a day for 15 consecutive days. The mice were weighed every day, and 24 hours after the last administration, the body fat and muscle content of the mice were detected using a Bruker desktop nuclear magnetic resonance spectrometer (model LF90II).
(4) Experimental results

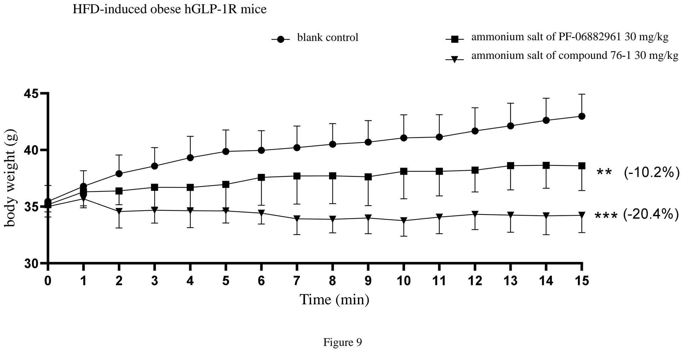

As shown in Figure 9, after high-fat diet (HFD) feeding, the body weight of the mice in the blank control group gradually increases, while administration of PF-06882961 ammonium salt or the ammonium salt of the compound 76-1 of the present invention can effectively inhibit the weight gain of the mice. Compared with the blank control group, after 15 days of administration, PF-06882961 ammonium salt at 30 mg/kg and compound 76-1 ammonium salt at 30 mg/kg are able to reduce body weight by 10.2% and 20.4%, respectively, indicating that at the same dose, the compound of the present invention has a better weight loss effect than PF-06882961.

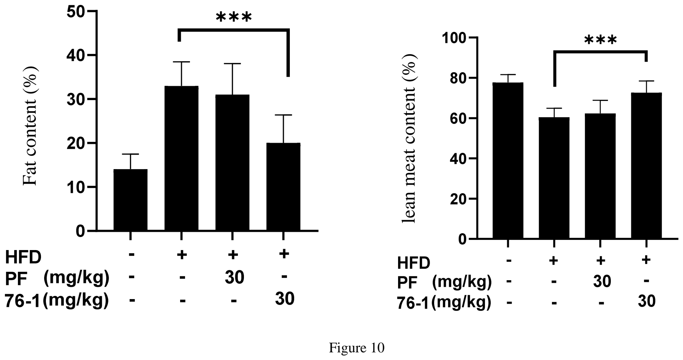

After 15 days of administration, the body fat and muscle content of mice were tested (Figure 10). Compared with healthy mice, the fat content of HFD mice increased significantly while the lean meat content decreased. Compound 76-1 ammonium salt (30 mg/kg) is able to effectively inhibit this change trend, showing good potential for the treatment of obesity.

### 3.5 Hypoglycemic effect of compound 76-1

### (1) Experimental methods

Mouse model: hGLP-1R transgenic mice, 6 female mice per experimental group.

Compound preparation: blank control group, ultrapure water, containing 1% DMSO; positive drug PF-06882961 ammonium salt, dose 0.3 mg/kg, dissolved in a small amount of DMSO and then added to pure water; compound 76-1 ammonium salt, dose 0.1, 0.3 mg/kg, dissolved in a small amount of DMSO and then added to pure water.

Experimental procedure: The mice were starved overnight, and the blood sugar in the tail of the mice was measured. 30 minutes after oral administration, the blood sugar was measured again as the blood sugar at zero point. Then, 2g/kg of sugar was given orally. 15min, 30min, 60min, and 90min after sugar administration, the blood sugar was measured using a blood glucose meter.

### (2) Experimental results

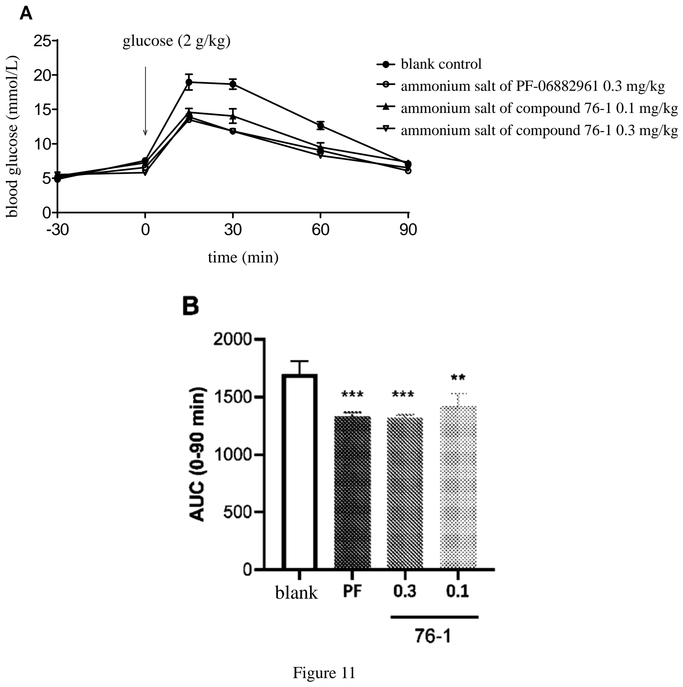

As shown in Figure 11A and B, the ammonium salt of compound 76-1 can dose-dependently reduce the blood glucose level and the area under the blood glucose curve (AUC) of mice, with an effective dose as low as 0.1 mg/kg, showing great potential for treating diabetes.

### 3.6 Effect of Tris salt of compounds 81-1 and 82-1 on diet inhibition

### (1) Experimental methods

Mouse model: hGLP-1R KI mice, 8 female mice per experimental group.

Compound preparation: The blank control group was an aqueous solution of 2:98 (v/v) Tween 80/0.5% (w/v) methylcellulose A4M (CMC-A4M); the positive compound PF-06882961 and compounds 81-1 and 82-1 were first prepared as Tris salts and then formulated into the above solvents, with a dosage of 30 mg/kg.

Experimental procedure: Mice were separated into cages and fasted 6 hours in advance, and were given drugs by oral gavage at the beginning of the night cycle (7:00 PM). The weight of food in each cage was weighed at 0, 2.5, 5, 12 (7:00 AM at the beginning of the day cycle), and 24 hours after drug administration to calculate the food intake of mice.

### (2) Experimental results

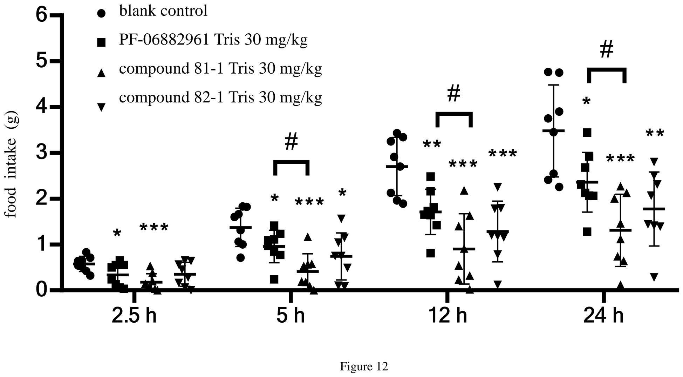

As shown in Figure 12, the Tris salts of compounds 81-1 and 82-1 are able to significantly reduce the food intake of mice after a single oral administration, and this effect last for 24 hours. The effect of the Tris salt of compound 81-1 is particularly prominent. Its inhibitory effect on eating at 5, 12 and 24 hours is significantly better than that of the Tris salt of the positive control PF-06882961 at the same dose, which indicates that the compound of the present invention has better potential for weight loss.

### 4. hERG channel inhibition test

### (1) Cell preparation

CHO cells expressing hERG protein were cultured in 175 cm² culture flasks. When the cell density grew to 60-80%, the culture medium was removed, the cells were washed once with 7 mL PBS, and then 3 mL Detachin was added for digestion. After digestion was complete, 7 mL of culture medium was added to neutralize, then centrifuged. The supernatant was aspirated, and 5 mL of culture medium was added to resuspend to ensure that the cell density is 2-5×10⁶/mL.

### (2) Solution preparation

| reagent | extracellular fluid (mM) | intracellular fluid (mM) |
|---|---|---|
| CaCl₂ | 2 | 5.374 |
| MgCl₂ | 1 | 1.75 |
| KCl | 4 | 120 |
| NaCl | 145 | - |
| glucose | 10 | - |
| HEPES | 10 | 10 |
| EGTA | - | 5 |
| Na-ATP | - | 4 |
| PH | 7.40(NaOH adjustment) osmotic pressure ~305mOsm | 7.25 (KOH adjustment) osmotic pressure ~290mOsm |

### (3) Electrophysiological recording process

Both the single-cell high-impedance sealing and whole-cell mode formation processes were automatically completed by the Qpatch instrument. After obtaining the whole-cell recording mode, the cell was clamped at -80 mV. Before a 5-second +20 mV depolarizing stimulus was given, a 50-ms -50 mV pre-voltage was given, and then the cell was repolarized to -50 mV for 5 seconds and then returned to -80 mV. This voltage stimulation was applied every 15 seconds. After recording for 2 minutes, the extracellular solution was recorded for 2 minutes. Then the drug administration process began. The compound concentration started from the lowest tested concentration. Each tested concentration was given for 2 minutes. After all concentrations were administered continuously, the positive control compound 10 µM Cisapride was administered. At least three cells were tested for each concentration (n≥3).

### (4) Preparation of compounds

The compound stock solution was diluted with extracellular fluid, 5 µL of 20 mM compound stock solution was added to 2495 µL extracellular fluid, diluted 500 times to 40 µM, and then serially diluted 3 times in extracellular fluid containing 0.2% DMSO (v/v) to obtain the final concentration to be tested. The highest tested concentration of the compound was 40 µM, followed by 6 concentrations including 40, 13.33, 4.44, 1.48, 0.49 and 0.16 µM. The highest tested concentration of the positive compound Cisapride was 3 µM, followed by 6 concentrations, namely 3, 1, 0.333, 0.111, 0.037 and 0.012 µM. The DMSO content in the final test concentration did not exceed 0.2% (v/v), and this concentration of DMSO had no effect on the hERG potassium channel.

### (5) Data analysis

The experimental data were analyzed by XLFit software.

### (6) Test results

As can be seen from Table 3, compared with PF-06882961, the compounds of the present invention have a smaller inhibitory effect on hERG potassium channels, especially when the A ring in the general formula (II) is 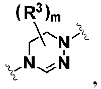 the IC₅₀ values of the compounds for inhibiting hERG are all over 40 µM. In the field of medicinal chemistry, hERG channel blockade is considered an indicator of cardiotoxicity risk and should be avoided as much as possible. The data in Table 3 show that the compounds of the present invention have a lower risk of cardiotoxicity.

**Table 3: Inhibitory effects of compounds on hERG potassium channels**

| Compound | IC₅₀(µM) |
|---|---|
| Cisapride | 0.035 |
| PF-06882961 | 2.62 |
| Compound 9 | >40 |
| Compound 17 | >40 |
| Compound 19 | 12.46 |
| Compound 20 | >40 |
| Compound 23 | >40 |
| Compound 61 | >40 |
| Compound 63 | >40 |
| Compound 67 | >40 |
| Compound 73 | >40 |
| Compound 76 | >40 |
| Compound 81 | >40 |
| Compound 82 | >40 |

All documents mentioned in the present application are incorporated by reference into this application as if each document was individually incorporated by reference. In addition, it should be understood that after reading the above teachings of the present invention, those skilled in the art may make various changes or modifications to the present invention, and these equivalent forms also fall within the scope defined by the claims attached to this application.

## Claims

1. A compound represented by general formula (II), stereoisomer, deuterated product or pharmaceutically acceptable salt thereof, 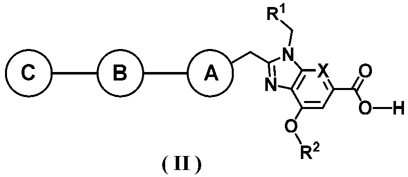
wherein, X is CR^{X} or N; R^{X} is H or halogen;
R¹ is 4-8 membered heterocyclyl, 5-8 membered heteroaryl or C3-C8 cycloalkyl, wherein the heterocyclyl, heteroaryl or cycloalkyl is unsubstituted or optionally substituted by one or more groups selected from a group consisting of halogen, C1-C6 alkyl, C1-C6 haloalkyl, C1-C6 alkyl substituted by cyano, C1-C6 alkoxy, C1-C6 haloalkoxy or cyano;
R² is C1-C6 alkyl, C1-C6 haloalkyl, deuterated C1-C6 alkyl, C1-C6 alkyl substituted by cyano, C3-C8 cycloalkyl, C1-C6 alkyl substituted by C3-C8 cycloalkyl, phenyl, C1-C6 alkyl substituted by phenyl, 5-8 membered heteroaryl, C1-C6 alkyl substituted by 5-8 membered heteroaryl, C1-C6 alkyl substituted by 4-8 membered heterocyclyl or 4-8 membered heterocyclyl, and the cycloalkyl, phenyl, heteroaryl or heterocyclyl is unsubstituted or optionally substituted by one or more groups selected from a group consisting of halogen, C1-C6 alkyl, C1-C6 alkoxy, C1-C6 haloalkyl, C1-C6 haloalkoxy, cyano; 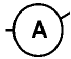
is selected from a group consisting of: 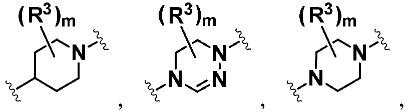 wherein N on the ring is linked to -CH₂; 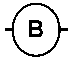
is selected from a group consisting of: 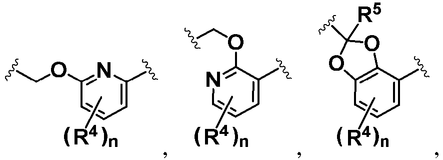 wherein a benzene ring or a pyridine ring is attached to 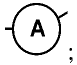 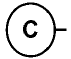
is selected from a group consisting of: 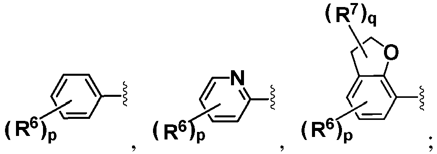
each R³, R⁴, R⁷ is independently H, halogen or C1-C6 alkyl;
R⁵ is H, C1-C6 alkyl or C1-C6 haloalkyl;
each R⁶ is independently H, halogen, cyano, C1-C6 alkyl, C1-C6 alkoxy, C1-C6 haloalkyl or C1-C6 haloalkoxy;
m, n, p, and q are each independently an integer of 1-4.

2. The compound of claim 1, wherein R¹ is a 4-6 membered heterocyclyl.

3. The compound of claim 1, wherein R² is C1-C4 alkyl, C1-C4 haloalkyl, deuterated C1-C4 alkyl, C3-C6 cycloalkyl, C1-C4 alkyl substituted by C3-C6 cycloalkyl, C1-C4 alkyl substituted by phenyl, 5-6 membered heteroaryl, C1-C4 alkyl substituted by 5-6 membered heteroaryl, or C1-C4 alkyl substituted by 5-6 membered heterocyclyl, wherein the cycloalkyl, heteroaryl, heterocyclyl is unsubstituted or optionally substituted by one or more groups selected from a group consisting of halogen, C1-C6 alkyl.

4. The compound of claim 1, wherein R⁵ is a C1-C3 alkyl.

5. The compound of claim 1, wherein each R⁶ is independently H, halogen, cyano, C1-C4 alkyl, C1-C4 alkoxy, C1-C4 haloalkyl or C1-C4 haloalkoxy.

6. The compound of claim 1, wherein the compound represented by general formula (II) has the following formulas (II-a) to (II-k): 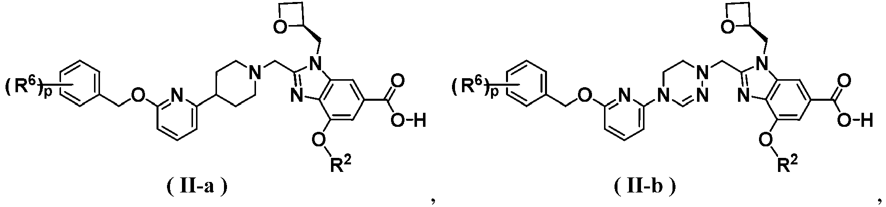 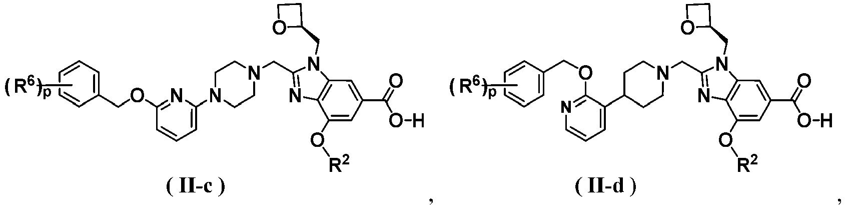 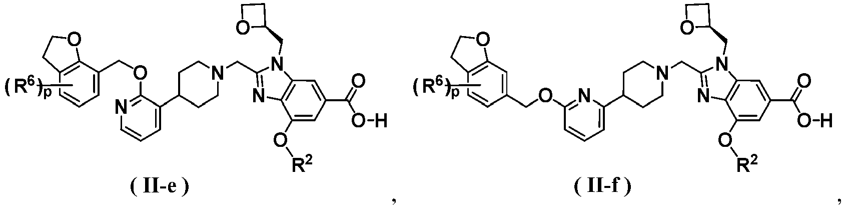 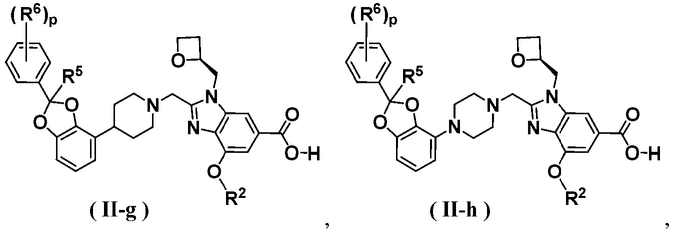 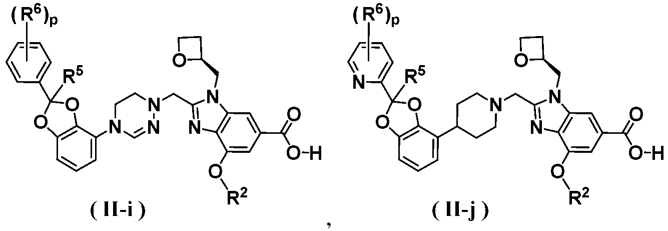 or 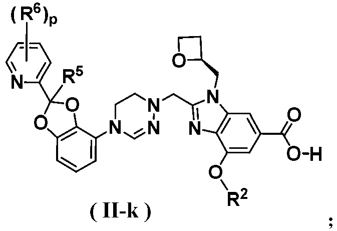 wherein, the definitions of R², R⁵, R⁶ and p are the same as those in formula (II).

7. The compound of claim 1, wherein the compound is selected from a group consisting of:

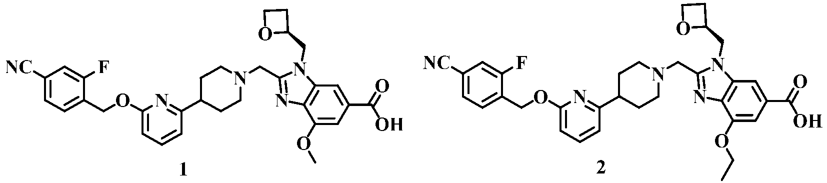

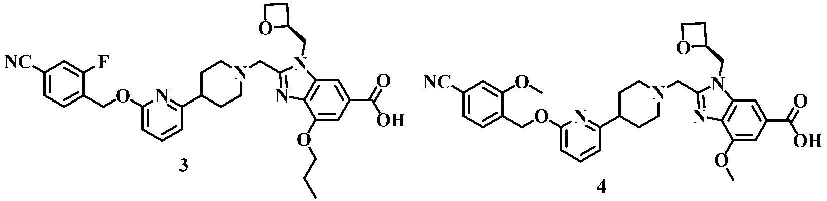

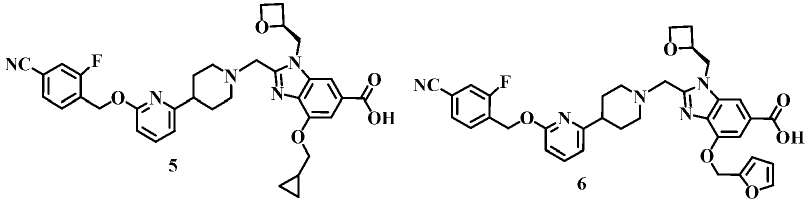

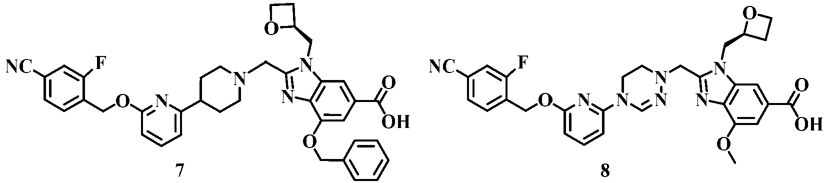

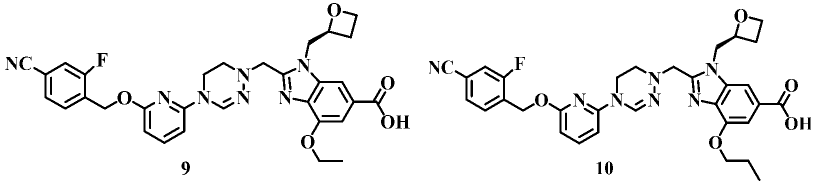

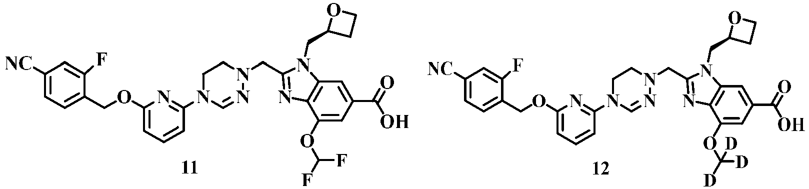

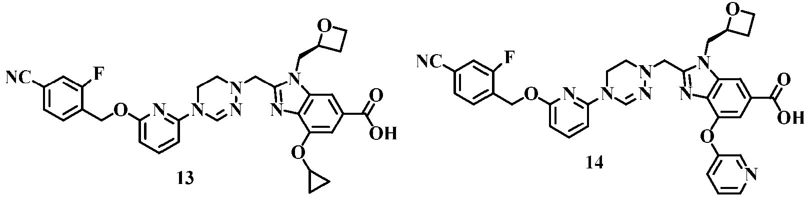

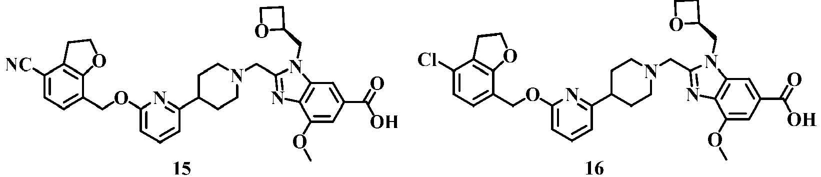

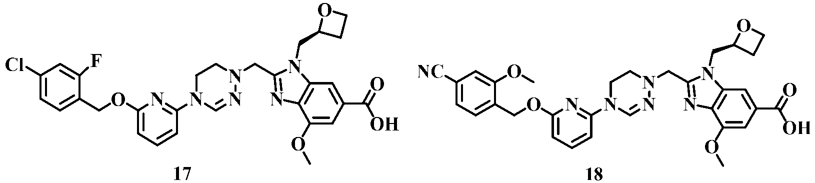

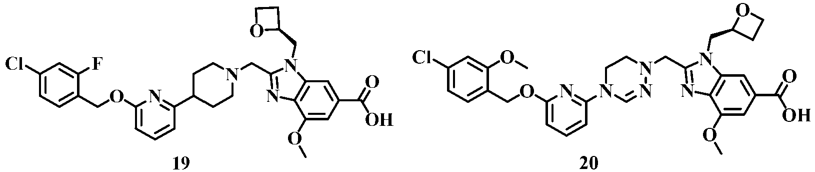

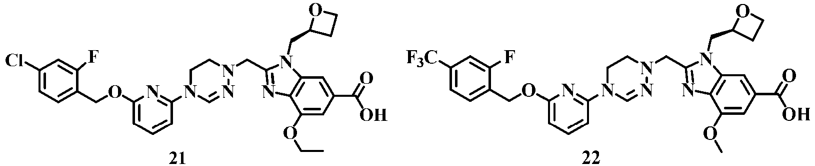

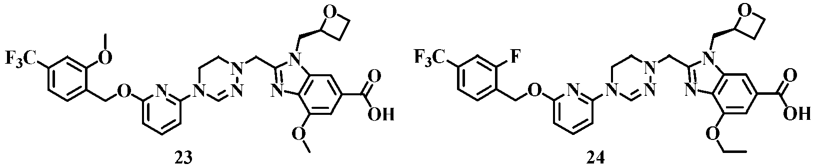

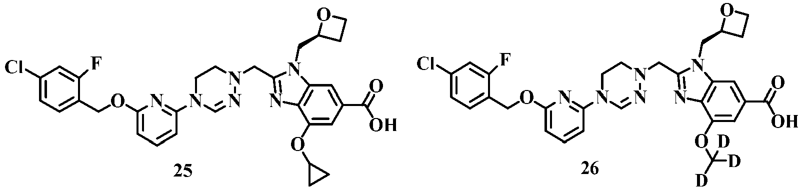

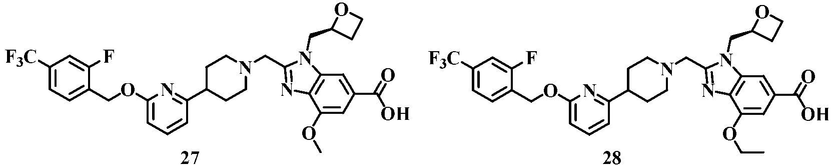

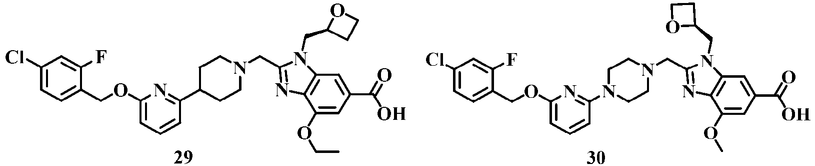

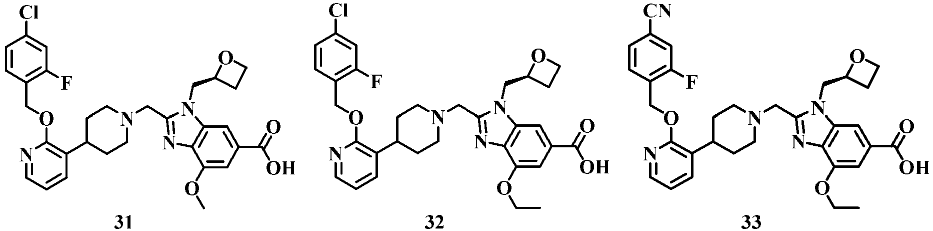

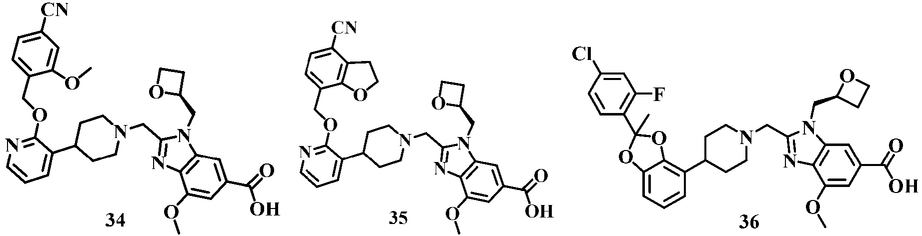

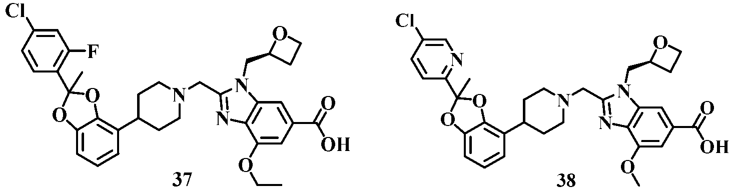

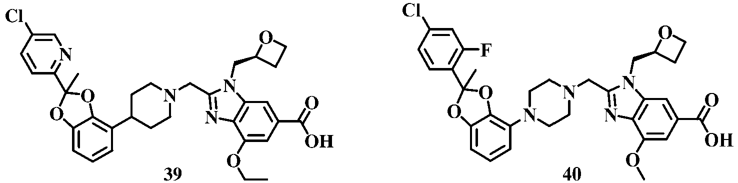

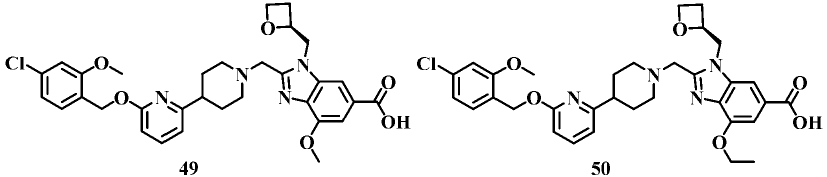

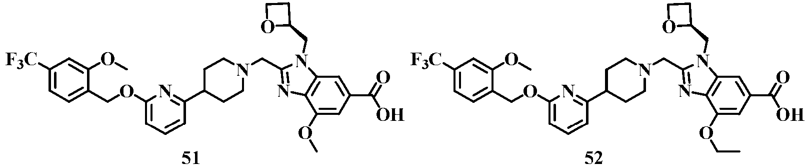

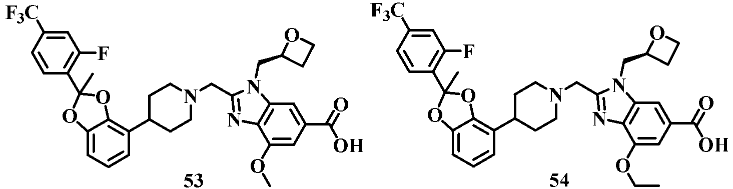

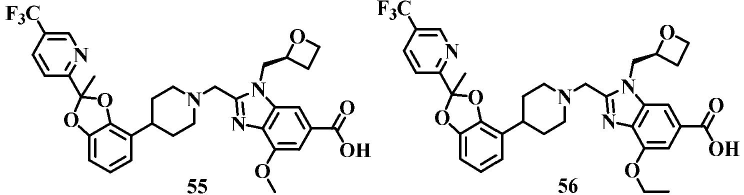

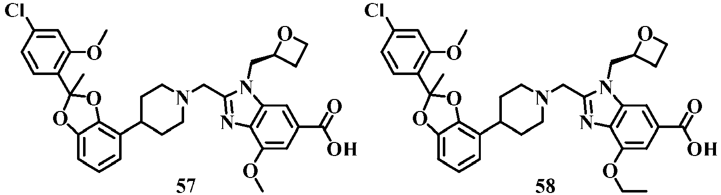

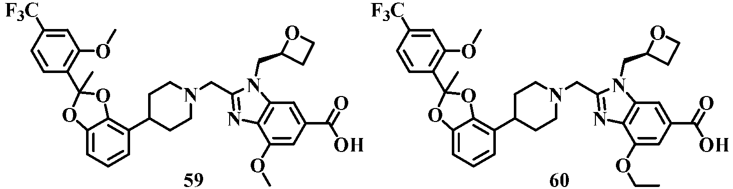

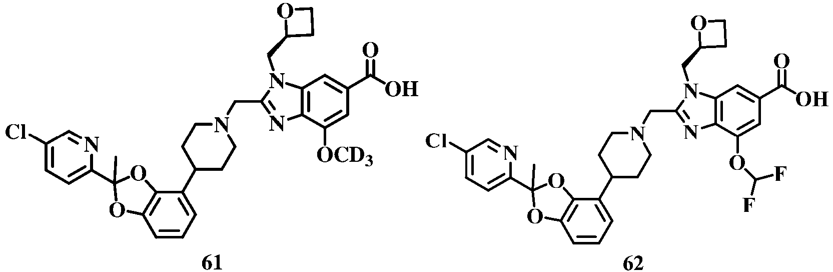

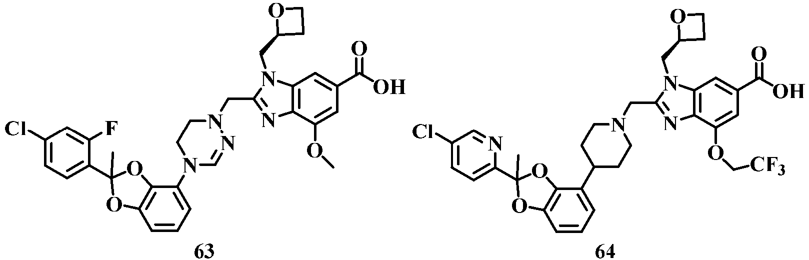

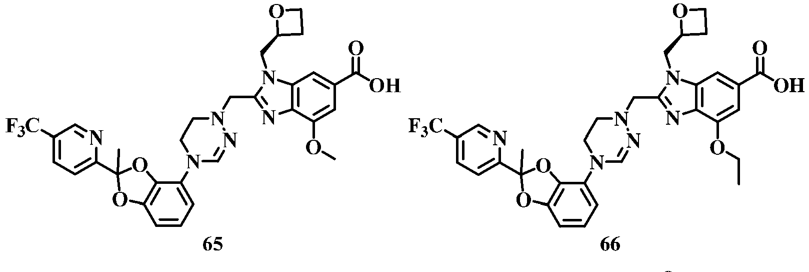

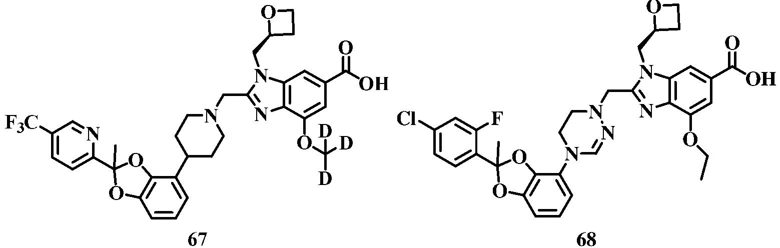

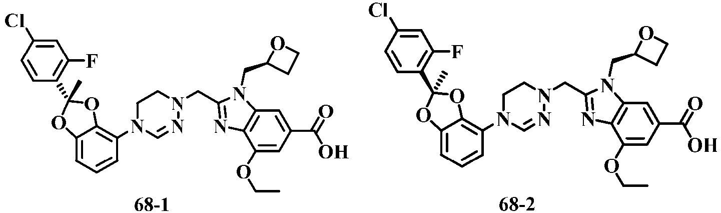

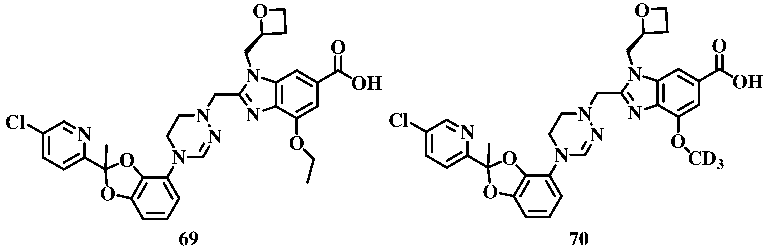

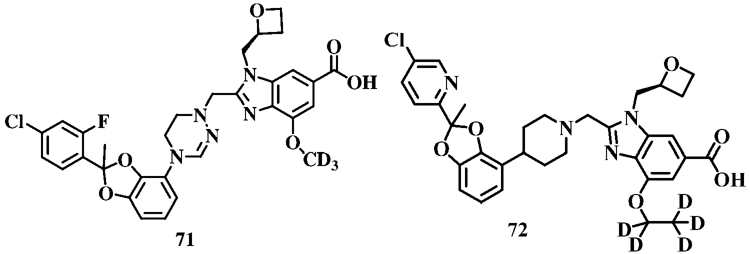

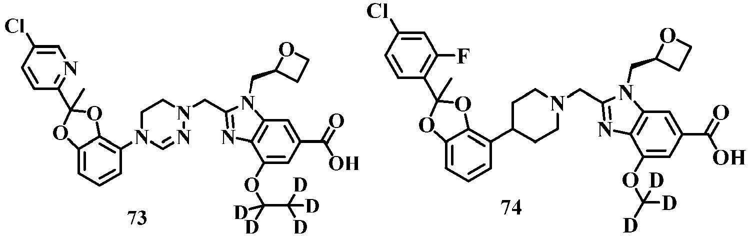

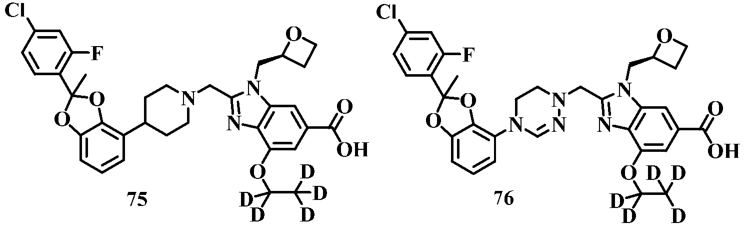

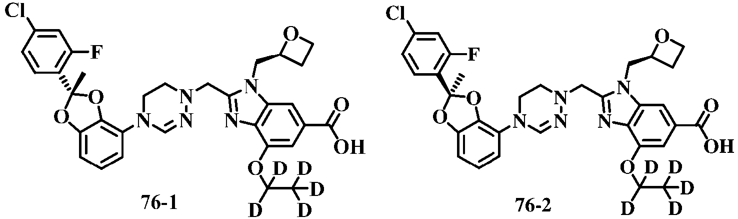

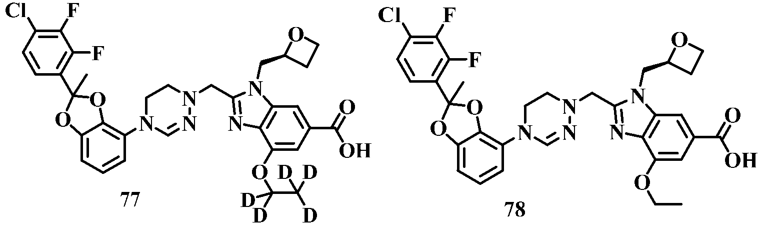

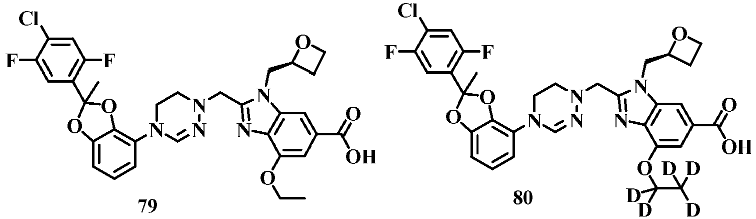

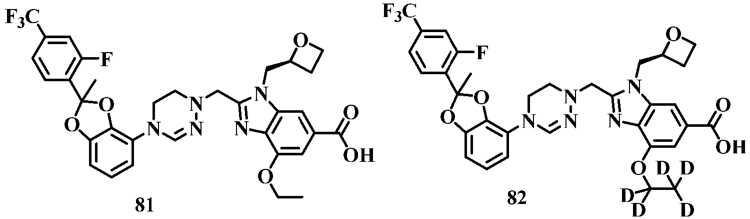

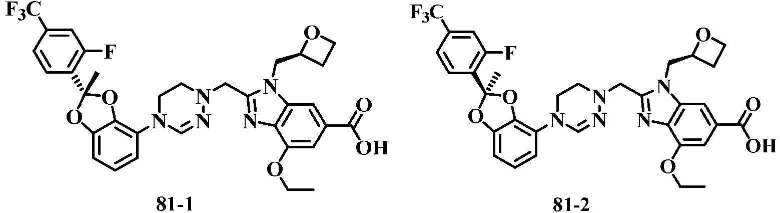

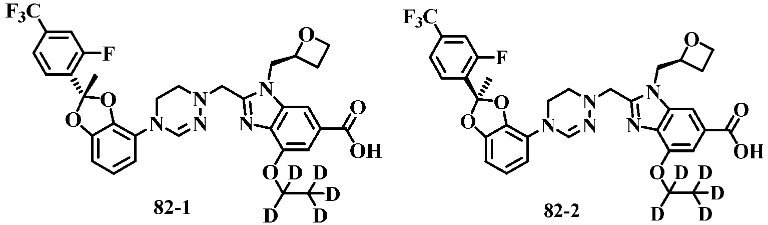

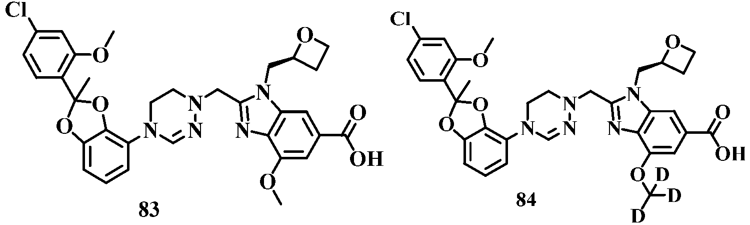

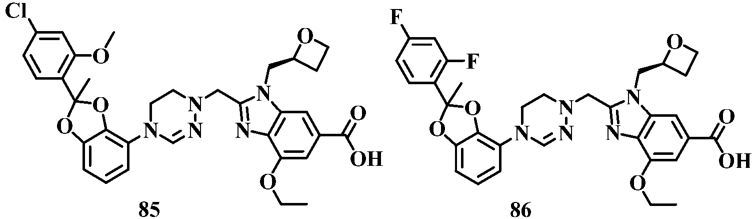

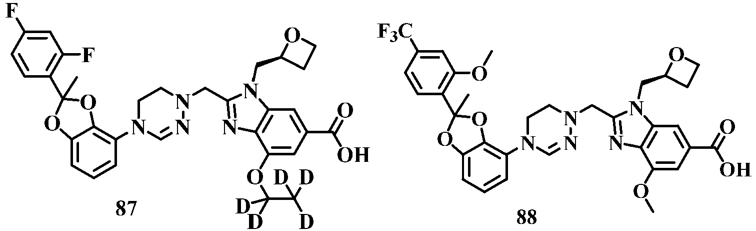

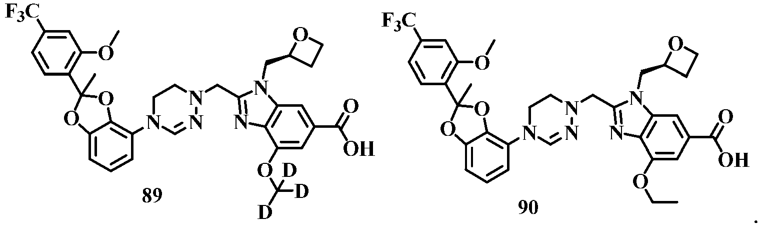

8. A pharmaceutical composition comprising:
the compound according to any one of claims 1 to 8, the stereoisomer, deuterated product or pharmaceutically acceptable salt thereof; and
a pharmaceutically acceptable excipient.

9. Use of the compound according to any one of claims 1 to 7 or the pharmaceutical composition of claim 8 for the manufacture of a GLP-1 receptor agonist, or for the manufacture of a medicament for preventing and/or treating a disease or symptom associated with the disorder of the GLP-1 receptor signaling pathway.

10. The use of claim 9, wherein the disease or symptom associated with the disorder of the GLP-1 receptor signaling pathway is selected from: diabetes, metabolic syndrome, diabetic complications, obesity, non-alcoholic fatty liver disease (NAFLD), non-alcoholic steatohepatitis (NASH), Parkinson's disease, dementia, hyperglycemia, glucose intolerance, atherosclerosis, hypertension, hyperlipidemia, coronary heart disease, cerebral infarction or stroke, preferably the disease or symptom is type II diabetes or obesity.

11. An intermediate (III) for preparing the compound represented by general formula (II): 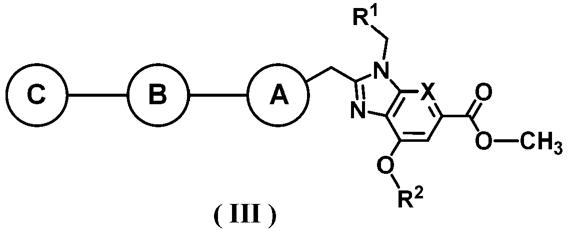 wherein, the definitions of each substituent are the same as those in the general formula (II).
